# EUROPEAN PATENT APPLICATION

(11) **EP 4 737 455 A1**
(43) Date of publication of application: **06.05.2026**
(21) Application number: 24830955.1
(22) Date of filing: 28.06.2024
(51) Int. Cl.: C07D 471/04, C07D 519/00, C07D 498/04, A61K 31/437, A61K 31/4985, A61P 3/10, A61P 3/06

(54) **COMPOUND CONTAINING MULTI-FUSED RING STRUCTURE**

(30) Priority: 30.06.2023 CN 202310802212; 03.11.2023 CN 202311497217; 20.06.2024 CN 202410808056
(71) Applicant: Chia Tai Tianqing Pharmaceutical Group Co., Ltd., Lianyungang, Jiangsu 222062 (CN)
(72) Inventor: LIU, Baomin, Lianyungang, Jiangsu 222062 (CN); ZHU, Yan, Lianyungang, Jiangsu 222062 (CN); HU, Jinfa, Lianyungang, Jiangsu 222062 (CN); HUANG, Yu, Lianyungang, Jiangsu 222062 (CN)
(74) Representative: Grünecker Patent- und Rechtsanwälte PartG mbB
(86) International application number: PCT/CN2024/102306
(87) International publication number: WO 2025/002326

(57) **Abstract**

The present invention is in the field of pharmaceutical chemistry, and relates to a compound containing a multi-fused ring structure, specifically to a compound of formula (I), a stereoisomer or pharmaceutically acceptable salt thereof, a preparation method therefor, or a pharmaceutical composition thereof, and a use thereof in the preparation of a drug for treating diabetes or obesity-related diseases.

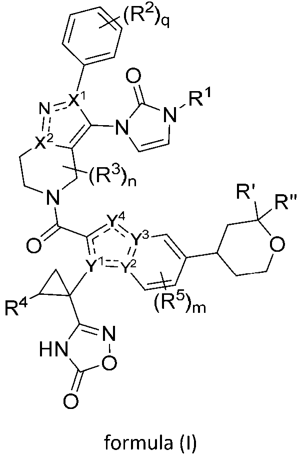

## Description

### CROSS-REFERENCE TO RELATED APPLICATIONS

The present application claims priority to and benefits of the Chinese Patent Application No. 202310802212.0 filed with China National Intellectual Property Administration on Jun. 30, 2023, the Chinese Patent Application No. 202311497217.3 filed with China National Intellectual Property Administration on Nov. 3, 2023, and the Chinese Patent Application No. 202410808056.3 filed with China National Intellectual Property Administration on Jun. 20, 2024, the contents of which are incorporated herein by reference in their entirety.

### TECHNICAL FIELD

The present disclosure relates to the field of pharmaceutical chemistry, and relates to a compound containing a polycyclic fused ring structure, a stereoisomer thereof or a pharmaceutically acceptable salt thereof, a preparation method thereof, or a pharmaceutical composition thereof, and use thereof for preparing a medicament for treating diabetes or obesity-associated diseases.

### BACKGROUND

Type 2 diabetes mellitus (T2DM) is a chronic metabolic disease characterized by elevated blood glucose concentrations, with high morbidity and mortality rates. Obesity is considered an important risk factor for T2DM, with approximately 85% of T2DM patients being overweight or obese. Glucagon-like peptide-1 (GLP-1) is an incretin secreted by L cells in the small intestine when nutrients pass through the digestive tract, and it is known that GLP-1 exhibits a variety of physiological effects through the GLP-1 receptor, such as promoting glucose-dependent insulin secretion, inhibiting glucagon secretion, delaying gastric emptying, and suppressing food intake. Although GLP-1 analogs have been commercialized as diabetes therapeutic agents and are considered one of the most effective diabetes therapeutic agents due to their effective effects in reducing HbAlc and body weight, they must be administered via subcutaneous injection, resulting in poor patient compliance. Therefore, the development of non-polypeptide small-molecule agonists targeting the GLP-1 receptor to improve patient compliance is of great significance, and has become one of the research hotspots in the field of diabetes.

### SUMMARY

The present application provides a compound of formula (I), a stereoisomer thereof, or a pharmaceutically acceptable salt thereof, 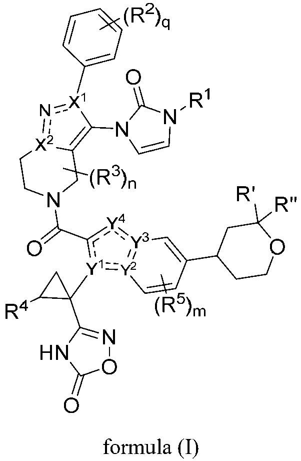 wherein
X¹ and X² are each independently selected from the group consisting of C and N;
Y¹, Y², Y³, and Y⁴ are each independently selected from the group consisting of CH, C, and N;
R¹ is selected from the group consisting of C₁₁₋₁₅ cycloalkyl, C₁₁₋₁₅ aryl, 11- to 15-membered heteroaryl, and 11- to 15-membered heterocyclyl, wherein the C₁₁₋₁₅ cycloalkyl, C₁₁₋₁₅ aryl, 11- to 15-membered heteroaryl, or 11- to 15-membered heterocyclyl is a tricyclic group, and the C₁₁₋₁₅ cycloalkyl, C₁₁₋₁₅ aryl, 11- to 15-membered heteroaryl, or 11- to 15-membered heterocyclyl may be optionally independently substituted with one or more R^{a};
or, R¹ is selected from the group consisting of C₃₋₇ cycloalkyl, phenyl, 5- to 6-membered heteroaryl, and 3- to 7-membered heterocyclyl, wherein R¹ is substituted with one C₂₋₄ alkynyl, R¹ may further be optionally independently substituted with one or more R^{a}, and the C₂₋₄ alkynyl may be optionally substituted with one or more R^{b};
R^{a} is each independently selected from the group consisting of deuterium, halogen, =O, deuterated C₁₋₆ alkyl, -OH, - CN, NH₂, -COOH, C₁₋₆ alkyl NH-, (C₁₋₆ alkyl)₂N-, C₁₋₆ alkyl, C₂₋₆ alkenyl, C₂₋₆ alkynyl, C₁₋₆ alkoxy, C₁₋₆ alkoxy C₁₋₃ alkylene, C₃₋₆ cycloalkyl, phenyl, 5- to 6-membered heteroaryl, and 3- to 6-membered heterocyclyl, wherein the deuterated C₁₋₆ alkyl, C₁₋₆ alkyl NH-, (C₁₋₆ alkyl)₂N-, C₁₋₆ alkyl, C₂₋₆ alkenyl, C₂₋₆ alkynyl, C₁₋₆ alkoxy, or C₁₋₆ alkoxy C₁₋₃ alkylene is optionally independently substituted with one or more R^{c1}, and the C₃₋₆ cycloalkyl, phenyl, 5- to 6-membered heteroaryl, or 3- to 6-membered heterocyclyl is optionally independently substituted with one or more R^{d1},
R² is each independently selected from the group consisting of halogen, -OH, -CN, NH₂, -COOH, C₁₋₆ alkyl NH-, (C₁₋₆ alkyl)₂N-, C₁₋₆ alkyl, deuterated C₁₋₆ alkyl, C₂₋₆ alkenyl, C₂₋₆ alkynyl, C₁₋₆ alkoxy, C₁₋₆ alkoxy C₁₋₃ alkylene, C₃₋₆ cycloalkyl, phenyl, 5- to 6-membered heteroaryl, and 3- to 6-membered heterocyclyl, wherein the C₁₋₆ alkyl NH-, (C₁₋₆ alkyl)₂N-, C₁₋₆ alkyl, deuterated C₁₋₆ alkyl, C₂₋₆ alkenyl, C₂₋₆ alkynyl, C₁₋₆ alkoxy, or C₁₋₆ alkoxy C₁₋₃ alkylene is optionally independently substituted with one or more R^{c2}, and the C₃₋₆ cycloalkyl, phenyl, 5- to 6-membered heteroaryl, or 3- to 6-membered heterocyclyl is optionally independently substituted with one or more R^{d2};
or, R² on two adjacent carbon atoms, together with the carbon atoms connected thereto, form C₄₋₆ cycloalkyl, phenyl, 5- to 6-membered heteroaryl, or 4- to 6-membered heterocyclyl, wherein the C₄₋₆ cycloalkyl, phenyl, 5- to 6-membered heteroaryl, or 4- to 6-membered heterocyclyl is optionally independently substituted with one or more R^{d3};
R³ is each independently selected from the group consisting of deuterium, halogen, -OH, -CN, NH₂, -COOH, C₁₋₆ alkyl NH-, (C₁₋₆ alkyl)₂N-, C₁₋₆ alkyl, deuterated C₁₋₆ alkyl, C₂₋₆ alkenyl, C₂₋₆ alkynyl, C₁₋₆ alkoxy, C₁₋₆ alkoxy C₁₋₃ alkylene, C₃₋₆ cycloalkyl, phenyl, 5- to 6-membered heteroaryl, and 3- to 6-membered heterocyclyl, wherein the C₁₋₆ alkyl NH-, (C₁₋₆ alkyl)₂N-, C₁₋₆ alkyl, deuterated C₁₋₆ alkyl, C₂₋₆ alkenyl, C₂₋₆ alkynyl, C₁₋₆ alkoxy, or C₁₋₆ alkoxy C₁₋₃ alkylene is optionally independently substituted with one or more R^{c3}, and the C₃₋₆ cycloalkyl, phenyl, 5- to 6-membered heteroaryl, or 3- to 6-membered heterocyclyl is optionally independently substituted with one or more R^{d4};
R⁴ is selected from the group consisting of H, deuterium, halogen, -CN, NH₂, -COOH, C₁₋₆ alkyl NH-, (C₁₋₆ alkyl)₂N-, C₁₋₆ alkyl, deuterated C₁₋₆ alkyl, C₁₋₆ alkoxy, and C₁₋₆ alkoxy C₁₋₃ alkylene, wherein the C₁₋₆ alkyl NH-, (C₁₋₆ alkyl)₂N-, C₁₋₆ alkyl, deuterated C₁₋₆ alkyl, C₁₋₆ alkoxy, or C₁₋₆ alkoxy C₁₋₃ alkylene is optionally independently substituted with one or more R^{c4};
R⁵ is each independently selected from the group consisting of halogen, -CN, -OH, -SH, -NH₂, C₁₋₆ alkyl NH-, (C₁₋₆ alkyl)₂N-, C₁₋₆ alkyl, deuterated C₁₋₆ alkyl, C₁₋₆ alkoxy, C₁₋₆ alkoxy C₁₋₃ alkylene, C₁₋₆ alkylthio, -CONH₂, -CONHC₁₋₃ alkyl, -NHCOC₁₋₃ alkyl, -SO₂NH₂, -SO₂NHC₁₋₃ alkyl, and -NHSO₂C₁₋₃ alkyl, wherein the C₁₋₆ alkyl NH-, (C₁₋₆ alkyl)₂N-, C₁₋₆ alkyl, deuterated C₁₋₆ alkyl, C₁₋₆ alkoxy, C₁₋₆ alkoxy C₁₋₃ alkylene, C₁₋₆ alkylthio, -CONH₂, -CONHC₁₋₃ alkyl, -NHCOC₁₋₃ alkyl, -SO₂NH₂, -SO₂NHC₁₋₃ alkyl, or -NHSO₂C₁₋₃ alkyl is optionally independently substituted with one or more R^{c5};
R' and R" are each independently selected from the group consisting of H, halogen, -CN, -OH, -SH, -NH₂, C₁₋₆ alkyl NH-, (C₁₋₆ alkyl)₂N-, C₁₋₆ alkyl, deuterated C₁₋₆ alkyl, C₁₋₆ alkoxy, C₁₋₆ alkoxy C₁₋₃ alkylene, C₁₋₆ alkylthio, -CONH₂, -CONHC₁₋₃ alkyl, -NHCOC₁₋₃ alkyl, -SO₂NH₂, -SO₂NHC₁₋₃ alkyl, and -NHSO₂C₁₋₃ alkyl, wherein the C₁₋₆ alkyl NH-, (C₁₋₆ alkyl)₂N-, C₁₋₆ alkyl, deuterated C₁₋₆ alkyl, C₁₋₆ alkoxy, C₁₋₆ alkoxy C₁₋₃ alkylene, C₁₋₆ alkylthio, -CONH₂, -CONHC₁₋₃ alkyl, -NHCOC₁₋₃ alkyl, -SO₂NH₂, -SO₂NHC₁₋₃ alkyl, or -NHSO₂C₁₋₃ alkyl is optionally independently substituted with one or more R^{c6};
or, R' and R", together with the carbon atom connected thereto, form C₃₋₆ cycloalkyl or 3- to 6-membered heterocycloalkyl, wherein the C₃₋₆ cycloalkyl or 3- to 6-membered heterocycloalkyl is optionally independently substituted with one or more R^{d5};
" 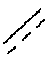 " is each independently selected from the group consisting of a single bond and a double bond;
R^{b} is each independently selected from the group consisting of deuterium, halogen, -CN, -OH, -NH₂, C₁₋₃ alkyl, and C₁₋₃ alkoxy, wherein the C₁₋₃ alkyl or C₁₋₃ alkoxy is optionally independently substituted with one or more substituents selected from the group consisting of deuterium, halogen, OH, CN, and NH₂;
R^{c1}, R^{c2}, R^{c3}, R^{c4}, R^{c5}, and R^{c6} are each independently selected from the group consisting of deuterium, halogen, - CN, -OH, and -NH₂;
R^{d1}, R^{d2}, R^{d3}, R^{d4}, and R^{d5} are each independently selected from the group consisting of deuterium, halogen, -CN, - OH, =O, -NH₂, C₁₋₃ alkyl, and C₁₋₃ alkoxy, wherein the C₁₋₃ alkyl or C₁₋₃ alkoxy is optionally independently substituted with one or more substituents selected from the group consisting of deuterium, halogen, OH, CN, and NH₂;
q is selected from the group consisting of 0, 1, 2, 3, and 4;
n is selected from the group consisting of 0, 1, 2, 3, and 4;
m is selected from the group consisting of 0, 1, 2, 3, and 4.

In some embodiments, the "substituted with one or more" is each independently selected from substitution with 1, 2, 3, 4, 5, or 6 substituents.

In some embodiments, the "substituted with one or more" is each independently selected from substitution with 1, 2, 3, 4, or 5 substituents.

In some embodiments, the "substituted with one or more" is each independently selected from substitution with 1, 2, 3, or 4 substituents.

In some embodiments, the "substituted with one or more" is each independently selected from substitution with 1, 2, or 3 substituents.

In some embodiments, the "hetero" in the heteroaryl or heterocyclyl is each independently a heteroatom selected from the group consisting of oxygen, sulfur, and nitrogen, wherein the nitrogen atom is optionally quaternized or oxidized to N(O), the sulfur atom is optionally oxidized to S(O) or S(O)₂, and the other variables are as defined in the present application.

In some embodiments, the tricyclic group is selected from the group consisting of a monocyclic group fused to a fused bicyclic group, a monocyclic group fused to a spiro bicyclic group, and a monocyclic group fused to a bridged bicyclic group.

In some embodiments, the tricyclic group is selected from the group consisting of a monocyclic group fused to a fused bicyclic group, and a monocyclic group fused to a spiro bicyclic group.

In some embodiments, the tricyclic group is selected from the group consisting of a monocyclic group fused to a fused bicyclic group, a monocyclic group fused to a spiro bicyclic group, and a monocyclic group fused to a bridged bicyclic group, wherein the monocyclic group is connected to the structural unit

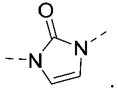

In some embodiments, in the tricyclic group, the aromatic ring is connected to the structural unit

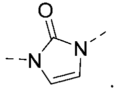

In some other embodiments, the tricyclic group is selected from the group consisting of a monocyclic group fused to a fused bicyclic group, a monocyclic group fused to a spiro bicyclic group, and a monocyclic group fused to a bridged bicyclic group, wherein the monocyclic group is an aromatic ring, and the monocyclic group is connected to the structural unit

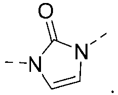

In some other embodiments, the tricyclic group is selected from the group consisting of a monocyclic group fused to a fused bicyclic group, a monocyclic group fused to a spiro bicyclic group, and a monocyclic group fused to a bridged bicyclic group, wherein the monocyclic group is a benzene ring or a 5- to 6-membered heteroaromatic ring, and the monocyclic group is connected to the structural unit

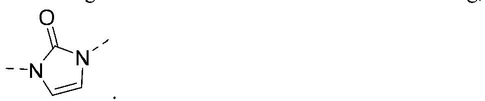

In some other embodiments, the tricyclic group is selected from the group consisting of a monocyclic group fused to a fused bicyclic group, a monocyclic group fused to a spiro bicyclic group, and a monocyclic group fused to a bridged bicyclic group, wherein the monocyclic group is a benzene ring or a 5- to 6-membered heteroaromatic ring containing 1-2 N atoms, and the monocyclic group is connected to the structural unit

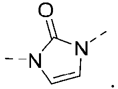

In some other embodiments, the tricyclic group is selected from the group consisting of a monocyclic group fused to a fused bicyclic group, a monocyclic group fused to a spiro bicyclic group, and a monocyclic group fused to a bridged bicyclic group, wherein the monocyclic group is a benzene ring or a 6-membered heteroaromatic ring containing 1-2 N atoms, and the monocyclic group is connected to the structural unit

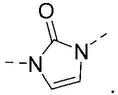

In some other embodiments, the tricyclic group is selected from the group consisting of a monocyclic group fused to a fused bicyclic group, a monocyclic group fused to a spiro bicyclic group, and a monocyclic group fused to a bridged bicyclic group, wherein the monocyclic group is a benzene ring, a pyridine ring, a pyrimidine ring, a pyrazine ring, or a pyridazine ring, and the monocyclic group is connected to the structural unit

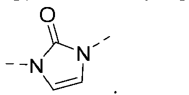

In some other embodiments, the tricyclic group is selected from the group consisting of a monocyclic group fused to a fused bicyclic group, a monocyclic group fused to a spiro bicyclic group, and a monocyclic group fused to a bridged bicyclic group, wherein the monocyclic group is a benzene ring or a pyridine ring, and the monocyclic group is connected to the structural unit

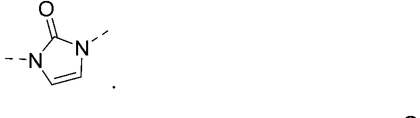

In the present application, when the ring connected to the structural unit 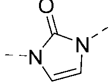 in R¹ is an aromatic ring, R¹ is defined as aryl or heteroaryl.

In some embodiments, R¹ is selected from the group consisting of C₁₁₋₁₅ aryl and 11- to 15-membered heteroaryl, wherein the C₁₁₋₁₅ aryl or 11- to 15-membered heteroaryl is a tricyclic group, and the C₁₁₋₁₅ aryl or 11- to 15-membered heteroaryl may be optionally independently substituted with one or more R^{a}.

In some embodiments, R¹ is selected from the group consisting of benzo C₇₋₁₁ fused bicycloalkyl, benzo 7- to 11-membered fused heterobicyclyl, benzo 7- to 11-membered fused heterobiaryl, pyridino C₇₋₁₁ fused bicycloalkyl, pyridino 7- to 11-membered fused heterobicyclyl, pyridino 7- to 11-membered fused heterobiaryl, pyrimido C₇₋₁₁ fused bicycloalkyl, pyrimido 7- to 11-membered fused heterobicyclyl, pyrimido 7- to 11-membered fused heterobiaryl, pyridazino C₇₋₁₁ fused bicycloalkyl, pyridazino 7- to 11-membered fused heterobicycloalkyl, pyridazino 7- to 11-membered fused heterobiaryl, pyrazino C₇₋₁₁ fused bicycloalkyl, pyrazino 7- to 11-membered fused heterobicycloalkyl, pyrazino 7- to 11-membered fused heterobiaryl, benzo C₇₋₁₁ spiro bicycloalkyl, benzo C₇₋₁₁ spiro heterobicyclyl, pyridino C₇₋₁₁ spiro bicycloalkyl, pyridino C₇₋₁₁ spiro heterobicyclyl, pyrimido C₇₋₁₁ spiro bicycloalkyl, pyrimido C₇₋₁₁ spiro heterobicyclyl, pyridazino C₇₋₁₁ spiro bicycloalkyl, pyridazino C₇₋₁₁ spiro heterobicyclyl, pyrazino C₇₋₁₁ spiro bicycloalkyl, and pyrazino C₇₋₁₁ spiro heterobicyclyl, wherein R¹ may be optionally independently substituted with one or more R^{a}.

In some embodiments, R¹ is selected from the group consisting of benzo C₇₋₉ fused bicycloalkyl, benzo 7- to 9-membered fused heterobicyclyl, benzo 7- to 9-membered fused heterobiaryl, pyridino C₇₋₉ fused bicycloalkyl, pyridino 7- to 9-membered fused heterobicyclyl, pyridino 7- to 9-membered fused heterobiaryl, pyrimido C₇₋₉ fused bicycloalkyl, pyrimido 7- to 9-membered fused heterobicyclyl, pyrimido 7- to 9-membered fused heterobiaryl, pyridazino C₇₋₉ fused bicycloalkyl, pyridazino 7- to 9-membered fused heterobicycloalkyl, pyridazino 7- to 9-membered fused heterobiaryl, pyrazino C₇₋₉ fused bicycloalkyl, pyrazino 7- to 9-membered fused heterobicycloalkyl, pyrazino 7- to 9-membered fused heterobiaryl, benzo C₇₋₉ spiro bicycloalkyl, benzo C₇₋₉ spiro heterobicyclyl, pyridino C₇₋₉ spiro bicycloalkyl, pyridino C₇₋₉ spiro heterobicyclyl, pyrimido C₇₋₉ spiro bicycloalkyl, pyrimido C₇₋₉ spiro heterobicyclyl, pyridazino C₇₋₉ spiro bicycloalkyl, pyridazino C₇₋₉ spiro heterobicyclyl, pyrazino C₇₋₉ spiro bicycloalkyl, and pyrazino C₇₋₉ spiro heterobicyclyl, wherein R¹ may be optionally independently substituted with one or more R^{a}.

In some embodiments, R¹ is selected from the group consisting of benzo 7- to 9-membered fused heterobicyclyl, benzo 7- to 9-membered fused heterobiaryl, benzo C₇₋₉ spiro bicycloalkyl, benzo C₇₋₉ spiro heterobicyclyl, and pyridino C₇₋₉ spiro bicycloalkyl, wherein R¹ may be optionally independently substituted with one or more R².

In some embodiments, R¹ is selected from the group consisting of benzo 7- to 9-membered fused heterobicyclyl, benzo 7- to 9-membered fused heterobiaryl, benzo C₇₋₉ spiro bicycloalkyl, benzo C₇₋₉ spiro heterobicyclyl, and pyridino C₇₋₉ spiro bicycloalkyl, wherein in R¹, the ring connected to the structural unit 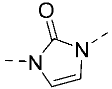 is a benzene ring or a pyridine ring, and R¹ may be optionally independently substituted with one or more R².

In some embodiments, R¹ is selected from the group consisting of benzo 7- to 9-membered fused heterobicyclyl, benzo C₇₋₉ spiro bicycloalkyl, and benzo C₇₋₉ spiro heterobicyclyl, wherein R¹ may be optionally independently substituted with one or more R^{a}.

In some embodiments, R¹ is selected from the group consisting of benzo 7- to 9-membered fused heterobicyclyl, benzo C₇₋₉ spiro bicycloalkyl, and benzo C₇₋₉ spiro heterobicyclyl, wherein in R¹ the ring connected to the structural unit 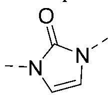 is a benzene ring, and R¹ may be optionally independently substituted with one or more R^{a}.

In some embodiments, R¹ is selected from the group consisting of benzo 5-membered cycloalkyl fused 3-membered cycloalkyl, benzo 5-membered cycloalkyl fused 4-membered cycloalkyl, benzo 5-membered cycloalkyl fused 5-membered cycloalkyl, benzo 6-membered cycloalkyl fused 4-membered cycloalkyl, benzo 6-membered cycloalkyl fused 5-membered cycloalkyl, benzo 5-membered cycloalkyl fused 3-membered heterocyclyl, benzo 5-membered cycloalkyl fused 4-membered heterocyclyl, benzo 5-membered cycloalkyl fused 5-membered heterocyclyl, benzo 6-membered cycloalkyl fused 4-membered heterocyclyl, benzo 6-membered cycloalkyl fused 5-membered heterocyclyl, benzo 5-membered heterocyclyl fused 3-membered cycloalkyl, benzo 5-membered heterocyclyl fused 4-membered cycloalkyl, benzo 5-membered heterocyclyl fused 5-membered cycloalkyl, benzo 6-membered heterocyclyl fused 4-membered cycloalkyl, benzo 6-membered heterocyclyl fused 5-membered cycloalkyl, benzo 5-membered heterocyclyl fused 3-membered heterocyclyl, benzo 5-membered heterocyclyl fused 4-membered heterocyclyl, benzo 5-membered heterocyclyl fused 5-membered heterocyclyl, benzo 6-membered heterocyclyl fused 4-membered heterocyclyl, benzo 6-membered heterocyclyl fused 5-membered heterocyclyl, benzo 5-membered heteroaryl fused 3-membered cycloalkyl, benzo 5-membered heteroaryl fused 4-membered cycloalkyl, benzo 5-membered heteroaryl fused 5-membered cycloalkyl, benzo 5-membered heteroaryl fused 6-membered cycloalkyl, benzo 6-membered heteroaryl fused 4-membered cycloalkyl, benzo 6-membered heteroaryl fused 5-membered cycloalkyl, benzo 5-membered heteroaryl fused 3-membered heterocyclyl, benzo 5-membered heteroaryl fused 4-membered heterocyclyl, benzo 5-membered heteroaryl fused 5-membered heterocyclyl, benzo 5-membered heteroaryl fused 6-membered heterocyclyl, benzo 6-membered heteroaryl fused 4-membered heterocyclyl, benzo 6-membered heteroaryl fused 5-membered heterocyclyl, benzo 5-membered cycloalkyl fused 5-membered heteroaryl, benzo 6-membered cycloalkyl fused 5-membered heteroaryl, benzo 5-membered cycloalkyl fused 6-membered heteroaryl, benzo 5-membered heterocyclyl fused 5-membered heteroaryl, benzo 6-membered heterocyclyl fused 5-membered heteroaryl, benzo 5-membered heterocyclyl fused 6-membered heteroaryl, pyridino 5-membered cycloalkyl fused 3-membered cycloalkyl, pyridino 5-membered cycloalkyl fused 4-membered cycloalkyl, pyridino 5-membered cycloalkyl fused 5-membered cycloalkyl, pyridino 6-membered cycloalkyl fused 4-membered cycloalkyl, pyridino 6-membered cycloalkyl fused 5-membered cycloalkyl, pyridino 5-membered cycloalkyl fused 3-membered heterocyclyl, pyridino 5-membered cycloalkyl fused 4-membered heterocyclyl, pyridino 5-membered cycloalkyl fused 5-membered heterocyclyl, pyridino 6-membered cycloalkyl fused 4-membered heterocyclyl, pyridino 6-membered cycloalkyl fused 5-membered heterocyclyl, pyridino 5-membered heterocyclyl fused 3-membered cycloalkyl, pyridino 5-membered heterocyclyl fused 4-membered cycloalkyl, pyridino 5-membered heterocyclyl fused 5-membered cycloalkyl, pyridino 6-membered heterocyclyl fused 4-membered cycloalkyl, pyridino 6-membered heterocyclyl fused 5-membered cycloalkyl, pyridino 5-membered heterocyclyl fused 3-membered heterocyclyl, pyridino 5-membered heterocyclyl fused 4-membered heterocyclyl, pyridino 5-membered heterocyclyl fused 5-membered heterocyclyl, pyridino 6-membered heterocyclyl fused 4-membered heterocyclyl, pyridino 6-membered heterocyclyl fused 5-membered heterocyclyl, pyridino 5-membered heteroaryl fused 3-membered cycloalkyl, pyridino 5-membered heteroaryl fused 4-membered cycloalkyl, pyridino 5-membered heteroaryl fused 5-membered cycloalkyl, pyridino 5-membered heteroaryl fused 6-membered cycloalkyl, pyridino 6-membered heteroaryl fused 4-membered cycloalkyl, pyridino 6-membered heteroaryl fused 5-membered cycloalkyl, pyridino 5-membered heteroaryl fused 3-membered heterocyclyl, pyridino 5-membered heteroaryl fused 4-membered heterocyclyl, pyridino 5-membered heteroaryl fused 5-membered heterocyclyl, pyridino 5-membered heteroaryl fused 6-membered heterocyclyl, pyridino 6-membered heteroaryl fused 4-membered heterocyclyl, pyridino 6-membered heteroaryl fused 5-membered heterocyclyl, pyridino 5-membered cycloalkyl fused 5-membered heteroaryl, pyridino 6-membered cycloalkyl fused 5-membered heteroaryl, pyridino 5-membered cycloalkyl fused 6-membered heteroaryl, pyridino 5-membered heterocyclyl fused 5-membered heteroaryl, pyridino 6-membered heterocyclyl fused 5-membered heteroaryl, pyridino 5-membered heterocyclyl fused 6-membered heteroaryl, benzo 5-membered cycloalkyl spiro 3-membered cycloalkyl, benzo 5-membered cycloalkyl spiro 4-membered cycloalkyl, benzo 5-membered cycloalkyl spiro 5-membered cycloalkyl, benzo 6-membered cycloalkyl spiro 4-membered cycloalkyl, benzo 6-membered cycloalkyl spiro 5-membered cycloalkyl, benzo 5-membered cycloalkyl spiro 3-membered heterocyclyl, benzo 5-membered cycloalkyl spiro 4-membered heterocyclyl, benzo 5-membered cycloalkyl spiro 5-membered heterocyclyl, benzo 6-membered cycloalkyl spiro 4-membered heterocyclyl, benzo 6-membered cycloalkyl spiro 5-membered heterocyclyl, benzo 5-membered heterocyclyl spiro 3-membered cycloalkyl, benzo 5-membered heterocyclyl spiro 4-membered cycloalkyl, benzo 5-membered heterocyclyl spiro 5-membered cycloalkyl, benzo 6-membered heterocyclyl spiro 4-membered cycloalkyl, benzo 6-membered heterocyclyl spiro 5-membered cycloalkyl, benzo 5-membered heterocyclyl spiro 3-membered heterocyclyl, benzo 5-membered heterocyclyl spiro 4-membered heterocyclyl, benzo 5-membered heterocyclyl spiro 5-membered heterocyclyl, benzo 6-membered heterocyclyl spiro 4-membered heterocyclyl, benzo 6-membered heterocyclyl spiro 5-membered heterocyclyl, benzo 5-membered heteroaryl spiro 3-membered cycloalkyl, pyridino 5-membered cycloalkyl spiro 3-membered cycloalkyl, pyridino 5-membered cycloalkyl spiro 4-membered cycloalkyl, pyridino 5-membered cycloalkyl spiro 5-membered cycloalkyl, pyridino 6-membered cycloalkyl spiro 4-membered cycloalkyl, pyridino 6-membered cycloalkyl spiro 5-membered cycloalkyl, pyridino 5-membered cycloalkyl spiro 3-membered heterocyclyl, pyridino 5-membered cycloalkyl spiro 4-membered heterocyclyl, pyridino 5-membered cycloalkyl spiro 5-membered heterocyclyl, pyridino 6-membered cycloalkyl spiro 4-membered heterocyclyl, pyridino 6-membered cycloalkyl spiro 5-membered heterocyclyl, pyridino 5-membered heterocyclyl spiro 3-membered cycloalkyl, pyridino 5-membered heterocyclyl spiro 4-membered cycloalkyl, pyridino 5-membered heterocyclyl spiro 5-membered cycloalkyl, pyridino 6-membered heterocyclyl spiro 4-membered cycloalkyl, pyridino 6-membered heterocyclyl spiro 5-membered cycloalkyl, pyridino 5-membered heterocyclyl spiro 3-membered heterocyclyl, pyridino 5-membered heterocyclyl spiro 4-membered heterocyclyl, pyridino 5-membered heterocyclyl spiro 5-membered heterocyclyl, pyridino 6-membered heterocyclyl spiro 4-membered heterocyclyl, pyridino 6-membered heterocyclyl spiro 5-membered heterocyclyl, and pyridino 5-membered heteroaryl spiro 3-membered cycloalkyl, wherein R¹ may be optionally independently substituted with one or more R².

In some other embodiments, R¹ is selected from the group consisting of benzo 5-membered heteroaryl fused 5-membered heterocyclyl, benzo 5-membered heteroaryl fused 6-membered heterocyclyl, benzo 5-membered heterocyclyl fused 5-membered heteroaryl, benzo 5-membered cycloalkyl spiro 3-membered cycloalkyl, benzo 5-membered heterocyclyl spiro 3-membered cycloalkyl, benzo 5-membered heterocyclyl spiro 4-membered cycloalkyl, benzo 5-membered cycloalkyl spiro 4-membered heterocyclyl, pyridino 5-membered heteroaryl fused 5-membered cycloalkyl, and pyridino 5-membered cycloalkyl spiro 3-membered cycloalkyl, wherein R¹ may be optionally independently substituted with one or more R^{a}.

In some other embodiments, R¹ is selected from the group consisting of benzo 5-membered heteroaryl fused 5-membered heterocyclyl, benzo 5-membered heteroaryl fused 6-membered heterocyclyl, benzo 5-membered heterocyclyl fused 5-membered heteroaryl, benzo 5-membered cycloalkyl spiro 3-membered cycloalkyl, benzo 5-membered heterocyclyl spiro 3-membered cycloalkyl, benzo 5-membered heterocyclyl spiro 4-membered cycloalkyl, benzo 5-membered cycloalkyl spiro 4-membered heterocyclyl, pyridino 5-membered heteroaryl fused 5-membered cycloalkyl, and pyridino 5-membered cycloalkyl spiro 3-membered cycloalkyl, wherein in R¹, the ring connected to the structural unit 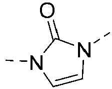 is a benzene ring or a pyridine ring, and R¹ may be optionally independently substituted with one or more R².

In some embodiments, R¹ is selected from the group consisting of benzo 5-membered heteroaryl fused 5-membered cycloalkyl, benzo 5-membered heteroaryl fused 5-membered heterocyclyl, benzo 5-membered heteroaryl fused 6-membered heterocyclyl, benzo 5-membered heterocyclyl fused 5-membered heteroaryl, benzo 5-membered cycloalkyl spiro 3-membered cycloalkyl, benzo 5-membered heterocyclyl spiro 3-membered cycloalkyl, benzo 5-membered heterocyclyl spiro 4-membered cycloalkyl, benzo 5-membered cycloalkyl spiro 4-membered heterocyclyl, and pyridino 5-membered cycloalkyl spiro 3-membered cycloalkyl, wherein R¹ may be optionally independently substituted with one or more R^{a}.

In some embodiments, R¹ is selected from the group consisting of benzo 5-membered heteroaryl fused 5-membered cycloalkyl, benzo 5-membered heteroaryl fused 5-membered heterocyclyl, benzo 5-membered heteroaryl fused 6-membered heterocyclyl, benzo 5-membered heterocyclyl fused 5-membered heteroaryl, benzo 5-membered cycloalkyl spiro 3-membered cycloalkyl, benzo 5-membered heterocyclyl spiro 3-membered cycloalkyl, benzo 5-membered heterocyclyl spiro 4-membered cycloalkyl, benzo 5-membered cycloalkyl spiro 4-membered heterocyclyl, and pyridino 5-membered cycloalkyl spiro 3-membered cycloalkyl, wherein in R¹, the ring connected to the structural unit 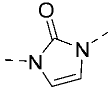 is a benzene ring or a pyridine ring, and R¹ may be optionally independently substituted with one or more R^{a}.

In some embodiments, R¹ is selected from the group consisting of benzo 5-membered cycloalkyl spiro 3-membered cycloalkyl and benzo 5-membered heterocyclyl spiro 3-membered cycloalkyl, wherein R¹ may be optionally independently substituted with one or more R^{a}.

In some embodiments, R¹ is selected from the group consisting of benzo 5-membered cycloalkyl spiro 3-membered cycloalkyl and benzo 5-membered heterocyclyl spiro 3-membered cycloalkyl, wherein in R¹, the ring connected to the structural unit 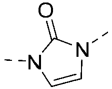 is a benzene ring, and R¹ may be optionally independently substituted with one or more R^{a}.

In some embodiments, R¹ is selected from the group consisting of 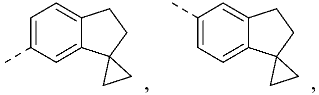 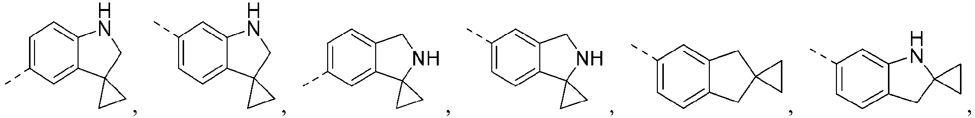 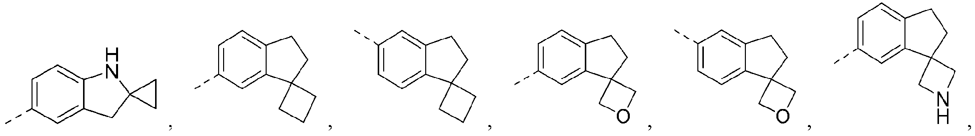 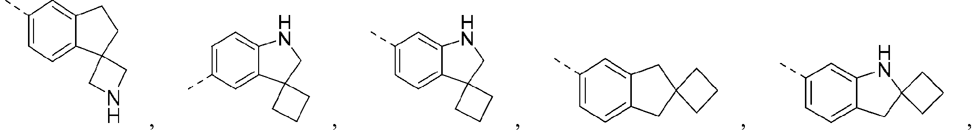 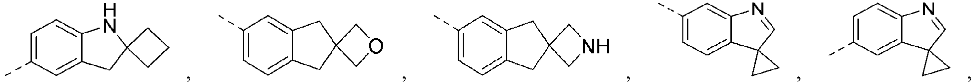 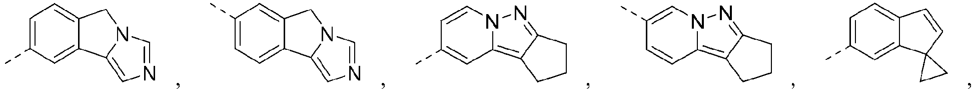 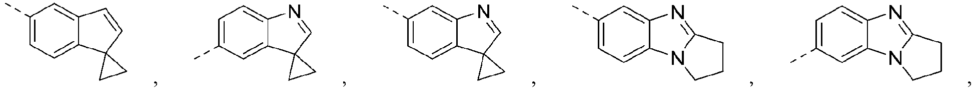 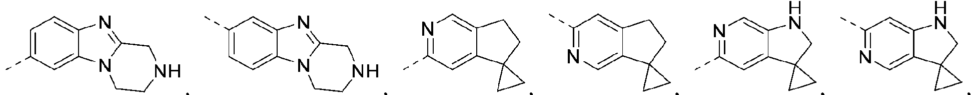 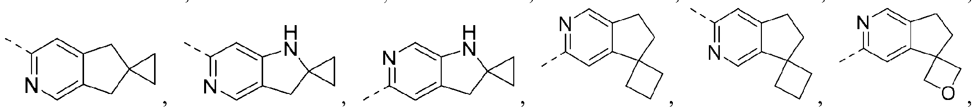 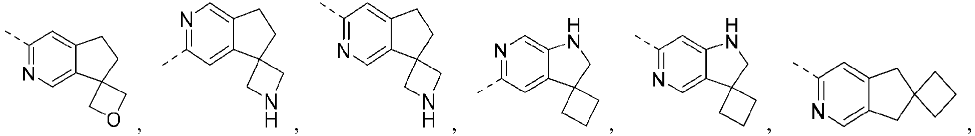 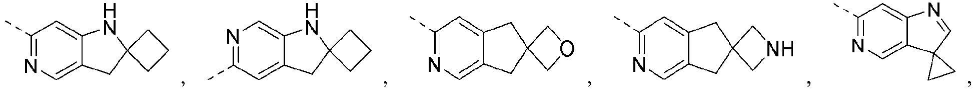 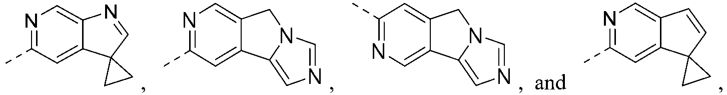 wherein R¹ may be optionally independently substituted with one or more R^{a}.

In some embodiments, R¹ is selected from the group consisting of 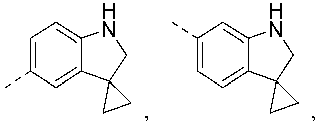 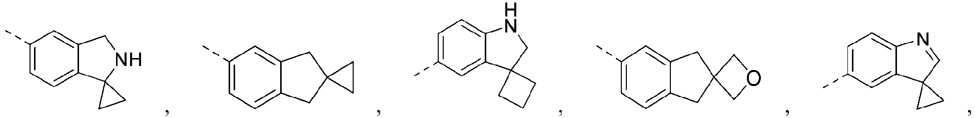 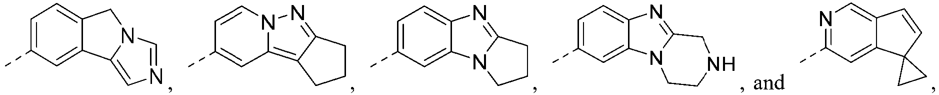 wherein R¹ may be ontionallv indenendentlv substituted with one or more R^{a}. In some other embodiments R¹ is selected from 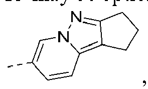 wherein R¹ may be optionally independently substituted with one or more R^{a}.

In some embodiments, R¹ is selected from the group consisting of 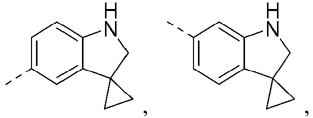 and 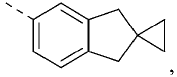 wherein R¹ may be optionally independently substituted with one or more R^{a}.

In some other embodiments, R¹ is selected from the group consisting of 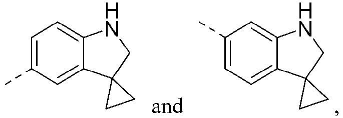 wherein R¹ may be optionally independently substituted with one or more R^{a}.

In some other embodiments, R¹ is selected from 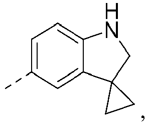 wherein R¹ may be optionally independently substituted with one or more R².

In some embodiments, R¹ is selected from the group consisting of phenyl and 5- to 6-membered heteroaryl, wherein R¹ is substituted with one C₂₋₄ alkynyl, R¹ may further be optionally independently substituted with one or more R^{a}, and the C₂₋₄ alkynyl may be optionally substituted with one or more R^{b}.

In some embodiments, R¹ is selected from the group consisting of phenyl, pyridinyl, pyrimidinyl, pyrazinyl, and pyridazinyl, wherein R¹ is substituted with one C₂₋₄ alkynyl, R¹ may further be optionally independently substituted with one or more R^{a}, and the C₂₋₄ alkynyl may be optionally substituted with one or more R^{b}.

In some embodiments, R¹ is selected from the group consisting of phenyl and pyridinyl, wherein R¹ is substituted with one C₂₋₄ alkynyl, R¹ may further be optionally independently substituted with one or more R^{a}, and the C₂₋₄ alkynyl may be optionally substituted with one or more R^{b}.

In some embodiments, R¹ is selected from the group consisting of phenyl and pyridinyl, wherein R¹ is substituted with one ethynyl or propynyl, R¹ may further be optionally independently substituted with one or more R^{a}, and the ethynyl or propynyl may be optionally substituted with one or more R^{b}.

In some embodiments, R¹ is selected from the group consisting of phenyl and pyridinyl, wherein R¹ is substituted with one ethynyl or 1-propynyl, R¹ may further be optionally independently substituted with one or more R^{a}, and the ethynyl or propynyl may be optionally substituted with one or more R^{b}.

In some embodiments, R^{a} is each independently selected from the group consisting of halogen, =O, deuterated C₁₋₅ alkyl, -OH, -CN, NH₂, C₁₋₅ alkyl, C₂₋₄ alkenyl, C₂₋₄ alkynyl, C₁₋₅ alkoxy, C₁₋₅ alkoxy C₁₋₃ alkylene, C₃₋₆ cycloalkyl, phenyl, 5- to 6-membered heteroaryl, and 3- to 6-membered heterocyclyl, wherein the deuterated C₁₋₅ alkyl, C₁₋₅ alkyl, C₂₋₄ alkenyl, C₂₋₄ alkynyl, C₁₋₆ alkoxy, or C₁₋₅ alkoxy C₁₋₃ alkylene is optionally independently substituted with one or more R^{c1}, and the C₃₋₆ cycloalkyl, phenyl, 5- to 6-membered heteroaryl, or 3- to 6-membered heterocyclyl is optionally independently substituted with one or more R^{d1}.

In some embodiments, R^{a} is each independently selected from the group consisting of halogen, =O, deuterated C₁₋₃ alkyl, -OH, -CN, NH₂, C₁₋₃ alkyl, C₂₋₄ alkenyl, C₂₋₄ alkynyl, C₁₋₃ alkoxy, C₁₋₃ alkoxymethylene, cyclopropyl, cyclobutyl, cyclopentyl, phenyl, 5- to 6-membered heteroaryl, and 3- to 5-membered heterocyclyl, wherein the deuterated C₁₋₃ alkyl, C₁₋₃ alkyl, C₂₋₄ alkenyl, C₂₋₄ alkynyl, C₁₋₃ alkoxy, or C₁₋₃ alkoxymethylene is optionally independently substituted with one or more R^{c1}, and the cyclopropyl, cyclobutyl, cyclopentyl, phenyl, 5- to 6-membered heteroaryl, or 3- to 5-membered heterocyclyl is optionally independently substituted with one or more R^{d1}.

In some embodiments, R^{a} is each independently selected from the group consisting of halogen, =O, deuterated C₁₋₃ alkyl, C₁₋₃ alkyl, and C₃₋₆ cycloalkyl, wherein the deuterated C₁₋₃ alkyl or C₁₋₃ alkyl is optionally independently substituted with one or more R^{c1}, and the C₃₋₆ cycloalkyl is optionally independently substituted with one or more R^{d1}.

In some embodiments, R^{a} is each independently selected from the group consisting of F, Cl, Br, I, =O, -CD₃, -C₂D₅, -OH, -CN, methyl, ethyl, propyl, ethenyl, propenyl, ethynyl, propynyl, methoxy, ethoxy, propoxy, methoxymethylene, ethoxymethylene, cyclopropyl, cyclobutyl, cyclopentyl, and phenyl, wherein the methyl, ethyl, propyl, ethenyl, propenyl, ethynyl, propynyl, methoxy, ethoxy, propoxy, methoxymethylene, or ethoxymethylene is optionally independently substituted with one or more R^{c1}, and the cyclopropyl, cyclobutyl, cyclopentyl, or phenyl is optionally independently substituted with one or more R^{d1}.

In some embodiments, R^{a} is each independently selected from the group consisting of F, Cl, Br, I, =O, -CD₃, -C₂D₅, -OH, -CN, methyl, ethyl, propyl, methoxymethylene, ethoxymethylene, cyclopropyl, cyclobutyl, and cyclopentyl, wherein the methyl, ethyl, propyl, methoxymethylene, or ethoxymethylene is optionally independently substituted with one or more R^{c1}, and the cyclopropyl, cyclobutyl, or cyclopentyl is optionally independently substituted with one or more R^{d1}.

In some embodiments, R^{a} is each independently selected from the group consisting of F, Cl, Br, =O, -CD₃, -C₂D₅, methyl, ethyl, propyl, cyclopropyl, cyclobutyl, and cyclopentyl, wherein the methyl, ethyl, or propyl is optionally independently substituted with one or more R^{c1}, and the cyclopropyl, cyclobutyl, or cyclopentyl is optionally independently substituted with one or more R^{d1}.

In some embodiments, R^{a} is each independently selected from the group consisting of F, methyl, =O, -CD₃, and cyclopropyl.

In some embodiments, R^{a} is each independently selected from the group consisting of methyl and =O.

In some embodiments, R^{a} is each independently selected from the group consisting of F and methyl.

In some embodiments, R¹ is selected from the group consisting of 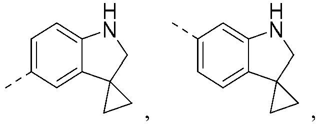 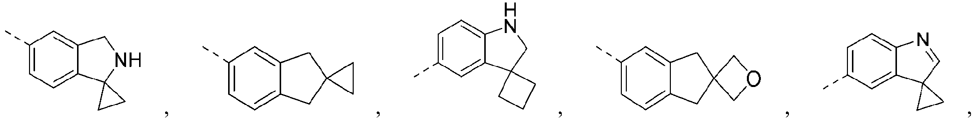 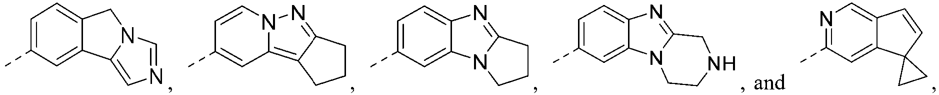 wherein R¹ may be optionally independently substituted with 1, 2, or 3 substituents selected from the group consisting of F, methyl, =O, -CD₃, and cyclopropyl. In some other embodiments, R¹ is selected from 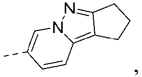 wherein R¹ may be optionally independently substituted with 1, 2, or 3 substituents selected from the group consisting of F, methyl, =O, -CD₃, and cyclopropyl.

In some other embodiments, R¹ is selected from the group consisting of 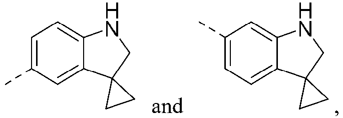 wherein R¹ may be optionally independently substituted with 1, 2, or 3 substituents selected from the group consisting of F, methyl, =O, -CD₃, and cyclopropyl.

In some other embodiments, R¹ is selected from 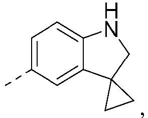 wherein R¹ may be optionally independently substituted with 1, 2, or 3 substituents selected from the group consisting of F, methyl, =O, -CD₃, and cyclopropyl.

In some embodiments, R¹ is selected from the group consisting of 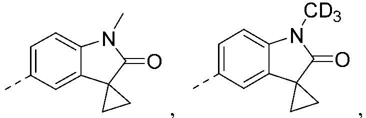 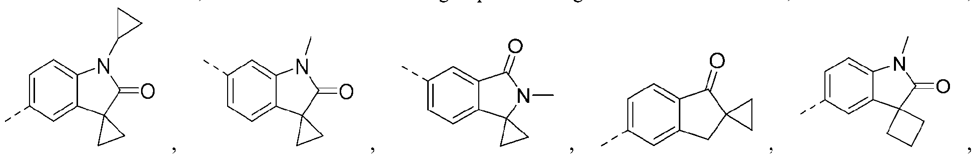 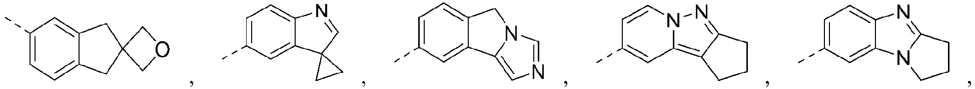 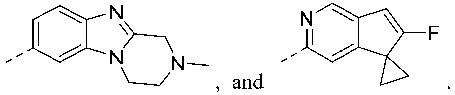 In some other embodiments, R¹ is selected from the group consisting of

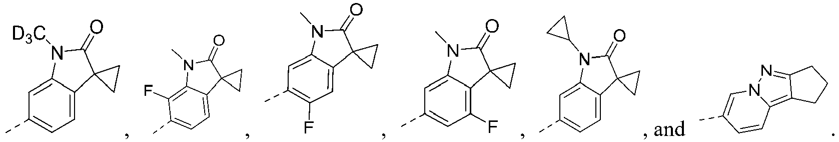

In some embodiments, R¹ is selected from the group consisting of 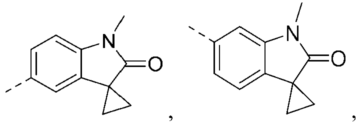 and

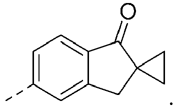

In some other embodiments, R¹ is selected from the group consisting of

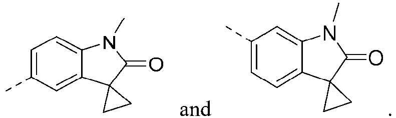

In some other embodiments, R¹ is selected from

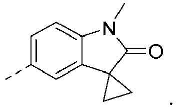

In some embodiments, R^{b} is each independently selected from the group consisting of halogen, -CN, -OH, -NH₂, methyl, ethyl, methoxy, and ethoxy, wherein the methyl, ethyl, methoxy, or ethoxy is optionally independently substituted with one or more substituents selected from the group consisting of deuterium, halogen, OH, CN, and NH₂.

In some embodiments, R^{b} is each independently selected from C₁₋₃ alkoxy, wherein the C₁₋₃ alkoxy is optionally independently substituted with one or more substituents selected from the group consisting of deuterium, halogen, OH, CN, and NH₂.

In some embodiments, R^{b} is each independently selected from the group consisting of F, Cl, Br, I, and methoxy.

In some embodiments, R^{b} is each independently selected from methoxy.

In some embodiments, R¹ is selected from the group consisting of phenyl and pyridinyl, wherein R¹ is substituted with one ethynyl or propynyl, R¹ may further be optionally independently substituted with 1, 2, or 3 substituents selected from the group consisting of F and methyl, and the ethynyl or propynyl may be optionally substituted with 1, 2, or 3 methoxy.

In some embodiments, R¹ is selected from the group consisting of phenyl and pyridinyl, wherein R¹ is substituted with one 1-propynyl, R¹ may further be optionally independently substituted with 1, 2, or 3 F, and the 1-propynyl may be optionally substituted with 1 methoxy.

In some embodiments, R¹ is selected from the group consisting of

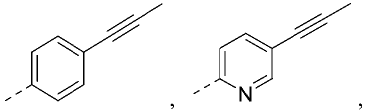

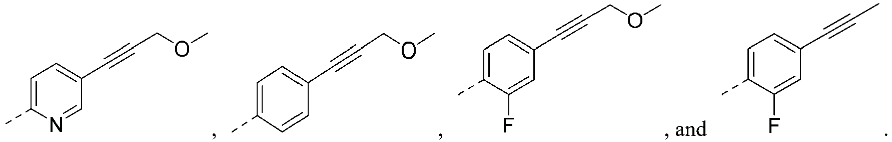

In some embodiments, R¹ is selected from the group consisting of

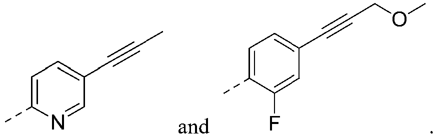

In some embodiments, R² is each independently selected from the group consisting of halogen, -OH, -CN, C₁₋₅ alkyl, C₂₋₆ alkenyl, C₂₋₆ alkynyl, C₁₋₅ alkoxy, C₃₋₆ cycloalkyl, phenyl, 5- to 6-membered heteroaryl, and 3- to 6-membered heterocyclyl, wherein the C₁₋₅ alkyl, C₂₋₆ alkenyl, C₂₋₆ alkynyl, or C₁₋₅ alkoxy is optionally independently substituted with one or more R^{c2}, and the C₃₋₆ cycloalkyl, phenyl, 5- to 6-membered heteroaryl, or 3- to 6-membered heterocyclyl is optionally independently substituted with one or more R^{d2}.

In some embodiments, R² is each independently selected from the group consisting of halogen, -OH, -CN, C₁₋₃ alkyl, C₂₋₄ alkenyl, C₂₋₄ alkynyl, C₁₋₃ alkoxy, C₃₋₅ cycloalkyl, phenyl, 5- to 6-membered heteroaryl, and 3- to 5-membered heterocyclyl, wherein the C₁₋₃ alkyl, C₂₋₄ alkenyl, C₂₋₄ alkynyl, or C₁₋₃ alkoxy is optionally independently substituted with one or more R^{c2}, and the C₃₋₆ cycloalkyl, phenyl, 5- to 6-membered heteroaryl, or 3- to 6-membered heterocyclyl is optionally independently substituted with one or more R^{d2}.

In some embodiments, R² is each independently selected from the group consisting of halogen, C₁₋₃ alkyl, and C₃₋₅ cycloalkyl, wherein the C₁₋₃ alkyl is optionally independently substituted with one or more R^{c2}, and the C₃₋₆ cycloalkyl is optionally independently substituted with one or more R^{d2}.

In some embodiments, R² is each independently selected from the group consisting of F, Cl, Br, I, methyl, ethyl, propyl, cyclopropyl, cyclobutyl, and cyclopentyl, wherein the methyl, ethyl, or propyl is optionally independently substituted with one or more R^{c2}, and the cyclopropyl, cyclobutyl, or cyclopentyl is optionally independently substituted with one or more R^{d2}.

In some embodiments, R² is each independently selected from the group consisting of F, methyl, and cyclopropyl, wherein the methyl is optionally independently substituted with one or more R^{c2}, and the cyclopropyl is optionally independently substituted with one or more R^{d2}.

In some embodiments, R² is each independently selected from the group consisting of F, methyl, and cyclopropyl.

In some embodiments, R² on two adjacent carbon atoms, together with the carbon atoms connected thereto, form C₅₋₆ cycloalkyl, phenyl, 5- to 6-membered heteroaryl, or 5- to 6-membered heterocyclyl, wherein the C₅₋₆ cycloalkyl, phenyl, 5- to 6-membered heteroaryl, or 5- to 6-membered heterocyclyl is optionally independently substituted with one or more R^{d3}.

In some embodiments, R² on two adjacent carbon atoms, together with the carbon atoms connected thereto, form C₅₋₆ cycloalkyl or 5- to 6-membered heterocyclyl, wherein the C₅₋₆ cycloalkyl or 5- to 6-membered heterocyclyl is optionally independently substituted with one or more R^{d3}.

In some embodiments, R² on two adjacent carbon atoms, together with the carbon atoms connected thereto, form C₅₋₆ cycloalkyl, wherein the C₅₋₆ cycloalkyl is optionally independently substituted with one or more R^{d3}.

In some embodiments, R² on two adjacent carbon atoms, together with the carbon atoms connected thereto, form cyclopentyl, pyrrolyl, tetrahydrofuranyl, or tetrahydrothienyl, wherein the cyclopentyl, pyrrolyl, tetrahydrofuranyl, or tetrahydrothienyl is optionally independently substituted with one or more R^{d3}. In some embodiments, R² on two adjacent carbon atoms, together with the carbon atoms connected thereto, form cyclopentyl, wherein the cyclopentyl is optionally independently substituted with one or more R^{d3}.

In some embodiments, R² on two adjacent carbon atoms, together with the carbon atoms connected thereto, form cyclopentyl.

In the present application, when R² on two adjacent carbon atoms, together with the carbon atoms connected thereto, form cyclopentyl, it should be understood that the resulting structural unit is 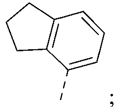 when R² on two adjacent carbon atoms, together with the carbon atoms connected thereto, form pyrrolyl, it should be understood that the resulting structural unit is

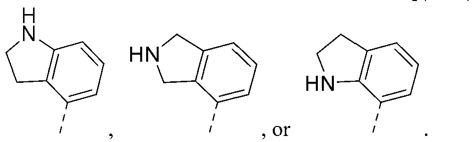

In some embodiments, R² on two adjacent carbon atoms, together with the carbon atoms connected thereto, form cyclopentyl, and the resulting structural unit is

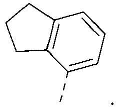

In some embodiments, q is selected from the group consisting of 0, 1, 2, and 3.

In some embodiments, q is selected from the group consisting of 1, 2, and 3.

In some embodiments, q is selected from 2.

In some embodiments, q is selected from 3.

In some embodiments, the structural unit 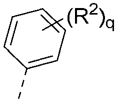 is selected from the group consisting of

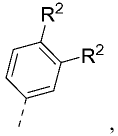

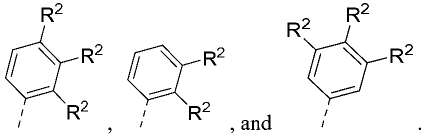

In some embodiments, the structural unit 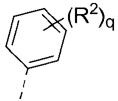 is selected from the group consisting of

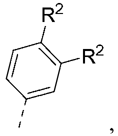

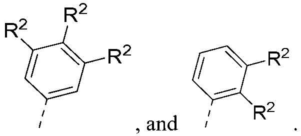

In some embodiments, the structural unit 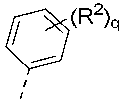 is selected from the group consisting of 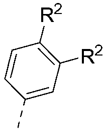 and

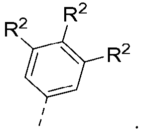

In some embodiments, the structural unit 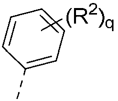 is selected from

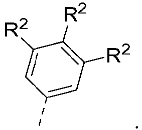

In some embodiments, the structural unit 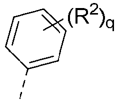 is selected from the group consisting of

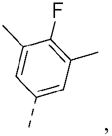

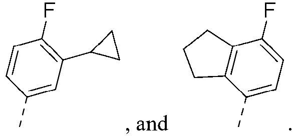

In some embodiments, the structural unit 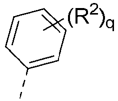 is selected from

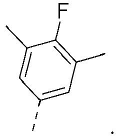

In some embodiments, one of X¹ and X² is selected from C, and the other is selected from N.

In some embodiments, the structural unit 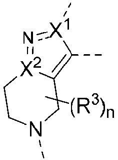 is selected from the group consisting of 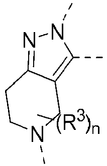 and

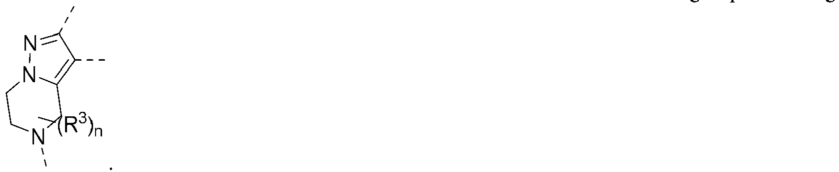

In some embodiments, the structural unit 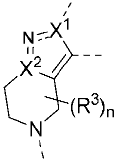 is selected from

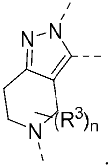

In some embodiments, Y¹, Y², and Y³ are each independently selected from the group consisting of C and N, and Y⁴ is selected from CH.

In some embodiments, at least one of Y¹, Y², and Y³ is selected from N, and Y⁴ is selected from CH.

In some embodiments, Y¹ is selected from C, Y² is selected from N, Y³ is selected from C, and Y⁴ is selected from CH.

In some embodiments, Y¹ is selected from C, Y² is selected from C, Y³ is selected from N, and Y⁴ is selected from CH.

In some embodiments, Y¹ is selected from N, Y² is selected from C, Y³ is selected from C, and Y⁴ is selected from CH.

In some embodiments, the structural unit 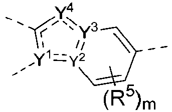 is selected from the group consisting of

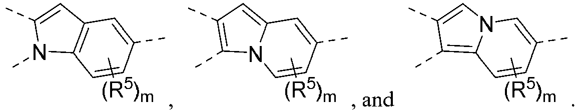

In some embodiments, the structural unit 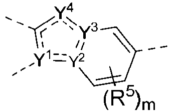 is selected from

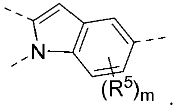

In some embodiments, R³ is each independently selected from the group consisting of deuterium, halogen, C₁₋₃ alkyl, C₂₋₄ alkenyl, C₂₋₄ alkynyl, C₃₋₆ cycloalkyl, phenyl, 5- to 6-membered heteroaryl, and 3- to 6-membered heterocyclyl, wherein the C₁₋₃ alkyl, C₂₋₄ alkenyl, or C₂₋₄ alkynyl is optionally independently substituted with one or more R^{c3}, and the C₃₋₆ cycloalkyl, phenyl, 5- to 6-membered heteroaryl, or 3- to 6-membered heterocyclyl is optionally independently substituted with one or more R^{d4}.

In some embodiments, R³ is each independently selected from the group consisting of deuterium, halogen, and C₁₋₃ alkyl, wherein the C₁₋₃ alkyl is optionally independently substituted with one or more R^{c3}.

In some embodiments, R³ is each independently selected from C₁₋₃ alkyl, wherein the C₁₋₃ alkyl is optionally independently substituted with one or more R^{c3}.

In some embodiments, R³ is each independently selected from the group consisting of deuterium, F, Cl, Br, **I,** methyl, ethyl, propyl, cyclopropyl, cyclobutyl, and cyclopentyl, wherein the methyl, ethyl, or propyl is optionally independently substituted with one or more R^{c3}, and the cyclopropyl, cyclobutyl, or cyclopentyl is optionally independently substituted with one or more R^{d4}.

In some embodiments, R³ is each independently selected from the group consisting of methyl, ethyl, and propyl, wherein the methyl, ethyl, or propyl is optionally substituted with one or more R^{c3}.

In some embodiments, R³ is selected from methyl.

In some embodiments, n is selected from the group consisting of 0, 1, 2, and 3.

In some embodiments, n is selected from the group consisting of 1, 2, and 3.

In some embodiments, n is selected from 1.

In some embodiments, R⁴ is selected from the group consisting of H, deuterium, halogen, -CN, C₁₋₃ alkyl, deuterated C₁₋₃ alkyl, and C₁₋₃ alkoxy, wherein the C₁₋₃ alkyl, deuterated C₁₋₃ alkyl, or C₁₋₃ alkoxy is optionally independently substituted with one or more R^{c4}.

In some embodiments, R⁴ is selected from the group consisting of H and C₁₋₃ alkyl, wherein the C₁₋₃ alkyl is optionally independently substituted with one or more R^{c4}.

In some embodiments, R⁴ is selected from the group consisting of H and methyl, wherein the methyl is optionally substituted with one or more R^{c4}.

In some embodiments, R⁴ is selected from the group consisting of H and methyl.

In some embodiments, R⁴ is selected from H.

In some embodiments, R⁴ is selected from methyl.

In some embodiments, R⁵ is selected from the group consisting of halogen, -CN, C₁₋₃ alkyl, deuterated C₁₋₃ alkyl, and C₁₋₃ alkoxy, wherein the C₁₋₃ alkyl, deuterated C₁₋₃ alkyl, or C₁₋₃ alkoxy is optionally independently substituted with one or more R^{c5}.

In some embodiments, R⁵ is selected from C₁₋₃ alkyl, wherein the C₁₋₃ alkyl is optionally independently substituted with one or more R^{c5}.

In some embodiments, R⁵ is selected from methyl.

In some embodiments, m is selected from the group consisting of 0, 1, 2, and 3.

In some embodiments, m is selected from the group consisting of 0, 1, and 2.

In some embodiments, m is selected from 0.

In some embodiments, m is selected from 1.

In some embodiments, R' and R" are each independently selected from the group consisting of H, deuterium, halogen, -CN, C₁₋₃ alkyl, deuterated C₁₋₃ alkyl, and C₁₋₃ alkoxy, wherein the C₁₋₃ alkyl, deuterated C₁₋₃ alkyl, or C₁₋₃ alkoxy is optionally independently substituted with one or more R^{c6}.

In some embodiments, R' and R" are each independently selected from the group consisting of H and C₁₋₃ alkyl, wherein the C₁₋₃ alkyl is optionally independently substituted with one or more R^{c6}.

In some embodiments, R' and R" are each independently selected from the group consisting of H and methyl, wherein the methyl is optionally substituted with one or more R^{c6}.

In some embodiments, R' and R" are selected from methyl.

In some embodiments, R' and R", together with the carbon atom connected thereto, form C₃₋₄ cycloalkyl or 3- to 4-membered heterocycloalkyl, wherein the C₃₋₄ cycloalkyl or 3- to 4-membered heterocycloalkyl is optionally independently substituted with one or more R^{d5}.

In some embodiments, R' and R", together with the carbon atom connected thereto, form cyclopropyl, wherein the cyclopropyl is optionally independently substituted with one or more R^{d5}.

In some embodiments, R' and R", together with the carbon atom connected thereto, form cyclopropyl.

In some embodiments, R^{c1}, R^{c2}, R^{c3}, R^{c4}, R^{c5}, and R^{c6} are each independently selected from the group consisting of halogen, -CN, -OH, and -NH₂.

In some embodiments, R^{c1}, R^{c2}, R^{c3}, R^{c4}, R^{c5}, and R^{c6} are each independently selected from the group consisting of F, -CN, -OH, and -NH₂.

In some embodiments, R^{c1}, R^{c2}, R^{c3}, R^{c4}, R^{c5}, and R^{c6} are each independently selected from the group consisting of F and OH.

In some embodiments, R^{c1}, R^{c2}, R^{c3}, R^{c4}, R^{c5}, and R^{c6} are each independently selected from F.

In some embodiments, R^{d1}, R^{d2}, R^{d3}, R^{d4}, and R^{d5} are each independently selected from the group consisting of halogen, -CN, -OH, -NH₂, C₁₋₃ alkyl, and C₁₋₃ alkoxy, wherein the C₁₋₃ alkyl or C₁₋₃ alkoxy is optionally independently substituted with one or more substituents selected from the group consisting of halogen, OH, CN, and NH₂.

In some embodiments, R^{d1}, R^{d2}, R^{d3}, R^{d4}, and R^{d5} are each independently selected from the group consisting of F, - CN, -OH, -NH₂, methyl, and methoxy, wherein the methyl or methoxy is optionally independently substituted with one or more substituents selected from the group consisting of F, OH, CN, and NH₂.

In some embodiments, R^{d1}, R^{d2}, R^{d3}, R^{d4}, and R^{d5} are each independently selected from the group consisting of F, - CN, -OH, -NH₂, methyl, and methoxy.

In some embodiments, R^{d1}, R^{d2}, R^{d3}, R^{d4}, and R^{d5} are each independently selected from the group consisting of F and methyl.

In some embodiments, R^{d1}, R^{d2}, R^{d3}, R^{d4}, and R^{d5} are each independently selected from F.

The compound of formula (I), the stereoisomer thereof, or the pharmaceutically acceptable salt thereof of the present application is selected from the group consisting of compounds of formulas (I-A), (I-B), (I-C), and (I-D), stereoisomers thereof, or pharmaceutically acceptable salts thereof, 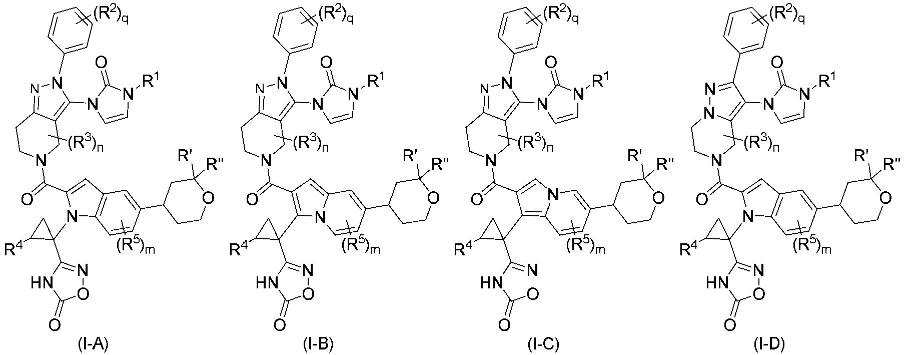 wherein R¹, R², R³, R⁴, R⁵, R', R", q, n, or m is as defined in the compound of formula (I).

The compound of formula (I), the stereoisomer thereof, or the pharmaceutically acceptable salt thereof of the present application is selected from the group consisting of compounds of formulas (II-A), (II-B), (II-C), (II-D), (II-E), (II-F), (II-G), (II-H), (II-I), (II-J), (II-K), and (II-L), stereoisomers thereof, or pharmaceutically acceptable salts thereof, 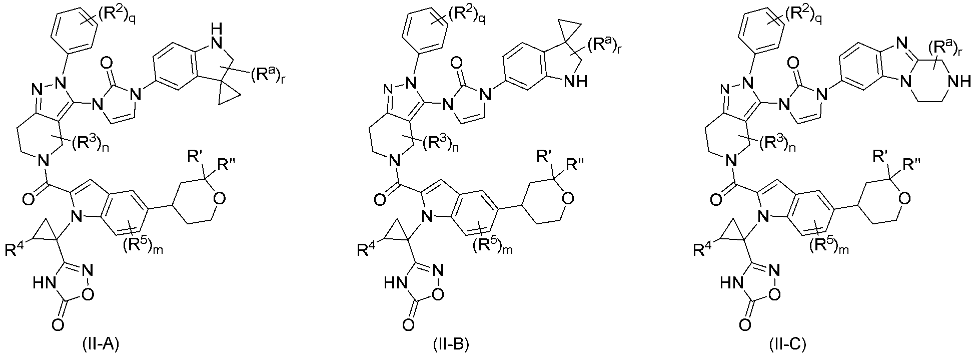 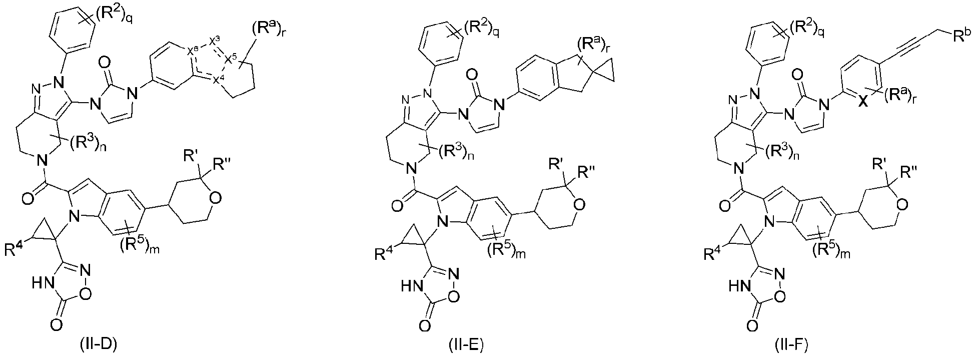 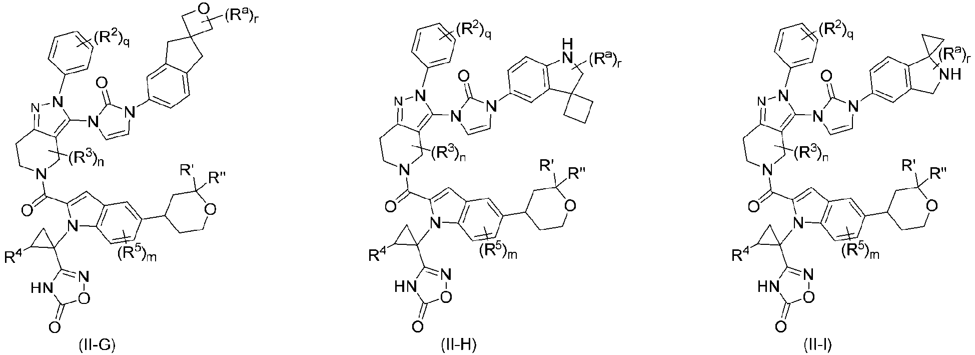 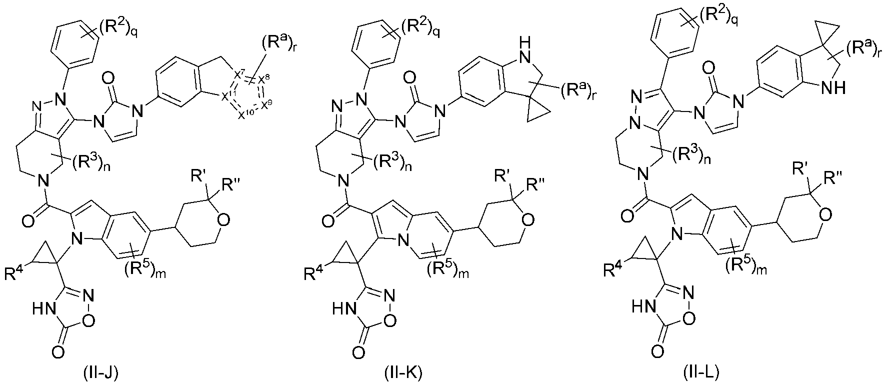
wherein " ", R¹, R², R³, R⁴, R⁵, R', R", R^{a}, q, n, or m is as defined in the compound of formula (I);
r is selected from the group consisting of 0, 1, 2, 3, and 4;
X is selected from the group consisting of C and N;
X³, X⁴, X⁵, and X⁶ are each independently selected from the group consisting of C and N;
X⁷, X⁸, X⁹, X¹⁰, and X¹¹ are each independently selected from the group consisting of C, CH, and N.

In some embodiments, X is selected from C. In some embodiments, X is selected from N.

In some embodiments, X³ is selected from N, and X⁴, X⁵, and X⁶ are each independently selected from the group consisting of C and N.

In some embodiments, X³ and X⁶ are selected from N, and X⁴ and X⁵ are selected from C.

In some embodiments, X³ and X⁴ are selected from N, and X⁵ and X⁶ are selected from C.

In some embodiments, one of X⁷ and X¹¹ is selected from N, and the other is selected from C.

In some embodiments, X⁷ and X⁹ are selected from N, X¹¹ is selected from C, and X⁸ and X¹⁰ are selected from CH.

In some embodiments, r is selected from the group consisting of 0, 1, and 2.

In some embodiments, r is selected from the group consisting of 0 and 2.

The present application further provides a compound of formula (I'), a stereoisomer thereof, or a pharmaceutically acceptable salt thereof, 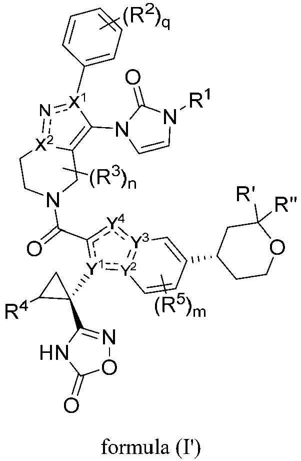 wherein X¹, X², Y¹, Y², Y³, Y⁴, " 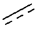 ",R¹, R², R³, R⁴, R⁵, R', R", q, n, or m is as defined in the compound of formula (I).

The compound of formula (I), the stereoisomer thereof, or the pharmaceutically acceptable salt thereof of the present application is selected from the group consisting of compounds of formulas (I-A'), (I-B'), (I-C'), and (I-D'), stereoisomers thereof, or pharmaceutically acceptable salts thereof, 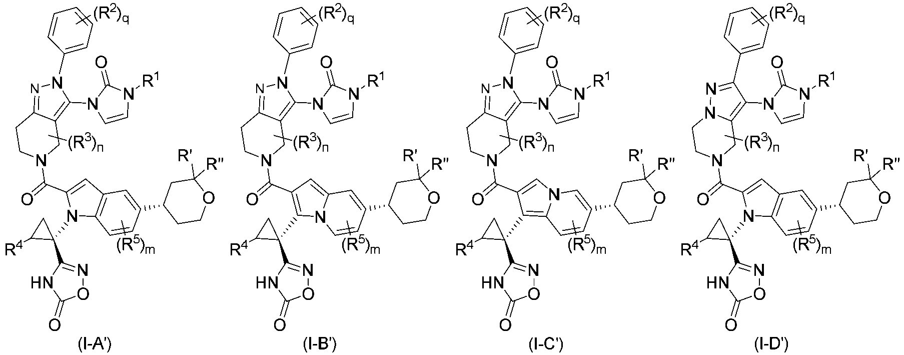 wherein R¹, R², R³, R⁴, R⁵, R', R", q, n, or m is as defined in the compound of formula (I).

The compound of formula (I), the stereoisomer thereof, or the pharmaceutically acceptable salt thereof of the present application is selected from the group consisting of compounds of formulas (II-A'), (II-B'), (II-C'), (II-D'), (II-E'), (II-F'), (II-G'), (II-H'), (II-I'), (II-J'), (II-K'), and (II-L'), stereoisomers thereof, or pharmaceutically acceptable salts thereof, 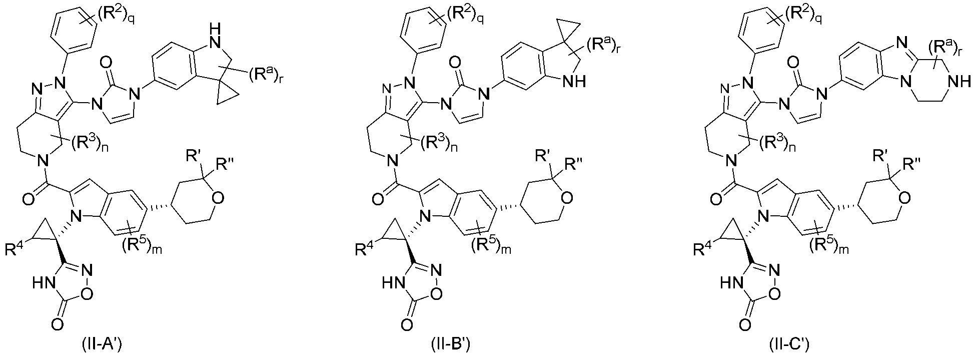 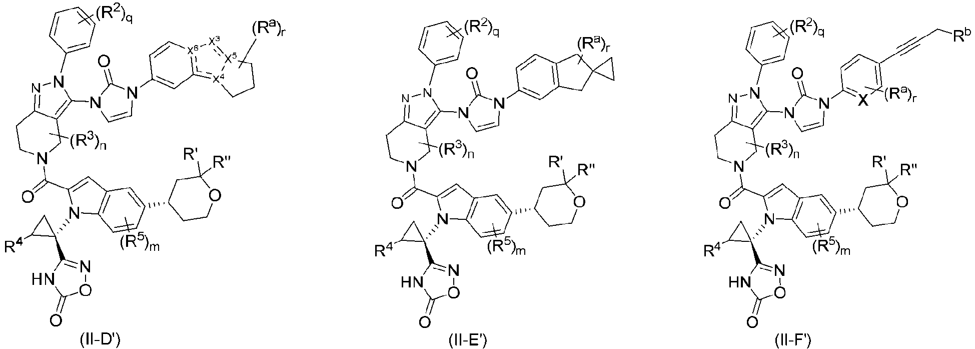 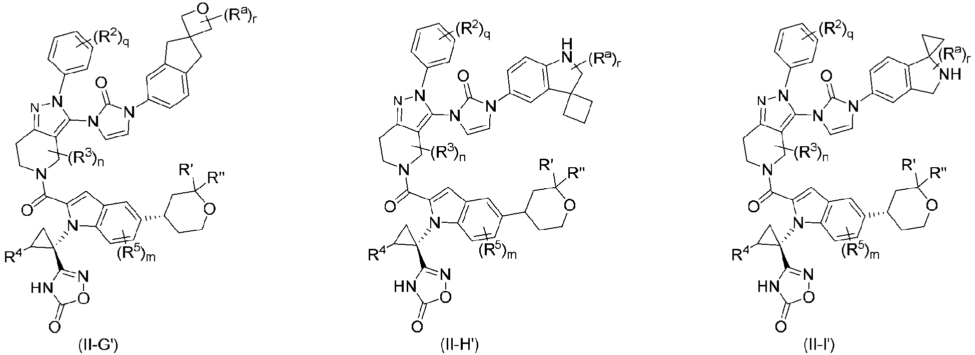 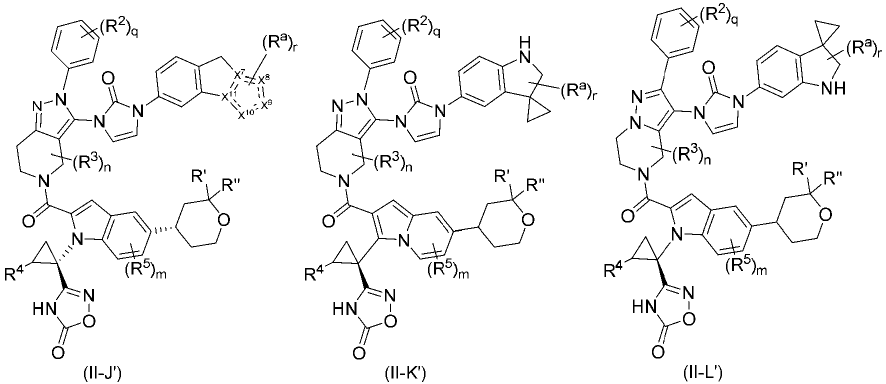
wherein " 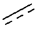 ", R¹, R², R³, R⁴, R⁵, R', R", R^{a}, q, n, or m is as defined in the compound of formula (I);
r is selected from the group consisting of 0, 1, 2, 3, and 4;
X is selected from the group consisting of C and N;
X³, X⁴, X⁵, and X⁶ are each independently selected from the group consisting of C and N;
X⁷, X⁸, X⁹, X¹⁰, and X¹¹ are each independently selected from the group consisting of C, CH, and N.

In some embodiments, X, X³, X⁴, X⁵, X⁶, X⁷, X⁸, X⁹, X¹⁰, or X¹¹ is as defined in the compound of formula (II-D), (II-F), or (II-J) described above.

The compound of formula (I), the stereoisomer thereof, or the pharmaceutically acceptable salt thereof of the present application is selected from a compound of formula (III-A), a stereoisomer thereof, or a pharmaceutically acceptable salt thereof, 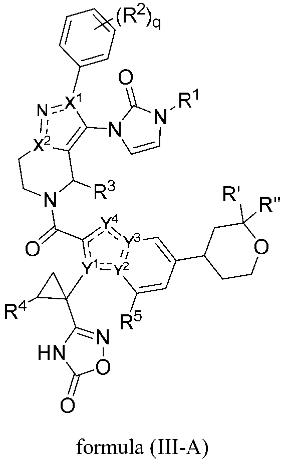 wherein X¹, X², Y¹, Y², Y³, Y⁴, " 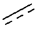 ", R¹, R², R³, R⁴, R⁵, R', R", and q are as defined in the compound of formula (I).

The compound of formula (I), the stereoisomer thereof, or the pharmaceutically acceptable salt thereof of the present application is selected from the group consisting of compounds of formulas (III-A'), (III-B'), and (III-C'), stereoisomers thereof, or pharmaceutically acceptable salts thereof, 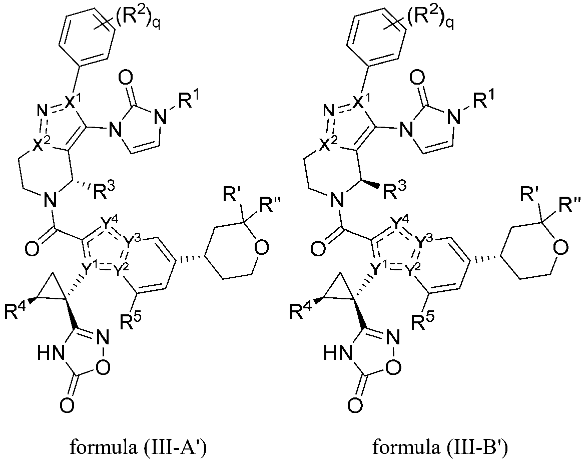 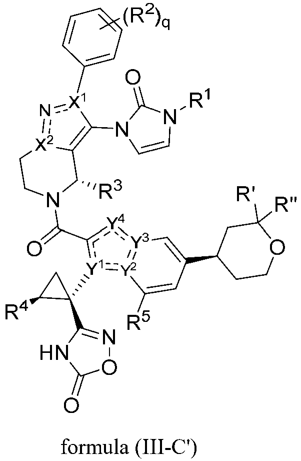 wherein X¹, X², Y¹, Y², Y³, Y⁴, " 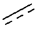 ", R¹, R², R³, R⁴, R⁵, R', R", and q are as defined in the compound of formula (I).

The compound of formula (I), the stereoisomer thereof, or the pharmaceutically acceptable salt thereof of the present application is selected from a compound of formula (IV-A), a stereoisomer thereof, or a pharmaceutically acceptable salt thereof, 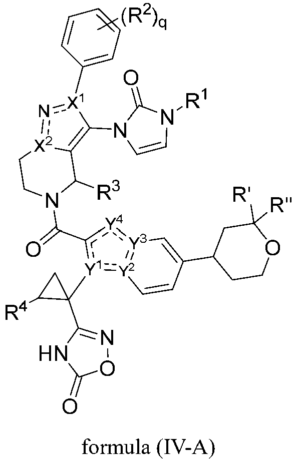 wherein X¹, X², Y¹, Y², Y³, Y⁴, " 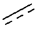 ", R¹, R², R³, R⁴, R', R", and q are as defined in the compound of formula (I).

The compound of formula (I), the stereoisomer thereof, or the pharmaceutically acceptable salt thereof of the present application is selected from the group consisting of compounds of formulas (IV-A'), (IV-B'), and (IV-C'), stereoisomers thereof, or pharmaceutically acceptable salts thereof, 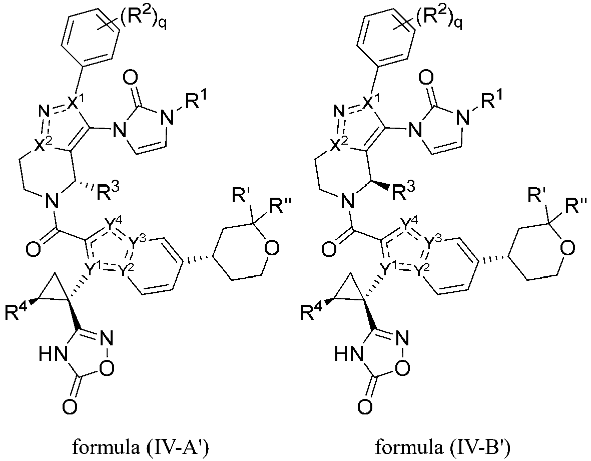 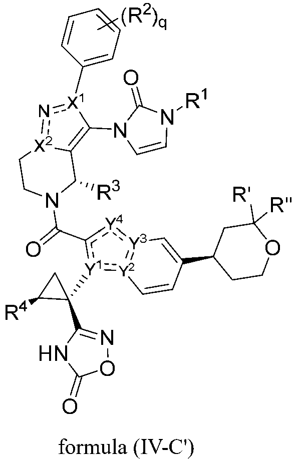 wherein X¹, X², Y¹, Y², Y³, Y⁴, " 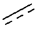 " R¹, R², R³, R⁴, R', R", and q are as defined in the compound of formula (I).

In some embodiments, the present disclosure encompasses the variables defined above and embodiments thereof, as well as any combination thereof.

In another aspect, the present disclosure provides a compound of one of the following formulas, a stereoisomer thereof, or a pharmaceutically acceptable salt thereof:

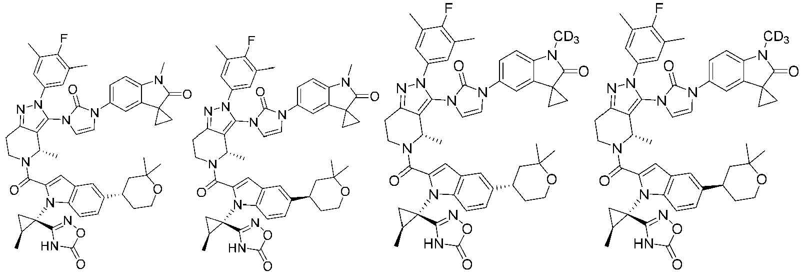

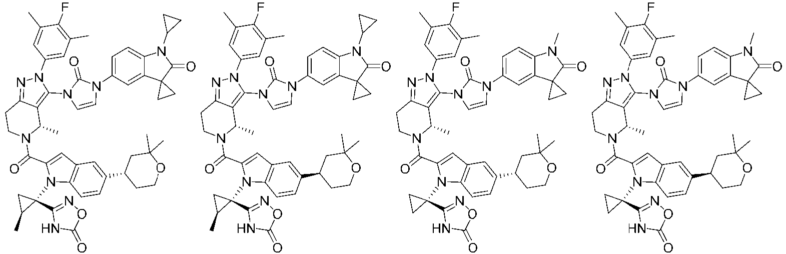

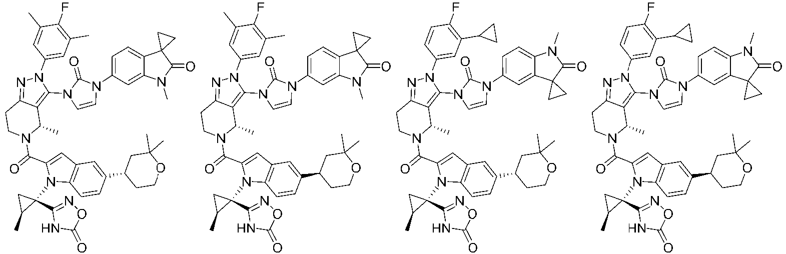

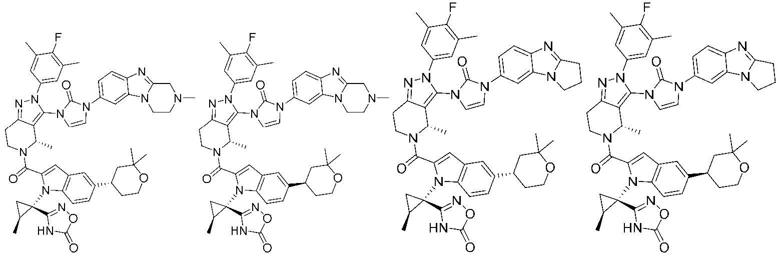

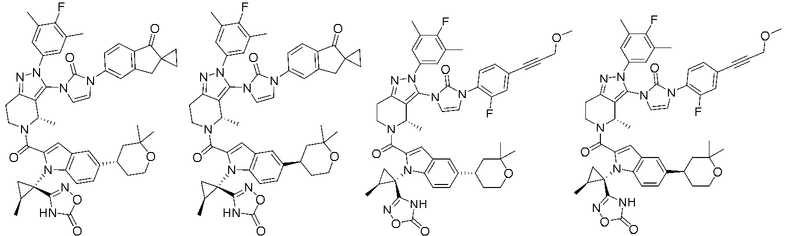

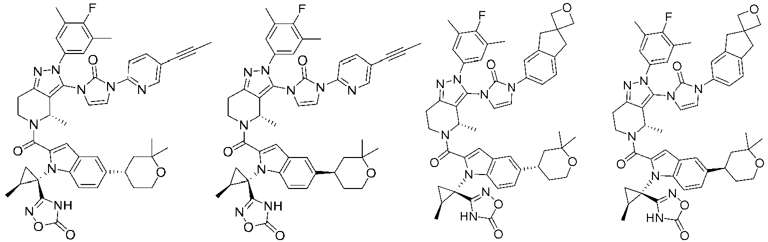

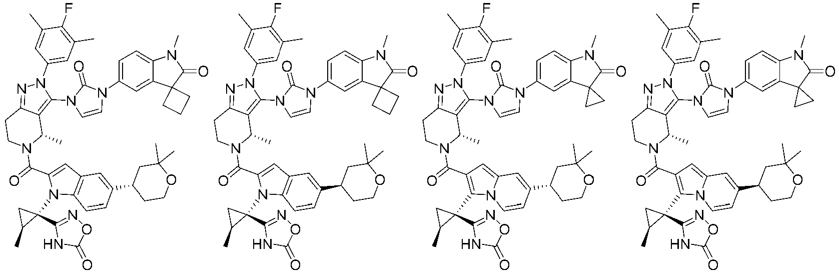

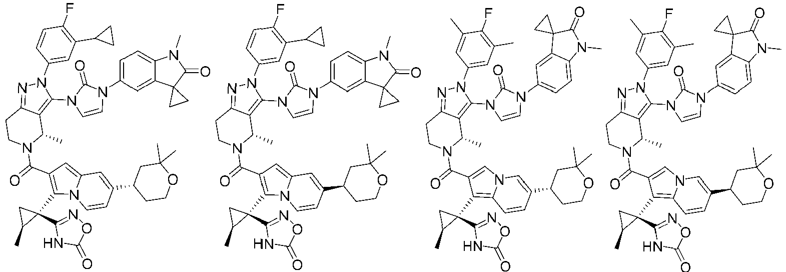

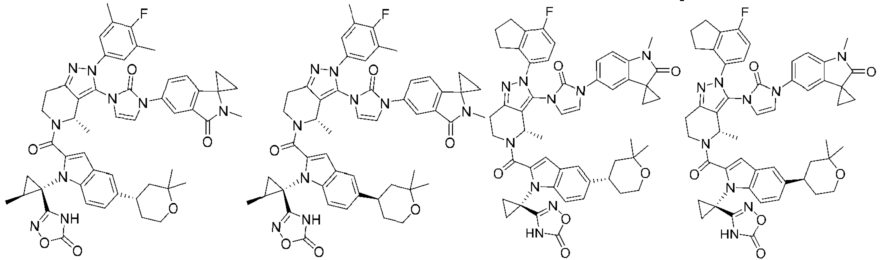

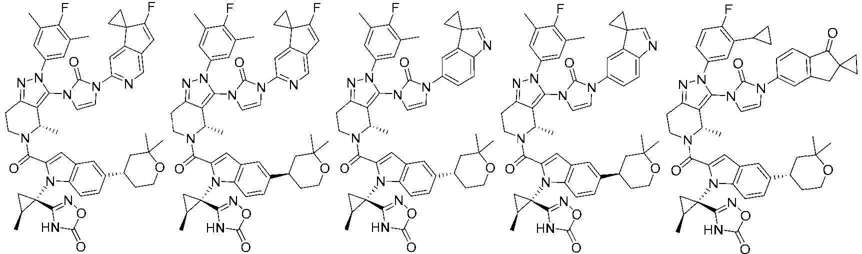

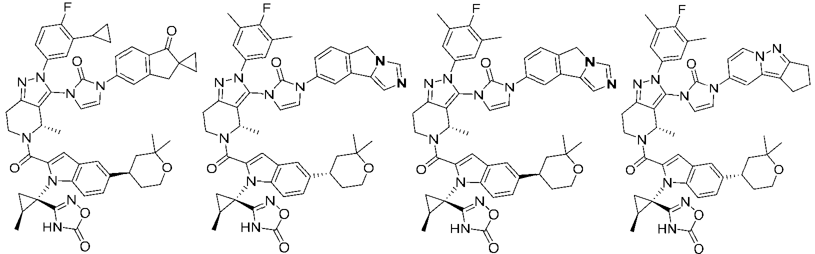

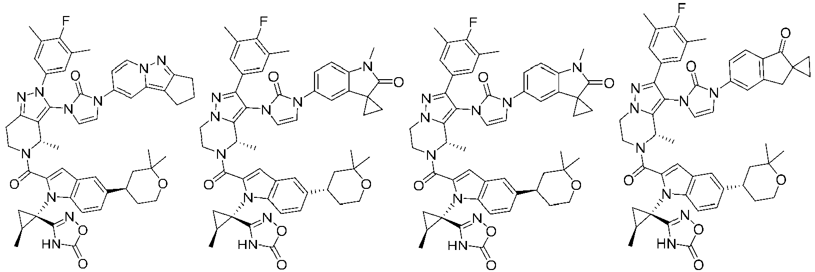

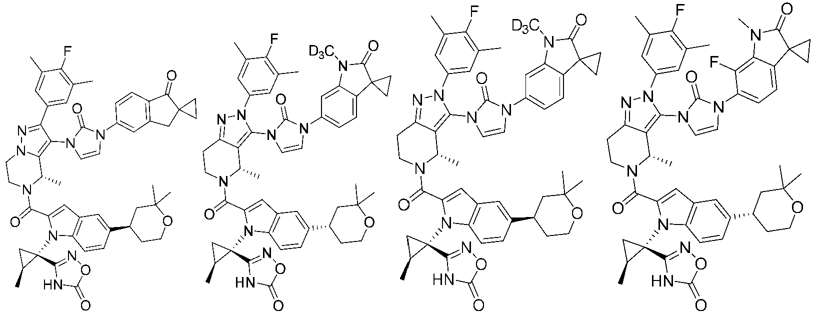

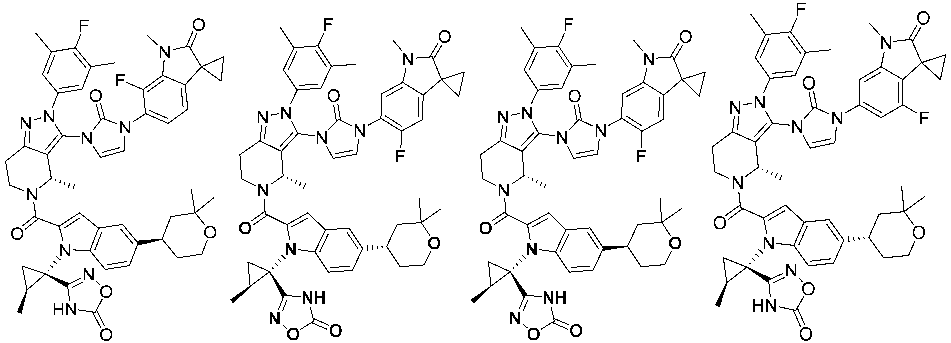

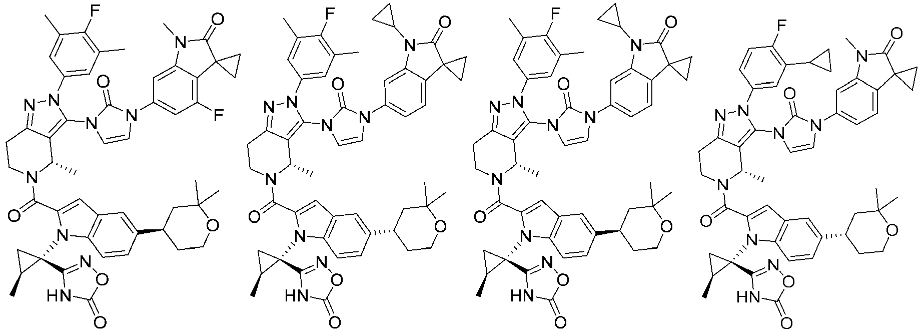

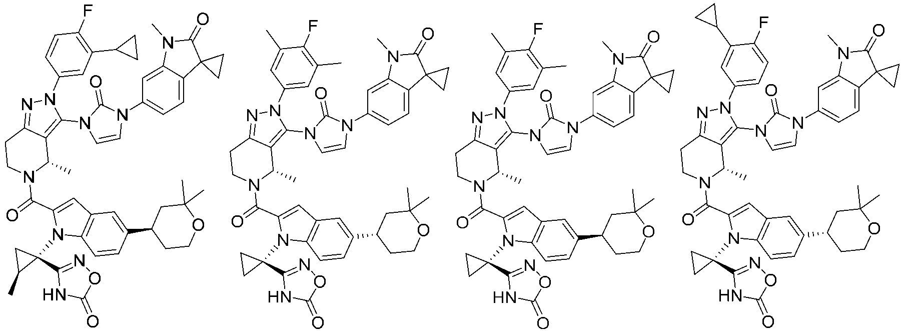

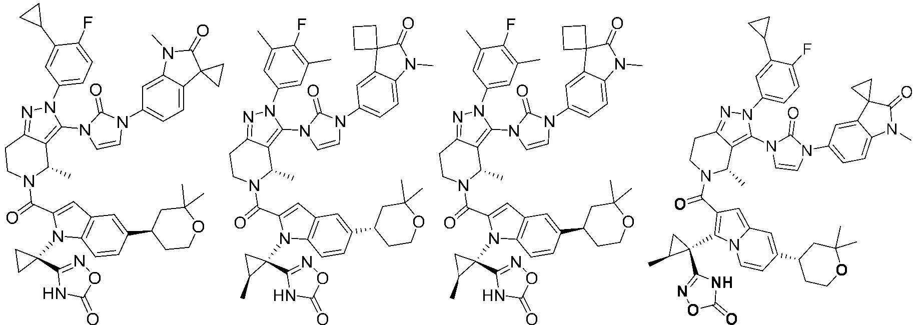

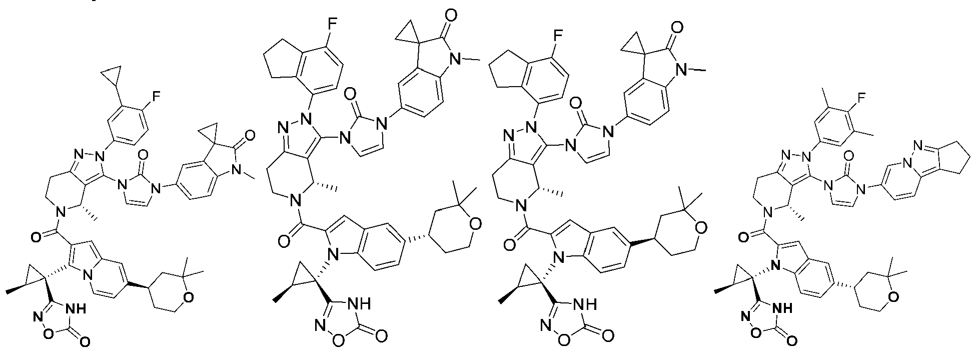

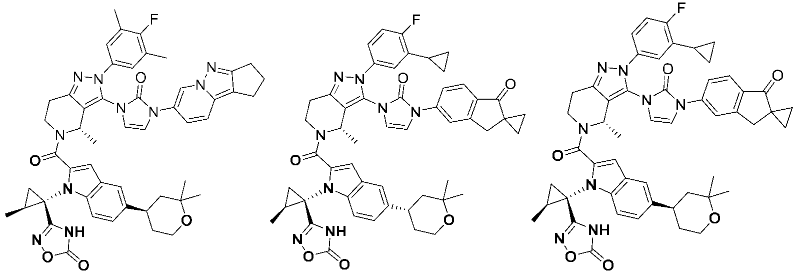

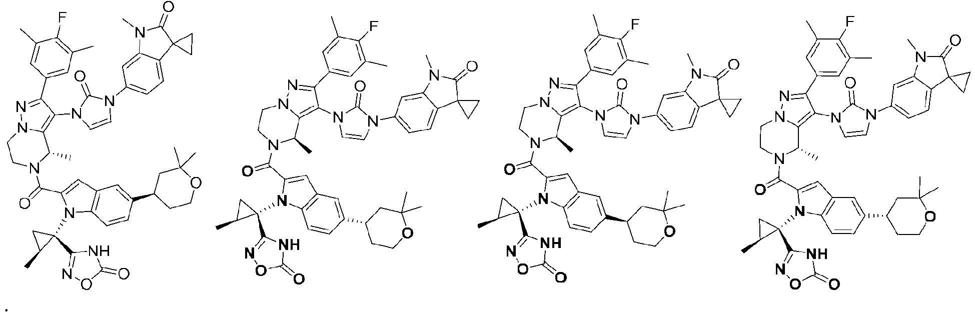

In another aspect, the present disclosure further provides a pharmaceutical composition, which comprises the compound, the stereoisomer thereof, or the pharmaceutically acceptable salt thereof described herein above. In some embodiments, the pharmaceutical composition of the present disclosure further comprises a pharmaceutically acceptable excipient.

In another aspect, the present disclosure further provides a method for treating various GLP-1-associated diseases, which comprises administering to a mammal, preferably a human, in need of the treatment a therapeutically effective amount of the compound, the stereoisomer thereof, or the pharmaceutically acceptable salt thereof, or the pharmaceutical composition thereof described herein above.

In another aspect, the present disclosure further provides use of the compound, the stereoisomer thereof, or the pharmaceutically acceptable salt thereof, or the pharmaceutical composition thereof described herein above for preparing a medicament for treating various GLP-1-associated diseases.

In another aspect, the present disclosure further provides use of the compound, the stereoisomer thereof, or the pharmaceutically acceptable salt thereof, or the pharmaceutical composition thereof described herein above for treating various GLP-1-associated diseases.

In another aspect, the present disclosure further provides the compound, the stereoisomer thereof, or the pharmaceutically acceptable salt thereof, or the pharmaceutical composition thereof described herein above for use in treating various GLP-1-associated diseases.

In some embodiments, the various GLP-1-associated diseases are selected from the group consisting of diabetes and obesity.

The compound of the present disclosure has good *in vivo* and *in vitro* agonist activity related to GLP-1 (e.g., *in vitro* enzymatic agonist activity for GLP-1) and metabolic stability (e.g., metabolic stability in liver microsomes).

### Definitions

Unless otherwise stated, the following terms used in the present disclosure shall have the following meanings. A certain term, unless otherwise specifically defined, should not be considered uncertain or unclear, but interpreted according to its common meaning in the art. When referring to a trade name, it is intended to refer to its corresponding commercial product or its active ingredient.

When certain structural units or groups in the present disclosure have a covalent bond that is not connected to a specific atom, it means that the covalent bond can be connected to any atom within that structural unit or group, as long as it adheres to the rules of valence bonding.

The term "substituted" means that any one or more hydrogen atoms on a specific atom are replaced by substituents, as long as the valence of the specific atom is normal and the resulting compound is stable. When the substituent is oxo (i.e., =O), it means that two hydrogen atoms are replaced; oxo is not possible on an aromatic group.

The term "optional" or "optionally" means that the subsequently described event or circumstance may or may not occur. The description includes instances where the event or circumstance occurs and instances where it does not. For example, ethyl "optionally" substituted with halogen means that the ethyl may be unsubstituted (CH₂CH₃), monosubstituted (e.g., CH₂CH₂F), polysubstituted (e.g., CHFCH₂F, CH₂CHF₂, etc.), or fully substituted (CF₂CF₃). It can be understood by those skilled in the art that for any group containing one or more substituents, no substitutions or substitution patterns that are spatially impossible and/or cannot be synthesized will be introduced.

Cₘ₋ₙ herein means that the moiety has an integer number of carbon atoms in the given range. For example, "C₁₋₆" means that the group may have 1 carbon atom, 2 carbon atoms, 3 carbon atoms, 4 carbon atoms, 5 carbon atoms, or 6 carbon atoms. For example, C₁₋₃ means that the group may have 1 carbon atom, 2 carbon atoms, or 3 carbon atoms.

When any variable (e.g., R) occurs more than once in the constitution or structure of a compound, the definition of the variable in each case is independent. Therefore, for example, if a group is substituted with 2 R, the definition of each R is independent.

When the number of connecting groups is 0, for example, -(CH2)₀-, it means that the connecting group is a covalent bond.

When one of the variables is selected from a covalent bond, it means that the two groups connected by it are directly linked. For example, in A-L'-Z, when L' represents a covalent bond, it means that the structure is actually A-Z.

When a bond of a substituent is cross-linked to two atoms on a ring, the substituent can be bonded to any atom on the ring. For example, ring A described above indicates that the bonds on both sides may be linked to any two different atoms on ring A.

The term "halo" or "halogen" refers to fluorine, chlorine, bromine, and iodine.

The term "alkyl" refers to hydrocarbyl with a general formula of CₙH₂ₙ₊₁, typically having 1 to 12, 1 to 8, 1 to 6, 1 to 5, 1 to 4, 1 to 3, or 1 to 2 carbon atoms. The alkyl may be linear or branched; for example, it may be "C₁₋₁₂ alkyl", "C₁₋₆ alkyl", or the like. For example, the term "C₁₋₁₂ alkyl" refers to alkyl containing 1 to 12 carbon atoms, including C₁, C₂, C₃, C₄, C₅, C₆, C₇, C₈, C₉, C₁₀, C₁₁, and C₁₂ alkyl, as well as combinations of any ranges thereof. For example, the term "C₁₋₆ alkyl" refers to alkyl containing 1 to 6 carbon atoms (e.g., methyl, ethyl, *n*-propyl, isopropyl, *n*-butyl, isobutyl, *sec*-butyl, *tert*-butyl, *n*-pentyl, 1-methylbutyl, 2-methylbutyl, 3-methylbutyl, neopentyl, hexyl, 2-methylpentyl, etc.). Similarly, the alkyl moieties (i.e., alkyl) of alkoxy, alkylamino, dialkylamino, alkylsulfonyl, and alkylthio have the same definition as described above. As another example, the term "C₁₋₃ alkyl" refers to alkyl containing 1 to 3 carbon atoms (e.g., methyl, ethyl, propyl, and isopropyl).

The term "deuterated C₁₋ₙ alkyl" refers to alkyl substituted with 1 to 2n+1 deuterium atoms, where the alkyl is as defined above; the maximum number of deuterium atoms that can be used for substitution depends on the maximum number of hydrogen atoms that can be present in the alkyl itself, and the substitution positions may be arbitrary. For example, the term "deuterated C₁₋₃ alkyl" means that alkyl containing 1 to 3 carbon atoms (e.g., methyl, ethyl, *n-*propyl, or isopropyl) is substituted with 1-7 deuterium atoms, wherein methyl may be substituted with 1, 2, or 3 deuterium atoms; ethyl may be substituted with 1, 2, 3, 4, or 5 deuterium atoms; *n*-propyl and isopropyl may be substituted with 1, 2, 3, 4, 5, 6, or 7 deuterium atoms.

The term "alkenyl" refers to a linear or branched unsaturated aliphatic hydrocarbyl consisting of carbon atoms and hydrogen atoms with at least one double bond, typically having 2 to 12, 2 to 8, 2 to 6, 2 to 4, or 2 to 3 carbon atoms; for example, it may be "C₂₋₁₂ alkenyl", "C₂₋₆ alkenyl", or the like. Non-limiting examples of the alkenyl include, but are not limited to, ethenyl, 1-propenyl, 2-propenyl, 1-butenyl, isobutenyl, 1,3-butadienyl, and the like.

The term "alkynyl" refers to a linear or branched unsaturated aliphatic hydrocarbyl consisting of carbon atoms and hydrogen atoms with at least one triple bond, typically having 2 to 12, 2 to 8, 2 to 6, 2 to 4, or 2 to 3 carbon atoms; for example, it may be "C₂₋₁₂ alkynyl", "C₂₋₆ alkynyl", or the like. Non-limiting examples of the alkynyl include, but are not limited to, ethynyl (-C≡CH), propynyl (1-propynyl (-C≡C-CH₃) and 2-propynyl (-CH₂-C≡CH)), butynyl (1,3-butadiynyl (-C≡C-C≡CH)), and the like.

The term "alkoxy" refers to -O-alkyl.

The term "alkylthio" refers to -S-alkyl.

The term "cycloalkyl" refers to a fully saturated or partially saturated cyclic hydrocarbon, which may exist as a monocyclic, bridged cyclic, fused cyclic, or spiro cyclic structure. Unless otherwise indicated, the carbocyclic ring is usually a 3- to 10-membered ring, a 4- to 8-membered ring, a 5- to 8-membered ring, or a 5- to 6-membered ring. Non-limiting examples of the cycloalkyl include, but are not limited to, cyclopropyl, cyclobutyl, cyclopentyl, cyclohexyl, norbornyl (bicyclo[2.2.1]heptyl), bicyclo[2.2.2]octyl, adamantyl, bicyclo[1.1.1]pentan-1-yl, and the like. For example, C₃₋₄ cycloalkyl includes cyclopropyl and cyclobutyl.

The term "heterocyclyl" refers to a fully saturated or partially saturated non-aromatic or partially non-aromatic ring that may exist as a monocyclic, bridged cyclic, fused cyclic, or spiro cyclic structure. Unless otherwise indicated, the heterocyclic ring is usually a 3- to 13-membered ring (e.g., a 3-, 4-, 5-, 6-, 7-, 8-, 9-, 10-, 11-, 12-, or 13-membered ring), a 10- to 13-membered ring, or an 11- to 13-membered ring, containing 1 to 3 heteroatoms (preferably 1 or 2 heteroatoms) independently selected from the group consisting of sulfur, oxygen, nitrogen, phosphorus, silicon, and/or boron. Non-limiting examples of the heterocyclyl include, but are not limited to, spiro[cyclopropane-1,3'-indolin]yl, 1,2,3,4-tetrahydrobenzo[4,5]imidazo[1,2-*a*]pyrazinyl, 2,3-dihydro-1*H*-benzo[*d*]pyrrolo[1,2-*a*]imidazolyl, 1',3'-dihydrospiro[cyclopropane-1,2'-inden]yl, phenyl, pyridinyl, 1,3-dihydrospiro[indene-2,3'-oxetane]yl, spiro[cyclobutane-1,3'-indolin]yl, spiro[cyclopropane-1,1'-isoindolin]yl, spiro[cyclopenta[*c*]pyridine-5,1'-cyclopropane]yl, spiro[cyclopropane-1,3'-indol]yl, 5*H*-imidazo[5,1-*a*]isoindolyl, 2,3-dihydro-1*H-*cyclopenta[3,4]pyrazolo[1,5-*a*]pyridinyl, or the like.

The term "aryl" refers to an aromatic carbocyclic group, and in addition to the aromatic moiety, it may also contain a non-aromatic moiety. The cyclic group may be monocyclic or it may be a bicyclic or tricyclic group formed by a benzene ring with an aromatic moiety or a non-aromatic moiety. For example, the aryl may have 6-20 carbon atoms, 6-15 carbon atoms, 6-14 carbon atoms, 6-12 carbon atoms, or 6-11 carbon atoms. Non-limiting examples of the aryl include, but are not limited to, phenyl, naphthyl, anthryl, 1,2,3,4-tetrahydronaphthalene, isochromanyl, 2,4-dihydro-1*H*-isoquinolin-3-onyl, or spiro[cyclopropane-1,2'-indene]-1'(3'*H*)-onyl.

The term "heteroaryl" refers to a monocyclic or polycyclic system that contains at least one ring atom selected from the group consisting of N, O, and S, with the remaining ring atoms being C, and that has at least one aromatic ring; in addition to the aromatic moiety, it may also contain a non-aromatic moiety. The cyclic system may be monocyclic or it may be a bicyclic or tricyclic system formed by a benzene ring or a monocyclic heteroaryl ring with an aromatic moiety or a non-aromatic moiety. Preferably, the heteroaryl has a single 5- to 8-membered (e.g., 5-, 6-, 7-, or 8-membered) ring, or has multiple fused rings containing 6 to 15, particularly 6 to 12, ring atoms (e.g., 6, 7, 8, 9, 10, 11, or 12 ring atoms). Non-limiting examples of the heteroaryl include, but are not limited to, pyrrolyl, furanyl, thienyl, imidazolyl, oxazolyl, pyrazolyl, pyridinyl, pyrimidinyl, pyrazinyl, quinolyl, isoquinolyl, tetrazolyl, triazolyl, triazinyl, benzofuranyl, benzothienyl, indolyl, isoindolyl, pyridopyrrolyl, or spiro[cyclopropane-1,3'-indolin]-2'-onyl.

The term "alkylene" refers to a divalent group formed by the removal of one hydrogen from any position of alkyl. For example, the term "C₁₋₃ alkylene" refers to alkylene containing 1 to 3 carbon atoms, including but not limited to methylene (-CH₂-), ethylene (-CH₂CH₂-), or propylene (-CH₂CH₂CH₂- or -CH₂CH(CH₃)-).

The compounds of the present disclosure may have particular geometric or stereoisomeric forms. The present disclosure contemplates all such compounds, including cis and trans isomers, (-)- and (+)-enantiomers, (R)- and (S)-enantiomers, diastereoisomers, (D)-isomers, (L)-isomers, and racemic mixtures and other mixtures thereof, such as enantiomerically or diastereomerically enriched mixtures, all of which fall within the scope of the present disclosure. Additional asymmetric carbon atoms may be present in substituents such as alkyl. All such isomers and mixtures thereof are encompassed within the scope of the present disclosure.

Unless otherwise stated, the absolute configuration of a stereogenic center is represented by a wedged solid bond **(** 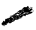 **)** and a wedged dashed bond ( 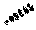 ), and the relative configuration of a stereogenic center is represented by a straight solid bond ( 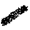 ) and a straight dashed bond ( 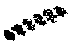 ). A wavy line ( 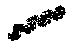 ) represents a wedged solid bond ( 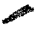 ) or a wedged dashed bond ( 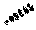 ), or a wavy line ( 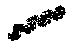 ) represents a straight solid bond ( 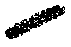 ) and a straight dashed bond ( 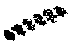 ).

Unless otherwise stated, when a double bond structure such as a carbon-carbon double bond, a carbon-nitrogen double bond, and a nitrogen-nitrogen double bond is present in the compound, and each atom on the double bond is connected to two different substituents (in the double bond including a nitrogen atom, a lone pair of electrons on the nitrogen atom is regarded as a substituent to which the nitrogen atom is connected), if the atom on the double bond of the compound and its substituents are connected using a wavy line ( 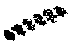 ), it means that the compound exists in the form of a (Z)-type isomer, an (E)-type isomer, or a mixture of the two isomers.

The term "treat", "treating", or "treatment" means administering the compound or formulation described in the present disclosure to ameliorate or eliminate a disease or one or more symptoms related to the disease, and includes:
(i) inhibiting the disease or disease state, i.e., arresting its progression; and
(ii) alleviating the disease or disease state, i.e., causing its regression.

The term "therapeutically or prophylactically effective amount" refers to an amount of the compound of the present disclosure for (i) treating or preventing a specific disease, condition, or disorder; (ii) relieving, ameliorating, or eliminating one or more symptoms of a specific disease, condition, or disorder, or (iii) preventing or delaying the onset of one or more symptoms of the specific disease, condition, or disorder described herein. The amount of the compound of the present disclosure constituting the "therapeutically effective amount" varies depending on the compound, the disease state and its severity, the mode of administration, and the age of the mammal to be treated, but can be determined routinely by those skilled in the art in accordance with their knowledge and the present disclosure.

The term "pharmaceutically acceptable" is used herein for those compounds, materials, compositions, and/or dosage forms that are, within the scope of sound medical judgment, suitable for use in contact with the tissues of human beings and animals without excessive toxicity, irritation, allergic response, or other problems or complications, and commensurate with a reasonable benefit/risk ratio.

A pharmaceutically acceptable salt, for example, may refer to a metal salt, an ammonium salt, a salt formed with an organic base, a salt formed with an inorganic acid, a salt formed with an organic acid, a salt formed with a basic or acidic amino acid, etc.

The term "pharmaceutical composition" refers to a mixture consisting of one or more of the compounds or the salts thereof of the present disclosure and a pharmaceutically acceptable excipient. The pharmaceutical composition is intended to facilitate the administration of the compound of the present disclosure to an organism.

The term "pharmaceutically acceptable excipient" refers to those excipients that do not have a significant irritating effect on an organism and do not impair the biological activity and properties of the active compound. Suitable excipients are well known to those skilled in the art, for example, carbohydrates, waxes, water-soluble and/or waterswellable polymers, hydrophilic or hydrophobic materials, gelatin, oils, solvents, water, etc.

The word "comprise" and variations thereof such as "comprises" or "comprising" will be understood in an open, non-exclusive sense, i.e., "including but not limited to".

The compounds and intermediates of the present disclosure may also exist in different tautomeric forms, and all such forms are included within the scope of the present disclosure. The term "tautomer" or "tautomeric form" refers to structural isomers of different energies that can interconvert via a low energy barrier. For example, a proton tautomer (also referred to as prototropic tautomer) includes interconversion via proton transfer, such as keto-enol isomerization and imine-enamine isomerization. A specific example of a proton tautomer is an imidazole moiety where a proton can transfer between two ring nitrogen atoms. A valence tautomer includes the interconversion via recombination of some bonding electrons.

The present disclosure also includes isotopically labeled compounds of the present disclosure, which are identical to those recited herein but have one or more atoms replaced by an atom having an atomic mass or mass number different from the atomic mass or mass number generally found in nature. Examples of isotopes that can be incorporated into the compounds of the present disclosure include isotopes of hydrogen, carbon, nitrogen, oxygen, phosphorus, sulfur, fluorine, iodine, and chlorine, such as ²H, ³H, ¹¹C, ¹³C, ¹⁴C, ¹³N, ¹⁵N, ¹⁵O, ¹⁷O, ¹⁸O, ³¹P, ³²P, ³⁵S, ¹⁸F, ¹²³I, ¹²⁵I, ³⁶Cl, and the like.

Certain isotopically labeled compounds of the present disclosure (e.g., those labeled with ³H and ¹⁴C) can be used in compound and/or substrate tissue distribution analysis. Tritiated (i.e., ³H) and carbon-14 (i.e., ¹⁴C) isotopes are particularly preferred for their ease of preparation and detectability. Positron emitting isotopes, such as ¹⁵O, ¹³N, ¹¹C, and ¹⁸F, can be used in positron emission tomography (PET) studies to determine substrate occupancy. The isotopically labeled compounds of the present disclosure can generally be prepared by following procedures analogous to those disclosed in the schemes and/or examples below while substituting a non-isotopically labeled reagent with an isotopically labeled reagent.

Furthermore, substitution with heavier isotopes such as deuterium (i.e., ²H) may provide certain therapeutic advantages (e.g., increased *in vivo* half-life or reduced dose) resulting from greater metabolic stability and hence may be preferred in some circumstances in which deuterium substitution may be partial or complete, wherein partial deuterium substitution refers to substitution of at least one hydrogen with at least one deuterium, and all such forms of the compounds are included within the scope of the present disclosure.

The compound of the present disclosure may be asymmetrical, for example, having one or more stereoisomers. Unless otherwise stated, all stereoisomers are included, for example, enantiomers and diastereoisomers. The compound containing asymmetric carbon atoms of the present disclosure may be isolated in an optically active pure form or in a racemic form. The optically active pure form may be obtained by resolution of a racemic mixture or may be synthesized using a chiral starting material or a chiral reagent.

The pharmaceutical composition of the present disclosure can be prepared by combining the compound of the present disclosure with a suitable pharmaceutically acceptable excipient, and can be formulated, for example, into a solid, semisolid, liquid, or gaseous formulation such as tablet, pill, capsule, powder, granule, ointment, emulsion, suspension, suppository, injection, inhalant, gel, microsphere, and aerosol.

Typical routes of administration of the compound or the pharmaceutically acceptable salt thereof or the pharmaceutical composition thereof of the present disclosure include, but are not limited to, oral, rectal, topical, inhalational, parenteral, sublingual, intravaginal, intranasal, intraocular, intraperitoneal, intramuscular, subcutaneous, and intravenous administration.

The pharmaceutical composition of the present disclosure can be manufactured using methods well known in the art, such as conventional mixing, dissolving, granulating, dragee-making, levigating, emulsifying, lyophilizing, etc.

In some embodiments, the pharmaceutical composition is in an oral form. For oral administration, the pharmaceutical composition can be formulated by mixing the active compound with pharmaceutically acceptable excipients well known in the art. These excipients enable the compound of the present disclosure to be formulated into tablets, pills, pastilles, dragees, capsules, liquids, gels, slurries, suspensions, etc., for oral administration to a patient.

A solid oral composition can be prepared by conventional mixing, filling, or tableting. For example, the solid oral composition can be obtained by the following method: mixing the active compound with solid excipients, optionally grinding the resulting mixture, adding additional suitable excipients if desired, and then processing the mixture into granules to obtain the core parts of tablets or dragees. Suitable excipients include, but are not limited to: binders, diluents, disintegrants, lubricants, glidants, sweeteners, flavoring agents, or the like.

The pharmaceutical composition may also be suitable for parenteral administration, such as sterile solutions, suspensions, or lyophilized products in suitable unit dosage forms.

The therapeutic dose of the compound of the present disclosure may be determined, for example, according to: the specific use of the treatment, the route of administration of the compound, the health and condition of the patient, and the judgment of the prescribing physician. The proportion or concentration of the compound of the present disclosure in the pharmaceutical composition may not be constant and depends on a variety of factors including the dose, chemical properties (e.g., hydrophobicity), and the route of administration. For example, the compound of the present disclosure may be provided for parenteral administration by a physiological buffered aqueous solution containing about 0.1%-10% w/v of the compound. Certain typical doses range from about 1 µg/kg body weight/day to about 1 g/kg body weight/day. In certain embodiments, the dose ranges from about 0.01 mg/kg body weight/day to about 100 mg/kg body weight/day. The dose is likely to depend on variables such as the type and degree of progression of the disease or disorder, the general health condition of a specific patient, the relative biological potency of the compound selected, the excipient formulation, and its route of administration. Effective doses can be extrapolated from dose-response curves derived from *in vitro* or animal model test systems.

The compounds of the present disclosure can be prepared using a variety of synthetic methods well known to those skilled in the art, including the specific embodiments listed below, embodiments formed by combinations thereof with other chemical synthetic methods, and equivalents thereof well known to those skilled in the art. The preferred embodiments include, but are not limited to, the examples of the present disclosure.

The chemical reactions in the specific embodiments of the present disclosure are conducted in a suitable solvent that must be suitable for the chemical changes in the present disclosure and the reagents and materials required. In order to obtain the compounds of the present disclosure, it is sometimes necessary for those skilled in the art to modify or select a synthetic procedure or a reaction process based on the existing embodiments.

An important consideration in synthetic route planning in the art is the selection of suitable protecting groups for reactive functional groups (e.g., amino in the present disclosure). For example, reference may be made to Greene's Protective Groups in Organic Synthesis (4th Ed.) Hoboken, New Jersey: John Wiley & Sons, Inc.

In some embodiments, the compounds of the present disclosure can be prepared by those skilled in the art of organic synthesis by reference to the following route:

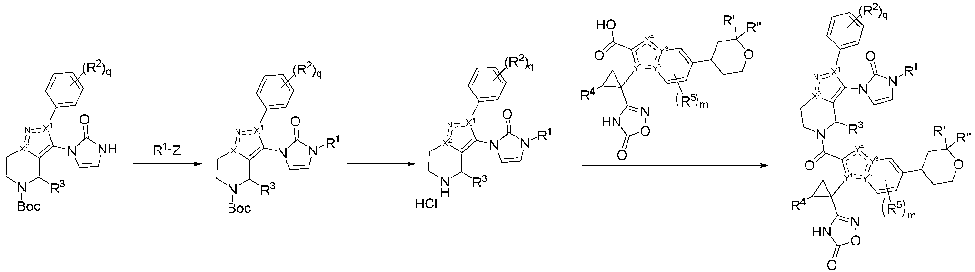

The following abbreviations are used in the present disclosure:
R¹, R², R³, R⁴, R⁵, q, X¹, X², Y¹, Y², Y³, Y⁴, R', or R" is as defined above, and Z is a leaving group, including but not limited to F, Cl, Br, I, etc.

The following abbreviations are used in the present disclosure:
NaHMDS for sodium bis(trimethylsilyl)amide; DCM for dichloromethane; and MeOH for methanol.

For clarity, the present disclosure is further described with the following examples, which are, however, not intended to limit the scope of the present disclosure. All the reagents used in the present disclosure are commercially available and can be used without further purification.

### Example 1

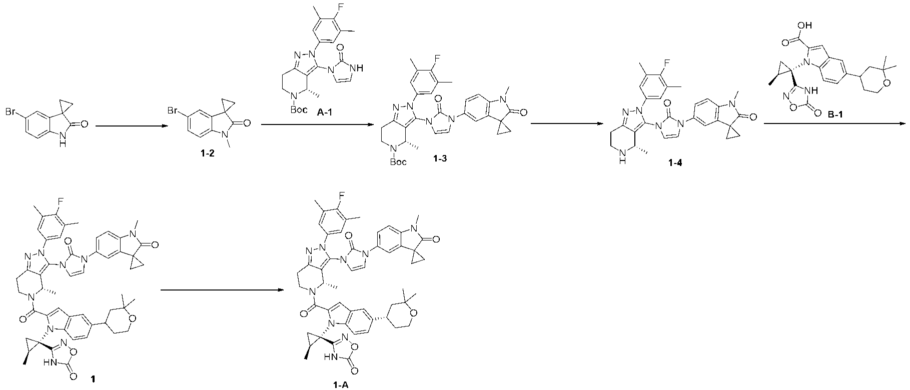

### Step 1: Synthesis of intermediate 1-2

5'-Bromo-1',2'-dihydrospiro[cyclopropane-1,3'-indolin]-2'-one (500 mg) and N,N-dimethylformamide (7 mL) were mixed, and sodium hydride (126 mg, 60% dispersion in mineral oil) was added in an ice bath. The mixture was stirred for 30 min, and then iodomethane (0.055 mL) was added dropwise to the reaction solution. The reaction mixture was transferred to room temperature and stirred for 1.5 h. The reaction solution was poured into a mixed system of ethyl acetate (30 mL) and water (50 mL). The aqueous phase was extracted with ethyl acetate (2 × 30 mL). The organic phases were combined, then washed with saturated brine, dried over anhydrous sodium sulfate, filtered under vacuum, and concentrated under reduced pressure to give 450 mg of intermediate **1-2.**

MS (ESI, [M+H]⁺) m/z: 252.05.

¹H-NMR (500 MHz, DMSO-*d*₆): *δ* 7.43 (dd, *J =* 8.3, 2.0 Hz, 1H), 7.28 (d, *J=* 2.0 Hz, 1H), 7.03 (d, *J =* 8.2 Hz, 1H), 3.20 (s, 3H), 1.69 (q, *J =* 3.8 Hz, 2H), 1.52 (q, *J =* 3.8 Hz, 2H).

### Step 2: Synthesis of intermediate 1-3

Intermediate **A-1** (250 mg), intermediate **1-2** (186 mg), (1*S*,2*S*)-*N*1,*N*2-dimethylcyclohexane-1,2-diamine (40.3 mg), potassium carbonate (235 mg), copper(I) iodide (21.57 mg), and N-methylpyrrolidone (4 mL) were mixed, and the mixture was stirred in an oil bath at 120 °C overnight under a nitrogen atmosphere. The reaction solution was then cooled to room temperature and poured into water (50 mL), and the resulting solution was extracted with ethyl acetate (30 mL). The organic phase was washed with water and saturated brine in sequence, dried over anhydrous sodium sulfate, concentrated, and separated and purified by column chromatography (petroleum ether:ethyl acetate = 3:2) to give 0.3 g of intermediate **1-3.**

MS (ESI, [M+H]⁺) m/z: 613.37.

¹H-NMR (500 MHz, DMSO-*d*₆): *δ* 7.49 (dd, *J =* 8.4, 2.2 Hz, 1H), 7.24 (d, *J =* 1.9 Hz, 1H), 7.15 (t, *J =* 5.9 Hz, 2H), 7.10 (d, *J =* 6.3 Hz, 2H), 6.91 (s, 1H), 5.18-5.03 (m, 1H), 4.37-4.13 (m, 1H), 3.23 (s, 3H), 3.19-3.06 (m, 1H), 2.77-2.61 (m, 2H), 2.19 (d, *J =* 1.7 Hz, 6H), 1.68-1.63 (m, 2H), 1.58-1.53 (m, 2H), 1.43 (s, 9H), 1.22-1.18 (m, 3H).

### Step 3: Synthesis of intermediate 1-4

Intermediate **1-3** (0.28 g), dichloromethane (2 mL), and a 4 N solution of hydrochloric acid in dioxane (2 mL) were mixed, and the mixture was stirred at room temperature for 1 h. The stirring was stopped, and the mixture was concentrated under reduced pressure to give 0.24 g of intermediate **1-4.**

MS (ESI, [M+H]⁺) m/z: 513.1.

### Step 6: Synthesis of compound 1 and compound 1-A

Intermediate **B-1** (0.1 g), *N,N*-dimethylformamide (5 mL), triethylamine (0.576 mL), and *O*-(7-azabenzotriazol-1-yl)-*N,N,N,N*-tetramethyluronium hexafluorophosphate (0.236 g) were stirred for 5 min, and then intermediate **1-4** (0.125 g) was added. The mixture was stirred at 30 °C. The reaction solution was poured into water (50 mL), and the resulting solution was extracted with ethyl acetate. The organic phase was washed with saturated brine, dried over anhydrous sodium sulfate, concentrated, and separated and purified by column chromatography (DCM:MeOH = 95:5) to give compound **1.** The above compound **1** was resolved by preparative chiral HPLC (chromatographic column: REFLECT I-Cellulose B, 30 × 250 mm, 10 µm; mobile phase: ethanol-dichloromethane (1:3):n-hexane = 25:75; flow rate: 40 mL/min) to give compound **1-A** (43 mg).

Compound **1-A:** Rₜ = 1.34 min (UPCC conditions: chromatographic column: CHIRALPAK IB-3, 4.6 × 100 mm, 3 µm; mobile phase: carbon dioxide:ethanol:isopropanol = 40:30:30; flow rate: 2.0 mL/min; column temperature: 40 °C)

HRMS: (ESI, [M+H]⁺) m/z: 906.4130.

¹H-NMR (500 MHz, DMSO-*d*₆): *δ* 11.73 (s, 1H), 7.58-7.31 (m, 3H), 7.30-7.03 (m, 6H), 7.01-6.68 (m, 2H), 5.61-5.49 (m, 1H), 4.46-4.29 (m, 1H), 3.77-3.66 (m, 2H), 3.66-3.58 (m, 1H), 3.28-3.15 (m, 4H), 3.08-2.98 (m, 1H), 2.94-2.83 (m, 1H), 2.29-2.13 (m, 6H), 1.84-1.62 (m, 6H), 1.58-1.50 (m, 4H), 1.42-1.37 (m, 2H), 1.27 (s, 3H), 1.25-1.13 (m, 8H).

### Example 2

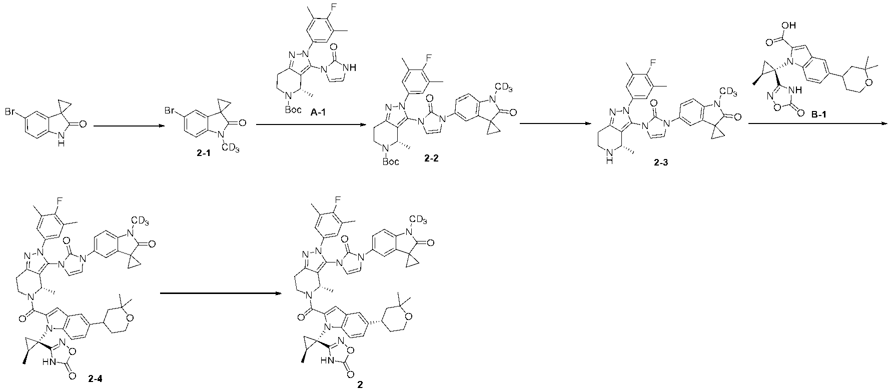

### Step 1: Synthesis of intermediate 2-1

5'-Bromo-1',2'-dihydrospiro[cyclopropane-1,3'-indolin]-2'-one (500 mg) and N,N-dimethylformamide (8 mL) were mixed, and sodium hydride (126 mg, 60% dispersion in mineral oil) was added in an ice bath. The mixture was stirred for 10 min, and then deuterated iodomethane (457 mg) was added. The mixture was warmed to room temperature and left to react for 2 h. After the reaction was completed, the reaction solution was poured into a saturated ammonium chloride solution (20 mL), and ethyl acetate (20 mL) was added for extraction. The organic phase was washed with saturated brine, dried over anhydrous sodium sulfate, and filtered under vacuum. The filtrate was concentrated under reduced pressure, and the residue was separated and purified by silica gel column chromatography (petroleum ether:ethyl acetate = 3:1) to give 400 mg of intermediate **2-1.**

MS (ESI, [M+H]⁺) m/z: 255.08.

¹H-NMR (500 MHz, DMSO-*d*₆): *δ* 7.42 (dd, *J =* 8.3, 2.0 Hz, 1H), 7.28 (d, *J =* 2.0 Hz, 1H), 7.02 (d, *J =* 8.2 Hz, 1H), 1.72-1.67 (m, 2H), 1.54-1.49 (m, 2H).

### Step 2: Synthesis of intermediate 2-2

Intermediate **A-1** (250 mg), intermediate **2-1** (188 mg), (1*S*,2*S*)-*N*1,*N*2-dimethylcyclohexane-1,2-diamine (40.3 mg), potassium carbonate (235 mg), copper(I) iodide (21.57 mg), and N-methylpyrrolidone (6 mL) were mixed, and the mixture was left to react at 120 °C for 12 h under a nitrogen atmosphere. After the reaction was completed, the reaction solution was poured into water, and ethyl acetate (50 mL) was added for extraction. The organic phase was washed with saturated brine, dried over anhydrous sodium sulfate, and filtered under vacuum. The filtrate was concentrated under reduced pressure, and the residue was separated and purified by silica gel column chromatography (petroleum ether:ethyl acetate = 70:30) to give 260 mg of intermediate **2-2.**

MS (ESI, [M+H]⁺) m/z: 616.39.

¹H NMR (500 MHz, DMSO-d₆): *δ* 7.49 (dd, *J* = 8.4, 2.2 Hz, 1H), 7.24 (d, *J =* 2.1 Hz, 1H), 7.19-7.14 (m, 2H), 7.10 (d*, J =* 6.2 Hz, 2H), 6.96-6.87 (m, 1H), 2.79-2.61 (m, 2H), 2.19 (d, *J =* 2.0 Hz, 6H), 1.69-1.62 (m, 2H), 1.58-1.53 (m, 2H), 1.44 (s, 9H), 1.38-1.22 (m, 3H), 1.21-1.14 (m, 3H).

### Step 3: Synthesis of intermediate 2-3

Intermediate 2-2 (260 mg) and a 4 N solution of hydrochloric acid in dioxane (5 mL) were mixed, and the mixture was left to react at room temperature for 2 h. After the reaction was completed, the reaction solution was concentrated under reduced pressure to give 230 mg of intermediate **2-3.**

### Step 4: Synthesis of intermediate 2-4 and compound 2

Intermediate **B-1** (0.1 g), *N,N*-dimethylformamide (5 mL), triethylamine (0.678 mL), and *O*-(7-azabenzotriazol-1-yl)-*N,N,N,N*-tetramethyluronium hexafluorophosphate (0.277 g) were mixed. The mixture was stirred at room temperature for 5 min, and then intermediate **2-3** (0.148 g) was added thereto. The resulting mixture was stirred at room temperature for 5 h. After the reaction was completed, the reaction solution was poured into water, and ethyl acetate (50 mL) was added for extraction. The organic phase was washed with saturated brine, dried over anhydrous sodium sulfate, and filtered under vacuum. The filtrate was concentrated under reduced pressure, and the residue was separated and purified by silica gel column chromatography (dichloromethane:methanol = 20:1) to give 70 mg of intermediate **2-4.** The above intermediate **2-4** was resolved by preparative chiral HPLC (chromatographic column: CHIRAL ART Cellulose-SB, 30 × 250 mm, 5 µm; mobile phase: ethanol-dichloromethane (1:1):n-hexane = 30:70; flow rate: 38 mL/min) to give compound **2** (35 mg).

Compound **2:** Rt = 2.80 min (UPCC conditions: chromatographic column: CHIRALPAK IB-3 (4.6 × 100 mm, 3 µm); mobile phase: carbon dioxide:methanol (containing 0.1% ammonium hydroxide) = 50:50; flow rate: 2.0 mL/min; column temperature: 40 °C)

HRMS: (ESI, [M+H]⁺) m/z: 909.4328.

¹H NMR (500 MHz, DMSO) *δ* 11.73 (s, 1H), 7.56-7.48 (m, 2H), 7.45-7.32 (m, 2H), 7.29-7.23 (m, 2H), 7.20 (s, 1H), 7.16-7.13 (m, 2H), 6.94 (s, 2H), 5.63-5.50 (m, 1H), 4.44-4.33 (m, 1H), 3.73-3.68 (m, 2H), 3.67-3.56 (m, 1H), 3.18 (s, 1H), 3.06-3.00 (m, 1H), 2.93-2.85 (m, 1H), 2.22 (s, 6H), 1.76-1.65 (m, 6H), 1.64-1.59 (m, 2H), 1.57-1.47 (m, 6H), 1.39 (d, *J =* 6.3 Hz, 2H), 1.24 (s, 4H), 1.18 (s, 3H).

### Example 3

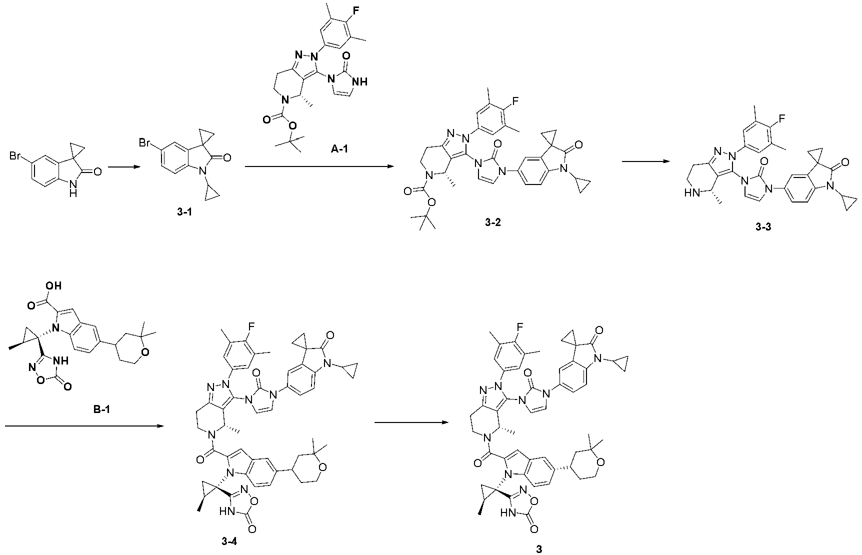

### Step 1: Synthesis of intermediate 3-1

5'-Bromo-1',2'-dihydrospiro[cyclopropane-1,3'-indolin]-2'-one (500 mg) and toluene (50 mL) were mixed, and cyclopropylboronic acid (360 mg), 4-dimethylaminopiperidine (800 mg), copper acetate (440 mg), and a 2 M solution of sodium bis(trimethylsilyl)amide in tetrahydrofuran (1.1 mL) were added in sequence. The mixture was heated to 95 °C and stirred for 16 h. The reaction solution was then diluted with ethyl acetate, filtered under vacuum, concentrated, and separated and purified by column chromatography (petroleum ether:ethyl acetate = 3:2) to give 0.5 g of intermediate **3-1.**

MS (ESI, [M+H]⁺) m/z: 278.09.

¹H-NMR (500 MHz, DMSO-d₆): *δ* 7.43 (dd, *J =* 8.3, 2.0 Hz, 1H), 7.25 (d, *J =* 2.0 Hz, 1H), 7.10 (d, *J =* 8.3 Hz, 1H), 2.73 (tt, *J =* 7.0, 3.8 Hz, 1H), 1.65 (q, *J =* 3.7 Hz, 2H), 1.49 (q, *J =* 3.7 Hz, 2H), 1.06-0.95 (m, 2H), 0.85-0.73 (m, 2H).

### Step 2: Synthesis of intermediate 3-2

Intermediate **A-1** (250 mg), intermediate **3-1** (250 mg), (1*S*,2*S*)-*N*1,*N*2-dimethylcyclohexane-1,2-diamine (50 mg), potassium carbonate (250 mg), copper(I) iodide (25 mg), and *N*-methylpyrrolidone (5 mL) were mixed, and the mixture was stirred in an oil bath at 120 °C overnight under a nitrogen atmosphere. The reaction solution was then cooled to room temperature and poured into water (50 mL), and the resulting solution was extracted with ethyl acetate (30 mL). The organic phase was washed with water and saturated brine in sequence, dried over anhydrous sodium sulfate, concentrated, and separated and purified by column chromatography (petroleum ether:ethyl acetate = 3:2) to give 0.3 g of intermediate **3-2.**

MS (ESI, [M+H]⁺) m/z: 639.33.

¹H-NMR (500 MHz, DMSO-d₆): *δ* 7.49 (dd, *J =* 8.4, 2.3 Hz, 1H), 7.24 (d, *J =* 8.4 Hz, 1H), 7.20 (d, *J =* 2.1 Hz, 1H), 7.15 (d, *J =* 3.2 Hz, 1H), 7.10 (d, *J =* 6.3 Hz, 2H), 6.91 (s, 1H), 5.12 (s, 1H), 4.21 (s, 1H), 3.15 (s, 1H), 2.80-2.61 (m, 3H), 2.19 (s, 6H), 1.67-1.57 (m, 2H), 1.51 (q, *J =* 3.4 Hz, 2H), 1.43 (s, 9H), 1.19 (d, *J =* 5.8 Hz, 3H), 1.07-0.99 (m, 2H), 0.86-0.78 (m, 2H).

### Step 3: Synthesis of intermediate 3-3

Intermediate **3-2** (0.3 g) and a 4 N solution of hydrochloric acid in dioxane (3 mL) were mixed, and the mixture was stirred at room temperature for 1 h. The stirring was stopped, and the mixture was concentrated under reduced pressure to give 0.26 g of intermediate **3-3.**

MS (ESI, [M+H]⁺) m/z: 539.32.

### Step 4: Synthesis of intermediate 3-4 and compound 3

Intermediate **B-1** (0.12 g), *N,N*-dimethylformamide (4 mL), triethylamine (0.068 g), and *O*-(7-azabenzotriazol-1-yl)-*N,N,N,N*-tetramethyluronium hexafluorophosphate (0.14 g) were stirred for 5 min, and then intermediate **3-3** (0.16 g) was added. The mixture was stirred at 30 °C. The reaction solution was poured into water (50 mL), and the resulting solution was extracted with ethyl acetate. The organic phase was washed with saturated brine, dried over anhydrous sodium sulfate, concentrated, and separated and purified by column chromatography (DCM:MeOH = 95:5) to give intermediate **3-4.** The above intermediate **3-4** was resolved by preparative chiral HPLC (chromatographic column: Pre-packed REGIS IB, 30 × 250 mm, 10 µm; mobile phase: ethanol-dichloromethane (1:3):n-hexane = 30:70; flow rate: 40 mL/min) to give 85 mg of compound **3.**

Compound 3: Rt = 2.02 min (UPCC conditions: chromatographic column: CHIRALPAK IB-3 (4.6 × 100 mm, 3 µm); mobile phase: carbon dioxide:methanol (containing 0.1% ammonium hydroxide) = 30:70; flow rate: 2.0 mL/min; column temperature: 40 °C)

HRMS: (ESI, [M+H]⁺) m/z: 932.4280.

¹H-NMR (500 MHz, DMSO-d₆): *δ* 11.73 (s, 1H), 7.55-7.49 (m, 2H), 7.42-7.36 (m, 1H), 7.28-7.13 (m, 6H), 6.95 (s, 2H), 5.55 (d*, J =* 6.0 Hz, 1H), 4.38 (d, *J =* 9.5 Hz, 1H), 3.72 (d, *J =* 10.4 Hz, 2H), 3.63 (t*, J =* 11.2 Hz, 1H), 3.53-3.39 (m, 1H), 3.24-3.11 (m, 1H), 3.03 (t, *J =* 11.9 Hz, 1H), 2.88 (d, *J =* 15.6 Hz, 1H), 2.81-2.69 (m, 2H), 2.22 (s, 6H), 1.73-1.62 (m, 6H), 1.56-1.51 (m, 4H), 1.39 (d, *J =* 6.9 Hz, 2H), 1.19-1.12 (m, 6H), 1.03 (t, *J =* 7.3 Hz, 3H), 0.89-0.78 (m, 4H).

### Example 4

### Synthesis of compound 4:

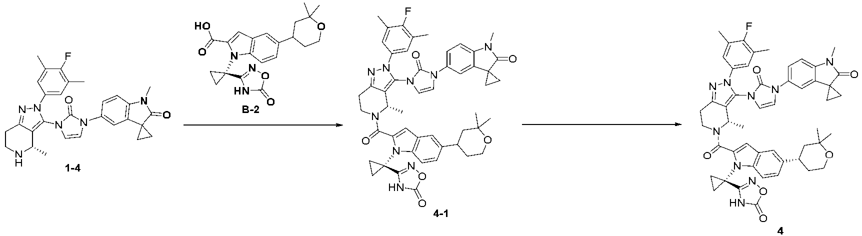

Intermediate **1-4** (133 mg), N,N-dimethylformamide (5 mL), triethylamine (492 mg), and *O*-(7-azabenzotriazol-1-yl)-*N,N,N,N*-tetramethyluronium hexafluorophosphate (277 mg) were mixed, and the mixture was stirred for 5 min. Intermediate **B-2** (100 mg) was then added, and the mixture was stirred at room temperature for 3 h. The reaction solution was poured into water (50 mL), and the resulting solution was extracted with ethyl acetate (30 mL). The organic phase was washed with saturated brine, dried over anhydrous sodium sulfate, and concentrated, and the residue was separated and purified by silica gel column chromatography (DCM:MeOH = 95:5) to give intermediate **4-1.** The above intermediate **4-1** was resolved by preparative chiral HPLC (chromatographic column: REGIS IB, 30 × 250 mm, 10 µm; mobile phase: n-hexane:dichloromethane:ethanol = 70:15:15; flow rate: 40 mL/min) to give 40 mg of compound **4.**

Compound **4:** Rt = 3.89 min (UPCC conditions: chromatographic column: CHIRALPAK IB-3, 4.6 × 100 mm, 3 µm; mobile phase: carbon dioxide:methanol (containing 0.1% ammonium hydroxide) = 60:40; flow rate: 2.0 mL/min; column temperature: 40 °C)

HRMS: (ESI, [M+H]⁺) m/z: 892.3949.

1H NMR (500 MHz, DMSO-*d*₆) *δ* 12.08 (s, 1H), 7.57 - 7.38 (m, 3H), 7.34 - 7.05 (m, 6H), 7.00-6.90 (m, 1H), 6.85-6.71 (m, 1H), 5.50 (s, 1H), 4.47-4.30 (m, 1H), 3.72 (d, J = 8.9 Hz, 2H), 3.55 (d, J = 36.8 Hz, 1H), 3.23 (s, 3H), 3.09-2.98 (m, 1H), 2.89-2.75 (m, 1H), 2.20 (d, J = 10.8 Hz, 6H), 1.76-1.51 (m, 9H), 1.36-1.22 (m, 10H), 1.21-1.16 (m, 3H).

### Example 5

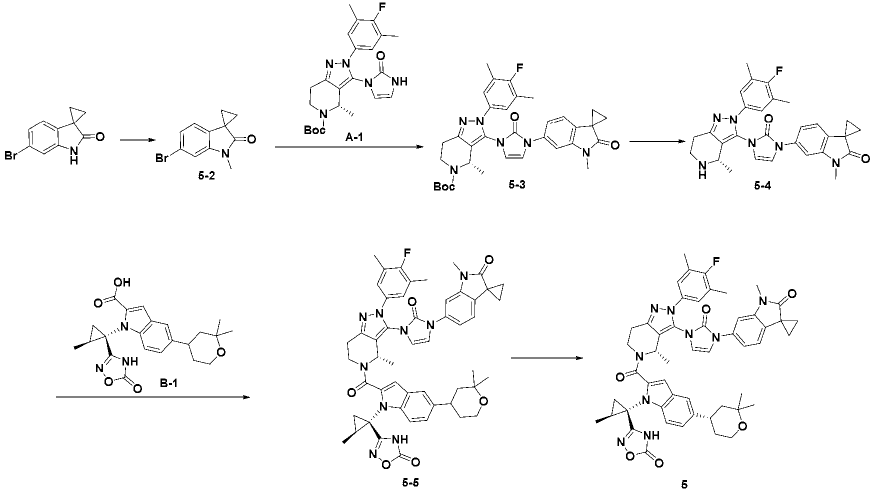

### Step 1: Synthesis of intermediate 5-2

6'-Bromospiro[cyclopropane-1,3'-indolin]-2'-one (0.5 g) was dissolved in N,N-dimethylformamide (10 mL), and cesium carbonate (1.38 g) and iodomethane (0.17 mL) were added in sequence. The mixture was heated to 80 °C and stirred for 1 h. Water was then added, and the mixture was extracted with ethyl acetate. The organic layer was washed with water and a saturated aqueous sodium chloride solution in sequence, dried over anhydrous sodium sulfate, filtered under vacuum, concentrated, and separated and purified by column chromatography (petroleum ether:ethyl acetate = 3:1) to give 0.51 g of intermediate 5-2.

MS (ESI, [M+H+2]⁺) m/z: 254.02.

¹H-NMR (500 MHz, CDCl₃): *δ* 7.15 (dd, *J* = 7.9, 1.7 Hz, 1H), 7.04 (d, *J =* 1.8 Hz, 1H), 6.69 (d, *J =* 7.9 Hz, 1H), 3.27 (s, 3H), 1.75 (q, *J =* 4.1 Hz, 2H), 1.51 (q, *J =* 4.2 Hz, 2H).

### Step 2: Synthesis of intermediate 5-3

Intermediate **5-2** (0.14 g), intermediate **A-1** (0.2 g), copper(I) iodide (17.25 mg), (1S,2S)-N1,N2-dimethylcyclohexane-1,2-diamine (25.8 mg), potassium carbonate (125 mg), and N-methylpyrrolidone (5 mL) were mixed. The mixture was heated to 130 °C and stirred for 6 h under a nitrogen atmosphere. Water and ethyl acetate were added for dilution, and the mixture was extracted with ethyl acetate. The organic layer was washed with water and a saturated aqueous NaCl solution in sequence, dried over anhydrous sodium sulfate, filtered under vacuum, concentrated, and separated and purified by column chromatography (petroleum ether:ethyl acetate = 3:1) to give 0.18 g of intermediate **5-3.**

MS (ESI, [M+H]⁺) m/z: 613.38.

¹H-NMR (500 MHz, CDCl₃): *δ* 7.26 (s, 1H), 7.09 (d, *J =* 6.2 Hz, 2H), 7.05 (d, *J =* 7.4 Hz, 1H), 6.87 (d, *J =* 7.9 Hz, 1H), 6.68 (d, *J =* 3.2 Hz, 1H), 6.29 (s, 1H), 5.43-5.16 (m, 1H), 4.59-4.21 (m, 1H), 3.31 (s, 3H), 3.23-3.06 (m, 1H), 2.82-2.75 (m, 2H), 2.22 (d, *J =* 2.2 Hz, 6H), 1.77 (q, *J =* 4.2 Hz, 2H), 1.54 (q, *J =* 4.1 Hz, 2H), 1.50 (s, 9H), 1.32 (d, *J* = 6.7 Hz, 3H).

### Step 3: Synthesis of intermediate 5-4

Intermediate **5-3** (0.17 g) was mixed with a 6 N solution of hydrochloric acid in dioxane (1 mL), and the mixture was stirred at room temperature for 1 h and then concentrated by evaporation to remove the solvent. A saturated aqueous sodium bicarbonate solution was then added, and the mixture was extracted with ethyl acetate. The organic layer was washed with water and a saturated aqueous sodium chloride solution in sequence, dried over anhydrous sodium sulfate, filtered under vacuum, and concentrated to give 0.11 g of intermediate **5-4.**

MS (ESI, [M+H]⁺) m/z: 513.36.

### Step 4: Synthesis of intermediate 5-5

Intermediate **B-1** (0.08 g) and *N,N*-dimethylformamide (2 mL) were mixed, and triethylamine (44 mg) and *O*-(7-azabenzotriazol-1-yl)-*N*,*N*,*N*',*N*'-tetramethyluronium hexafluorophosphate (110 mg) were added in sequence. The mixture was stirred for 10 min, and then intermediate **5-4** (0.082 g) was added. The resulting mixture was stirred at 40 °C for 2 h. Water and ethyl acetate were added for dilution, and the mixture was extracted with ethyl acetate. The organic layer was washed with water and a saturated aqueous sodium chloride solution in sequence, dried over anhydrous sodium sulfate, and filtered. The filtrate was concentrated, and the residue was separated and purified by column chromatography (dichloromethane:methanol = 98:2) to give 60 mg of intermediate **5-5.** The above intermediate **5-5** was resolved by preparative chiral HPLC (chromatographic column: CHIRALART Cellulose-SB, 30 × 250 mm, 10 µm; mobile phase: *n*-hexane:dichloromethane:ethanol = 72:21:7; flow rate: 40 mL/min) to give 17 mg of compound **5.**

Compound 5: Rt = 2.08 min (UPCC conditions: chromatographic column: CHIRALPAK IB-3 (4.6 × 100 mm, 3 µm); mobile phase: carbon dioxide:methanol (containing 0.1% ammonium hydroxide) = 30:70; flow rate: 2.0 mL/min; column temperature: 40 °C)

HRMS: (ESI, [M+H]⁺) m/z: 906.4103.

¹H-NMR (500 MHz, DMSO-d₆): *δ* 11.73 (s, 1H), 7.53 (s, 1H), 7.41-7.32 (m, 3H), 7.31-7.24 (m, 2H), 7.17-7.11 (m, 3H), 7.00-6.95 (m, 2H), 5.57 (d, *J =* 6.8 Hz, 1H), 4.38 (d, *J =* 13.9 Hz, 1H), 3.71 (d, *J =* 8.9 Hz, 2H), 3.24 (s, 3H), 3.09-3.03 (m, 1H), 2.89 (d, *J =* 16.7 Hz, 1H), 2.22-2.18(m, 7H), 1.64-1.62 (m, 2H), 1.55-1.53 (m, 2H), 1.40 (d, *J =* 6.4 Hz, 2H), 1.36-1.33 (m, 4H), 1.30 (s, 2H), 1.27 (s, 3H), 1.24 (s, 4H), 1.18 (s, 3H), 1.15 (s, 2H).

### Example 6

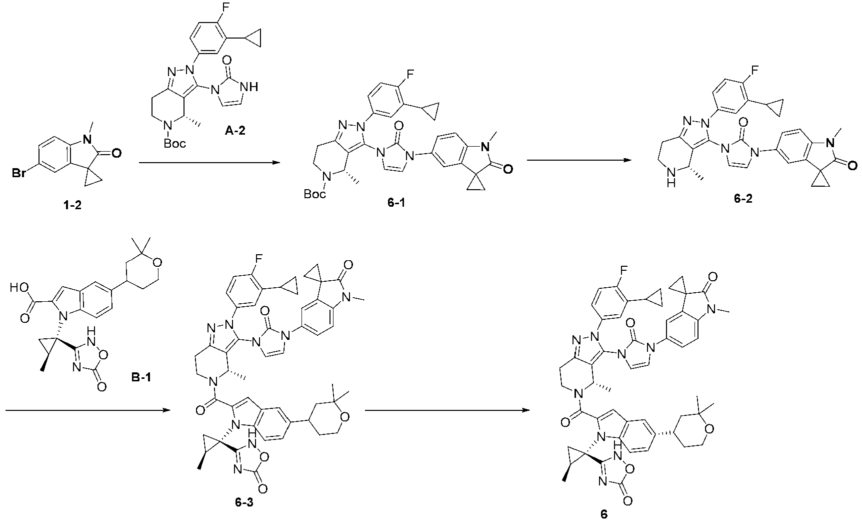

### Step 1: Synthesis of intermediate 6-1

Intermediate **A-2** (700 mg), intermediate **1-2** (506 mg), (1*S*,2*S*)-*N*1,*N*2-dimethylcyclohexane-1,2-diamine (132 mg), potassium carbonate (640 mg), copper(I) iodide (88 mg), and *N*-methylpyrrolidone (10 mL) were mixed, and the mixture was left to react at 120 °C for 12 h under a nitrogen atmosphere. After the reaction was completed, the reaction solution was poured into water, and ethyl acetate (50 mL) was added for extraction. The organic phase was washed with saturated brine, dried over anhydrous sodium sulfate, and filtered under vacuum. The filtrate was concentrated under reduced pressure, and the residue was separated and purified by silica gel column chromatography (dichloromethane:methanol = 20: 1) to give 0.75 g of intermediate **6-1.**

MS (ESI, [M+H]⁺) m/z: 625.42.

### Step 2: Synthesis of intermediate 6-2

Intermediate **6-1** (0.75 g) and a 4 N solution of hydrochloric acid in dioxane (10 mL) were mixed, and the mixture was stirred at room temperature for 2 h. After the reaction was completed, the reaction solution was directly concentrated under reduced pressure to give 0.65 g of intermediate **6-2.**

### Step 3: Synthesis of intermediate 6-3 and compound 6

Intermediate **B-1** (0.1 g), *N,N*-dimethylformamide (5 mL), triethylamine (0.678 mL), and *O*-(7-azabenzotriazol-1-yl)-*N,N,N,N-*tetramethyluronium hexafluorophosphate (0.277 g) were mixed. The mixture was stirred at room temperature for 5 min, and then intermediate **6-2** (0.15 g) was added thereto. The resulting mixture was stirred at room temperature for 5 h. After the reaction was completed, the reaction solution was poured into water, and ethyl acetate (50 mL) was added for extraction. The organic phase was washed with saturated brine, dried over anhydrous sodium sulfate, and filtered under vacuum. The filtrate was concentrated under reduced pressure, and the residue was separated and purified by silica gel column chromatography (dichloromethane:methanol = 20:1) to give 90 mg of intermediate **6-3.** The above intermediate **6-3** was resolved by preparative chiral HPLC (chromatographic column: CHIRALART Cellulose-SB, 30 × 250 mm, 5 µm; mobile phase: ethanol-dichloromethane (1:1):*n*-hexane = 30:70; flow rate: 40 mL/min) to give compound **6** (40 mg).

Compound 6: Rt = 5.58 min (UPCC conditions: chromatographic column: CHIRALPAK IB-3 (4.6 × 100 mm, 3 µm); mobile phase: carbon dioxide:methanol (containing 0.1% ammonium hydroxide) = 60:40; flow rate: 2.0 mL/min; column temperature: 40 °C)

HRMS: (ESI, [M+H]⁺) m/z: 918.4083.

¹H-NMR (500 MHz, DMSO-*d*₆): *δ* 11.73 (s, 1H), 7.57-7.50 (m, 2H), 7.42-7.36 (m, 2H), 7.32-7.26 (m, 4H), 7.26-7.22 (m, 2H), 6.93-6.87 (m, 2H), 5.58-5.51 (m, 1H), 4.41-4.34 (m, 1H), 3.73-3.70 (m, 2H), 3.66-3.58 (m, 1H), 3.26-3.23 (m, 3H), 3.21 (s, 1H), 3.05- 3.00 (m, 1H), 2.92-2.87 (m, 1H), 2.11-2.00 (m, 2H), 1.72-1.64 (m, 6H), 1.63-1.59 (m, 2H), 1.58-1.51 (m, 6H), 1.41-1.38 (m, 2H), 1.34-1.33 (m, 3H), 1.30 (s, 3H), 0.99-0.96 (m, 2H), 0.60-0.55 (m, 2H).

### Example 7

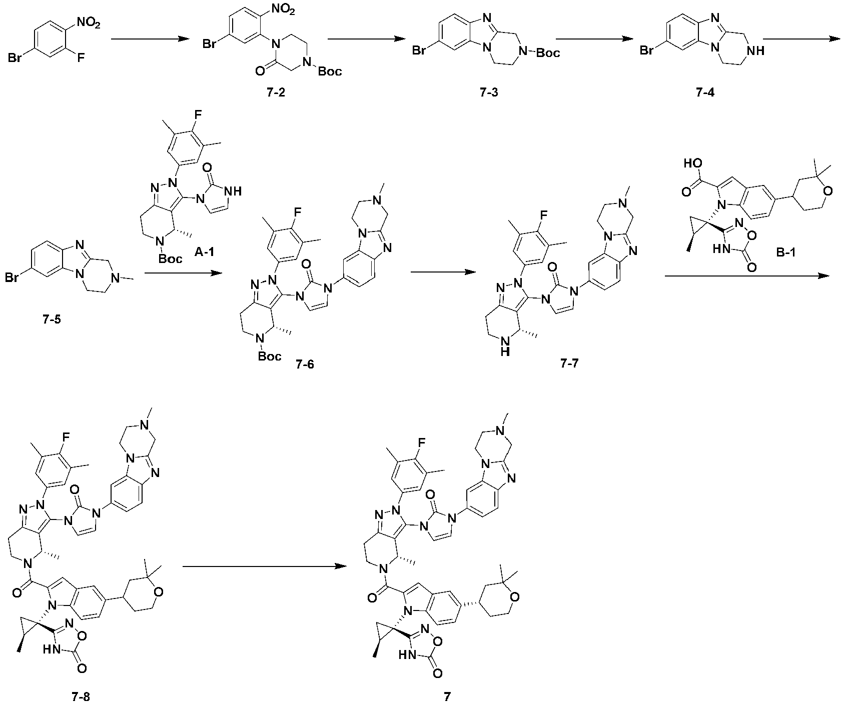

### Step 1: Synthesis of intermediate 7-2

*tert*-Butyl 3-oxopiperazine-1-carboxylate (3.52 g) and N,N-dimethylformamide (80 mL) were mixed, and sodium hydride (0.960 g, 60% dispersion in mineral oil) was added in an ice bath. The mixture was stirred at room temperature for 10 min, and then 2-fluoro-4-bromonitrobenzene (3.52 g) was added thereto. After the addition, the resulting mixture was stirred at room temperature for 12 h. After the reaction was completed, the reaction solution was poured into a saturated ammonium chloride solution (100 mL), and ethyl acetate (100 mL) was added for extraction. The organic phase was washed with saturated brine, dried over anhydrous sodium sulfate, and filtered under vacuum. The filtrate was concentrated under reduced pressure, and the residue was separated and purified by silica gel column chromatography (petroleum ether:ethyl acetate = 3:1) to give 2.29 g of intermediate **7-2.**

¹H-NMR (500 MHz, CDCl*₃*) *δ* 7.93 (d*, J =* 8.5 Hz, 1H), 7.64 (dd, *J* = 8.5, 2.0 Hz, 1H), 7.52 (d, *J* = 2.0 Hz, 1H), 4.32-4.14 (m, 2H), 4.00-3.86 (m, 2H), 3.85-3.67 (m, 2H), 1.51 (s, 9H).

### Step 2: Synthesis of intermediate 7-3

Intermediate **7-2** (1.7 g), acetic acid (34 mL), and iron powder (1.2 g) were mixed, and the mixture was heated to 120 °C and left to react for 1 h under a nitrogen atmosphere. After the reaction was completed, the reaction solution was cooled to room temperature and filtered through diatomite under vacuum, and the filtrate was directly concentrated. The residue was dissolved in ethyl acetate (100 mL), and the resulting solution was washed with a saturated sodium bicarbonate solution and saturated brine, dried over anhydrous sodium sulfate, and filtered under vacuum. The filtrate was concentrated under reduced pressure to give 1.26 g of intermediate **7-3.**

MS (ESI, [M+H]⁺) m/z: 352.14.

### Step 3: Synthesis of intermediate 7-4

Intermediate **7-3** (1.26 g) and a 4 N aqueous hydrochloric acid solution (12 mL) were mixed, and the mixture was heated to 100 °C and left to react for 1 h under a nitrogen atmosphere. After the reaction was completed, the reaction solution was poured into a saturated sodium bicarbonate solution to adjust the pH to 8-9, and dichloromethane (100 mL) was added for extraction. The organic phase was washed with saturated brine, dried over anhydrous sodium sulfate, and filtered under vacuum. The filtrate was concentrated under reduced pressure, and the residue was separated and purified by silica gel column chromatography (dichloromethane:methanol = 15:1) to give 0.64 g of intermediate **7-4.**

MS (ESI, [M+H]⁺) m/z: 252.13.

¹H-NMR (500 MHz, DMSO-d₆): *δ* 7.74 (d, *J =* 1.5 Hz, 1H), 7.49 (d, *J =* 8.5 Hz, 1H), 7.30 (dd, *J =* 8.5, 2 Hz, 1H), 4.03 (t, *J=* 5.5 Hz, 4H), 3.18 (t, *J =* 5.5 Hz, 2H), 2.87 (s, 1H).

### Step 4: Synthesis of intermediate 7-5

Intermediate **7-4** (0.22 g), methanol (7 mL), a 37% aqueous formaldehyde solution (0.354 g), and acetic acid (0.150 mL) were mixed, and the mixture was stirred at room temperature for 10 min. Sodium triacetoxyborohydride (0.925 g) was then added, and the mixture was stirred at room temperature for 12 h. After the reaction was completed, the reaction solution was poured into a saturated sodium bicarbonate solution, and dichloromethane (100 mL) was added for extraction. The organic phase was washed with saturated brine, dried over anhydrous sodium sulfate, and filtered under vacuum. The filtrate was concentrated under reduced pressure, and the residue was separated and purified by silica gel column chromatography (dichloromethane:methanol = 20:1) to give 0.2 g of intermediate **7-5.**

MS (ESI, [M+H]⁺) m/z: 266.13.

¹H-NMR (500 MHz, DMSO-d₆): *δ* 7.76 (d, *J* = 2 Hz, 1H), 7.51 (d, *J =* 8.5 Hz, 1H), 7.32 (dd, *J* = 8.5, 2 Hz, 1H), 4.13 (t, *J =* 5.5 Hz, 2H), 3.74 (s, 2H), 2.92 (t, *J =* 5.5 Hz, 2H), 2.44 (s, 3H).

### Step 5: Synthesis of intermediate 7-6

Intermediate **A-1** (200 mg), intermediate **7-5** (181 mg), (1*S*,2*S*)-*N*1,*N*2-dimethylcyclohexane-1,2-diamine (32.2 mg), potassium carbonate (188 mg), copper(I) iodide (17.25 mg), and N-methylpyrrolidone (5 mL) were mixed, and the mixture was left to react at 120 °C for 12 h under a nitrogen atmosphere. After the reaction was completed, the reaction solution was poured into water, and ethyl acetate (50 mL) was added for extraction. The organic phase was washed with saturated brine, dried over anhydrous sodium sulfate, and filtered under vacuum. The filtrate was concentrated under reduced pressure, and the residue was separated and purified by silica gel column chromatography (dichloromethane:methanol = 20:1) to give 0.245 g of intermediate **7-6.**

MS (ESI, [M+H]⁺) m/z: 627.48.

¹H-NMR (500 MHz, DMSO-d₆): *δ* 7.74 (s, 1H), 7.63 (d, *J =* 8.5 Hz, 1H), 7.40 (dd, *J =* 8.5, 2.5 Hz, 1H), 7.26 (d, *J =* 3 Hz, 1H), 7.12 (d, *J =* 6 Hz, 2H), 6.95 (s, 1H), 5.22-5.05 (m, 1H), 4.36-4.18 (m, 1H), 4.14 (t, *J =* 5.5 Hz, 2H), 3.77 (s, 2H), 3.20-3.05 (m, 1H), 2.94 (t, *J =* 5.5 Hz, 2H), 2.77-2.71 (m, 1H), 2.70-2.62 (m, 1H), 2.45 (s, 3H), 2.20 (d, *J =* 1.5 Hz, 6H), 1.44 (s, 9H), 1.22-1.18 (m, 3H).

### Step 6: Synthesis of intermediate 7-7

Intermediate **7-6** (0.24 g), dichloromethane (2 mL), and a 4 N solution of hydrochloric acid in dioxane (2 mL) were mixed, and the mixture was stirred at room temperature for 1 h. After the reaction was completed, the reaction solution was directly concentrated under reduced pressure to give 0.2 g of intermediate **7-7.**

MS (ESI, [M+H]⁺) m/z: 527.34.

### Step 7: Synthesis of intermediate 7-8 and compound 7

Intermediate **B-1** (0.1 g), *N,N*-dimethylformamide (5 mL), triethylamine (0.678 mL), and *O*-(7-azabenzotriazol-1-yl)-*N,N,N,N*-tetramethyluronium hexafluorophosphate (0.277 g) were mixed. The mixture was stirred at room temperature for 5 min, and then intermediate 7-7 (0.151 g) was added thereto. The resulting mixture was stirred at room temperature for 5 h. After the reaction was completed, the reaction solution was poured into water, and ethyl acetate (50 mL) was added for extraction. The organic phase was washed with saturated brine, dried over anhydrous sodium sulfate, and filtered under vacuum. The filtrate was concentrated under reduced pressure, and the residue was separated and purified by silica gel column chromatography (dichloromethane:methanol = 20: 1) to give 0.12 g of intermediate **7-8.** The above intermediate **7-8** was subjected to chiral resolution by supercritical fluid chromatography (chromatographic column: CHIRALART Cellulose-SB, 20 × 100 mm, 5 µm; mobile phase: carbon dioxide:ethanol (0.1% ammonium hydroxide) = 68:32; flow rate: 60 mL/min) to give 54 mg of compound **7.**

Compound 7: Rt = 2.97 min (UPCC conditions: chromatographic column: CHIRALPAK IB-3 (4.6 × 100 mm, 3 µm); mobile phase: carbon dioxide:methanol (containing 0.1% ammonium hydroxide) = 50:50; flow rate: 2.0 mL/min; column temperature: 40 °C)

HRMS: (ESI, [M+H]⁺) m/z: 920.4379.

¹H NMR (500 MHz, DMSO-*d*₆) *δ* 11.74 (s, 1H), 7.78 (s, 1H), 7.65 (d, *J =* 8.5 Hz, 1H), 7.53 (s, 1H), 7.44-7.38 (m, 2H), 7.29-7.24 (m, 2H), 7.20-7.14 (m, 2H), 6.97-6.91 (m, 2H), 5.62-5.55 (m, 1H), 4.42-4.34 (m, 1H), 4.19-4.12 (m, 2H), 4.08-3.92 (m, 1H), 3.79-3.76 (m, 2H), 3.73-3.70 (m, 2H), 3.66-3.56 (m, 1H), 3.24-3.15 (m, 1H), 3.06-3.00 (m, 1H), 2.96-2.92 (m, 2H), 2.45 (s, 3H), 2.23 (s, 6H), 1.80-1.75 (m, 1H), 1.73-1.64 (m, 4H), 1.62-1.57 (m, 2H), 1.55-1.49 (m, 2H), 1.41-1.39 (m, 1H), 1.28-1.22 (m, 6H), 1.18 (s, 3H).

### Example 8

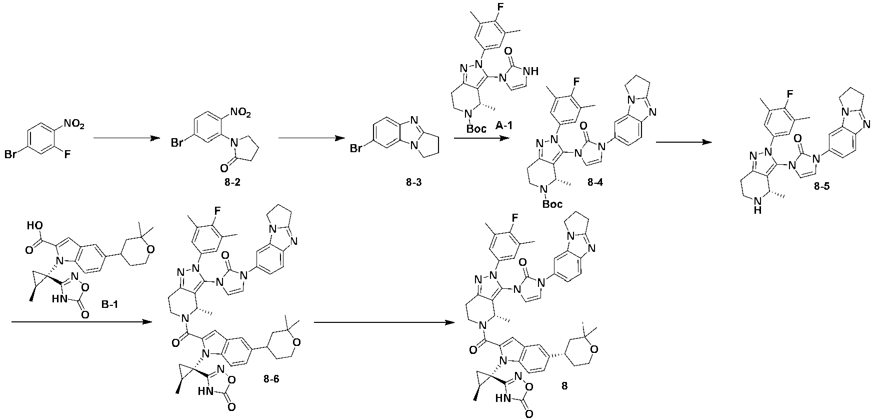

### Step 1: Synthesis of intermediate 8-2

2-Pyrrolidone (0.426 g) and *N,N*-dimethylformamide (15 mL) were mixed, and sodium hydride (0.273 g, 60% dispersion in mineral oil) was added in an ice bath. The mixture was stirred at room temperature for 10 min, and then 2-fluoro-4-bromonitrobenzene (1 g) was added thereto. After the addition, the resulting mixture was stirred at room temperature for 2 h. After the reaction was completed, the reaction solution was poured into a saturated ammonium chloride solution (100 mL), and ethyl acetate (100 mL) was added for extraction. The organic phase was washed with saturated brine, dried over anhydrous sodium sulfate, and filtered under vacuum. The filtrate was concentrated under reduced pressure, and the residue was separated and purified by silica gel column chromatography (petroleum ether:ethyl acetate = 3:1) to give 0.83 g of intermediate **8-2.**

MS (ESI, [M+H]⁺) m/z: 285.02.

¹H-NMR (500 MHz, DMSO-d₆): *δ* 7.91 (d, *J =* 8.5 Hz, 1H), 7.89 (d, *J =* 2 Hz, 1H), 7.70 (dd, *J =* 8.5, 2 Hz, 1H), 3.93 (t, *J =* 7 Hz, 2H), 2.41 (t, *J =* 8.0 Hz, 2H), 2.17-2.11 (m, 2H).

### Step 2: Synthesis of intermediate 8-3

Intermediate **8-2** (0.7 g), acetic acid (20 mL), and iron powder (0.7 g) were mixed, and the mixture was heated to 120 °C and left to react for 4 h under a nitrogen atmosphere. After the reaction was completed, the reaction solution was cooled to room temperature and filtered through diatomite under vacuum, and the filtrate was directly concentrated. The residue was dissolved in ethyl acetate (100 mL), and the resulting solution was washed with a saturated sodium bicarbonate solution and saturated brine, dried over anhydrous sodium sulfate, and filtered under vacuum. The filtrate was concentrated under reduced pressure to give 0.5 g of intermediate **8-3.**

MS (ESI, [M+H]⁺) m/z: 237.12.

¹H-NMR (500 MHz, DMSO-d₆): *δ* 7.71 (d, *J* = 2 Hz, 1H), 7.48 (d, *J =* 8.5 Hz, 1H), 7.26 (dd, *J* = 8.5, 2 Hz, 1H), 4.10 (t, *J =* 7 Hz, 2H), 2.94 (t, *J =* 7.5 Hz, 2H), 2.65-2.59 (m, 2H).

### Step 3: Synthesis of intermediate 8-4

Intermediate **A-1** (200 mg), intermediate **8-3** (0.129 g), (1*S*,2*S*)-*N*1,*N*2-dimethylcyclohexane-1,2-diamine (32.2 mg), potassium carbonate (188 mg), copper(I) iodide (17.25 mg), and N-methylpyrrolidone (5 mL) were mixed, and the mixture was left to react at 120 °C for 12 h under a nitrogen atmosphere. After the reaction was completed, the reaction solution was poured into water, and ethyl acetate (50 mL) was added for extraction. The organic phase was washed with saturated brine, dried over anhydrous sodium sulfate, and filtered under vacuum. The filtrate was concentrated under reduced pressure, and the residue was separated and purified by silica gel column chromatography (dichloromethane:methanol = 20: 1) to give 0.25 g of intermediate **8-4.**

MS (ESI, [M+H]⁺) m/z: 598.39.

¹H-NMR (500 MHz, DMSO-d₆): *δ* 7.76-7.70 (m, 1H), 7.66-7.57 (m, 1H), 7.34 (d, *J =* 7 Hz, 1H), 7.25 (d, *J =* 3 Hz, 1H), 7.12 (d, *J =* 6 Hz, 2H), 6.97 (s, 1H), 5.22-5.06 (m, 1H), 4.36-4.18 (m, 1H), 4.12 (t, *J =* 7 Hz, 2H), 3.21-3.08 (m, 1H), 2.99-2.90 (m, 2H), 2.77-2.71 (m, 1H), 2.69-2.59 (m, 3H), 2.20 (d, *J =* 1.5 Hz, 6H), 1.44 (s, 9H), 1.23-1.19 (m, 3H).

### Step 4: Synthesis of intermediate 8-5

Intermediate **8-4** (0.25 g), dichloromethane (2 mL), and a 4 N solution of hydrochloric acid in dioxane (2 mL) were mixed, and the mixture was stirred at room temperature for 1 h. After the reaction was completed, the reaction solution was directly concentrated under reduced pressure to give 0.2 g of intermediate **8-5.**

MS (ESI, [M+H]⁺) m/z: 498.40.

### Step 5: Synthesis of intermediates 8-6 and compound 8

Intermediate **B-1** (0.1 g), *N,N*-dimethylformamide (5 mL), triethylamine (0.678 mL), and *O*-(7-azabenzotriazol-1-yl)-*N,N,N,N*-tetramethyluronium hexafluorophosphate (0.277 g) were mixed. The mixture was stirred at room temperature for 5 min, and then intermediate **8-5** (0.130 g) was added thereto. The resulting mixture was stirred at room temperature for 5 h. After the reaction was completed, the reaction solution was poured into water, and ethyl acetate (50 mL) was added for extraction. The organic phase was washed with saturated brine, dried over anhydrous sodium sulfate, and filtered under vacuum. The filtrate was concentrated under reduced pressure, and the residue was separated and purified by silica gel column chromatography (dichloromethane:methanol = 20: 1) to give 0.12 g of intermediate **8-6.** The above intermediate **8-6** was subjected to chiral resolution by supercritical fluid chromatography (chromatographic column: CHIRALART Cellulose-SB, 20 × 100 mm, 5 µm; mobile phase: carbon dioxide:ethanol (0.1% ammonium hydroxide) = 53:47; flow rate: 60 mL/min) to give 30 mg of compound **8.**

Compound **8:** Rt = 3.57 min (UPCC conditions: chromatographic column: CHIRALPAK IB-3 (4.6 × 100 mm, 3 µm); mobile phase: carbon dioxide:methanol (containing 0.1% ammonium hydroxide) = 50:50; flow rate: 2.0 mL/min; column temperature: 40 °C)

HRMS: (ESI, [M+H]⁺) m/z: 891.4119.

¹H NMR (500 MHz, DMSO-*d*₆) *δ* 11.74 (s, 1H), 7.75 (s, 1H), 7.64-7.59 (m, 1H), 7.53 (s, 1H), 7.41-7.34 (m, 2H), 7.29-7.24 (m, 2H), 7.19-7.14 (m, 2H), 7.00-6.91 (m, 2H), 5.62-5.55 (m, 1H), 4.42-4.35 (m, 1H), 4.17-4.11 (m, 2H), 3.76-3.67 (m, 2H), 3.22-3.16 (m, 1H), 3.08-3.00 (m, 1H), 2.99-2.93 (m, 2H), 2.92-2.85 (m, 1H), 2.68-2.59 (m, 3H), 2.22 (s, 6H), 1.82-1.75 (m, 1H), 1.71-1.64 (m, 4H), 1.62-1.57 (m, 2H), 1.55-1.52 (m, 2H), 1.41-1.39 (m, 1H), 1.28-1.22 (m, 6H), 1.18 (s, 3H).

### Example 9

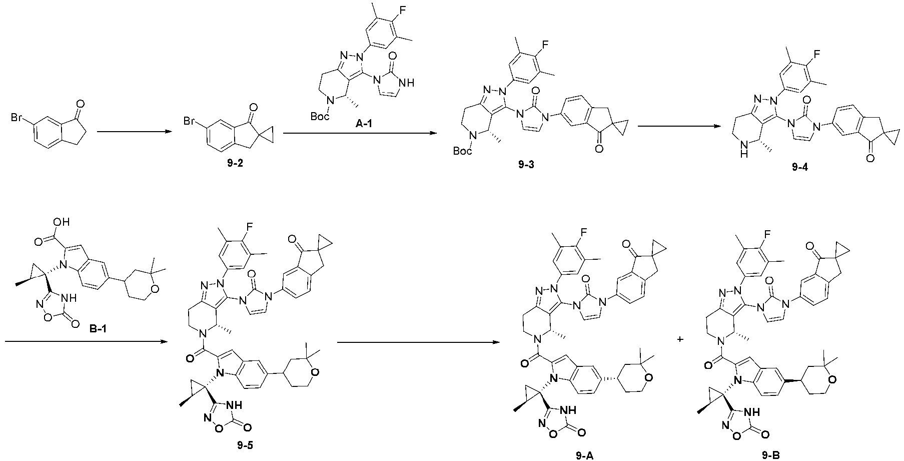

### Step 1: Synthesis of intermediate 9-2

Sodium hydride (0.995 g, 60% dispersion in mineral oil) and *N,N*-dimethylformamide (10 mL) were mixed, and the mixture was stirred in an ice-water bath for 10 min under a nitrogen atmosphere. A solution of 6-bromoindanone (1 g) and 1,2-dibromoethane (3.12 g) in *N,N*-dimethylformamide (10 mL) was then added dropwise to the mixture. After the dropwise addition, the resulting mixture was stirred with the temperature maintained. The reaction was stopped, and the reaction solution was poured into water (150 mL). The resulting solution was extracted with ethyl acetate (50 mL). The organic phase was washed with saturated brine, dried over anhydrous sodium sulfate, concentrated, and separated and purified by column chromatography (petroleum ether:ethyl acetate = 95:5) to give 0.69 g of intermediate **9-2.**

¹H-NMR (500 MHz, DMSO-d₆): *δ* 7.90 (s, 1H), 7.65 (d, *J =* 7.9 Hz, 1H), 7.59 (d, *J =* 8.0 Hz, 1H), 3.25 (s, 2H), 1.24 (d, *J =* 10.1 Hz, 4H).

### Step 2: Synthesis of intermediate 9-3

Intermediate **A-1** (250 mg), intermediate **9-2** (175 mg), (1*R*,2*R*)-(-)-*N*,*N*-dimethyl-1,2-cyclohexanediamine (40.3 mg), potassium carbonate (235 mg), copper(I) iodide (21.57 mg), and *N*-methylpyrrolidone (4 mL) were mixed, and the mixture was stirred in an oil bath at 120 °C overnight under a nitrogen atmosphere. The reaction was stopped, and the reaction solution was cooled to room temperature and poured into water (50 mL). The resulting solution was extracted with ethyl acetate (30 mL). The organic phase was washed with water and saturated brine in sequence, dried over anhydrous sodium sulfate, concentrated, and separated and purified by column chromatography (petroleum ether:ethyl acetate = 85:15) to give 0.27 g of intermediate **9-3.**

MS (ESI, [M+H]⁺) m/z: 598.34.

¹H-NMR (500 MHz, DMSO-d₆): *δ* 7.99 (s, 1H), 7.84 (d, *J =* 8.5 Hz, 1H), 7.75 (d, *J =* 8.4 Hz, 1H), 7.48 (d, *J =* 3.3 Hz, 1H), 7.11 (d, *J =* 6.3 Hz, 2H), 7.07 (s, 1H), 5.21-5.01 (m, 1H), 4.39-4.11 (m, 1H), 3.27 (s, 2H), 3.22-3.04 (m, 1H), 2.77-2.62 (m, 2H), 2.18 (d, *J =* 1.3 Hz, 6H), 1.44 (s, 9H), 1.27-1.18 (m, 7H).

### Step 3: Synthesis of intermediate 9-4

Intermediate **9-3** (0.25 g), dichloromethane (2 mL), and a 4 N solution of hydrochloric acid in dioxane (2 mL) were mixed, and the mixture was stirred at room temperature for 1 h. After the reaction was completed, the reaction solution was directly concentrated under reduced pressure to give 0.21 g of intermediate **9-4.**

MS (ESI, [M+H]⁺) m/z: 498.0.

### Step 4: Synthesis of intermediate 9-5, compound 9-A, and compound 9-B

Intermediate **B-1** (0.14 g), *N,N*-dimethylformamide (5 mL), triethylamine (0.806 mL), and *O*-(7-azabenzotriazol-1-yl)-*N,N,N,N*-tetramethyluronium hexafluorophosphate (0.330 g) were mixed and stirred for 5 min, and then intermediate **9-4** (0.170 g) was added. The mixture was stirred at room temperature. The reaction solution was poured into water (50 mL), and the resulting solution was extracted with ethyl acetate (30 mL). The organic phase was washed with saturated brine, dried over anhydrous sodium sulfate, concentrated, and separated and purified by column chromatography (DCM:MeOH = 95:5) to give 130 mg of intermediate **9-5.** The above intermediate **9-5** was resolved by preparative chiral HPLC (chromatographic column: REFLECT I-Cellulose B, 30 × 250 mm, 10 µm; mobile phase: ethanol-dichloromethane (1:4):*n*-hexane = 25:75; flow rate: 40 mL/min) to give compound **9-A** (26 mg) and compound **9-B** (30 mg).

Compound **9-A:** Rₜ = 2.59 min (UPCC conditions: chromatographic column: CHIRALPAK IB-3, 4.6 × 100 mm, 3 µm; mobile phase: carbon dioxide:ethanol:isopropanol = 40:30:30; flow rate: 2.0 mL/min; column temperature: 40 °C)

HRMS: (ESI, [M+H]⁺) m/z: 891.4030.

¹H-NMR (500 MHz, DMSO-*d*₆): *δ* 11.73 (s, 1H), 8.06-7.74 (m, 3H), 7.57-7.37 (m, 3H), 7.28-6.94 (m, 5H), 5.65-5.53 (m, 1H), 4.46-4.31 (m, 1H), 3.79-3.59 (m, 3H), 3.29 (s, 3H), 3.10-2.98 (m, 1H), 2.94-2.85 (m, 1H), 2.23-2.15 (m, 6H), 1.83-1.47 (m, 8H), 1.44-1.37 (m, 2H), 1.28 (s, 3H), 1.25-1.15 (m, 10H).

Compound **9-B:** Rₜ = 2.03 min (UPCC conditions: chromatographic column: CHIRALPAK IB-3, 4.6 × 100 mm, 3 µm; mobile phase: carbon dioxide:ethanol:isopropanol = 40:30:30; flow rate: 2.0 mL/min; column temperature: 40 °C)

HRMS: (ESI, [M+H]⁺) m/z: 891.4026.

¹H-NMR (500 MHz, DMSO-*d*₆): *δ* 11.73 (s, 1H), 8.07-7.68 (m, 3H), 7.56-7.37 (m, 3H), 7.29-6.85 (m, 5H), 5.64-5.54 (m, 1H), 4.48-4.32 (m, 1H), 3.77-3.59 (m, 3H), 3.30-3.13 (m, 3H), 3.09-2.99 (m, 1H), 2.96-2.86 (m, 1H), 2.24-2.13 (m, 6H), 1.83-1.46 (m, 8H), 1.43-1.36 (m, 2H), 1.27 (s, 3H), 1.25-1.14 (m, 10H).

### Example 10

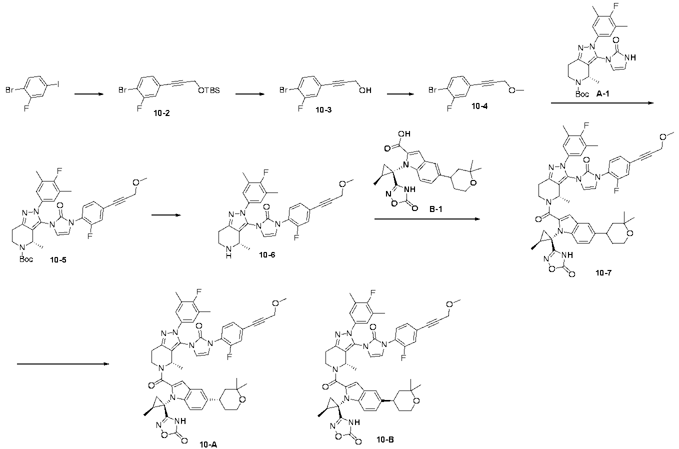

### Step 1: Synthesis of intermediate 10-2

1-Bromo-2-fluoro-4-iodobenzene (2 g), copper(I) iodide (0.076 g), bis(triphenylphosphine)palladium(II) dichloride (0.140 g), diisopropylamine (20 mL), and *tert*-butyldimethyl(prop-2-yn-1-yloxy)silane (1.359 g) were mixed, and the mixture was stirred at room temperature overnight. The reaction solution was then added to a mixed system of ethyl acetate (30 mL) and water (40 mL), and the two phases were separated. The aqueous phase was extracted with ethyl acetate (2 × 30 mL). The organic phases were combined, then washed with saturated brine, dried over anhydrous sodium sulfate, filtered, concentrated under vacuum, and separated and purified by column chromatography (petroleum ether:ethyl acetate = 98:2) to give 1.8 g of intermediate **10-2.**

¹H-NMR (500 MHz, DMSO-*d*₆): δ 7.60 (t, *J =* 7.8 Hz, 1H), 7.32 (dd, *J* = 9.5, 2.0 Hz, 1H), 7.09 (dd, *J =* 8.3, 1.9 Hz, 1H), 4.42 (s, 2H), 0.76 (s, 9H), -0.00 (s, 6H).

### Step 2: Synthesis of intermediate 10-3

Intermediate **10-2** (1.8 g), tetrabutylammonium fluoride (1 M, 10.49 mL), and tetrahydrofuran (10 mL) were mixed, and the mixture was stirred at room temperature for 1 h. The reaction system was directly concentrated to dryness by rotary evaporation, and separated and purified by column chromatography (dichloromethane:methanol = 100:0) to give 1.67 g of intermediate **10-3.**

MS (ESI, [M+H]⁺) m/z: 228.23.

¹H-NMR (500 MHz, DMSO-*d*₆): δ 7.79-7.72 (m, 1H), 7.49 (dd, *J* = 9.5, 1.9 Hz, 1H), 7.26 (dd, *J* = 8.3, 1.9 Hz, 1H), 5.42 (s, 1H), 4.39-4.30 (m, 2H).

### Step 3: Synthesis of intermediate 10-4

Intermediate **10-3** (500 mg), tetrahydrofuran (10 mL), and sodium hydride (175 mg, 60% dispersion in mineral oil) were mixed, and the reaction system was placed in an ice-water bath. Iodomethane (372 mg) was then added dropwise to the reaction solution, and the reaction mixture was transferred to room temperature and stirred for 2 h. The reaction solution was quenched with water and then added to a mixed system of ethyl acetate (30 mL) and water (40 mL), and the two phases were separated. The aqueous phase was extracted with ethyl acetate (2 × 30 mL). The organic phases were combined, then washed with saturated brine, dried over anhydrous sodium sulfate, filtered, and concentrated under vacuum to give 320 mg of intermediate **10-4.**

¹H-NMR (500 MHz, DMSO-*d*₆): δ 7.77-7.71 (m, 1H), 7.52 (dd, *J* = 9.5, 1.9 Hz, 1H), 7.26 (dd, *J* = 8.2, 2.1 Hz, 1H), 4.33 (s, 2H), 3.33 (s, 3H).

### Step 4: Synthesis of intermediate 10-5

Copper(I) iodide (21.57 mg), potassium carbonate (235 mg), (1*S*,2*S*)-*N*1,*N*2-dimethylcyclohexane-1,2-diamine (40.3 mg), intermediate **A-1** (250 mg), intermediate **10-4** (210 mg), and N-methylpyrrolidone (4 mL) were mixed, and the reaction system was placed under a nitrogen atmosphere and then left to react in an oil bath at 130 °C for 3 h. The reaction solution was added to a mixed system of ethyl acetate (30 mL) and water (40 mL), and the two phases were separated. The aqueous phase was extracted with ethyl acetate (2 × 30 mL). The organic phases were combined, then washed with saturated brine, dried over anhydrous sodium sulfate, filtered, concentrated under vacuum, and separated and purified by column chromatography (petroleum ether:ethyl acetate = 1:1) to give 220 mg of intermediate **10-5.**

MS (ESI, [M+H]⁺) m/z: 604.33.

### Step 5: Synthesis of intermediate 10-6

Intermediate **10-5** (210 mg, 0.348 mmol), dichloromethane (2 mL), and a 4 M solution of hydrochloric acid in dioxane (1.5 mL) were mixed, and the mixture was stirred at room temperature for 2 h. The reaction system was concentrated under reduced pressure to give 190 mg of intermediate **10-6.**

MS (ESI, [M+H]⁺) m/z: 504.26.

### Step 6: Synthesis of intermediate 10-7, compound 10-A, and compound 10-B

Intermediate **B-1** (100 mg), *N,N*-dimethylformamide (5 mL), *O*-(7-azabenzotriazol-1-yl)-*N*,*N*,*N*',*N*'-tetramethyluronium hexafluorophosphate (236 mg), and triethylamine (418 mg) were stirred for 5 min, and then intermediate **10-6** (123 mg) was added. The mixture was stirred at room temperature for 3 h. The reaction solution was poured into water (50 mL), and the resulting solution was extracted with ethyl acetate (30 mL). The organic phase was washed with saturated brine, dried over anhydrous sodium sulfate, concentrated, and purified by preparative high-pressure chromatography to give 80 mg of compound **10-7.** The above compound **10-7** was resolved by preparative chiral HPLC (chromatographic column: REGIS IB, 30 × 250 mm, 10 µm; mobile phase: ethanol-dichloromethane (1:1):n-hexane = 27:73; flow rate: 42 mL/min) to give compound **10-A** (26 mg) and compound **10-B** (30 mg).

Compound **10-A:** Rt = 2.790 min (UPCC conditions: chromatographic column: CHIRALPAK IB-3, 4.6 × 100 mm, 3 µm; mobile phase: carbon dioxide:ethanol:isopropanol = 50:25:25; flow rate: 2.0 mL/min; column temperature: 40 °C)

HRMS: (ESI, [M+H]⁺) m/z: 897.3933.

¹H-NMR (500 MHz, DMSO-d₆): δ 11.73 (s, 1H), 7.59 (t, J = 8.6 Hz, 2H), 7.53 (s, 1H), 7.45 (d, J = 8.3 Hz, 1H), 7.40 (d, J = 8.6 Hz, 1H), 7.26 (d, J = 8.4 Hz, 1H), 7.17-7.06 (m, 3H), 6.95 (d, J = 10.8 Hz, 2H), 5.57 (d, J = 7.1 Hz, 1H), 4.36 (s, 2H), 3.72 (d, J = 9.5 Hz, 3H), 3.65-3.60 (m, 1H), 3.2-3.15 (m, 1H), 3.02 (d, J = 12.3 Hz, 1H), 2.22 (d, J = 15.4 Hz, 6H), 1.99 (s, 1H), 1.75-1.63 (m, 4H), 1.57-1.47 (m, 2H), 1.41 (d, J = 6.7 Hz, 3H), 1.29 (d, J = 12.2 Hz, 5H), 1.24 (d, J = 6.7 Hz, 3H), 1.21-1.12 (m, 5H).

Compound **10-B:** Rt = 2.169 min (UPCC conditions: chromatographic column: CHIRALPAK IB-3, 4.6 × 100 mm, 3 µm; mobile phase: carbon dioxide:ethanol:isopropanol = 50:25:25; flow rate: 2.0 mL/min; column temperature: 40 °C)

HRMS: (ESI, [M+H]⁺) m/z: 897.3928.

¹H-NMR (500 MHz, DMSO-d₆): δ 11.73 (s, 1H), 7.60 (d, J = 9.6 Hz, 2H), 7.53 (s, 1H), 7.44 (d, J = 8.3 Hz, 1H), 7.40 (d, J = 8.5 Hz, 1H), 7.26 (d, J = 8.6 Hz, 1H), 7.15 (d, J = 6.3 Hz, 2H), 7.09 (s, 1H), 6.95 (d, J = 13.0 Hz, 2H), 5.63-5.52 (m, 1H), 4.36 (s, 2H), 3.72 (d, J = 9.6 Hz, 2H), 3.65-3.59 (m, 1H), 3.35 (s, 3H), 3.20-3.15 (s, 2H), 3.02 (d, J = 12.6 Hz, 1H), 2.90 (t, J = 15.9 Hz, 1H), 2.22 (d, J = 15.4 Hz, 6H), 1.69-1.65 (m, J = 8.9 Hz, 3H), 1.53-1.48 (m, 2H), 1.29-1.27 (m, 6H),1.24-1.23 (m, 3H), 1.18-1.16 (m, 5H).

### Example 11

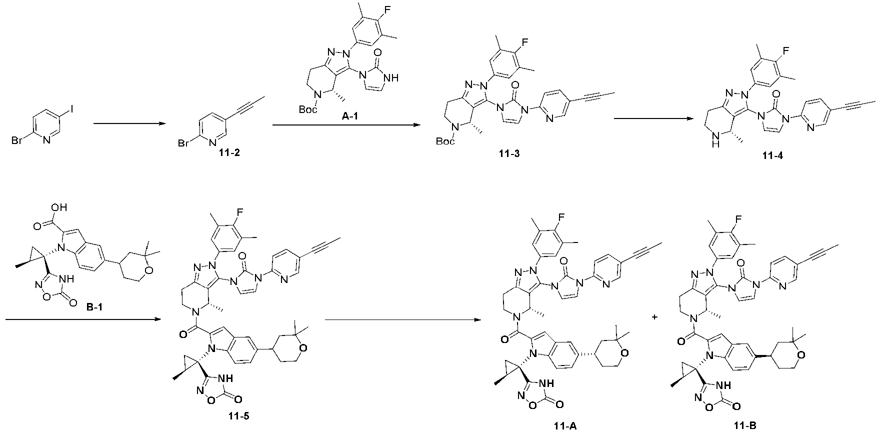

### Step 1: Synthesis of intermediate 11-2

2-Bromo-5-iodopyridine (2 g), copper(I) iodide (0.040 g), bis(triphenylphosphine)palladium(II) dichloride (0.198 g), tetrahydrofuran (30 mL), triethylamine (10.86 g), and propyne (0.367 g) were mixed, and the reaction solution was stirred at room temperature for 2.5 h. The reaction solution was poured into a mixed system of ethyl acetate (30 mL) and water (40 mL), and the two phases were separated. The aqueous phase was extracted with ethyl acetate (2 × 30 mL). The organic phases were combined, then washed with saturated brine, dried over anhydrous sodium sulfate, filtered, concentrated under vacuum, and separated and purified by column chromatography (petroleum ether:ethyl acetate = 97:3) to give 1.2 g of intermediate **11-2.**

MS (ESI, [M+H]⁺) m/z: 196.05.

¹H-NMR (500 MHz, DMSO-*d*₆): *δ* 8.41 (d, J = 2.4 Hz, 1H), 7.75 (dd, J = 8.3, 2.5 Hz, 1H), 7.64 (d, J = 9.0 Hz, 1H), 2.08 (s, 3H).

### Step 2: Synthesis of intermediate 11-3

Intermediate **A-1** (370 mg), intermediate **11-2** (246 mg), (1*R*,2*R*)-(-)-*N,N*-dimethyl-1,2-cyclohexanediamine (69 mg), copper(I) iodide (32 mg), potassium carbonate (232 mg), and *N*-methylpyrrolidone (4 mL) were mixed. The mixture was purged with nitrogen, and then heated and stirred in an oil bath at 130 °C for 3 h. The reaction solution was added to a mixed system of ethyl acetate (30 mL) and water (40 mL), and the two phases were separated. The aqueous phase was extracted with ethyl acetate (2 × 30 mL). The organic phases were combined, then washed with saturated brine, dried over anhydrous sodium sulfate, filtered, concentrated under vacuum, and separated and purified by column chromatography (petroleum ether:ethyl acetate = 1:1) to give 420 mg of intermediate **11-3.**

### Step 3: Synthesis of intermediate 11-4

Intermediate **11-3** (450 mg), dichloromethane (2 mL), and a 4 N solution of hydrochloric acid in dioxane (2 mL) were mixed, and the mixture was stirred at room temperature for 1 h. The stirring was stopped, and the mixture was concentrated under reduced pressure to give 347 mg of intermediate **11-4.**

MS (ESI, [M+H]⁺) m/z: 457.30.

### Step 4: Synthesis of intermediate 11-5, compound 11-A, and compound 11-B

Intermediate **11-4** (132 mg), intermediate **B-1** (100 mg), triethylamine (492 mg), *O*-(7-azabenzotriazol-1-yl)-*N,N,N,N*-tetramethyluronium hexafluorophosphate (277 mg), and *N,N-*dimethylformamide (5 mL) were mixed, and the mixture was stirred at room temperature for 3 h. The reaction solution was poured into water (50 mL), and the resulting solution was extracted with ethyl acetate (30 mL). The organic phase was washed with saturated brine, dried over anhydrous sodium sulfate, concentrated, and separated and purified by column chromatography (dichloromethane:methanol = 95:5) to give 70 mg of **11-5.** The above intermediate **11-5** was resolved by preparative chiral HPLC (chromatographic column: REFLECT I-Cellulose B, 30 × 250 mm, 10 µm; mobile phase: ethanol-dichloromethane (1:3):n-hexane = 20:80; flow rate: 40 mL/min) to give compound **11-A** (15 mg) and compound **11-B** (12 mg).

Compound **11-A:** Rt = 1.58 min (UPCC conditions: chromatographic column: CHIRALPAK IB-3, 4.6 × 100 mm, 3 µm; mobile phase: carbon dioxide:ethanol:isopropanol = 40:30:30; flow rate: 2.0 mL/min; column temperature: 40 °C).

HRMS: (ESI, [M+H]⁺) m/z: 850.3866.

¹H-NMR (500 MHz, DMSO-d₆): δ 11.73 (s, 1H), 8.53-7.88 (m, 2H), 7.63-6.67 (m, 9H), 5.76-5.54 (m, 1H), 4.51-4.35 (m, 1H), 3.76-3.51 (m, 3H), 3.24-3.14 (m, 1H), 3.08-2.99 (m, 1H), 2.96-2.85 (m, 1H), 2.24-2.14 (m, 6H), 2.11-2.04 (m, 3H), 1.80-1.44 (m, 8H), 1.34 (s, 3H), 1.24 -1.17 (m, 8H).

Compound **11-B:** Rt = 1.31 min (UPCC conditions: chromatographic column: CHIRALPAK IB-3, 4.6 × 100 mm, 3 µm; mobile phase: carbon dioxide:ethanol:isopropanol = 40:30:30; flow rate: 2.0 mL/min; column temperature: 40 °C).

HRMS: (ESI, [M+H]⁺) m/z: 850.3867.

¹H-NMR (500 MHz, DMSO-*d*₆): δ 11.73 (s, 1H), 8.56-7.91 (m, 2H), 7.66-6.64 (m, 9H), 5.76-5.52 (m, 1H), 4.50-4.33 (m, 1H), 3.83-3.54 (m, 3H), 3.26-3.15 (m, 1H), 3.09-2.96 (m, 1H), 2.96-2.84 (m, 1H), 2.28-2.14 (m, 6H), 2.13-2.03 (m, 3H), 1.81-1.45 (m, 8H), 1.37-1.32 (m, 3H), 1.25-1.17 (m, 8H).

### Example 12

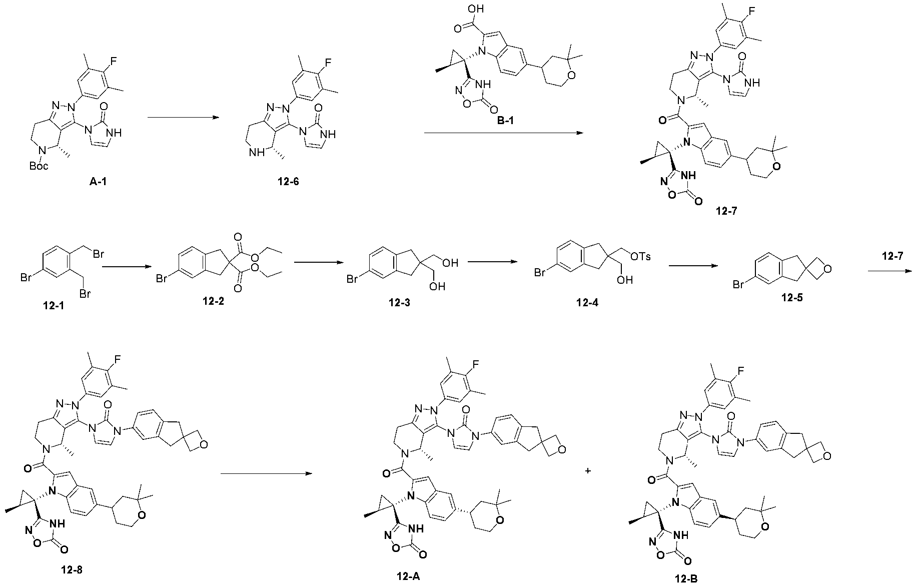

### Step 1: Synthesis of intermediate 12-2

Sodium hydride (1.167 g, 60% dispersion in mineral oil) was added to a mixed solvent of tetrahydrofuran (60 mL) and ethanol (20 mL) at 0 °C, and the mixture was stirred for 10 min. Intermediate **12-1** (5 g) and diethyl malonate (2.336 g) were added to the reaction solution, and the reaction solution was left to react at room temperature for 4 h. A saturated ammonium chloride solution was added to the reaction solution to quench the reaction, and the mixture was extracted with ethyl acetate. The organic phase was dried, concentrated, and separated and purified by column chromatography (petroleum ether:ethyl acetate = 90:10) to give 4.3 g of intermediate **12-2.**

¹H-NMR (500 MHz, DMSO-*d₆*): δ 7.45 (d, J = 1.8 Hz, 1H), 7.34 (dd, J = 8.0, 1.9 Hz, 1H), 7.19 (d, J = 8.1 Hz, 1H), 4.15 (q, J = 7.1 Hz, 4H), 3.49 (s, 2H), 3.43 (s, 2H), 1.17 (t, J = 7.1 Hz, 6H).

### Step 2: Synthesis of intermediate 12-3

Intermediate **12-2** (0.5 g), THF (6 mL), and lithium borohydride (0.064 g) were mixed, and the mixture was left to react overnight at room temperature. The reaction solution was poured into a saturated ammonium chloride solution (50 mL) and the mixture was extracted with ethyl acetate (50 mL × 3). The organic phases were combined, washed with saturated brine, dried over anhydrous sodium sulfate, filtered, concentrated, and separated and purified by column chromatography (dichloromethane:methanol = 95:5) to give 0.24 g of intermediate **12-3.**

¹H-NMR (500 MHz, DMSO-*d₆*): δ 7.34 (s, 1H), 7.25 (d, J = 8.0 Hz, 1H), 7.10 (d, J = 8.0 Hz, 1H), 4.62 (t, J *=* 5.3 Hz, 2H), 3.34 (d, J = 5.3 Hz, 4H), 2.71 (s, 2H), 2.65 (s, 2H).

### Step 3: Synthesis of intermediate 12-4

Intermediate **12-3** (1 g) and tetrahydrofuran (15 mL) were mixed, and sodium hydride (0.187 g, 60% dispersion in mineral oil) was added at 0 °C. The mixture was left to react for half an hour, and then p-toluenesulfonyl chloride (0.741 g) was added. The mixture was left to react for another 1.5 h. A saturated ammonium chloride solution was added to the reaction solution to quench the reaction, and the mixture was extracted with ethyl acetate. The organic phase was dried, concentrated, and separated and purified by column chromatography (petroleum ether:ethyl acetate = 80:20) to give 1.2 g of intermediate **12-4.**

¹H-NMR (500 MHz, DMSO-*d₆*): δ 7.76 (d, J = 8.3 Hz, 2H), 7.46 (d, J = 8.0 Hz, 2H), 7.34 -7.25 (m, 2H), 7.08 (d, J = 8.0 Hz, 1H), 4.94 (s, 1H), 3.97 (d, J = 4.3 Hz, 2H), 3.28 (s, 2H), 2.77 -2.58 (m, 4H), 2.42 (s, 3H).

### Step 4: Synthesis of intermediate 12-5

Intermediate **12-4** (500 mg) and tetrahydrofuran (10 mL) were mixed, and sodium hydride (97 mg, 60% dispersion in mineral oil) was added at 0 °C. The mixture was left to react overnight at 70 °C in an oil bath. A saturated ammonium chloride solution was added to the reaction solution to quench the reaction, and the mixture was extracted with ethyl acetate. The organic phase was dried, concentrated, and separated and purified by column chromatography (petroleum ether:ethyl acetate = 80:20) to give 0.19 g of intermediate **12-5.**

¹H-NMR (500 MHz, DMSO-*d*₆): δ 7.43-7.38 (m, 1H), 7.33-7.27 (m, 1H), 7.17 (d, J = 8.0 Hz, 1H), 4.52 (s, 4H), 3.21 (s, 2H), 3.15 (s, 2H).

### Step 5: Synthesis of intermediate 12-6

Intermediate **A-1** (0.1 g), dichloromethane (1 mL), and a 4 N solution of hydrochloric acid in dioxane (1 mL) were mixed, and the mixture was left to react at room temperature for 1 h. The stirring was stopped, and the mixture was concentrated under reduced pressure to give 0.1 g of intermediate **12-6.**

MS (ESI, [M+H]⁺) m/z: 342.19.

### Step 6: Synthesis of intermediate 12-7

Intermediate **12-6** (0.25 g), *N,N*-dimethylformamide (4 mL), intermediate **B-1** (0.251 g), *N,N*-diisopropylethylamine (0.358 g), and *O*-(7-azabenzotriazol-1-yl)-*N*,*N*,*N*',*N*'-tetramethyluronium hexafluorophosphate (0.253 g) were mixed, and the mixture was left to react overnight at room temperature. The reaction solution was poured into water (50 mL) and the mixture was extracted with ethyl acetate (30 mL × 3). The organic phases were combined, washed with saturated brine, dried over anhydrous sodium sulfate, filtered, concentrated, and separated and purified by column chromatography (dichloromethane:methanol = 95:5) to give 0.2 g of intermediate **12-7.**

MS (ESI, [M+H]⁺) m/z: 735.44.

### Step 7: Synthesis of intermediate 12-8, compound 12-A, and compound 12-B

Intermediate **12-7** (0.2 g), N-methylpyrrolidinone (4 mL), **12-5** (0.130 g), potassium carbonate (0.113 g), (1*R*,2*R*)-(-)-*N*,*N*'-dimethyl-1,2-cyclohexanediamine (0.015 g), and copper(I) iodide (10.37 mg) were mixed, and the mixture was heated to 130 °C and left to react for 3 h under a nitrogen atmosphere. The reaction solution was poured into water (50 mL) and the mixture was extracted with ethyl acetate (50 mL × 3). The organic phases were combined, washed with saturated brine, dried over anhydrous sodium sulfate, filtered, and concentrated. The resulting crude product was mixed with silica gel and separated and purified by column chromatography (dichloromethane:methanol = 95:5) to give 80 mg of intermediate **12-8.** The above intermediate **12-8** was resolved by preparative chiral HPLC (chromatographic column: REGIS IB, 30 × 250 mm, 10 µm; mobile phase: *n*-hexane:dichloromethane:ethanol = 70:15:15; flow rate: 40 mL/min) to give compound **12-A** (29 mg) and compound **12-B** (25 mg).

Compound **12-A:** Rₜ = 2.22 min (UPCC conditions: chromatographic column: CHIRALPAK IB-3, 4.6 × 100 mm, 3 µm; mobile phase: carbon dioxide:ethanol:isopropanol = 40:30:30; flow rate: 2.0 mL/min; column temperature: 40 °C)

HRMS: (ESI, [M+H]⁺) m/z: 893.4172.

¹H-NMR (500 MHz, DMSO-d₆): *δ* 11.73 (s, 1H), 7.55-7.28 (m, 5H), 7.27-7.06 (m, 4H), 7.02-6.77 (m, 2H), 5.63-5.49 (m, 1H), 4.61-4.50 (m, 4H), 4.43-4.34 (m, 1H), 3.77-3.57 (m, 3H), 3.27-3.11 (m, 5H), 3.08-3.00 (m, 1H), 2.95-2.84 (m, 1H), 2.26-2.14 (m, 6H), 1.87-1.44 (m, 8H), 1.40-1.35 (m, 2H), 1.29-1.27 (m, 3H), 1.25-1.17 (m, 6H).

Compound **12-B:** Rₜ = 1.76 min (UPCC conditions: chromatographic column: CHIRALPAK IB-3, 4.6 × 100 mm, 3 µm; mobile phase: carbon dioxide:ethanol:isopropanol = 40:30:30; flow rate: 2.0 mL/min; column temperature: 40 °C)

HRMS: (ESI, [M+H]⁺) m/z: 893.4167.

¹H-NMR (500 MHz, DMSO-*d₆*): δ 11.73 (s, 1H), 7.58-7.28 (m, 5H), 7.27-7.07 (m, 4H), 6.99-6.75 (m, 2H), 5.62-5.52 (m, 1H), 4.61-4.50 (m, 4H), 4.45-4.33 (m, 1H), 3.79-3.56 (m, 3H), 3.27-3.12 (m, 5H), 3.07-2.97 (m, 1H), 2.92-2.83 (m, 1H), 2.26-2.16 (m, 6H), 1.81-1.46 (m, 8H), 1.40-1.35 (m, 2H), 1.29-1.26 (m, 3H), 1.24-1.15 (m, 6H).

### Example 13

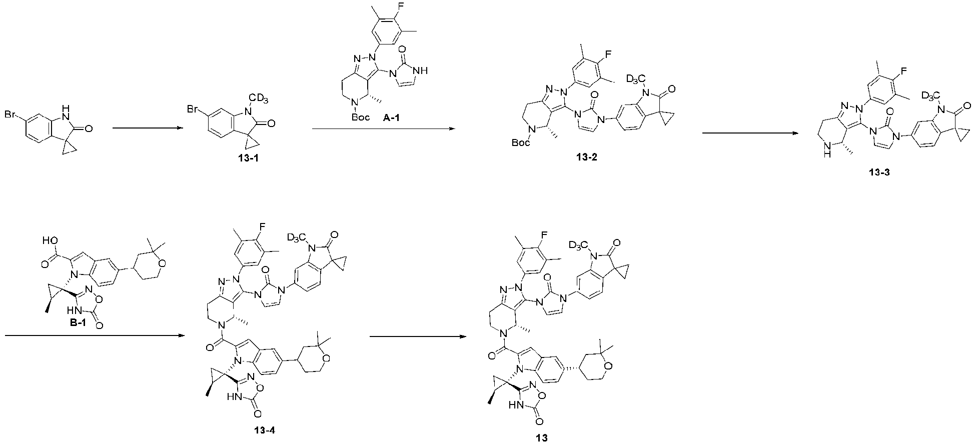

### Step 1: Synthesis of intermediate 13-1

6'-Bromo-1',2'-dihydrospiro[cyclopropane-l,3'-indol]-2'-one (0.5 g) and N,N-dimethylformamide (5 mL) were mixed, and the mixture was cooled in an ice-water bath, and sodium hydride (0.13 g, 60% dispersion in mineral oil) was added in portions. After the addition, the ice bath was removed, and the mixture was naturally warmed to room temperature and stirred for 10 min. Then the mixture was cooled in an ice-water bath, and deuterated iodomethane (0.46 g) was added. After the addition, the ice bath was removed, and the mixture was stirred at room temperature for 2 h. The reaction solution was poured into a saturated ammonium chloride solution (50 mL), and ethyl acetate was added for extraction. The organic phases were combined, washed with saturated brine, dried over anhydrous sodium sulfate, filtered under vacuum, and concentrated under reduced pressure, and the resulting crude product was separated and purified by column chromatography (petroleum ether:ethyl acetate = 5:1) to give 0.53 g of intermediate **13-1.**

¹H-NMR (500 MHz, DMSO-*d*₆): *δ* 7.31 (d, *J =* 1.8 Hz, 1H), 7.18 (dd, *J =* 7.9, 1.8 Hz, 1H), 6.98 (d, *J =* 7.9 Hz, 1H), 1.65-1.61 (m, 2H), 1.55-1.50 (m, 2H).

### Step 2: Synthesis of intermediate 13-2

Intermediate **A-1** (0.2 g), *N*-methylpyrrolidinone (4 mL), intermediate **13-2** (0.19 g), (1*R*,2*R*)-(-)-*N,N*-dimethyl-1,2-cyclohexanediamine (0.032 g), potassium carbonate (0.19 g), and copper(I) iodide (0.017 g) were mixed, and the mixture was heated to 130 °C and left to react for 2 h under a nitrogen atmosphere. The reaction solution was poured into water (50 mL) and the mixture was extracted with ethyl acetate. The organic phases were combined, washed with saturated brine, dried over anhydrous sodium sulfate, filtered under vacuum, and concentrated under reduced pressure. The resulting crude product was separated and purified by column chromatography (dichloromethane:methanol = 95:5) to give 0.13 g of intermediate **13-2.**

MS (ESI, [M+H]⁺) m/z: 616.42.

¹H-NMR (500 MHz, DMSO-*d*₆): *δ* 7.35 (s, 1H), 7.30 (d, *J =* 3.4 Hz, 1H), 7.27 (dd, *J =* 8.1, 2.0 Hz, 1H), 7.14-7.07 (m, 3H), 6.97 (s, 1H), 5.14 (s, 1H), 4.21 (s, 1H), 3.14 (s, 1H), 2.80-2.70 (m, 2H), 2.19 (s, 6H), 1.66-1.60 (m, 2H), 1.56-1.51 (m, 2H), 1.44 (s, 9H), 1.22-1.16 (m, 3H).

### Step 3: Synthesis of intermediate 13-3

Intermediate **13-2** (0.13 g), dichloromethane (2 mL), and a 4 N solution of hydrochloric acid in dioxane (2 mL) were mixed, and the mixture was stirred at room temperature for 1.5 h. The reaction solution was concentrated under reduced pressure to give 0.11 g of intermediate **13-3.**

MS (ESI, [M+H]⁺) m/z: 516.36.

### Step 4: Synthesis of intermediate 13-4 and compound 13

Intermediate **B-1** (0.08 g), *N,N*-dimethylformamide (2 mL), *O*-(7-azabenzotriazol-1-yl)-*N*,*N*,*N*',*N*'-tetramethyluronium hexafluorophosphate (0.22 g), and triethylamine (0.39 g) were mixed, and the mixture was stirred at room temperature for 5 min. Then a solution of intermediate **1-3** (0.12 g) in *N,N*-dimethylformamide (1 mL) was added. After the addition, the mixture was stirred at room temperature overnight. The reaction solution was poured into water (50 mL) and the mixture was extracted with ethyl acetate. The organic phases were combined, washed with saturated brine, dried over anhydrous sodium sulfate, filtered under vacuum, and concentrated under reduced pressure. The resulting crude product was separated and purified by column chromatography (dichloromethane:methanol = 95:5) to give 80 mg of intermediate **13-4.** Intermediate **13-4** was resolved by preparative chiral HPLC (chromatographic column: REGIS IB, 30 × 250 mm, 10 µm; mobile phase: *n*-hexane-ethanol (80:20):dichloromethane = 1:1; flow rate: 40 mL/min) to give compound **13** (38 mg).

Compound **13:** Rt = 2.077 min (UPCC conditions: chromatographic column: CHIRALPAK IB-3, 4.6 × 100 mm, 3 µm; mobile phase: carbon dioxide:methanol = 30:70; flow rate: 2.0 mL/min; column temperature: 40 °C)

HRMS: (ESI, [M+H]⁺) m/z: 909.4308.

¹H-NMR (500 MHz, DMSO-*d₆*): *δ* 11.73 (s, 1H), 7.57-7.46 (m, 1H), 7.43-7.23 (m, 5H), 7.20-7.06 (m, 3H), 7.04-6.77 (m, 2H), 5.77-5.53 (m, 1H), 4.46-4.32 (m, 1H), 3.80-3.54 (m, 3H), 3.24-3.12 (m, 1H), 3.08-2.96 (m, 1H), 2.95-2.85 (m, 1H), 2.26-2.15 (m, 6H), 1.82-1.45 (m, 12H), 1.43-1.36 (m, 2H), 1.35-1.30 (m, 2H), 1.29-1.25 (m, 4H), 1.19-1.15 (m, 3H).

### Example 14

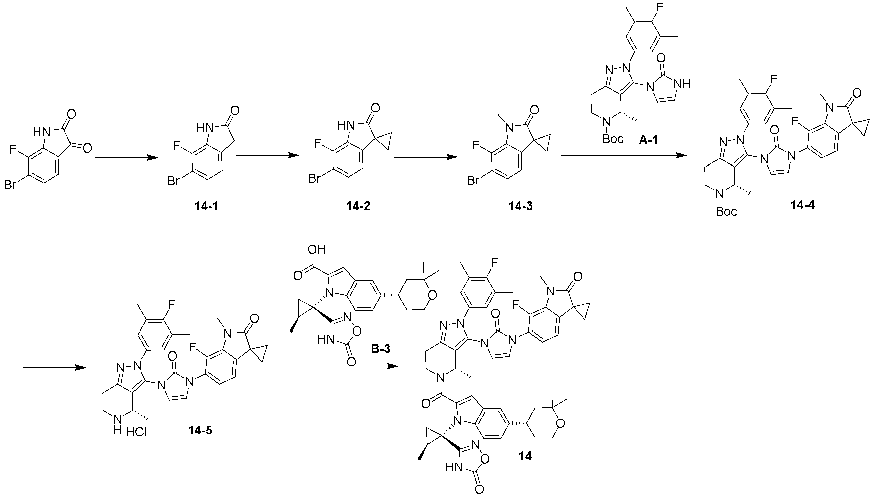

### Step 1: Synthesis of intermediate 14-1

6-Bromo-7-fluoroindoline-2,3-dione (8.4 g), ethanol (150 mL), and 85% hydrazine hydrate solution (1.5 mL) were mixed, and the mixture was heated to 85 °C and left to react for 1 h under a nitrogen atmosphere. After the reaction was completed, the reaction solution was cooled to room temperature and filtered. The filter cake, ethanol (150 mL), and potassium *tert*-butoxide (12 g) were mixed, and the mixture was heated to 85 °C and left to react for 2 h under a nitrogen atmosphere. After the reaction was completed, the reaction solution was poured into water, and a 2 N aqueous hydrochloric acid solution (50 mL) was added to adjust the pH to 2-3. The mixture was filtered, and the filter cake was dried to give 4.2 g of intermediate **14-1.**

MS (ESI, [M-H]⁻) m/z: 227.95.

¹H-NMR (500 MHz, DMSO-*d*₆): *δ* 11.03 (s, 1H), 7.24-7.19 (m, 1H), 7.01 (d, *J =* 7.5 Hz, 1H), 3.55 (s, 2H).

### Step 2: Synthesis of intermediate 14-2

At -40 °C, a 2 N solution of lithium diisopropylamide in tetrahydrofuran (8.69 mL) was added to a mixture of intermediate **14-1** (1 g) and tetrahydrofuran (20 mL), and the mixture was stirred for 30 min. 1,2-Dibromoethane (2.45 g) was added to the reaction solution, and the reaction solution was left to react at room temperature for 12 h. A saturated ammonium chloride solution was added to the reaction solution to quench the reaction, and the mixture was extracted with ethyl acetate. The organic phase was dried and concentrated, and the residue was separated and purified by column chromatography (petroleum ether:ethyl acetate = 90:10) to give 0.27 g of intermediate **14-2.**

¹H-NMR (500 MHz, DMSO-d₆): *δ* 11.24 (s, 1H), 7.24-7.19 (m, 1H), 6.81 (d, *J =* 8.0 Hz, 1H), 1.66-1.64 (m, 2H), 1.56-1.52 (m, 2H).

### Step 3: Synthesis of intermediate 14-3

Intermediate **14-2** (0.27 g), N,N-dimethylformamide (5 mL), iodomethane (0.19 g), and cesium carbonate (0.68 g) were mixed, and the mixture was heated to 80 °C and left to react for 1 h under a nitrogen atmosphere. After the reaction was completed, the reaction solution was poured into water, and ethyl acetate (50 mL) was added for extraction. The organic phase was washed with saturated brine, dried over anhydrous sodium sulfate, and filtered under vacuum. The filtrate was concentrated under reduced pressure, and the residue was separated and purified by silica gel column chromatography (petroleum ether:ethyl acetate = 90:10) to give 0.13 g of intermediate **14-3.**

¹H-NMR (500 MHz, DMSO-*d*₆): *δ* 7.29-7.26 (m, 1H), 6.85 (d, *J =* 8.0 Hz, 1H), 3.38 (d, *J =* 3.0 Hz, 3H), 1.70-1.68 (m, 2H), 1.59-1.57 (m, 2H).

### Step 4: Synthesis of intermediate 14-4

Intermediate **A-1** (200 mg), intermediate **14-3** (0.13 g), (1*S*,2*S*)-*N*1,*N*2-dimethylcyclohexane-1,2-diamine (32 mg), potassium carbonate (188 mg), copper(I) iodide (17.25 mg), and *N*-methylpyrrolidone (5 mL) were mixed, and the mixture was left to react at 100 °C for 12 h under a nitrogen atmosphere. After the reaction was completed, the reaction solution was poured into water, and ethyl acetate (50 mL) was added for extraction. The organic phase was washed with saturated brine, dried over anhydrous sodium sulfate, and filtered under vacuum. The filtrate was concentrated under reduced pressure, and the residue was separated and purified by silica gel column chromatography (dichloromethane:methanol = 20: 1) to give 0.14 g of intermediate **14-4.**

MS (ESI, [M+H]⁺) m/z: 631.39.

¹H-NMR (500 MHz, DMSO-d₆): *δ* 7.12 (d, *J =* 6.5 Hz, 2H), 7.08-7.04 (m, 1H), 6.99-6.97 (m, 2H), 6.92 (s, 1H), 5.19-5.04 (m, 1H), 4.39-4.14 (m, 1H), 3.40 (d, *J =* 2.5 Hz, 3H), 3.23-3.06 (m, 1H), 2.75-2.69 (m, 1H), 2.67-2.62 (m, 1H), 2.23 (s, 6H), 1.72-1.70 (m, 2H), 1.62-1.59 (m, 2H), 1.44 (s, 9H), 1.21 (d, *J =* 7.0 Hz, 3H).

### Step 5: Synthesis of intermediate 14-5

Intermediate **14-4** (0.14 g), dichloromethane (2 mL), and a 4 N solution of hydrochloric acid in dioxane (2 mL) were mixed, and the mixture was stirred at room temperature for 1 h. After the reaction was completed, the reaction solution was directly concentrated under reduced pressure to give 0.11 g of intermediate **14-5.**

MS (ESI, [M+H]⁺) m/z: 531.31.

### Step 6: Synthesis of compound 14

Intermediate **B-3** (0.07 g), *N,N*-dimethylformamide (4 mL), triethylamine (0.47 mL), and *O*-(7-azabenzotriazol-1-yl)-*N,N,N,N*-tetramethyluronium hexafluorophosphate (0.19 g) were mixed. The mixture was stirred at room temperature for 5 min, and then intermediate **14-5** (0.1 g) was added thereto. The resulting mixture was stirred at room temperature for 5 h. After the reaction was completed, the reaction solution was poured into water, and ethyl acetate (50 mL) was added for extraction. The organic phase was washed with saturated brine, dried over anhydrous sodium sulfate, and filtered under vacuum. The filtrate was concentrated under reduced pressure, and the residue was separated and purified by silica gel column chromatography (dichloromethane:methanol = 20: 1) to give 0.048 g of compound **14.**

HRMS: (ESI, [M+H]⁺) m/z: 924.4014.

¹H-NMR (500 MHz, DMSO-*d*₆) *δ* 11.73 (s, 1H), 7.53 (s, 1H), 7.42-7.39 (m, 1H), 7.37-7.36 (m, 1H), 7.29-7.24 (m, 1H), 7.19-7.15 (m, 2H), 7.12-7.09 (m, 1H), 7.01 (s, 1H), 6.94 (s, 2H), 5.59-5.52 (m, 1H), 4.42-4.34 (m, 1H), 3.72-3.70 (m, 2H), 3.41 (s, 3H), 3.25-3.14 (m, 1H), 3.07-2.99 (m, 1H), 2.93-2.85 (m, 1H), 2.75-2.70(m, 1H), 2.25 (s, 6H), 1.75-1.71 (m, 2H), 1.69-1.64 (m, 2H), 1.64-1.60 (m, 2H), 1.52-1.49 (m, 2H), 1.43-1.38 (m, 3H), 1.34 (s, 3H), 1.30 (s, 3H), 1.24 (s, 3H), 1.18 (s, 3H).

### Example 15

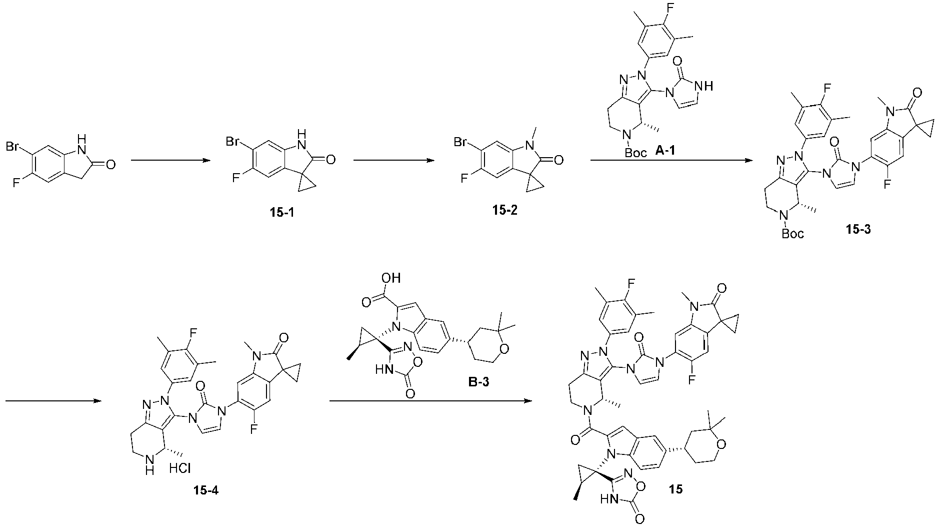

### Step 1: Synthesis of intermediate 15-1

At -60 °C, a 2 N solution of lithium diisopropylamide in tetrahydrofuran (15.7 mL) was added to a mixture of 6-bromo-5-fluoroindoline-2-one (1.8 g) and tetrahydrofuran (6 mL), and the mixture was stirred for 30 min. 1,2-Dibromoethane (4.41 g) was added to the reaction solution, and the reaction solution was left to react at room temperature for 12 h. A saturated ammonium chloride solution was added to the reaction solution to quench the reaction, and the mixture was extracted with ethyl acetate. The organic phase was dried and concentrated, and the residue was separated and purified by silica gel column chromatography (petroleum ether:ethyl acetate = 3:2) to give 0.72 g of intermediate **15-1.**

MS (ESI, [M-H]⁻) m/z: 253.96.

¹H-NMR (500 MHz, DMSO-d₆): *δ* 10.64 (s, 1H), 7.16-7.12 (m, 1H), 7.10-7.07 (m, 1H), 1.67-1.62 (m, 2H), 1.54-1.49 (m, 2H).

### Step 2: Synthesis of intermediate 15-2

Intermediate **15-1** (0.7 g) and *N,N*-dimethylformamide (15 mL) were mixed, and sodium hydride (0.33 g, 60% dispersion in mineral oil) was added in an ice bath. The mixture was stirred for 30 min, and then iodomethane (1.94 g) was added dropwise to the reaction solution. The reaction mixture was transferred to room temperature and stirred for 2 h. After the reaction was completed, the reaction solution was poured into a saturated aqueous ammonium chloride solution (100 mL), and ethyl acetate (100 mL) was added for extraction. The organic phase was washed with saturated brine, dried over anhydrous sodium sulfate, and filtered under vacuum. The filtrate was concentrated under reduced pressure, and the residue was separated and purified by silica gel column chromatography (petroleum ether:ethyl acetate = 1:4) to give 0.40 g of intermediate **15-2.**

MS (ESI, [M+H]⁺) m/z: 269.87.

¹H-NMR (500 MHz, DMSO-*d*₆): *δ* 7.41 (d, *J* = 6.0 Hz, 1H), 7.20 (d, *J* = 8.5 Hz, 1H), 3.20 (s, 3H), 1.73-1.66 (m, 2H), 1.59-1.53 (m, 2H).

### Step 3: Synthesis of intermediate 15-3

Intermediate **A-1** (400 mg), intermediate **15-2** (367 mg), (1*S*,2*S*)-*N*1,*N*2-dimethylcyclohexane-1,2-diamine (90 mg), potassium carbonate (376 mg), copper(I) iodide (50 mg), and *N*-methylpyrrolidone (10 mL) were mixed, and the mixture was left to react at 100 °C for 12 h under a nitrogen atmosphere. After the reaction was completed, the reaction solution was poured into water, and ethyl acetate (50 mL) was added for extraction. The organic phase was washed with saturated brine, dried over anhydrous sodium sulfate, and filtered under vacuum. The filtrate was concentrated under reduced pressure, and the residue was separated and purified by silica gel column chromatography (petroleum ether:ethyl acetate = 3:2) to give 0.52 g of intermediate **15-3.**

MS (ESI, [M+H]⁺) m/z: 631.43.

¹H-NMR (500 MHz, DMSO-*d*₆): *δ* 7.24 (d, *J =* 9.5 Hz, 1H), 7.22-7.19 (m, 1H), 7.12 (d, *J =* 6.5 Hz, 2H), 6.98 (d, *J =* 3.0 Hz, 1H), 6.91 (s, 1H), 5.19-5.03 (m, 1H), 4.37-4.14 (m, 1H), 3.22 (s, 3H), 3.17-3.07 (m, 1H), 2.76-2.70 (m, 1H), 2.69-2.62 (m, 1H), 2.22 (s, 6H), 1.74-1.70 (m, 2H), 1.60-1.55 (m, 2H), 1.44 (s, 9H), 1.22 (d, *J =* 6.0 Hz, 3H).

### Step 4: Synthesis of intermediate 15-4

Intermediate **15-3** (0.25 g), dichloromethane (5 mL), and a 4 N solution of hydrochloric acid in dioxane (5 mL) were mixed, and the mixture was stirred at room temperature for 1 h. After the reaction was completed, the reaction solution was directly concentrated under reduced pressure to give 0.22 g of intermediate **15-4.**

MS (ESI, [M+H]⁺) m/z: 531.32.

### Step 5: Synthesis of compound 15

Intermediate **B-3** (0.07 g), *N,N*-dimethylformamide (3 mL), triethylamine (0.47 mL), and *O*-(7-azabenzotriazol-1-yl)-*N,N,N,N*-tetramethyluronium hexafluorophosphate (0.2 g) were mixed. The mixture was stirred at room temperature for 5 min, and then intermediate **15-4** (0.1 g) was added thereto. The resulting mixture was stirred at room temperature for 5 h. After the reaction was completed, the reaction solution was poured into water, and ethyl acetate (50 mL) was added for extraction. The organic phase was washed with saturated brine, dried over anhydrous sodium sulfate, and filtered under vacuum. The filtrate was concentrated under reduced pressure, and the residue was separated and purified by silica gel column chromatography (dichloromethane:methanol = 20: 1) to give 0.06 g of compound **15.**

HRMS: (ESI, [M+H]⁺) m/z: 924.4020.

¹H-NMR (500 MHz, DMSO-d₆): *δ* 11.77 (s, 1H), 7.52 (s, 1H), 7.41-7.40 (m, 1H), 7.28-7.26 (m, 2H), 7.24-7.20 (m, 3H), 7.03-6.99 (m, 1H), 6.93-6.89 (m, 2H), 5.61-5.51 (m, 1H), 4.45-4.34 (m, 1H), 3.76-3.71 (m, 2H), 3.23 (s, 3H), 3.19-3.13 (m, 2H), 3.04-3.00 (m, 1H), 2.91-2.84 (m, 1H), 2.24 (s, 6H), 1.76-1.70 (m, 4H), 1.60-1.56 (m, 4H), 1.46-1.40 (m, 3H), 1.30-1.26 (m, 6H), 1.20-1.16 (m, 6H).

### Example 16

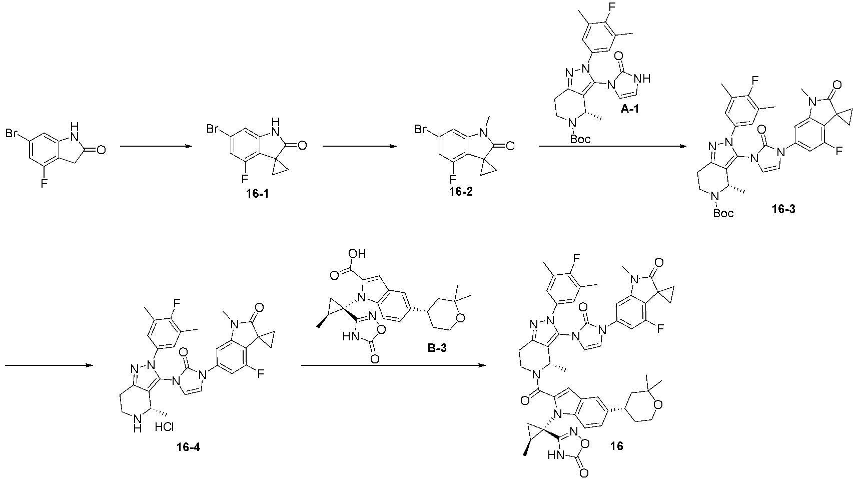

### Step 1: Synthesis of intermediate 16-1

At -78 °C, a 2 N solution of lithium diisopropylamide in tetrahydrofuran (9 mL) was added to a mixed solution of 6-bromo-4-fluorodihydro-2-one (1 g) and tetrahydrofuran (5 mL), and the mixture was stirred for 30 min with the temperature maintained. A solution of 1,2-dibromoethane (2.44 g) in tetrahydrofuran (5 mL) was added dropwise thereto, and after the dropwise addition, the mixture was slowly warmed to room temperature and stirred overnight. The reaction was stopped, and the reaction solution was poured into a saturated ammonium chloride solution (100 mL). The resulting solution was extracted with ethyl acetate (50 mL). The organic phase was washed with saturated brine, dried over anhydrous sodium sulfate, filtered under vacuum, concentrated under reduced pressure, and separated and purified by column chromatography (petroleum ether:ethyl acetate = 1:1) to give 0.54 g of intermediate **16-1.**

MS (ESI, [M-H]⁻) m/z: 253.91.

¹H-NMR (500 MHz, DMSO-d₆): *δ* 10.91 (s, 1H), 7.07 (dd, *J =* 9.3, 1.5 Hz, 1H), 6.93 (d, *J =* 1.5 Hz, 1H), 1.78 (q, *J* = 4.0 Hz, 2H), 1.48 (q, *J =* 3.9 Hz, 2H).

### Step 2: Synthesis of intermediate 16-2

Intermediate **16-1** (540 mg) and *N,N*-dimethylformamide (11 mL) were mixed, and sodium hydride (132 mg, 60% dispersion in mineral oil) was added in an ice bath. The mixture was stirred for 30 min, and then iodomethane (935 mg) was added dropwise to the reaction solution. The reaction mixture was transferred to room temperature and stirred for 2 h. The reaction solution was poured into a mixed system of ethyl acetate (50 mL) and water (50 mL). The aqueous phase was extracted with ethyl acetate (2 × 50 mL). The organic phases were combined, then washed with saturated brine, dried over anhydrous sodium sulfate, filtered under vacuum, and concentrated under reduced pressure to give 500 mg of intermediate **16-2.**

MS (ESI, [M+H]⁺) m/z: 269.98.

¹H-NMR (500 MHz, DMSO-*d*₆): *δ* 7.25 (d, *J =* 1.7 Hz, 1H), 7.15 (dd, *J =* 9.3, 1.5 Hz, 1H), 3.21 (s, 3H), 1.82 (q, *J =* 4.0 Hz, 2H), 1.53 (q, *J =* 4.0 Hz, 2H).

### Step 3: Synthesis of intermediate 16-3

Intermediate **A-1** (500 mg), intermediate **16-2** (500 mg), (1*S*,2*S*)-*N*1,*N*2-dimethylcyclohexane-1,2-diamine (100 mg), potassium carbonate (300 mg), copper(I) iodide (70 mg), and *N*-methylpyrrolidone (10 mL) were mixed, and the mixture was stirred in microwave at 150 °C for 1 h under a nitrogen atmosphere. The reaction solution was then cooled to room temperature and poured into water (50 mL), and the resulting solution was extracted with ethyl acetate (50 mL). The organic phase was washed with water and saturated brine in sequence, dried over anhydrous sodium sulfate, concentrated, and separated and purified by column chromatography (petroleum ether:ethyl acetate = 1:1) to give 0.4 g of intermediate **16-3.**

MS (ESI, [M+H]⁺) m/z: 631.44.

¹H-NMR (500 MHz, DMSO-d₆): *δ* 7.42 (d, *J =* 3.4 Hz, 1H), 7.36-7.28 (m, 2H), 7.11 (d, *J =* 6.3 Hz, 2H), 7.03 (s, 1H), 5.14 (s, 1H), 4.22 (s, 1H), 3.23 (s, 3H), 3.19-3.02 (m, 1H), 2.67 (m, 1H), 2.19 (s, 6H), 1.82 (m, 2H), 1.53 (q, *J=* 3.9 Hz, 2H), 1.44 (s, 9H), 1.18 (t, *J =* 7.2 Hz, 4H).

### Step 4: Synthesis of intermediate 16-4

Intermediate **16-3** (0.4 g), dichloromethane (2 mL), and a 4 N solution of hydrochloric acid in dioxane (4 mL) were mixed, and the mixture was stirred at room temperature for 1 h. The stirring was stopped, and the mixture was concentrated under reduced pressure to give 0.2 g of intermediate **16-4.**

MS (ESI, [M+H]⁺) m/z: 531.32.

### Step 5: Synthesis of compound 16

Intermediate **B-3** (0.15 g), *N,N*-dimethylformamide (4 mL), triethylamine (0.72 g), and *O*-(7-azabenzotriazol-1-yl)-*N,N,N,N*-tetramethyluronium hexafluorophosphate (0.4 g) were mixed, and the mixture was stirred for 5 min. Then intermediate **16-4** (0.2 g) was added. The mixture was stirred at 30 °C overnight. The reaction solution was poured into water (50 mL), and the resulting solution was extracted with ethyl acetate. The organic phase was washed with saturated brine, dried over anhydrous sodium sulfate, concentrated, and separated and purified by column chromatography (dichloromethane:methanol = 95:5) to give 80 mg of compound **16.**

HRMS: (ESI, [M+H]⁺) m/z: 924.4023.

¹H-NMR (500 MHz, DMSO-d₆): *δ* 11.73 (s, 1H), 7.50 (d, *J =* 35.6 Hz, 2H), 7.43-7.23 (m, 5H), 7.16 (d, *J =* 6.1 Hz, 2H), 6.95 (s, 1H), 5.58 (s, 1H), 4.39 (d, *J =* 10.2 Hz, 1H), 3.71 (d, *J =* 8.2 Hz, 2H), 3.24 (s, 4H), 3.04 (d, *J =* 10.2 Hz, 2H), 2.89 (d, *J =* 14.8 Hz, 1H), 2.25 (s, 6H), 1.82 (d, *J =* 7.6 Hz, 2H), 1.69 (d, *J =* 12.5 Hz, 5H), 1.58 (d, *J =* 31.6 Hz, 7H), 1.39 (d, *J =* 6.4 Hz, 3H), 1.21-1.10 (m, 6H).

### Example 17

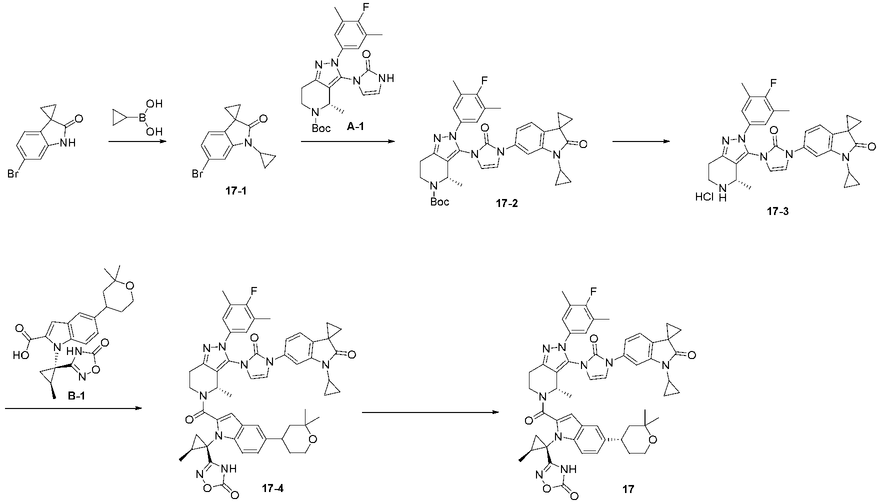

### Step 1: Synthesis of intermediate 17-1

6'-Bromo-1',2'-dihydrospiro[cyclopropane-1,3'-indol]-2'-one (0.72 g), copper acetate (0.80 g), 4-dimethylaminopyridine (1.54 g), and toluene (20 mL) were mixed, and then a 2 N solution of sodium bis(trimethylsilyl)amide in tetrahydrofuran (2.21 mL) was added dropwise to the reaction solution. The reaction solution was stirred at 95 °C for 16 h and then naturally cooled to room temperature. The reaction solution was poured into a mixed system of ethyl acetate (30 mL) and water (70 mL), and the two phases were separated. The aqueous phase was extracted with ethyl acetate (30 mL × 2). The organic phases were combined, then washed with a saturated sodium chloride solution, dried over anhydrous sodium sulfate, filtered under vacuum, and concentrated under reduced pressure, and the resulting crude product was separated and purified by column chromatography (petroleum ether:ethyl acetate = 90:10) to give 350 mg of intermediate **17-1.**

¹H-NMR (500 MHz, DMSO-d₆): *δ* 7.28 (d, *J =* 1.8 Hz, 1H), 7.18 (dd, *J =* 7.9, 1.8 Hz, 1H), 6.95 (d, *J =* 7.9 Hz, 1H), 2.72 (tt, *J =* 7.2, 3.8 Hz, 1H), 1.58 (q, *J =* 4.0 Hz, 2H), 1.48 (q, *J =* 4.0 Hz, 2H), 1.03-0.98 (m, 2H), 0.82-0.77 (m, 2H).

### Step 2: Synthesis of intermediate 17-2

Copper(I) iodide (43.1 mg), potassium carbonate (391 mg), (1*R*,2*R)*-(-)-*N,N'*-dimethyl-1,2-cyclohexanediamine (40.3 mg), intermediate **A-1** (250 mg), intermediate **17-1** (205 mg), and *N*-methylpyrrolidone (5 mL) were mixed. The reaction solution was stirred at 125 °C for 16 h under a nitrogen atmosphere and then naturally cooled to room temperature. The reaction solution was poured into a mixed system of ethyl acetate (20 mL) and water (30 mL), and the two phases were separated. The aqueous phase was extracted with (20 mL × 2). The organic phases were combined, then washed with a saturated sodium chloride solution, dried over anhydrous sodium sulfate, filtered under vacuum, and concentrated under reduced pressure, and the resulting crude product was separated and purified by column chromatography (petroleum ether:ethyl acetate = 80:20) to give 210 mg of intermediate **17-2.**

MS (ESI, [M+H]⁺) m/z: 639.48.

### Step 3: Synthesis of intermediate 17-3

Intermediate **17-2** (200 mg), dichloromethane (2 mL), and a 4 N solution of hydrochloric acid in dioxane (4 mL) were mixed, and the mixture was stirred at room temperature for 1.5 h and concentrated under reduced pressure to give 180 mg of intermediate **17-3.**

MS (ESI, [M+H]⁺) m/z: 539.35.

### Step 4: Synthesis of intermediate 17-4 and compound 17

Intermediate **B-1** (114 mg), *O*-(7-azabenzotriazol-1-yl)-*N*,*N*,*N*',*N*'-tetramethyluronium hexafluorophosphate (317 mg), triethylamine (563 mg), and *N,N*-dimethylformamide (3 mL) were mixed, and the mixture was stirred at room temperature for 10 min. Then intermediate **17-3** (160 mg) was added, and the mixture was stirred at room temperature for 16 h. The reaction solution was poured into water (50 mL), and the resulting solution was extracted with ethyl acetate (30 mL). The organic phase was washed with saturated brine, dried over anhydrous sodium sulfate, and concentrated. The resulting residue was separated and purified by column chromatography (dichloromethane:methanol = 95:5) to give 60 mg of intermediate **17-4.** Intermediate **17-4** was resolved by preparative chiral HPLC (chromatographic column: CHIRALART Cellulose-SB, 30 × 250 mm, 5 µm; mobile phase: ethanol/0.1% ammonium hydroxide:n-hexane = 30:70, isocratic elution) to give compound **17** (18 mg).

Compound **17:** Rₜ= 2.01 min (UPCC conditions: chromatographic column: CHIRALPAK IB, 4.6 × 100 mm, 3 µm; mobile phase: carbon dioxide:methanol/0.1% ammonium hydroxide = 30:70; flow rate: 0.5 mL/min; column temperature: 40 °C)

HRMS: (ESI, [M+H]⁺) m/z: 932.4291.

¹H NMR (500 MHz, DMSO-d₆): *δ* 11.76 (s, 1H), 7.51 (d, *J =* 17.9 Hz, 1H), 7.40 (dd, *J =* 8.6, 4.0 Hz, 1H), 7.35 (d, *J* = 17.7 Hz, 2H), 7.28-7.20 (m, 2H), 7.17 (d, *J =* 6.7 Hz, 2H), 7.09 (s, 1H), 6.98 (d, *J =* 28.8 Hz, 2H), 5.58 (d, *J =* 6.3 Hz, 1H), 4.38 (d*, J =* 11.9 Hz, 1H), 3.71 (d, *J =* 7.9 Hz, 2H), 3.51 (s, 1H), 3.18 (s, 1H), 3.03 (t, *J =* 12.1 Hz, 1H), 2.89 (d, *J =* 16.5 Hz, 1H), 2.75 (s, 1H), 2.21 (d, *J =* 16.5 Hz, 5H), 1.68 (s, 2H), 1.59 (d, *J =* 3.5 Hz, 2H), 1.50 (t, *J =* 3.7 Hz, 2H), 1.41 (d, *J* = 3.5 Hz, 2H), 1.34 (d, *J =* 7.8 Hz, 3H), 1.30 (s, 3H), 1.28 (s, 2H), 1.26 (s, 4H), 1.23 (s, 4H), 1.19 (d, *J =* 2.6 Hz, 4H).

### Example 18

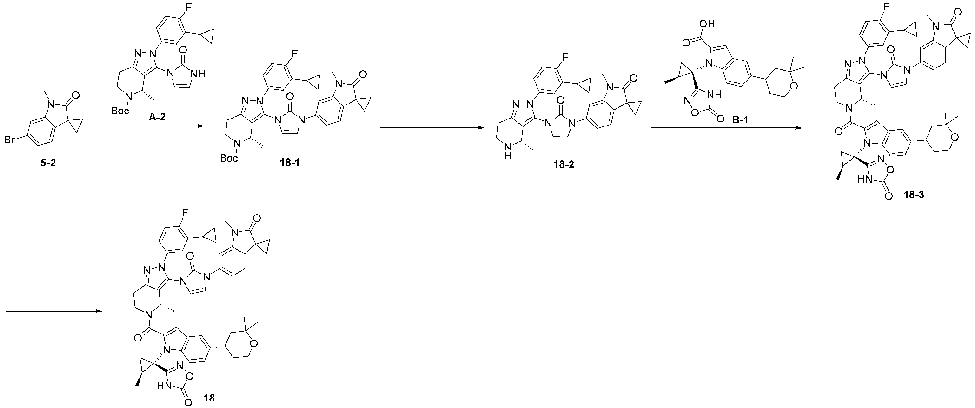

### Step 1: Synthesis of intermediate 18-1

Intermediate **A-2** (400 mg), intermediate **5-2** (320 mg), (1*S*,2*S*)-*N*¹,*N*²-dimethylcyclohexane-1,2-diamine (64 mg), potassium carbonate (400 mg), copper(I) iodide (38 mg), and *N*-methylpyrrolidone (8 mL) were mixed, and the mixture was left to react in microwave at 140 °C for 1 h under a nitrogen atmosphere. The reaction solution was then cooled to room temperature and poured into water (50 mL), and the resulting solution was extracted with ethyl acetate (50 mL). The organic phase was washed with water and saturated brine in sequence, dried over anhydrous sodium sulfate, concentrated, and separated and purified by column chromatography (petroleum ether:ethyl acetate = 1:1) to give 0.45 g of intermediate **18-1.**

MS (ESI, [M+H]⁺) m/z: 625.43.

¹H-NMR (500 MHz, DMSO-d₆): *δ* 7.37 (s, 1H), 7.34 (d, *J =* 3.2 Hz, 1H), 7.31-7.28 (m, 1H), 7.27-7.23 (m, 2H), 7.11 (d, *J =* 7.9 Hz, 1H), 6.98 (s, 1H), 6.87 (dd, *J =* 6.8, 2.5 Hz, 1H), 5.13 (s, 1H), 4.21 (s, 1H), 3.25 (s, 3H), 3.19-3.00 (m, 1H), 2.83-2.61 (m, 2H), 2.10-2.01 (m, 1H), 1.64 (q, *J =* 3.9 Hz, 2H), 1.53 (q, *J =* 3.7 Hz, 2H), 1.43 (s, 9H), 1.21-1.17 (m, 3H), 0.95 (m, 2H), 0.63-0.49 (m, 2H).

### Step 2: Synthesis of intermediate 18-2

Intermediate **18-1** (0.45 g), dichloromethane (2 mL), and a 4 N solution of hydrochloric acid in dioxane (10 mL) were mixed, and the mixture was stirred at room temperature for 1 h. The stirring was stopped, and the mixture was concentrated under reduced pressure to give 0.33 g of intermediate **18-2.**

MS (ESI, [M+H]⁺) m/z: 525.23.

¹H-NMR (500 MHz, DMSO-*d₆*): *δ* 7.36 (d, *J =* 2.0 Hz, 1H), 7.33 (d, *J =* 3.4 Hz, 1H), 7.29 (dd, *J =* 8.0, 2.1 Hz, 1H), 7.27-7.22 (m, 2H), 7.10 (d, *J =* 8.1 Hz, 1H), 6.95-6.89 (m, 1H), 6.89 -6.84 (m, 1H), 4.10 (s, 1H), 4.00 (q, *J =* 6.6 Hz, 1H), 3.27 (q, *J =* 3.9 Hz, 1H), 3.17 (s, 3H), 2.98 -2.85 (m, 1H), 2.77-2.60 (m, 2H), 2.11-1.97 (m, 1H), 1.63 (q, *J*= 4.0 Hz, 2H), 1.53 (q, *J =* 3.8 Hz, 2H), 1.13 (d, *J =* 6.6 Hz, 3H), 0.95 (dd, *J =* 8.4, 2.1 Hz, 2H), 0.67-0.49 (m, 2H).

### Step 3: Synthesis of intermediate 18-3 and compound 18

Intermediate **B-1** (0.155 g), *N,N*-dimethylformamide (4 mL), triethylamine (0.075 g), and *O*-(7-azabenzotriazol-1-yl)-*N,N,N,N*-tetramethyluronium hexafluorophosphate (0.18 g) were mixed, and the mixture was stirred for 5 min. Then intermediate **18-2** (0.16 g) was added. The mixture was stirred at 30 °C. The reaction solution was poured into water (50 mL), and the resulting solution was extracted with ethyl acetate. The organic phase was washed with saturated brine, dried over anhydrous sodium sulfate, concentrated, and separated and purified by column chromatography (dichloromethane:methanol = 95:5) to give intermediate **18-3.** The above intermediate **18-3** was resolved by preparative chiral HPLC (mobile phase A: ethanol-dichloromethane (1:1), B: *n*-hexane, mobile phase A:B = 30%:70%, flow rate: 40 mL/min; column temperature: 25 °C; chromatographic column: CHIRALART Cellulose-SB) to give 80 mg of compound **18.**

Compound **18:** Rt = 2.08 min (UPCC conditions: chromatographic column: CHIRALPAK IB-3 (4.6 × 100 mm, 3 µm); mobile phase: carbon dioxide:methanol (0.1% ammonium hydroxide) = 30:70; flow rate: 2 mL/min; column temperature: 40 °C)

HRMS: (ESI, [M+H]⁺) m/z: 918.4135.

¹H-NMR (500 MHz, DMSO-*d₆*): *δ* 11.74 (s, 1H), 7.53 (s, 1H), 7.42-7.35 (m, 3H), 7.29 (dq, *J =* 19.5, 9.0 Hz, 5H), 7.12 (d, *J=* 8.0 Hz, 1H), 7.03-6.89 (m, 3H), 5.65-5.49 (m, 1H), 4.47 -4.31 (m, 1H), 3.71 (d, *J=* 8.2 Hz, 3H), 3.66-3.54 (m, 1H), 3.24 (s, 3H), 3.22-3.13 (m, 1H), 3.03 (t, *J=* 12.2 Hz, 1H), 2.90 (d, *J=* 15.2 Hz, 1H), 2.83-2.66 (m, 1H), 2.13-1.95 (m, 2H), 1.70 -1.61 (m, 6H), 1.56-1.53 (m, 3H), 1.40 (d, *J=* 5.9 Hz, 2H), 1.20-1.13 (m, 6H), 0.99 (s, 3H), 0.60 (dd, *J =* 8.8, 4.1 Hz, 3H).

### Example 19

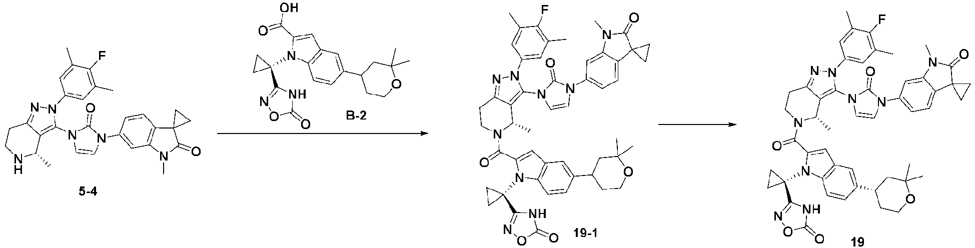

### Step 1: Synthesis of intermediate 19-1 and compound 19

Intermediate **B-2** (0.07 g) and *N,N*-dimethylformamide (2 mL) were mixed, and triethylamine (178 mg) and O-(7-azabenzotriazol-1-yl)-*N*,*N*,*N*',*N*'-tetramethyluronium hexafluorophosphate (134 mg) were added in sequence. The mixture was stirred for 10 min, and then intermediate **5-4** (0.09 g) was added. The resulting mixture was stirred at room temperature overnight. Water and ethyl acetate were added for dilution, and the mixture was extracted with ethyl acetate. The organic layer was washed with water and a saturated aqueous sodium chloride solution in sequence, dried over anhydrous sodium sulfate, and filtered. The filtrate was concentrated and separated and purified by column chromatography (dichloromethane:methanol = 98:2) to give 60 mg of intermediate **19-1.** The above intermediate **19-1** was resolved by preparative chiral HPLC (mobile phase: n-hexane:ethanol = 80:20; flow rate: 40 mL/min; column temperature: 25 °C; chromatographic column: YMC CHIRALART Cellulose-SB 30 × 250) to give 20 mg of compound **19.**

Compound **19:** Rt = 3.55 min (UPCC conditions: chromatographic column: CHIRALPAK IB-3 (4.6 × 100 mm, 3 µm); mobile phase: carbon dioxide:methanol (0.1% ammonium hydroxide) = 60:40; flow rate: 2 mL/min; column temperature: 40 °C)

HRMS: (ESI, [M+H]⁺) m/z: 892.3960.

¹H-NMR (500 MHz, DMSO-*d*₆): *δ* 12.08 (s, 1H), 7.48 (d, *J =* 10.0 Hz, 1H), 7.40 (d, *J =* 8.7 Hz, 1H), 7.38-7.35 (m, 1H), 7.34-7.32 (m, 1H), 7.29 (d, *J =* 7.9 Hz, 1H), 7.26 (dd, *J =* 8.6, 2.5 Hz, 1H), 7.22 (d, *J =* 8.5 Hz, 1H), 7.17-7.10 (m, 2H), 6.91-6.89 (m, 1H), 6.70 (s, 1H), 3.72 (d, *J =* 8.9 Hz, 2H), 3.24 (s, 3H), 3.08-3.02 (m, 1H), 2.86-2.79 (m, 1H), 2.72 (t, *J =* 8.0 Hz,1H), 2.55-2.52 (m, 1H), 2.19 (d, *J =* 15.4 Hz, 6H), 1.65-1.62 (m, 2H), 1.53-1.51 (m, 2H), 1.36-1.35 (m, 3H), 1.34-1.33 (m, 2H), 1.31-1.29 (m, 3H), 1.28-1.27 (m, 3H), 1.26-1.25 (m, 2H), 1.24-1.23 (m, 2H), 1.23-1.22 (m, 2H), 1.18 (d, *J =* 4.4 Hz, 2H).

### Example 20

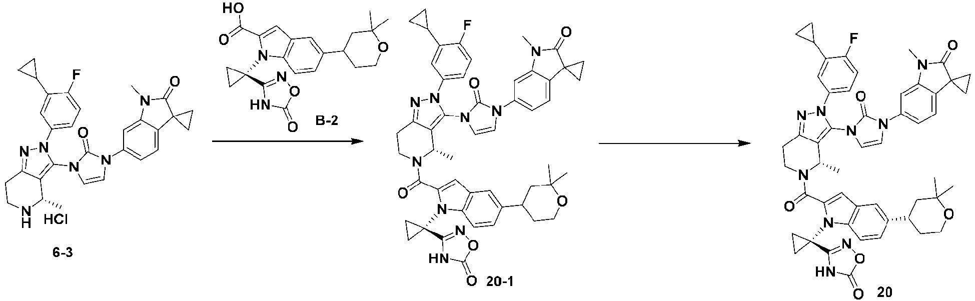

### Step 1: Synthesis of intermediate 20-1 and compound 20

Intermediate **B-2** (0.083 g), *N,N*-dimethylformamide (2 mL), triethylamine (0.265 mL), and *O*-(7-azabenzotriazol-1-yl)-*N,N,N,N*-tetramethyluronium hexafluorophosphate (0.109 g) were mixed. The mixture was stirred at room temperature for 5 min, and then intermediate **6-3** (0.10 g) was added thereto. The resulting mixture was stirred at room temperature for 5 h. After the reaction was completed, the reaction solution was poured into water, and ethyl acetate (50 mL) was added for extraction. The organic phase was washed with saturated brine, dried over anhydrous sodium sulfate, and filtered under vacuum. The filtrate was concentrated under reduced pressure, and the residue was separated and purified by silica gel column chromatography (dichloromethane:methanol = 20: 1) to give 0.12 g of intermediate **20-1.** The above intermediate **20-1** was resolved by preparative chiral HPLC (chromatographic column: Pre-packed REGIS IB, 30 × 250 mm, 10 µm; mobile phase: ethanol-dichloromethane (1:1):*n*-hexane = 30:70; flow rate: 40 mL/min) to give 33 mg of compound **20.**

Compound **20:** Rt = 7.47 min (UPCC conditions: chromatographic column: CHIRALPAK IB-3 (4.6 × 100 mm, 3 µm); mobile phase: carbon dioxide:methanol (containing 0.1% ammonium hydroxide) = 30:70; flow rate: 2.0 mL/min; column temperature: 40 °C)

HRMS: (ESI, [M+H]⁺) m/z: 904.3973.

¹H-NMR (500 MHz, DMSO-d₆): *δ* 12.09 (s, 1H), 7.52-7.43 (m, 2H), 7.42-7.35 (m, 2H), 7.34-7.18 (m, 4H), 7.15-7.09 (m, 1H), 7.03-6.97 (m, 1H), 6.93-6.87 (m, 1H), 6.85-6.65 (m, 1H), 3.78-3.64 (m, 2H), 3.24 (s, 1H), 3.04 (s, 1H), 2.91-2.77 (m, 1H), 2.10-1.96 (m, 1H), 1.82 (s, 2H), 1.75-1.58 (m, 6H), 1.57-1.45 (m, 4H), 1.37-1.34 (m, 3H), 1.31-1.27 (m, 5H), 1.26 (s, 3H), 1.20-1.17 (m, 2H), 1.02-0.91 (m, 2H), 0.90-0.79 (m, 2H), 0.66-0.50 (m, 2H).

### Example 21

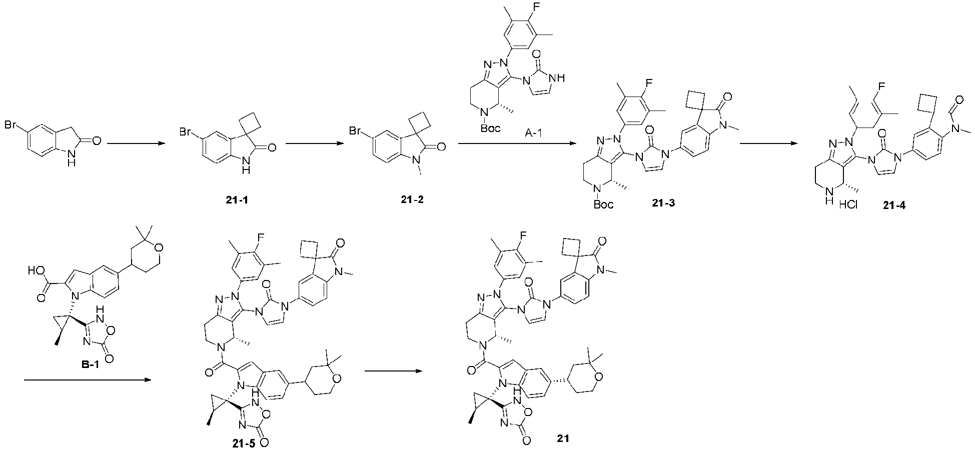

### Step 1: Synthesis of intermediate 21-1

At -5 °C, a 2 N solution of lithium diisopropylamide in tetrahydrofuran (46.7 mL) was added to a mixture of 5-bromo-2-hydroxyindole (5 g) and tetrahydrofuran (50 mL), and the mixture was stirred for 30 min. 1,3-Dibromopropane (14.28 g) was added to the reaction solution, and the reaction solution was left to react at room temperature for 12 h. A saturated ammonium chloride solution was added to the reaction solution to quench the reaction, and the mixture was extracted with ethyl acetate. The organic phase was dried and concentrated, and the residue was separated and purified on a silica gel column (petroleum ether:ethyl acetate = 3:1) to give 1.9 g of intermediate **21-1.**

MS (ESI, [M+H]⁺) m/z: 251.98.

¹H NMR (500 MHz, DMSO-d₆): *δ* 10.34 (s, 1H), 7.75 (d, *J =* 2.1 Hz, 1H), 7.33 (dd, *J =* 8.2, 2.1 Hz, 1H), 6.74 (d, *J* = 8.1 Hz, 1H), 2.44-2.37 (m, 2H), 2.36-2.29 (m, 2H), 2.22-2.14 (m, 2H).

### Step 2: Synthesis of intermediate 21-2

Intermediate **21-1** (0.7 g) and *N,N-*dimethylformamide (10 mL) were mixed, and sodium hydride (0.14 g, 60% dispersion in mineral oil) was added in an ice bath. The mixture was stirred for 10 min, and then iodomethane (0.51 g) was added dropwise to the reaction solution. The reaction mixture was transferred to room temperature and stirred for 2 h. After the reaction was completed, the reaction solution was poured into a saturated aqueous ammonium chloride solution (100 mL), and ethyl acetate (100 mL) was added for extraction. The organic phase was washed with saturated brine, dried over anhydrous sodium sulfate, and filtered under vacuum. The filtrate was concentrated under reduced pressure, and the residue was separated and purified by silica gel column chromatography (petroleum ether:ethyl acetate = 88:12) to give 0.58 g of intermediate **21-2.**

MS (ESI, [M+H]⁺) m/z: 266.06.

¹H NMR (500 MHz, DMSO-*d*₆): *δ* 7.82 (d, *J =* 2.0 Hz, 1H), 7.45 (dd, *J =* 8.3, 2.0 Hz, 1H), 6.92 (d, *J =* 8.2 Hz, 1H), 3.09 (s, 3H), 2.46-2.39 (m, 2H), 2.38-2.31 (m, 2H), 2.25-2.17 (m, 2H).

### Step 3: Synthesis of intermediate 21-3

Intermediate **A-1** (220 mg), intermediate **21-2** (172 mg), (1*S*,2*S*)-*N*1,*N*2-dimethylcyclohexane-1,2-diamine (42.5 mg), potassium carbonate (207 mg), copper(I) iodide (28.5 mg), and N-methylpyrrolidone (6 mL) were mixed, and the mixture was left to react at 130 °C for 12 h under a nitrogen atmosphere. After the reaction was completed, the reaction solution was poured into water, and ethyl acetate (50 mL) was added for extraction. The organic phase was washed with saturated brine, dried over anhydrous sodium sulfate, and filtered under vacuum. The filtrate was concentrated under reduced pressure, and the residue was separated and purified by silica gel column chromatography (petroleum ether:ethyl acetate = 55:45) to give 0.26 g of intermediate **21-3.**

MS (ESI, [M+H]⁺) m/z: 627.45.

¹H NMR (500 MHz, DMSO-*d*₆): *δ* 7.81 (d, *J =* 2.2 Hz, 1H), 7.50 (dd, *J =* 8.4, 2.2 Hz, 1H), 7.27 (d, *J =* 3.2 Hz, 1H), 7.12 (d, *J =* 6.2 Hz, 2H), 7.04 (d, *J =* 8.4 Hz, 1H), 6.94 (s, 1H), 5.14 (s, 1H), 4.23 (s, 1H), 3.13 (s, 3H), 2.77-2.72 (m, 1H), 2.70 (s, 2H), 2.48-2.41 (m, 2H), 2.40-2.32 (m, 2H), 2.20 (d, *J =* 2.0 Hz, 6H), 2.17 (d, *J =* 8.2 Hz, 2H), 1.92-1.88 (m, 1H), 1.44 (s, 9H), 1.23-1.20 (m, 2H).

### Step 4: Synthesis of intermediate 21-4

Intermediate **21-3** (0.25 g), dichloromethane (5 mL), and a 4 N solution of hydrochloric acid in dioxane (5 mL) were mixed, and the mixture was stirred at room temperature for 1 h. After the reaction was completed, the reaction solution was directly concentrated under reduced pressure to give 0.21 g of intermediate **21-4.**

MS (ESI, [M+H]⁺) m/z: 527.35.

### Step 5: Synthesis of intermediate 21-5 and compound 21

Intermediate **B-1** (100 mg), *N,N*-dimethylformamide (5 mL), triethylamine (0.678 mL), and *O*-(7-azabenzotriazol-1-yl)-*N,N,N,N*-tetramethyluronium hexafluorophosphate (277 mg) were mixed. The mixture was stirred at room temperature for 5 min, and then intermediate **21-5** (151 mg) was added thereto. The resulting mixture was stirred at room temperature for 5 h. After the reaction was completed, the reaction solution was poured into water, and ethyl acetate (50 mL) was added for extraction. The organic phase was washed with saturated brine, dried over anhydrous sodium sulfate, and filtered under vacuum. The filtrate was concentrated under reduced pressure, and the residue was separated and purified by silica gel column chromatography (dichloromethane:methanol = 97:3) to give 0.18 g of intermediate **21-5.** The above intermediate **21-5** was resolved by preparative chiral HPLC (chromatographic column: CHIRAL ART Cellulose-SB, 30 × 250 mm, 5 µm; mobile phase: ethanol-dichloromethane (3:2):*n*-hexane = 27:73; flow rate: 40 mL/min) to give 60 mg of compound **21.**

Compound **21:** Rt = 2.78 min (UPCC conditions: chromatographic column: CHIRALPAK IB-3 (4.6 × 100 mm, 3 µm); mobile phase: carbon dioxide:methanol (containing 0.1% ammonium hydroxide) = 50:50; flow rate: 2.0 mL/min; column temperature: 40 °C)

HRMS: (ESI, [M+H]⁺) m/z: 920.4281.

¹H-NMR (500 MHz, DMSO-d₆): *δ* 11.74 (s, 1H), 7.83 (s, 1H), 7.57-7.50 (m, 2H), 7.42-7.38 (m, 1H), 7.31 (d, *J* = 3.2 Hz, 1H), 7.26 (d, *J =* 8.4 Hz, 1H), 7.19-7.15 (m, 2H), 7.05 (d, *J =* 8.4 Hz, 1H), 7.00-6.90 (m, 2H), 5.64-5.52 (m, 1H), 4.43-4.34 (m, 1H), 3.74-3.69 (m, 2H), 3.13 (s, 3H), 3.11-3.06 (m, 1H), 3.05-2.99 (m, 1H), 2.92-2.86 (m, 1H), 2.69 (s, 1H), 2.47-2.41 (m, 2H), 2.40-2.35 (m, 2H), 2.23 (s, 6H), 2.21-2.18 (m, 2H), 1.74-1.65 (m, 4H), 1.62-1.48 (m, 5H), 1.42 (d, *J =* 6.6 Hz, 2H), 1.34 (s, 2H), 1.30 (s, 2H), 1.18 (s, 4H).

### Example 22

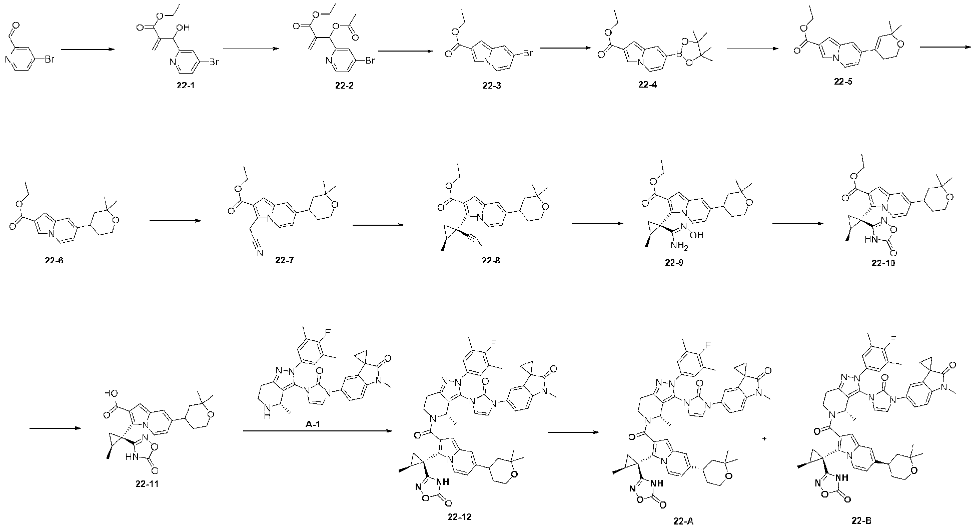

### Step 1: Synthesis of intermediate 22-1

4-Bromopyridine-2-carbaldehyde (25 g), ethyl acrylate (26.9 g), and triethylenediamine (9.8 g) were mixed, and the mixture was stirred at room temperature overnight and concentrated under reduced pressure. The resulting residue was separated and purified by column chromatography (petroleum ether:ethyl acetate = 80:20) to give 34.2 g of intermediate **22-1.**

MS (ESI, [M+H]⁺) m/z: 286.04.

¹H-NMR (500 MHz, DMSO-d₆): *δ* 8.35 (d, *J =* 5.3 Hz, 1H), 7.68 (d, *J =* 2.0 Hz, 1H), 7.54 (dd, *J =* 5.2, 2.0 Hz, 1H), 6.22 (s, 1H), 6.06 (d, *J =* 5.7 Hz, 1H), 5.90 (t, *J* = 1.5 Hz, 1H), 5.48 (d, *J =* 5.7 Hz, 1H), 4.16 - 3.99 (m, 2H), 1.10 (t, *J =* 7.1 Hz, 3H).

### Step 2: Synthesis of intermediate 22-2

Intermediate **22-1** (33.2 g) and dichloromethane (300 mL) were mixed and cooled in an ice-water bath. Pyridine (9.18 g) was added, and acetyl chloride (9.11 g) was slowly and dropwise added. After the addition, the mixture was stirred at room temperature for 2 h. The reaction solution was poured into water (200 mL), and the organic phase was separated, washed with saturated brine, dried over anhydrous sodium sulfate, filtered, and concentrated under reduced pressure to give 35 g of intermediate **22-2.**

MS (ESI, [M+H+2]⁺) m/z: 330.04.

¹H-NMR (500 MHz, DMSO-*d₆*): *δ* 8.42 (d, *J =* 5.3 Hz, 1H), 7.73 (d, *J =* 1.8 Hz, 1H), 7.65 (dd, *J =* 5.3, 1.9 Hz, 1H), 6.53 (s, 1H), 6.37 (s, 1H), 5.87 (s, 1H), 4.12-4.08 (m, 2H), 2.15 (s, 3H), 1.14 (t, *J =* 7.1 Hz, 3H).

### Step 3: Synthesis of intermediate 22-3

Intermediate **22-2** (39 g) and toluene (250 mL) were mixed, and the mixture was heated to 120 °C and left to react overnight. The heating was stopped, and the mixture was concentrated under reduced pressure. The residue was separated and purified by column chromatography (petroleum ether:ethyl acetate = 80:20) to give 6.4 g of intermediate **22-3.**

MS (ESI, [M+H+2]⁺) m/z: 270.05.

¹H-NMR (500 MHz, DMSO-*d₆*): *δ* 8.26 (d, *J =* 7.4 Hz, 1H), 8.13 (d, *J =* 1.6 Hz, 1H), 7.78 (d, *J =* 2.1 Hz, 1H), 6.78 (dd, *J =* 7.4, 2.1 Hz, 1H), 6.74 (s, 1H), 4.27 (q, *J =* 7.1 Hz, 2H), 1.30 (t, *J =* 7.1 Hz, 3H).

### Step 4: Synthesis of intermediate 22-4

Intermediate **22-3** (3.6 g), 1,4-dioxane (50 mL), bis(pinacolato)diboron (4.43 g), potassium acetate (2.64 g), and [1,1-bis(diphenylphosphino)ferrocene]dichloropalladium(II) (0.49 g) were mixed, and the mixture was heated to 90 °C and left to react overnight under a nitrogen atmosphere. The heating was stopped, and the reaction solution was cooled to room temperature, poured into water (100 mL), and extracted with ethyl acetate (50 mL × 3). The organic phases were combined, washed with saturated brine, dried over anhydrous sodium sulfate, filtered, and concentrated. The residue was separated and purified by column chromatography (petroleum ether:ethyl acetate = 80:20) to give 4.1 g of intermediate **22-4.**

MS (ESI, [M+H]⁺) m/z: 316.19.

¹H-NMR (500 MHz, DMSO-*d*₆): *δ* 8.24 (d, *J =* 7.0 Hz, 1H), 8.16 (d, *J =* 1.7 Hz, 1H), 7.88 (s, 1H), 6.94 (s, 1H), 6.74 (dd, *J* = 7.0, 1.2 Hz, 1H), 4.28 (q, *J =* 7.1 Hz, 2H), 1.32-1.28 (m, 15H).

### Step 5: Synthesis of intermediate 22-5

Intermediate **22-4** (4.1 g), 1,4-dioxane (2 mL), water (0.2 mL), a mixture of 6,6-dimethyl-3,6-dihydro-2*H*-pyran-4-yl trifluoromethanesulfonate and 2,2-dimethyl-3,6-dihydro-2*H*-pyran-4-yl trifluoromethanesulfonate (5.08 g), potassium carbonate (3.60 g), and [1,1-bis(diphenylphosphino)ferrocene]dichloropalladium(II) (0.48 g) were mixed, and the mixture was heated to 90 °C and left to react under a nitrogen atmosphere for 3 h. The reaction solution was poured into water (100 mL) and extracted with ethyl acetate (30 mL × 3). The organic phases were combined, washed with saturated brine, dried over anhydrous sodium sulfate, filtered, and concentrated. The residue was separated and purified by column chromatography (petroleum ether:ethyl acetate = 80:20) to give 3.6 g of intermediate **22-5.**

MS (ESI, [M+H]⁺) m/z: 300.19.

### Step 6: Synthesis of intermediate 22-6

Intermediate **22-5** (4.1 g) and methanol (80 mL) were mixed, 10% Pd/C (1.46 g) was added, and the mixture was stirred overnight under a hydrogen atmosphere. The mixture was filtered and concentrated, and the residue was separated and purified by column chromatography (petroleum ether:ethyl acetate = 80:20) to give 2.6 g of intermediate **22-6.**

MS (ESI, [M+H]⁺) m/z: 302.20.

¹H-NMR (500 MHz, DMSO-d₆): *δ* 8.21 (d, *J =* 7.3 Hz, 1H), 7.99 (d, *J=* 1.6 Hz, 1H), 7.26-7.20 (m, 1H), 6.62-6.61 (m, 2H), 4.25 (q, *J =* 7.0 Hz, 2H), 3.73-3.64 (m, 2H), 2.85 (tt, *J =* 12.4, 3.6 Hz, 1H), 1.72-1.65 (m, 2H), 1.55-1.39 (m, 2H), 1.29 (t, *J =* 7.1 Hz, 3H), 1.24 (s, 3H), 1.18 (s, 3H).

### Step 7: Synthesis of intermediate 22-7

Intermediate **22-6** (2.35 g), dimethyl sulfoxide (40 mL), iodoacetonitrile (3.91 g), and ferrous sulfate heptahydrate (1.08 g) were mixed, and the mixture was cooled in an ice-water bath, before hydrogen peroxide (4.78 mL) was added dropwise thereto. After the addition, the mixture was stirred for another 2 h. The reaction solution was poured into a mixture of a saturated sodium sulfite solution (100 mL) and ethyl acetate (100 mL). The phases were separated, and the organic phase was washed with saturated brine, dried over anhydrous sodium sulfate, filtered, and concentrated. The resulting residue was separated and purified by column chromatography (petroleum ether:ethyl acetate = 80:20) to give 1 g of intermediate **22-7.**

MS (ESI, [M+H]⁺) m/z: 341.27.

¹H-NMR (500 MHz, DMSO-d₆): *δ* 8.26 (d, *J=* 7.4 Hz, 1H), 7.35 (s, 1H), 6.85 (dd, *J =* 7.4, 1.9 Hz, 1H), 6.76 (s, 1H), 4.66 (s, 2H), 4.31 (q, *J =* 7.1 Hz, 2H), 3.76-3.67 (m, 2H), 2.92 (tt, *J =* 12.5, 3.6 Hz, 1H), 1.74-1.65 (m, 2H), 1.57-1.42 (m, 2H), 1.33 (t, *J =* 7.1 Hz, 3H), 1.26 (s, 3H), 1.19 (s, 3H).

### Step 8: Synthesis of intermediate 22-8

Intermediate **22-7** (0.31 g), tetrahydrofuran (6 mL), (4*R*)-4-methyl-1,3,2-dioxolane 2,2-dioxide (0.38 g), and *N,N-*dimethylpropyleneurea (0.23 g) were mixed, and the mixture was cooled to 0 °C in an ice-brine bath under a nitrogen atmosphere. A 1 N solution of lithium bis(trimethylsilyl)amide in tetrahydrofuran (5.46 mL) was then slowly and dropwise added. After the addition, the mixture was stirred at 0 °C for 1 h. The reaction solution was poured into a saturated ammonium chloride solution (100 mL) and extracted with ethyl acetate (20 mL × 3). The organic phases were separately washed with saturated brine, dried over anhydrous sodium sulfate, filtered, and concentrated. The resulting residue was separated and purified by column chromatography (petroleum ether:ethyl acetate = 3:1) to give 0.51 g of an isomer. The above resulting isomer was subjected to preparative HPLC (device: YMC high pressure preparative chromatograph; chromatographic column: Ultimate XB-C18, 30 × 250 mm, 10 µm; mobile phase: wateracetonitrile (35:65)) to give 0.19 g of intermediate **22-8.**

**Compound 22-8:** Rt = 2.73 min (UPLC conditions: chromatographic column: Waters UPLC BEH C18 (50 × 2.1 mm, 1.7 µm), mobile phase: 10 mM ammonium acetate buffer (containing 0.1% glacial acetic acid)-acetonitrile (50:50):acetonitrile = 10:90, flow rate: 0.6 mL/min, column temperature: 40 °C)

MS (ESI, [M+H]⁺) m/z: 381.34.

¹H-NMR (500 MHz, DMSO-*d₆*): *δ* 8.16 (d, *J =* 6.8 Hz, 1H), 7.37 (s, 1H), 6.87 (dd, *J =* 7.4, 1.8 Hz, 1H), 6.75 (s, 1H), 4.37-4.26 (m, 2H), 3.77-3.65 (m, 2H), 2.94 (tt, *J =* 12.3, 3.6 Hz, 1H), 1.70-1.67 (m, 4H), 1.61-1.42 (m, 6H), 1.34 (t, *J =* 7.1 Hz, 3H), 1.26 (s, 3H), 1.19 (s, 3H).

### Step 9: Synthesis of intermediate 22-9

Intermediate **22-8** (0.73 g), dimethyl sulfoxide (5 mL), hydroxylamine hydrochloride (0.67 g), and sodium bicarbonate (0.81 g) were mixed, and the mixture was heated to 65 °C and left to react overnight. The heating was stopped, and the reaction solution was cooled to room temperature, poured into water (50 mL), and extracted with ethyl acetate (20 mL × 3). The organic phases were combined, washed with saturated brine, dried over anhydrous sodium sulfate, filtered, and concentrated to give 0.72 g of intermediate **22-9.**

MS (ESI, [M+H]⁺) m/z: 414.42.

### Step 10: Synthesis of intermediate 22-10

Intermediate **22-9** (0.72 g), dimethyl sulfoxide (7 mL), N,N'-carbonyldiimidazole (0.57 g), and 1,8-diazabicyclo[5.4.0]undec-7-ene (0.66 g) were mixed, and the mixture was stirred at room temperature for 3 h. The stirring was stopped, and the reaction solution was poured into water (50 mL) and extracted with ethyl acetate (20 mL). The aqueous phase was adjusted to pH 6-7 with 2 N hydrochloric acid solution and extracted with ethyl acetate (20 mL × 2). The organic phases were combined, washed with saturated brine, dried over anhydrous sodium sulfate, filtered, and concentrated to give 0.6 g of intermediate **22-10.**

MS (ESI, [M+H]⁺) m/z: 440.31.

### Step 11: Synthesis of intermediate 22-11

Intermediate **22-10** (0.6 g), tetrahydrofuran (6 mL), water (1 mL), and potassium hydroxide (0.31 g) were mixed, and the mixture was heated to 70 °C and left to react for 8 h. The heating was stopped, and the reaction solution was cooled to room temperature, poured into water (80 mL), and extracted with ethyl acetate (20 mL). The aqueous phase was collected, adjusted to pH 4-5 with 2 N hydrochloric acid solution, and extracted with ethyl acetate (30 mL × 3). The organic phases were combined, washed with saturated brine, dried over anhydrous sodium sulfate, filtered, and concentrated to give 0.35 g of intermediate **22-11.**

MS (ESI, [M+H]⁺) m/z: 412.34.

¹H-NMR (500 MHz, DMSO-*d*₆): *δ* 12.58-11.62 (m, 2H), 8.06-7.92 (m, 1H), 7.36-7.23 (m, 1H), 6.79-6.73 (m, 1H), 6.72-6.63 (m, 1H), 3.75-3.64 (m, 2H), 2.96-2.85 (m, 1H), 1.94-1.78 (m, 1H), 1.76-1.63 (m, 3H), 1.57-1.36 (m, 3H), 1.25 (s, 6H), 1.18 (s, 3H).

### Step 12: Synthesis of intermediate 22-12, compound 22-A, and compound 22-B

Intermediate **22-11** (0.1 g), intermediate **A-1** (0.15 g), *N,N*-dimethylformamide (3 mL), triethylamine (0.49 g), and *O*-(7-azabenzotriazol-1-yl)-*N*,*N*,*N*',*N*'-tetramethyluronium hexafluorophosphate (0.28 g) were mixed, and after the addition, the mixture was stirred at room temperature for 4 h. The stirring was stopped, and the reaction solution was poured into water (50 mL) and extracted with ethyl acetate (20 mL × 3). The organic phases were combined, washed with saturated brine, dried over anhydrous sodium sulfate, filtered, and concentrated, and the resulting residue was separated and purified by column chromatography (dichloromethane:methanol = 95:5) to give 65 mg of intermediate **22-12.** The above intermediate 22-12 was resolved by preparative chiral HPLC (chromatographic column: REGIS IB, 30 × 250 mm, 10 µm; mobile phase: carbon dioxide:ethanol (containing 0.1% ammonium hydroxide) = 65:35; flow rate: 80 mL/min) to give compound **22-A** (25 mg) and compound **22-B** (22 mg).

**Compound 22-A:** Rt = 2.06 min (UPCC condition: chromatographic column: CHIRALPAK IB-3, 4.6 × 100 mm, 3 µm; mobile phase: carbon dioxide:ethanol:isopropanol (containing 0.1% ammonium hydroxide) = 50:25:25; flow rate: 2.0 mL/min; column temperature: 40 °C)

HRMS: (ESI, [M+H]⁺) m/z: 906.4132.

¹H-NMR (500 MHz, DMSO-*d*₆): *δ* 11.91 (s, 1H), 8.09-8.00 (m, 1H), 7.58-7.48 (m, 1H), 7.36-7.23 (m, 2H), 7.22-7.03 (m, 4H), 6.98-6.43 (m, 3H), 5.63 (q, *J =* 6.6 Hz, 1H), 4.16-4.03 (m, 1H), 3.78-3.64 (m, 2H), 3.52-3.41 (m, 1H), 3.26-3.19 (m, 3H), 3.02-2.83 (m, 2H), 2.80-2.69 (m, 1H), 2.25-2.13 (m, 6H), 1.76-1.63 (m, 4H), 1.60-1.49 (m, 5H), 1.38-1.34 (m, 3H), 1.27-1.23 (m, 5H), 1.19 (s, 3H), 1.17-1.12 (m, 3H).

**Compound 22-B:** Rt = 1.74 min (UPCC condition: chromatographic column: CHIRALPAK IB-3, 4.6 × 100 mm, 3 µm; mobile phase: carbon dioxide:ethanol:isopropanol (containing 0.1% ammonium hydroxide) = 50:25:25; flow rate: 2.0 mL/min; column temperature: 40 °C)

HRMS: (ESI, [M+H]⁺) m/z: 906.4122.

¹H-NMR (500 MHz, DMSO-d₆): *δ* 11.95 (s, 1H), 8.10-8.00 (m, 1H), 7.57-7.50 (m, 1H), 7.37-7.24 (m, 2H), 7.22-6.92 (m, 5H), 6.82-6.41 (m, 2H), 5.63 (q, *J =* 6.7 Hz, 1H), 4.19-3.99 (m, 1H), 3.79-3.62 (m, 2H), 3.54-3.40 (m, 1H), 3.27-3.19 (m, 3H), 3.03-2.84 (m, 2H), 2.83-2.71 (m, 1H), 2.26-2.15 (m, 6H), 1.76-1.63 (m, 4H), 1.61-1.51 (m, 5H), 1.40-1.35 (m, 3H), 1.28-1.21 (m, 5H), 1.19 (s, 3H), 1.17-1.11 (m, 3H).

### Example 23

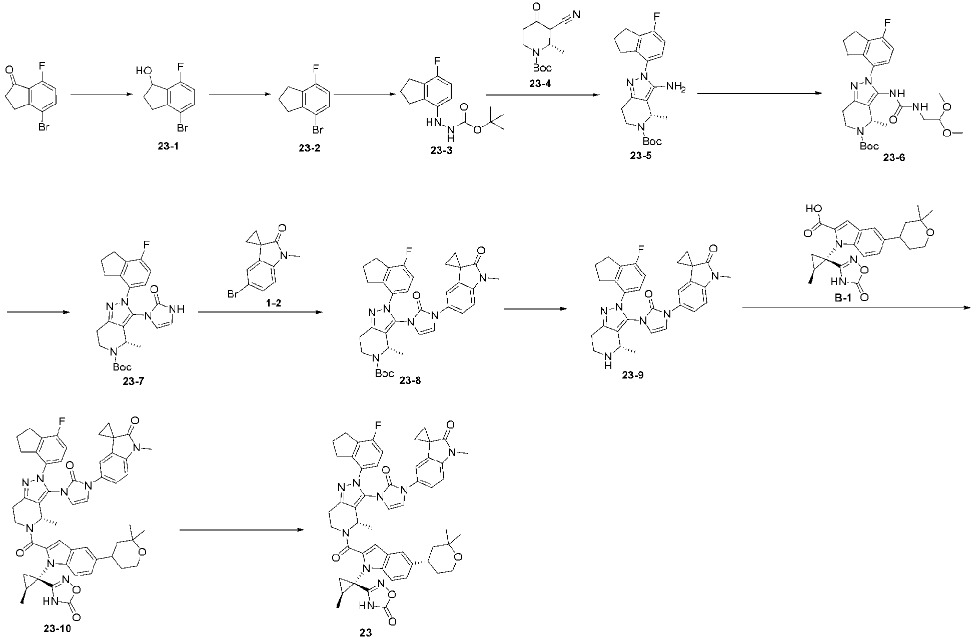

### Step 1: Synthesis of intermediate 23-1

4-Bromo-7-fluoroindanone (1 g) and methanol (10 mL) were mixed, and the mixture was cooled in an ice-water bath, before sodium borohydride (0.330 g) was added in portions. After the addition, the ice-water bath was removed, and the mixture was stirred at room temperature for 2 h. The reaction solution was poured into water (100 mL) and stirred, and a large amount of solid was precipitated. The mixture was filtered, and the filter cake was collected and dried under vacuum to give 0.7 g of intermediate **23-1.**

¹H-NMR (500 MHz, DMSO-*d*₆): *δ* 7.49 (dd, *J =* 8.6, 4.4 Hz, 1H), 7.04-6.96 (m, 1H), 5.43-5.38 (m, 1H), 5.36-5.29 (m, 1H), 3.06-2.95 (m, 1H), 2.81-2.66 (m, 1H), 2.38-2.25 (m, 1H), 1.96-1.85 (m, 1H).

### Step 2: Synthesis of intermediate 23-2

Intermediate **23-1** (5 g) and dichloromethane (60 mL) were mixed, and the mixture was cooled to -78 °C under a nitrogen atmosphere, before triethylsilane (7.55 g) and a solution of boron trifluoride diethyl etherate (8.23 mL) were dropwise added. After the addition, the mixture was naturally warmed to room temperature and stirred overnight. The reaction solution was poured into a saturated sodium bicarbonate solution (100 mL) and extracted with ethyl acetate (40 mL × 3). The organic phase was separately washed with a saturated sodium bicarbonate solution and saturated brine, dried over anhydrous sodium sulfate, filtered, and concentrated. The resulting crude product was separated and purified by column chromatography (petroleum ether:ethyl acetate = 100:0) to give 4.3 g of intermediate **23-2.**

¹H-NMR (500 MHz, DMSO-d₆): *δ* 7.40-7.34 (m, 1H), 7.00-6.94 (m, 1H), 3.01 (t, *J =* 7.6 Hz, 2H), 2.90 (t, *J =* 7.6 Hz, 2H), 2.09 (p, *J =* 7.6 Hz, 2H).

### Step 3: Synthesis of intermediate 23-3

Intermediate **23-2** (2 g), *tert*-butyl carbazate (2.46), toluene (10 mL), sodium tert-butoxide (1.79 g), 5-di-*tert-*butylphosphine-1',3',5'-triphenyl-1'*H*-[1,4']bipyrazole (0.94 g), and palladium(π-cinnamyl) chloride dimer (0.24 g) were mixed, and the mixture was heated to 115 °C and left to react under a nitrogen atmosphere for 5 h. The heating was stopped, and the reaction solution was cooled to room temperature, poured into water (100 mL), and extracted with ethyl acetate (50 mL × 3). The organic phase was washed with saturated brine, dried over anhydrous sodium sulfate, filtered, and concentrated. The resulting crude product was separated and purified by column chromatography (petroleum ether:ethyl acetate = 80:20) to give 1.7 g of intermediate **23-3.**

¹H-NMR (500 MHz, DMSO-d₆): *δ* 8.75 (s, 1H), 7.02 (s, 1H), 6.77 (t, *J =* 8.8 Hz, 1H), 6.52-6.26 (m, 1H), 2.82 (t, *J* = 7.5 Hz, 2H), 2.73 (t, *J =* 7.5 Hz, 2H), 2.03 (p, *J =* 7.6 Hz, 2H), 1.41 (s, 9H).

### Step 4: Synthesis of intermediate 23-5

Intermediate **23-3** (1.7 g), dichloromethane (20 mL), and trifluoroacetic acid (8.85 mL) were mixed, and the mixture was stirred at room temperature for 1 h and concentrated under reduced pressure. The resulting residue was redissolved in ethanol (20 mL), and then intermediate **23-4** (1.52 g) and pyridine hydrochloride (0.074 g) were added. The mixture was heated to 85 °C and stirred for 3 h. The heating was stopped, and the reaction solution was cooled to room temperature, poured into water (100 mL), and extracted with ethyl acetate (40 mL × 3). The organic phase was separately washed with a saturated sodium bicarbonate solution and saturated brine, dried over anhydrous sodium sulfate, filtered, and concentrated. The resulting crude product was separated and purified by column chromatography (petroleum ether:ethyl acetate = 60:40) to give 2.1 g of intermediate **23-5.**

MS (ESI, [M+H]⁺) m/z: 387.33.

¹H-NMR (500 MHz, DMSO-*d*₆): δ 7.13 (dd, J = 8.6, 4.7 Hz, 1H), 7.04 (t, *J =* 8.5 Hz, 1H), 5.17-4.91 (m, 3H), 4.21-3.98 (m, 1H), 3.13-2.90 (m, 3H), 2.90-2.81 (m, 1H), 2.80-2.69 (m, 1H), 2.48-2.40 (m, 2H), 2.10-2.00 (m, 2H), 1.43 (s, 9H), 1.27-1.20 (m, 3H).

### Step 5: Synthesis of intermediate 23-6

Intermediate **23-5** (2.0 g) and tetrahydrofuran (60 mL) were mixed, and the mixture was cooled in an ice-water bath, before triethylamine (2.62 g) and triphosgene (1.55 g) were added. After the addition, the mixture was naturally warmed to room temperature and stirred for 1 h. The mixture was cooled in an ice-water bath before aminoacetaldehyde dimethyl acetal (2.72 g) was added thereto, and again naturally warmed to room temperature and stirred for 2 h. The reaction solution was poured into water (100 mL), and the mixture was extracted with ethyl acetate (40 mL × 3). The organic phase was separately washed with a saturated sodium bicarbonate solution and saturated brine, dried over anhydrous sodium sulfate, filtered, and concentrated. The resulting crude product was separated and purified by column chromatography (petroleum ether:ethyl acetate = 60:40) to give 1.38 g of intermediate **23-6.**

MS (ESI, [M+H]⁺) m/z: 518.4.

### Step 6: Synthesis of intermediate 23-7

Intermediate **23-6** (1.38 g), tetrahydrofuran (30 mL), and methanesulfonic acid (0.13 g) were mixed, and the mixture was heated to 60 °C and left to react for 6 h. The reaction solution was poured into water (50 mL) and the mixture was extracted with ethyl acetate (50 mL × 3). The organic phases were combined, washed with saturated brine, dried over anhydrous sodium sulfate, filtered, and concentrated. The resulting crude product was separated and purified by column chromatography (dichloromethane:methanol = 95:5) to give 0.9 g of intermediate **23-7.**

MS (ESI, [M+H]⁺) m/z: 454.38.

### Step 7: Synthesis of intermediate 23-8

Intermediate **23-7** (0.4 g), intermediate **1-2** (0.33 g), *N*-methylpyrrolidone (4 mL), potassium carbonate (0.37 g), (1R,2R)-(-)-N,N'-dimethyl-1,2-cyclohexanediamine (0.063 g), and copper(I) iodide (0.067 g) were mixed, and the mixture was heated to 130 °C and left to react for 3 h under a nitrogen atmosphere. The reaction solution was poured into water (50 mL) and the mixture was extracted with ethyl acetate (50 mL × 3). The organic phases were combined, washed with saturated brine, dried over anhydrous sodium sulfate, filtered, and concentrated. The resulting crude product was separated and purified by column chromatography (dichloromethane:methanol = 95:5) to give 0.56 g of intermediate **23-8.**

MS (ESI, [M+H]⁺) m/z: 625.42.

### Step 8: Synthesis of intermediate 23-9

Intermediate **23-8** (0.56 g), dichloromethane (5 mL), and a 4 N solution of hydrochloric acid in dioxane (4 mL) were mixed. The mixture was stirred at room temperature for 2 h and concentrated under reduced pressure to give 0.56 g of intermediate **23-9.**

MS (ESI, [M+H]⁺) m/z: 525.35.

### Step 9: Synthesis of intermediate 23-10 and compound 23

Intermediate **B-1** (0.2 g), *N,N*-dimethylformamide (5 mL), *O*-(7-azabenzotriazol-1-yl)-*N,N,N',N'-*tetramethyluronium hexafluorophosphate (0.55 g), and triethylamine (0.98 g) were mixed, and the mixture was stirred at room temperature for 5 min. Then a solution of intermediate **23-9** (0.31 g) in *N,N*-dimethylformamide (1 mL) was added. After the addition, the mixture was stirred at room temperature overnight. The reaction solution was poured into water (50 mL) and the mixture was extracted with ethyl acetate (20 mL × 3). The organic phases were combined, washed with saturated brine, dried over anhydrous sodium sulfate, filtered, and concentrated. The resulting crude product was separated and purified by column chromatography (dichloromethane:methanol = 95:5) to give 65 mg of intermediate **23-10.** The above intermediate **23-10** was resolved by preparative chiral HPLC (chromatographic column: REGIS IB, 30 × 250 mm, 10 µm; mobile phase: *n*-hexane-ethanol (70:30):dichloromethane = 2:1; flow rate: 40 mL/min) to give compound **23** (23 mg).

Compound **23:** Rt = 3.31 min (UPCC conditions: chromatographic column: CHIRALPAK IB-3, 4.6 × 100 mm, 3 µm; mobile phase: carbon dioxide:methanol (containing 0.1% ammonium hydroxide) = 50:50; flow rate: 2.0 mL/min; column temperature: 40 °C)

HRMS: (ESI, [M+H]⁺) m/z: 918.4122.

¹H-NMR (500 MHz, DMSO-*d*₆): *δ* 11.74 (s, 1H), 7.59-7.22 (m, 5H), 7.20-7.04 (m, 3H), 7.02-6.93 (m, 1H), 6.92-6.65 (m, 2H), 5.66-5.52 (m, 1H), 4.44-4.31 (m, 1H), 3.81-3.58 (m, 3H), 3.26-3.16 (m, 3H), 3.08-2.99 (m, 1H), 2.97-2.88 (m, 2H), 2.88-2.81 (m, 2H), 2.76-2.69 (m, 1H), 2.59-2.53 (m, 1H), 2.10-1.89 (m, 3H), 1.83-1.44 (m, 11H), 1.39-1.37 (m, 1H), 1.34-1.33 (m, 2H), 1.25-1.23 (m, 4H), 1.20-1.17 (m, 2H), 1.16-1.12 (m, 2H).

### Example 24

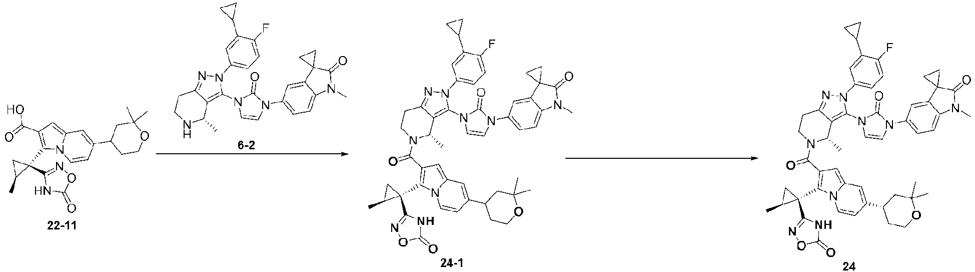

Intermediate **22-11** (0.1 g), intermediate **6-2** (0.15 g), *N,N*-dimethylformamide (3 mL), triethylamine (0.49 g), and *O*-(7-azabenzotriazol-1-yl)-*N*,*N*,*N*',*N*'-tetramethyluronium hexafluorophosphate (0.28 g) were mixed, and after the addition, the mixture was stirred at room temperature overnight. The stirring was stopped, and the reaction solution was poured into water (50 mL) and extracted with ethyl acetate (20 mL × 3). The organic phases were combined, washed with saturated brine, dried over anhydrous sodium sulfate, filtered, and concentrated, and the resulting residue was separated and purified by column chromatography (dichloromethane:methanol = 95:5) to give 65 mg of intermediate **24-1.** The above intermediate **24-1** was resolved by preparative chiral HPLC (chromatographic column: REGIS IB, 30 × 250 mm, 10 µm; mobile phase: *n*-hexane:ethanol = 56:44; flow rate: 40 mL/min) to give compound **24** (21 mg).

Compound **24:** Rt = 1.28 min (UPCC conditions: chromatographic column: CHIRALPAK IB-3, 4.6 × 100 mm, 3 µm; mobile phase: carbon dioxide:ethanol:isopropanol (containing 0.1% ammonium hydroxide) = 30:35:35; flow rate: 2.0 mL/min; column temperature: 40 °C)

HRMS: (ESI, [M+H]⁺) m/z: 918.4085.

¹H-NMR (500 MHz, DMSO-*d*₆): *δ* 11.97 (s, 1H), 8.13-7.97 (m, 1H), 7.60-7.51 (m, 1H), 7.36-7.09 (m, 6H), 7.07-6.50 (m, 4H), 5.78-5.54 (m, 1H), 4.31-4.05 (m, 1H), 3.83-3.65 (m, 2H), 3.53-3.41 (m, 1H), 3.27-3.18 (m, 3H), 3.04-2.88 (m, 2H), 2.85-2.73 (m, 1H), 2.10-1.98 (m, 1H), 1.72-1.64 (m, 3H), 1.61-1.51 (m, 5H), 1.39-1.33 (m, 3H), 1.28-1.22 (m, 6H), 1.19 (s, 3H), 1.17-1.11 (m, 3H), 1.01- 0.90 (m, 2H), 0.64-0.51 (m, 2H).

### Example 25

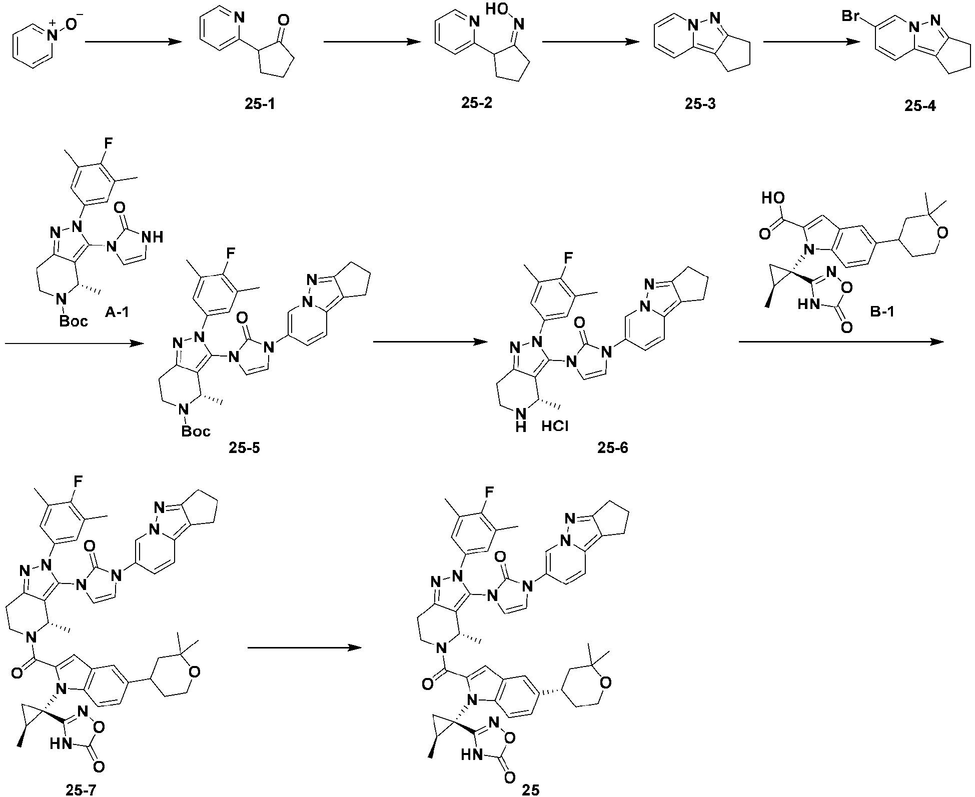

### Step 1: Synthesis of intermediate 25-1

Pyridine-N-oxide (2 g) and dichloromethane (15 mL) were mixed, and benzoyl chloride (2.96 g) was added in an ice bath. The mixture was stirred for 30 min, and 1-morpholinecyclopentene (4.19 g) was added. The mixture was heated to 40 °C and left to react for 12 h. After the reaction was completed, the reaction solution was poured into a 20% aqueous hydrochloric acid solution, and the aqueous phase was adjusted to pH 8-9 with 5 N aqueous sodium hydroxide solution and extracted with dichloromethane (100 mL). The organic phase was washed with saturated brine, dried over anhydrous sodium sulfate, and filtered under vacuum. The filtrate was concentrated under reduced pressure, and the residue was separated and purified by silica gel column chromatography (petroleum ether:ethyl acetate = 15:1) to give 1.56 g of intermediate **25-1.**

MS (ESI, [M+H]⁺) m/z: 162.12.

### Step 2: Synthesis of intermediate 25-2

Intermediate **25-1** (1.37 g), methanol (60 mL), hydroxylamine hydrochloride (0.95 g), and potassium acetate (1.67 g) were mixed, and the mixture was stirred at 70 °C for 1 h. After the reaction was completed, the reaction solution was poured into a saturated sodium bicarbonate solution, and ethyl acetate (100 mL) was added for extraction. The organic phase was washed with saturated brine, dried over anhydrous sodium sulfate, and filtered under vacuum. The filtrate was concentrated under reduced pressure to give 1.43 g of intermediate **25-2.**

MS (ESI, [M+H]⁺) m/z: 177.32.

¹H-NMR (500 MHz, DMSO-*d*₆): *δ* 10.35 (s, 1H), 8.49-8.45 (m, 1H), 7.71-7.68 (m, 1H), 7.28-7.26 (m, 1H), 7.21-7.18 (m, 1H), 3.85-3.84 (m, 1H), 2.55-2.52 (m, 1H), 2.41-2.34 (m, 1H), 2.18-2.09 (m, 1H), 2.07-2.01 (m, 1H), 1.93-1.89 (m, 1H), 1.73-1.67 (m, 1H).

### Step 3: Synthesis of intermediate 25-3

Intermediate **25-2** (1.43 g) and dichloromethane (20 mL) were mixed, and *p*-toluenesulfonyl chloride (1.70 g) and triethylamine (1.23 g) were added in an ice bath. The mixture was stirred at room temperature for 12 h. After the reaction was completed, the reaction solution was poured into water, and dichloromethane (100 mL) was added for extraction. The organic phase was washed with saturated brine, dried over anhydrous sodium sulfate, and filtered under vacuum. The filtrate was concentrated under reduced pressure, and the residue was separated and purified by silica gel column chromatography (petroleum ether:ethyl acetate = 20:1) to give 0.68 g of intermediate **25-3.**

MS (ESI, [M+H]⁺) m/z: 159.12.

¹H-NMR (500 MHz, DMSO-*d*₆): *δ* 8.54-8.51 (m, 1H), 7.46-7.42 (m, 1H), 7.12-7.07 (m, 1H), 6.70-6.66 (m, 1H), 2.85-2.77 (m, 4H), 2.48-2.42 (m, 2H).

### Step 4: Synthesis of intermediate 25-4

Intermediate **25-3** (0.6 g), chloroform (12 mL), and liquid bromine (0.67 g) were mixed, and the mixture was left to react at 50 °C for 12 h. After the reaction was completed, the reaction solution was poured into a saturated sodium sulfite solution, and dichloromethane (100 mL) was added for extraction. The organic phase was washed with saturated brine, dried over anhydrous sodium sulfate, and filtered under vacuum. The filtrate was concentrated under reduced pressure, and the residue was separated and purified by silica gel column chromatography (petroleum ether:ethyl acetate = 40:1) to give 0.10 g of intermediate **25-4.**

MS (ESI, [M+H]⁺) m/z: 237.14.

¹H-NMR (500 MHz, DMSO-*d*₆): *δ* 8.89 (s, 1H), 7.46 (d, *J=* 9.5 Hz, 1H), 7.21 (dd, *J=* 9.5, 2 Hz, 1H), 2.83-2.74 (m, 4H), 2.48-2.43 (m, 2H).

### Step 5: Synthesis of intermediate 25-5

Intermediate **A-1** (170 mg), intermediate **25-4** (100 mg), (1*S*,2*S*)-*N*1,*N*2-dimethylcyclohexane-1,2-diamine (27.4 mg), potassium carbonate (160 mg), copper(I) iodide (14.67 mg), and *N*-methylpyrrolidone (5 mL) were mixed, and the mixture was left to react at 120 °C for 12 h under a nitrogen atmosphere. After the reaction was completed, the reaction solution was poured into water, and ethyl acetate (50 mL) was added for extraction. The organic phase was washed with saturated brine, dried over anhydrous sodium sulfate, and filtered under vacuum. The filtrate was concentrated under reduced pressure, and the residue was separated and purified by silica gel column chromatography (dichloromethane:methanol = 20:1) to give 0.183 g of intermediate **25-5.**

¹H-NMR (500 MHz, DMSO-*d*₆): δ 8.96 (s, 1H), 7.59 (d, *J=* 9.5 Hz, 1H), 7.39-7.37 (m, 1H), 7.31 (d, *J=* 3.5 Hz, 1H), 7.11 (d, *J=* 6.5 Hz, 2H), 6.98 (s, 1H), 5.24-5.05 (m, 1H), 4.38-4.14 (m, 1H), 3.24-2.98 (m, 1H), 2.86-2.81 (m, 4H), 2.76-2.72 (m, 1H), 2.69-2.62 (m, 1H), 2.49-2.44 (m, 2H), 2.19 (s, 6H), 1.44 (s, 9H), 1.23-1.19 (m, 3H).

### Step 6: Synthesis of intermediate 25-6

Intermediate **25-5** (0.18 g), dichloromethane (2 mL), and a 4 N solution of hydrochloric acid in dioxane (2 mL) were mixed, and the mixture was stirred at room temperature for 1 h. After the reaction was completed, the reaction solution was directly concentrated under reduced pressure to give 0.15 g of intermediate **25-6.**

MS (ESI, [M+H]⁺) m/z: 498.38.

### Step 7: Synthesis of intermediate 25-7 and compound 25

Intermediate **B-1** (0.1 g), N,N-dimethylformamide (5 mL), triethylamine (0.68 mL), and *O*-(7-azabenzotriazol-1-yl)-N,N,N,N-tetramethyluronium hexafluorophosphate (0.28 g) were mixed. The mixture was stirred at room temperature for 5 min, and then intermediate **25-6** (0.13 g) was added thereto. The resulting mixture was stirred at room temperature for 5 h. After the reaction was completed, the reaction solution was poured into water, and ethyl acetate (50 mL) was added for extraction. The organic phase was washed with saturated brine, dried over anhydrous sodium sulfate, and filtered under vacuum. The filtrate was concentrated under reduced pressure, and the residue was separated and purified by silica gel column chromatography (dichloromethane:methanol = 20:1) to give 0.12 g of intermediate **25-7.** The above intermediate **25-7** was resolved by preparative chiral HPLC (chromatographic column: CHIRALART Cellulose-SB, 30 × 250 mm, 5 µm; mobile phase: ethanol:n-hexane = 43:57; flow rate: 40 mL/min) to give 26 mg of compound **25.**

Compound **25:** Rt = 4.57 min (UPCC conditions: chromatographic column: CHIRALPAK IB-3 (4.6 × 100 mm, 3 µm); mobile phase: carbon dioxide:methanol (containing 0.1% ammonium hydroxide) = 60:40; flow rate: 2.0 mL/min; column temperature: 40 °C)

HRMS: (ESI, [M+H]⁺) m/z: 891.4098.

¹H-NMR (500 MHz, DMSO-*d*₆): *δ* 11.74 (s, 1H), 8.98 (s, 1H), 7.60 (d, *J =* 9.5 Hz, 1H), 7.53 (s, 1H), 7.44-7.39 (m, 2H), 7.35-7.33 (m, 1H), 7.26 (d, *J =* 8.5 Hz, 1H), 7.18-7.14 (m, 2H), 7.02-6.93 (m, 2H), 5.63-5.56 (m, 1H), 4.41-4.35 (m, 1H), 3.73-3.69 (m, 2H), 3.22-3.15 (m, 1H), 3.07-3.02 (m, 1H), 2.87-2.80 (m, 6H), 2.48-2.42 (m, 2H), 2.23 (s, 6H), 1.70-1.66 (m, 2H), 1.60-1.56 (m, 2H), 1.43-1.39 (m, 3H), 1.28 (s, 3H), 1.24 (s, 3H), 1.18 (s, 3H), 1.16-1.13 (m, 3H).

### Example 26

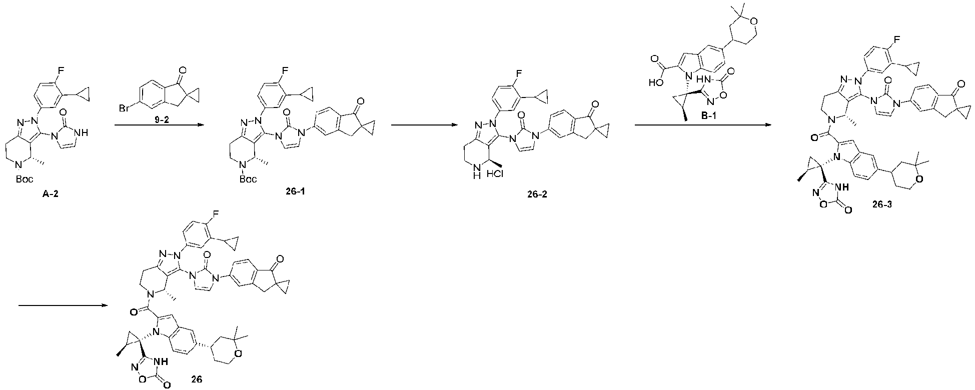

### Step 1: Synthesis of intermediate 26-1

Copper(I) iodide (42.0 mg), potassium carbonate (229 mg), (1*S*,2*S*)-*N1*,*N2*-dimethylcyclohexane-1,2-diamine (39.2 mg), intermediate **A-2** (250 mg), intermediate **9-2** (196 mg), and N-methylpyrrolidone (4 mL) were mixed. The reaction mixture was heated to 130 °C and stirred for 5 h. The reaction solution was poured into a mixture of ethyl acetate (30 mL) and water (30 mL). The two phases were separated, and the aqueous phase was extracted with ethyl acetate (2 × 20 mL). The organic phases were combined, washed with a saturated sodium chloride solution, dried over anhydrous sodium sulfate, filtered under vacuum, and concentrated under reduced pressure. The residue was separated by silica gel column chromatography (petroleum ether:ethyl acetate = 50:50) to give 190 mg of intermediate **26-1.**

MS (ESI, [M+H]⁺) m/z: 610.41.

### Step 2: Synthesis of intermediate 26-2

Intermediate **26-1** (190 mg) and a 4 N solution of hydrochloric acid in dioxane (10 mL) were mixed, and the mixture was left to react at room temperature for 1 h. The reaction solution was concentrated under reduced pressure to give 130 mg of intermediate **26-2.**

MS (ESI, [M+H]⁺) m/z: 510.34.

### Step 3: Synthesis of intermediate 26-3 and compound 26

Intermediate **B-1** (100 mg), *N,N*-dimethylformamide (5 mL), triethylamine (492 mg), and *O*-(7-azabenzotriazol-1-yl)-*N,N,N',N'*-tetramethyluronium hexafluorophosphate (277 mg) were mixed. The mixture was stirred for 5 min, and **26-3** (130 mg) was added. The mixture was stirred at room temperature for 4 h. The reaction solution was poured into water (50 mL), and the resulting solution was extracted with ethyl acetate (30 mL). The organic phase was washed with saturated brine, dried over anhydrous sodium sulfate, and concentrated. The residue was separated by preparative C18 column chromatography (chromatographic column: Xtimate C18, 21.2 × 250 mm, 5 µm; mobile phase: water (0.1% acetic acid):acetonitrile = 25:75, isocratic elution) to give 70 mg of intermediate **26-4.** The above intermediate **26-4** was resolved by preparative chiral HPLC (chromatographic column: CHIRALART Cellulose-SB, 30 × 250 mm, 5 µm; mobile phase: ethanol-dichloromethane (1:1):n-hexane = 27:73, isocratic elution) to give 30 mg of compound **26.**

Compound **26:** Rt = 2.98 min (UPCC conditions: chromatographic column: CHIRALPAK IB, 4.6 × 100 mm, 3 µm; mobile phase: carbon dioxide:isopropanol (containing 0.1% ammonium hydroxide):ethanol = 50:25:25; flow rate: 0.5 mL/min; column temperature: 40 °C).

HRMS: (ESI, [M+H]⁺) m/z: 903.4006.

¹H-NMR (500 MHz, DMSO-*d*₆): δ 11.73 (s, 1H), 8.03 (s, 1H), 7.88 (d, *J=* 8.4 Hz, 1H), 7.78 (d, *J =* 8.4 Hz, 1H), 7.54 (s, 1H), 7.42-7.35 (m, 2H), 7.32-7.21 (m, 4H), 6.97-6.88 (m, 2H), 5.58 (d, *J =* 6.7 Hz, 1H), 4.39 (s, 1H), 3.72 (d, *J =* 10.2 Hz, 2H), 3.24-3.17 (m, 2H), 3.03-3.01 (m, 1H), 2.91 (d, *J =* 15.4 Hz, 1H), 2.74 (t, *J =* 7.5 Hz, 1H), 2.10-1.96 (m, 2H), 1.75-1.47 (m, 9H), 1.44-1.09 (m, 13H), 0.96 (d, *J =* 8.4 Hz, 2H), 0.85 (t, *J =* 6.9 Hz, 3H).

### Example 27

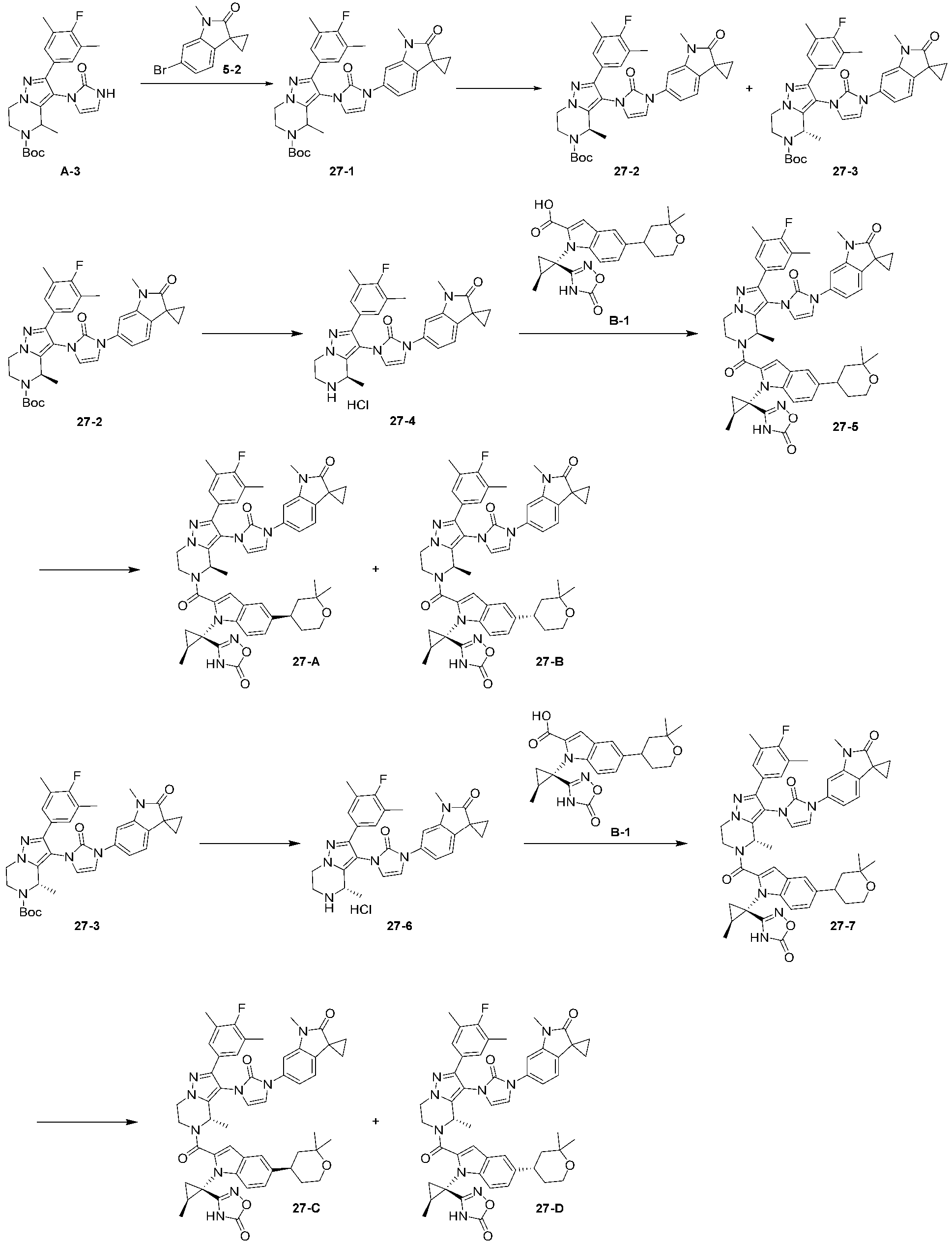

### Step 1: Synthesis of intermediate 27-1, intermediate 27-2, and intermediate 27-3

Intermediate **A-3** (800 mg), intermediate **5-2** (548 mg), (1*S*,2*S*)-*N*1,*N*2-dimethylcyclohexane-1,2-diamine (258 mg), potassium carbonate (751 mg), copper(I) iodide (173 mg), and *N*-methylpyrrolidone (12 mL) were mixed, and the mixture was left to react at 100 °C for 4 h under a nitrogen atmosphere. After the reaction was completed, the reaction solution was poured into water, and ethyl acetate (50 mL) was added for extraction. The organic phase was washed with saturated brine, dried over anhydrous sodium sulfate, and filtered under vacuum. The filtrate was concentrated under reduced pressure, and the residue was separated and purified by silica gel column chromatography (petroleum ether:ethyl acetate = 1:1) to give 940 mg of intermediate **27-1.** The above intermediate **27-1** was resolved by preparative chiral HPLC (chromatographic column: CHIRALART Cellulose-SB, 30 × 250 mm, 5 µm; mobile phase: ethanol:*n*-hexane = 43:57; flow rate: 40 mL/min) to give 450 mg of intermediate **27-2** and 460 mg of intermediate **27-3.**

Intermediate **27-2:** Rt = 3.73 min (UPCC conditions: chromatographic column: CHIRALPAK IB-3 (4.6 × 100 mm, 3 µm); mobile phase: carbon dioxide:methanol (containing 0.1% ammonium hydroxide) = 40:60; flow rate: 2.0 mL/min; column temperature: 40 °C)

MS (ESI, [M+H]⁺) m/z: 613.44.

¹H-NMR (500 MHz, DMSO-*d*₆): δ 7.46 (d, *J =* 2.1 Hz, 1H), 7.38 (dd, *J =* 8.1, 2.0 Hz, 1H), 7.33 (d, *J =* 3.2 Hz, 1H), 7.19 (d, *J =* 7.0 Hz, 2H), 7.12 (d, *J =* 8.1 Hz, 1H), 6.85 (d, *J =* 3.1 Hz, 1H), 5.27 (d, *J =* 6.0 Hz, 1H), 4.31 (s, 1H), 4.27-4.21 (m, 1H), 4.11-4.03 (m, 1H), 3.51 (s, 1H), 3.24 (s, 3H), 2.17 (d, *J =* 2.1 Hz, 6H), 1.66-1.62 (m, 2H), 1.55-1.52 (m, 2H), 1.45 (s, 9H), 1.29-1.26 (m, 3H).

Intermediate **27-3:** Rt = 4.78 min (UPCC conditions: chromatographic column: CHIRALPAK IB-3 (4.6 × 100 mm, 3 µm); mobile phase: carbon dioxide:methanol (containing 0.1% ammonium hydroxide) = 40:60; flow rate: 2.0 mL/min; column temperature: 40 °C)

MS (ESI, [M+H]⁺) m/z: 613.43.

¹H-NMR (500 MHz, DMSO-*d*₆): δ 7.46 (d, *J =* 2.0 Hz, 1H), 7.38 (dd, *J =* 8.1, 2.0 Hz, 1H), 7.33 (d, *J* = 3.2 Hz, 1H), 7.19 (d, *J =* 7.0 Hz, 2H), 7.12 (d, *J =* 7.9 Hz, 1H), 6.85 (d, *J =* 3.2 Hz, 1H), 5.27 (d, *J =* 6.3 Hz, 1H), 4.31 (s, 1H), 4.24 (dd, *J =* 12.7, 3.5 Hz, 1H), 4.12-4.03 (m, 1H), 3.51 (d, *J =* 1.1 Hz, 1H), 3.24 (s, 3H), 2.17 (d, *J =* 2.1 Hz, 6H), 1.67-1.62 (m, 2H), 1.56-1.51 (m, 2H), 1.45 (s, 9H), 1.29-1.26 (m, 3H).

### Step 2: Synthesis of intermediate 27-4

Intermediate **27-2** (0.2 g), dichloromethane (5 mL), and a 4 N solution of hydrochloric acid in dioxane (5 mL) were mixed, and the mixture was stirred at room temperature for 2 h. After the reaction was completed, the reaction solution was directly concentrated under reduced pressure to give 0.16 g of intermediate **27-4.**

MS (ESI, [M+H]⁺) m/z: 513.34.

### Step 3: Synthesis of intermediate 27-5, compound 27-A, and compound 27-B

Intermediate **B-1** (0.12 g), *N,N-*dimethylformamide (5 mL), triethylamine (0.393 mL), and *O*-(7-azabenzotriazol-1-yl)-*N,N,N,N*-tetramethyluronium hexafluorophosphate (0.22 g) were mixed. The mixture was stirred at room temperature for 5 min, and then intermediate **27-4** (0.16 g) was added thereto. The resulting mixture was stirred at room temperature for 5 h. After the reaction was completed, the reaction solution was poured into water, and ethyl acetate (50 mL) was added for extraction. The organic phase was washed with saturated brine, dried over anhydrous sodium sulfate, and filtered under vacuum. The filtrate was concentrated under reduced pressure, and the residue was separated and purified by silica gel column chromatography (dichloromethane:methanol = 20:1) to give 0.2 g of intermediate **27-5.** The above intermediate **27-5** was resolved by preparative chiral HPLC (chromatographic column: CHIRALART Cellulose-SB, 30 × 250 mm, 5 µm; mobile phase: ethanol-dichloromethane (1:3):n-hexane = 40:60; flow rate: 40 mL/min) to give 78 mg of compound **27-A** and 80 mg of compound **27-B.**

Compound **27-A:** Rt = 1.09 min (UPCC conditions: chromatographic column: CHIRALPAK IB-3 (4.6 × 100 mm, 3 µm); mobile phase: carbon dioxide:methanol/acetonitrile (1:1) (containing 0.1% ammonium hydroxide) = 30:70; flow rate: 2.0 mL/min; column temperature: 40 °C)

HRMS: (ESI, [M+H]⁺) m/z: 906.4119.

¹H-NMR (500 MHz, DMSO-*d*₆): *δ* 12.16 (s, 1H), 7.56-7.34 (m, 6H), 7.30-7.17 (m, 5H), 7.12 (d, *J =* 7.2 Hz, 1H), 6.95-6.81 (m, 3H), 4.59-4.33 (m, 3H), 3.89 (s, 1H), 3.77-3.63 (m, 3H), 3.27-3.22 (m, 3H), 3.11-2.98 (m, 2H), 2.19 (s, 6H), 1.56-1.49 (m, 5H), 1.46-1.38 (m, 4H), 1.28 (s, 3H), 1.23 (s, 3H), 1.18 (s, 3H).

Compound **27-B:** Rt = 1.44 min (UPCC conditions: chromatographic column: CHIRALPAK IB-3 (4.6 × 100 mm, 3 µm); mobile phase: carbon dioxide:methanol/acetonitrile (1:1) (containing 0.1% ammonium hydroxide) = 30:70; flow rate: 2.0 mL/min; column temperature: 40 °C)

HRMS: (ESI, [M+H]⁺) m/z: 906.4116.

¹H-NMR (500 MHz, DMSO-*d*₆): *δ* 12.15 (s, 1H), 7.53-7.34 (m, 6H), 7.31-7.18 (m, 5H), 7.16-7.08 (m, 1H), 6.96-6.78 (m, 3H), 4.59-4.31 (m, 3H), 3.89 (s, 1H), 3.71 (s, 3H), 3.25 (s, 3H), 3.11-2.94 (m, 2H), 2.19 (s, 6H), 1.57-1.49 (m, 5H), 1.47-1.39 (m, 4H), 1.30 (s, 3H), 1.28 (s, 3H), 1.26 (s, 3H).

### Step 4: Synthesis of intermediate 27-6

Intermediate **27-3** (0.2 g), dichloromethane (5 mL), and a 4 N solution of hydrochloric acid in dioxane (5 mL) were mixed, and the mixture was stirred at room temperature for 2 h. After the reaction was completed, the reaction solution was directly concentrated under reduced pressure to give 0.16 g of intermediate **27-6.**

### Step 5: Synthesis of intermediate 27-7, compound 27-C, and compound 27-D

Intermediate **B-1** (0.12 g), *N,N*-dimethylformamide (5 mL), triethylamine (0.39 mL), and *O*-(7-azabenzotriazol-1-yl)-*N,N,N,N*-tetramethyluronium hexafluorophosphate (0.22 g) were mixed. The mixture was stirred at room temperature for 5 min, and then intermediate **27-6** (0.16 g) was added thereto. The resulting mixture was stirred at room temperature for 5 h. After the reaction was completed, the reaction solution was poured into water, and ethyl acetate (50 mL) was added for extraction. The organic phase was washed with saturated brine, dried over anhydrous sodium sulfate, and filtered under vacuum. The filtrate was concentrated under reduced pressure, and the residue was separated and purified by silica gel column chromatography (dichloromethane:methanol = 20:1) to give 0.2 g of intermediate **27-7.** The above intermediate **27-7** was resolved by preparative chiral HPLC (chromatographic column: CHIRALART Cellulose-SB, 30 × 250 mm, 5 µm; mobile phase: ethanol-dichloromethane (1:3):*n*-hexane = 40:60; flow rate: 40 mL/min) to give 74 mg of compound **27-C** and 78 mg of compound **27-D.**

Compound **27-C:** Rt = 1.10 min (UPCC conditions: chromatographic column: CHIRALPAK IB-3 (4.6 × 100 mm, 3 µm); mobile phase: carbon dioxide:methanol/acetonitrile (1:1) (containing 0.1% ammonium hydroxide) = 30:70; flow rate: 2.0 mL/min; column temperature: 40 °C)

HRMS: (ESI, [M+H]⁺) m/z: 906.4132.

¹H-NMR (500 MHz, DMSO-*d*₆): *δ* 12.15 (s, 1H), 7.47 (d, *J =* 5.8 Hz, 2H), 7.44-7.32 (m, 4H), 7.31-7.18 (m, 5H), 7.12 (d, *J=* 5.8 Hz, 1H), 7.00-6.81 (m, 3H), 4.58-4.31 (m, 3H), 3.95-3.82 (m, 1H), 3.71 (s, 3H), 3.25 (s, 3H), 3.14-2.99 (m, 2H), 2.19 (s, 6H), 1.59-1.51 (m, 5H), 1.46-1.38 (m, 4H), 1.28 (s, 3H), 1.26 (s, 3H), 1.23 (s, 3H).

Compound **27-D:** Rt = 1.20 min (UPCC conditions: chromatographic column: CHIRALPAK IB-3 (4.6 × 100 mm, 3 µm); mobile phase: carbon dioxide:methanol/acetonitrile (1:1) (containing 0.1% ammonium hydroxide) = 30:70; flow rate: 2.0 mL/min; column temperature: 40 °C)

HRMS: (ESI, [M+H]⁺) m/z: 906.4144.

¹H-NMR (500 MHz, DMSO-*d*₆): *δ* 11.68 (s, 1H), 7.54-7.46 (m, 2H), 7.42-7.34 (m, 4H), 7.29-7.16 (m, 5H), 7.14-7.01 (m, 2H), 6.90-6.82 (m, 2H), 4.59 (s, 2H), 4.40 (s, 1H), 4.14 (s, 1H), 3.71 (s, 3H), 3.25 (s, 3H), 3.04 (s, 2H), 2.19 (s, 6H), 1.66-1.59 (m, 5H), 1.54 (s, 4H), 1.35 (s, 3H), 1.30 (s, 3H), 1.26 (s, 3H).

### Test Example 1: In vitro cellular cAMP expression activity assay

HEK293/CRE-Luc/GLP1R cells (manufacturer: GenScript Biotech Corporation) in a good growth state were taken, washed with PBS, and digested with pancreatin. The digestion was terminated with a complete medium. The cells were collected into a centrifuge tube, adjusted to a cell density of 4 × 10⁵ cells/mL using a DMEM + 2% FBS medium, seeded in a 96-well plate (100 µL/well), and incubated at 37 °C for 1 h. After 1 h, the compounds were added using a nanoliter pipettor such that the final concentrations of the compounds were 10 nM-0.0006 nM. The compounds were added in duplicate, and a control was set. After the cells were incubated in a cell incubator for another 6 h, a detection reagent Luciferase (manufacturer: Vazyme Biotech, 50 µL/well) was added. The plate was left to stand at room temperature for 3 min to fully lyse the cells, and the luminescence was detected on a PerkinElmer Envision microplate reader. A dose-response curve was fitted by four-parameter analysis, and the EC₅₀ was calculated.

Some of the compounds of the present disclosure exhibited good *in vitro* cellular cAMP expression activity, as shown in Table 1.

**Table 1. In vitro cAMP expression activity of some compounds in cells**

| **No.** | **HEK293/CRE -Luc/GLP-1R EC₅₀ (nM)** |
|---|---|
| **Compound 1-A** | <0.5 |
| **Compound 2** | <0.5 |
| **Compound 3** | <0.5 |
| **Compound 4** | <0.5 |
| **Compound 5** | <0.5 |
| **Compound 6** | <0.5 |
| **Compound 7** | <0.5 |
| **Compound 8** | <0.5 |
| **Compound 9-A** | <0.5 |
| **Compound 10-A** | <0.5 |
| **Compound 12-A** | <0.5 |
| **Compound 13** | <0.5 |
| **Compound 14** | <0.5 |
| **Compound 15** | <0.5 |
| **Compound 16** | <0.5 |
| **Compound 17** | <0.5 |
| **Compound 18** | <0.5 |
| **Compound 19** | <0.5 |
| **Compound 20** | <0.5 |
| **Compound 21** | <0.5 |
| **Compound 22-A** | <0.5 |
| **Compound 23** | <0.5 |
| **Compound 24** | <0.5 |
| **Compound 26** | <0.5 |
| **Compound 27-D** | <0.5 |

### Test Example 2: In vitro stability assay in liver microsome

Liver microsome incubation samples were prepared by mixing a PBS buffer (pH 7.4), a liver microsome solution (0.5 mg/mL), a test compound, and an NADPH + MgCl₂ solution, and incubated at 37 °C and 300 rpm for 1 h. Zerohour samples were prepared by mixing a PBS buffer (pH 7.4), a liver microsome solution (0.5 mg/mL), and a test compound. An acetonitrile solution containing an internal standard was added to the samples, and supernatants were prepared by protein precipitation, diluted, and then assayed by LC/MS/MS. The *in vitro* stability in liver microsome of some of the compounds is shown in Table 2.

**Table 2. In vitro stability in liver microsome**

| **No.** | **HLM Remaining (T=60min)** | **MLM Remaining (T=60min)** |
|---|---|---|
| **Compound 2** | 88.76% | 88.89% |
| **Compound 3** | 80.83% | 90.13% |
| **Compound 4** | 91.85% | 95.46% |
| **Compound 5** | 98.60% | 94.32% |
| **Compound 13** | 84.57% | 84.44% |
| **Compound 14** | 89.53% | 90.40% |
| **Compound 15** | 87.69% | 87.72% |
| **Compound 18** | 89.14% | 91.41% |
| **Compound 19** | 92.40% | 77.17% |
| **Compound 20** | 95.39% | 77.59% |
| **Compound 27-D** | 86.20% | 87.54% |

Some of the compounds of the present disclosure exhibited good *in vitro* stability in liver microsome.

### Test Example 3: In vivo pharmacokinetic evaluation in mice

ICR mice of 18-22 g were randomized into 4 groups of 9 mice after 3-5 days of acclimatization. The test compounds were administered intragastrically (IG) at a dose of 10 mg/kg or intravenously (IV) at a dose of 1 mg/kg.

The test animals (ICR mice) were fasted from 12 h pre-dose to 4 h post-dose, and given free access to water throughout the study.

Orbital blood samples of about 0.1 mL were collected at 15 min, 30 min, 1 h, 2 h, 3 h, 4 h, 6 h, 8 h, 10 h, and 24 h post-dose for the intragastric administration, and at 5 min, 10 min, 30 min, 1 h, 2 h, 4 h, 6 h, 8 h, 10 h, and 24 h post-dose for the intravenous administration. The blood sampling was performed at 3-4 time points for each mouse, 3 mice per time point. The collected whole blood was transferred into EDTA-K2-containing centrifuge tubes and stored at 4 °C, and, within 1 h, centrifuged at 4 °C at 4000 rpm for 10 min to separate the plasma. All the plasma samples were immediately stored after collection at -20 °C for testing.

30 µL of the plasma sample to be tested and a standard curve sample were pipetted and added with 300 µL of an acetonitrile solution containing an internal standard (20 ng/mL of diazepam). The mixture was shaken to mix well for 5 min, and centrifuged at 13,000 rpm for 10 min. 80 µL of the supernatant was taken and diluted with 80 µL of ultrapure water. After a thorough mixing, 2 µL of the resulting sample was taken for liquid chromatography-mass spectrometry, and the chromatogram was recorded.

The *in vivo* pharmacokinetic properties of some of the compounds in mice are shown in Table 3.

**Table 3. In vivo pharmacokinetic properties in mice**

| | | **Compound 14** | | **Compound 15** | | **Compound 18** | |
|---|---|---|---|---|---|---|---|
| **Route of administration** | | iv | ig | iv | ig | iv | ig |
| **Dose (mg/kg)** | | 0.5 | 3 | 0.5 | 3 | 0.5 | 3 |
| **AUC(0-24)** | **(h•ng/mL)** | 2056 | 4558 | 2505 | 6215 | 2933 | 5837 |
| **AUC(0-∞)** | **(h•ng/mL)** | 2166 | 4909 | 2569 | 6539 | 3004 | 6028 |
| **MRT(0-24)** | **h** | 6.78 | 10.1 | 5.00 | 9.28 | 5.48 | 8.36 |
| **t1/2** | **h** | 5.77 | 5.54 | 5.35 | 5.40 | 4.55 | 4.29 |
| **Absolute bioavailability F** | | / | 37.77% | / | 42.42% | / | 33.45% |

| | | **Compound 2** | | **Compound 3** | | **Compound 5** | |
|---|---|---|---|---|---|---|---|
| **Route of administration** | | iv | ig | iv | ig | iv | ig |
| **Dose (mg/kg)** | | 0.5 | 3 | 0.5 | 3 | 0.5 | 3 |
| **AUC(0-24)** | **(h•ng/mL)** | 1824 | 4789 | 2363 | 4799 | 2217 | 4644 |
| **AUC(0-∞)** | **(h•ng/mL)** | 1852 | 4875 | 2452 | 4960 | 2262 | 4777 |
| **MRT(0-24)** | **h** | 5.13 | 7.73 | 6.13 | 8.42 | 5.16 | 7.80 |
| **t1/2** | **h** | 4.10 | 3.53 | 5.73 | 4.15 | 4.60 | 4.27 |
| **Absolute bioavailability F** | | / | 43.86% | / | 33.72% | / | 35.20% |

Some of the compounds of the present disclosure exhibited good *in vivo* pharmacokinetic properties in mice.

### Test Example 4: Evaluation of hypoglycemic effect in mice

GLP-1R humanized C57BL/6J mice of 20-22 g were used in this study after 3 days of acclimatization. All the animals were deprived of food but not water until the end of study. Blood samples were collected from the tail tip of the mice.

The test animals were deprived of food but not water overnight before the study. The next day, blood was collected from all the animals for blood glucose testing, and the animals were randomized into a normal control group, a positive control group, and test compound groups by blood glucose and body weight, with 6 animals in each group. The groups were then given intragastric doses, and 5 h after the administration, an intraperitoneal injection of glucose solution (2 g/kg) was given. The blood glucose concentration was measured at 0.25 h, 0.5 h, 1 h, 2 h, and 3 h after the glucose injection. The blood glucose was recorded throughout the study, and the area under the blood glucosetime curve AUC0-180min Glu was calculated.

The hypoglycemic effects of some of the compounds on mice are shown in Table 4.

**Table 4. Evaluation of hypoglycemic effect in mice**

| **No.** | **Dose (mg/kg)** | **AUC0-180min Glu reduction (%)** |
|---|---|---|
| **Compound 5** | 1 | 83 |
| **Compound 14** | 1 | 79 |
| **Compound 15** | 1 | 80 |
| **Compound 27-D** | 1 | 75 |

Some of the compounds of the present disclosure exhibited good hypoglycemic effects in mice.

## Claims

1. A compound of formula (I), a stereoisomer thereof, or a pharmaceutically acceptable salt thereof, 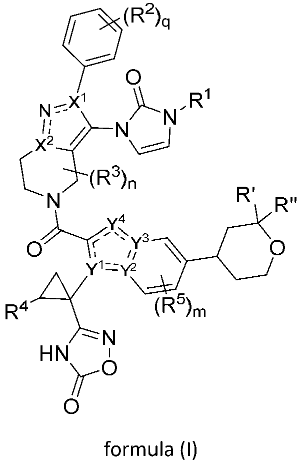 wherein
X¹ and X² are each independently selected from the group consisting of C and N;
Y¹, Y², Y³, and Y⁴ are each independently selected from the group consisting of CH, C, and N;
R¹ is selected from the group consisting of C₁₁₋₁₅ cycloalkyl, C₁₁₋₁₅ aryl, 11- to 15-membered heteroaryl, and 11- to 15-membered heterocyclyl, wherein the C₁₁₋₁₅ cycloalkyl, C₁₁₋₁₅ aryl, 11- to 15-membered heteroaryl, or 11- to 15-membered heterocyclyl is a tricyclic group, and the C₁₁₋₁₅ cycloalkyl, C₁₁₋₁₅ aryl, 11- to 15-membered heteroaryl, or 11- to 15-membered heterocyclyl may be optionally independently substituted with one or more R^{a};
or, R¹ is selected from the group consisting of C₃₋₇ cycloalkyl, phenyl, 5- to 6-membered heteroaryl, and 3- to 7-membered heterocyclyl, wherein R¹ is substituted with one C₂₋₄ alkynyl, R¹ may further be optionally independently substituted with one or more R^{a}, and the C₂₋₄ alkynyl may be optionally substituted with one or more R^{b};
R^{a} is each independently selected from the group consisting of deuterium, halogen, =O, deuterated C₁₋₆ alkyl, -OH, -CN, NH₂, -COOH, C₁₋₆ alkyl NH-, (C₁₋₆ alkyl)₂N-, C₁₋₆ alkyl, C₂₋₆ alkenyl, C₂₋₆ alkynyl, C₁₋₆ alkoxy, C₁₋₆ alkoxy C₁₋₃ alkylene, C₃₋₆ cycloalkyl, phenyl, 5- to 6-membered heteroaryl, and 3- to 6-membered heterocyclyl, wherein the deuterated C₁₋₆ alkyl, C₁₋₆ alkyl NH-, (C₁₋₆ alkyl)₂N-, C₁₋₆ alkyl, C₂₋₆ alkenyl, C₂₋₆ alkynyl, C₁₋₆ alkoxy, or C₁₋₆ alkoxy C₁₋₃ alkylene is optionally independently substituted with one or more R^{c1}, and the C₃₋₆ cycloalkyl, phenyl, 5- to 6-membered heteroaryl, or 3- to 6-membered heterocyclyl is optionally independently substituted with one or more R^{d1};
R² is each independently selected from the group consisting of halogen, -OH, -CN, NH₂, -COOH, C₁₋₆ alkyl NH-, (C₁₋₆ alkyl)₂N-, C₁₋₆ alkyl, deuterated C₁₋₆ alkyl, C₂₋₆ alkenyl, C₂₋₆ alkynyl, C₁₋₆ alkoxy, C₁₋₆ alkoxy C₁₋₃ alkylene, C₃₋₆ cycloalkyl, phenyl, 5- to 6-membered heteroaryl, and 3- to 6-membered heterocyclyl, wherein the C₁₋₆ alkyl NH-, (C₁₋₆ alkyl)₂N-, C₁₋₆ alkyl, deuterated C₁₋₆ alkyl, C₂₋₆ alkenyl, C₂₋₆ alkynyl, C₁₋₆ alkoxy, or C₁₋₆ alkoxy C₁₋₃ alkylene is optionally independently substituted with one or more R^{c2}, and the C₃₋₆ cycloalkyl, phenyl, 5- to 6-membered heteroaryl, or 3-to 6-membered heterocyclyl is optionally independently substituted with one or more R^{d2};
or, R² on two adjacent carbon atoms, together with the carbon atoms connected thereto, form C₄₋₆ cycloalkyl, phenyl, 5- to 6-membered heteroaryl, or 4- to 6-membered heterocyclyl, wherein the C₄₋₆ cycloalkyl, phenyl, 5- to 6-membered heteroaryl, or 4- to 6-membered heterocyclyl is optionally independently substituted with one or more R^{d3};
R³ is each independently selected from the group consisting of deuterium, halogen, -OH, -CN, NH₂, -COOH, C₁₋₆ alkyl NH-, (C₁₋₆ alkyl)₂N-, C₁₋₆ alkyl, deuterated C₁₋₆ alkyl, C₂₋₆ alkenyl, C₂₋₆ alkynyl, C₁₋₆ alkoxy, C₁₋₆ alkoxy C₁₋₃ alkylene, C₃₋₆ cycloalkyl, phenyl, 5- to 6-membered heteroaryl, and 3- to 6-membered heterocyclyl, wherein the C₁₋₆ alkyl NH-, (C₁₋₆ alkyl)₂N-, C₁₋₆ alkyl, deuterated C₁₋₆ alkyl, C₂₋₆ alkenyl, C₂₋₆ alkynyl, C₁₋₆ alkoxy, or C₁₋₆ alkoxy C₁₋₃ alkylene is optionally independently substituted with one or more R^{c3}, and the C₃₋₆ cycloalkyl, phenyl, 5- to 6-membered heteroaryl, or 3-to 6-membered heterocyclyl is optionally independently substituted with one or more R^{d4};
R⁴ is selected from the group consisting of H, deuterium, halogen, -CN, NH₂, -COOH, C₁₋₆ alkyl NH-, (C₁₋₆ alkyl)₂N-, C₁₋₆ alkyl, deuterated C₁₋₆ alkyl, C₁₋₆ alkoxy, and C₁₋₆ alkoxy C₁₋₃ alkylene, wherein the C₁₋₆ alkyl NH-, (C₁₋₆ alkyl)₂N-, C₁₋₆ alkyl, deuterated C₁₋₆ alkyl, C₁₋₆ alkoxy, or C₁₋₆ alkoxy C₁₋₃ alkylene is optionally independently substituted with one or more R^{c4};
R⁵ is each independently selected from the group consisting of halogen, -CN, -OH, -SH, -NH₂, C₁₋₆ alkyl NH-, (C₁₋₆ alkyl)₂N-, C₁₋₆ alkyl, deuterated C₁₋₆ alkyl, C₁₋₆ alkoxy, C₁₋₆ alkoxy C₁₋₃ alkylene, C₁₋₆ alkylthio, -CONH₂, -CONHC₁₋₃ alkyl, -NHCOC₁₋₃ alkyl, -SO₂NH₂, -SO₂NHC₁₋₃ alkyl, and -NHSO₂C₁₋₃ alkyl, wherein the C₁₋₆ alkyl NH-, (C₁₋₆ alkyl)₂N-, C₁₋₆ alkyl, deuterated C₁₋₆ alkyl, C₁₋₆ alkoxy, C₁₋₆ alkoxy C₁₋₃ alkylene, C₁₋₆ alkylthio, -CONH₂, -CONHC₁₋₃ alkyl, -NHCOC₁₋₃ alkyl, - SO₂NH₂, -SO₂NHC₁₋₃ alkyl, or -NHSO₂C₁₋₃ alkyl is optionally independently substituted with one or more R^{c5};
R' and R" are each independently selected from the group consisting of H, halogen, -CN, -OH, -SH, -NH₂, C₁₋₆ alkyl NH-, (C₁₋₆ alkyl)₂N-, C₁₋₆ alkyl, deuterated C₁₋₆ alkyl, C₁₋₆ alkoxy, C₁₋₆ alkoxy C₁₋₃ alkylene, C₁₋₆ alkylthio, -CONH₂, - CONHC₁₋₃ alkyl, -NHCOC₁₋₃ alkyl, -SO₂NH₂, -SO₂NHC₁₋₃ alkyl, and -NHSO₂C₁₋₃ alkyl, wherein the C₁₋₆ alkyl NH-, (C₁₋₆ alkyl)₂N-, C₁₋₆ alkyl, deuterated C₁₋₆ alkyl, C₁₋₆ alkoxy, C₁₋₆ alkoxy C₁₋₃ alkylene, C₁₋₆ alkylthio, -CONH₂, -CONHC₁₋₃ alkyl, -NHCOC₁₋₃ alkyl, -SO₂NH₂, -SO₂NHC₁₋₃ alkyl, or -NHSO₂C₁₋₃ alkyl is optionally independently substituted with one or more R^{c6};
or, R' and R", together with the carbon atom connected thereto, form C₃₋₆ cycloalkyl or 3- to 6-membered heterocycloalkyl, wherein the C₃₋₆ cycloalkyl or 3- to 6-membered heterocycloalkyl is optionally independently substituted with one or more R^{d5};
" 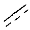 " is each independently selected from the group consisting of a single bond and a double bond;
R^{b} is each independently selected from the group consisting of deuterium, halogen, -CN, -OH, -NH₂, C₁₋₃ alkyl, and C₁₋₃ alkoxy, wherein the C₁₋₃ alkyl or C₁₋₃ alkoxy is optionally independently substituted with one or more substituents selected from the group consisting of deuterium, halogen, OH, CN, and NH₂;
R^{c1}, R^{c2}, R^{c3}, R^{c4}, R^{c5}, and R^{c6} are each independently selected from the group consisting of deuterium, halogen, -CN, -OH, and -NH₂;
R^{d1}, R^{d2}, R^{d3}, R^{d4}, and R^{d5} are each independently selected from the group consisting of deuterium, halogen, -CN, - OH, =O, -NH₂, C₁₋₃ alkyl, and C₁₋₃ alkoxy, wherein the C₁₋₃ alkyl or C₁₋₃ alkoxy is optionally independently substituted with one or more substituents selected from the group consisting of deuterium, halogen, OH, CN, and NH₂;
q is selected from the group consisting of 0, 1, 2, 3, and 4;
n is selected from the group consisting of 0, 1, 2, 3, and 4;
m is selected from the group consisting of 0, 1, 2, 3, and 4.

2. The compound, the stereoisomer thereof, or the pharmaceutically acceptable salt thereof according to claim 1, wherein R¹ is selected from the group consisting of C₁₁₋₁₅ aryl and 11- to 15-membered heteroaryl, wherein the C₁₁₋₁₅ aryl or 11- to 15-membered heteroaryl is a tricyclic group, and the C₁₁₋₁₅ aryl or 11- to 15-membered heteroaryl may be optionally independently substituted with one or more R^{a};
or, R¹ is selected from the group consisting of benzo C₇₋₁₁ fused bicycloalkyl, benzo 7- to 11-membered fused heterobicyclyl, benzo 7- to 11-membered fused heterobiaryl, pyridino C₇₋₁₁ fused bicycloalkyl, pyridino 7- to 11-membered fused heterobicyclyl, pyridino 7- to 11-membered fused heterobiaryl, pyrimido C₇₋₁₁ fused bicycloalkyl, pyrimido 7- to 11-membered fused heterobicyclyl, pyrimido 7- to 11-membered fused heterobiaryl, pyridazino C₇₋₁₁ fused bicycloalkyl, pyridazino 7- to 11-membered fused heterobicycloalkyl, pyridazino 7- to 11-membered fused heterobiaryl, pyrazino C₇₋₁₁ fused bicycloalkyl, pyrazino 7- to 11-membered fused heterobicycloalkyl, pyrazino 7- to 11-membered fused heterobiaryl, benzo C₇₋₁₁ spiro bicycloalkyl, benzo C₇₋₁₁ spiro heterobicyclyl, pyridino C₇₋₁₁ spiro bicycloalkyl, pyridino C₇₋₁₁ spiro heterobicyclyl, pyrimido C₇₋₁₁ spiro bicycloalkyl, pyrimido C₇₋₁₁ spiro heterobicyclyl, pyridazino C₇₋₁₁ spiro bicycloalkyl, pyridazino C₇₋₁₁ spiro heterobicyclyl, pyrazino C₇₋₁₁ spiro bicycloalkyl, and pyrazino C₇₋₁₁ spiro heterobicyclyl, wherein R¹ may be optionally independently substituted with one or more R^{a};
or, R¹ is selected from the group consisting of benzo C₇₋₉ fused bicycloalkyl, benzo 7- to 9-membered fused heterobicyclyl, benzo 7- to 9-membered fused heterobiaryl, pyridino C₇₋₉ fused bicycloalkyl, pyridino 7- to 9-membered fused heterobicyclyl, pyridino 7- to 9-membered fused heterobiaryl, pyrimido C₇₋₉ fused bicycloalkyl, pyrimido 7- to 9-membered fused heterobicyclyl, pyrimido 7- to 9-membered fused heterobiaryl, pyridazino C₇₋₉ fused bicycloalkyl, pyridazino 7- to 9-membered fused heterobicycloalkyl, pyridazino 7- to 9-membered fused heterobiaryl, pyrazino C₇₋₉ fused bicycloalkyl, pyrazino 7- to 9-membered fused heterobicycloalkyl, pyrazino 7- to 9-membered fused heterobiaryl, benzo C₇₋₉ spiro bicycloalkyl, benzo C₇₋₉ spiro heterobicyclyl, pyridino C₇₋₉ spiro bicycloalkyl, pyridino C₇₋₉ spiro heterobicyclyl, pyrimido C₇₋₉ spiro bicycloalkyl, pyrimido C₇₋₉ spiro heterobicyclyl, pyridazino C₇₋₉ spiro bicycloalkyl, pyridazino C₇₋₉ spiro heterobicyclyl, pyrazino C₇₋₉ spiro bicycloalkyl, and pyrazino C₇₋₉ spiro heterobicyclyl, wherein R¹ may be optionally independently substituted with one or more R^{a};
or, R¹ is selected from the group consisting of benzo 7- to 9-membered fused heterobicyclyl, benzo 7- to 9-membered fused heterobiaryl, benzo C₇₋₉ spiro bicycloalkyl, benzo C₇₋₉ spiro heterobicyclyl, and pyridino C₇₋₉ spiro bicycloalkyl, wherein R¹ may be optionally independently substituted with one or more R^{a};
or, R¹ is selected from the group consisting of benzo 7- to 9-membered fused heterobicyclyl, benzo 7- to 9-membered fused heterobiaryl, benzo C₇₋₉ spiro bicycloalkyl, benzo C₇₋₉ spiro heterobicyclyl, and pyridino C₇₋₉ spiro bicycloalkyl, wherein in R¹, the ring connected to the structural unit 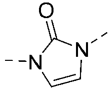 is a benzene ring or a pyridine ring, and R¹ may be optionally independently substituted with one or more R^{a};
or, R¹ is selected from the group consisting of benzo 7- to 9-membered fused heterobicyclyl, benzo C₇₋₉ spiro bicycloalkyl, and benzo C₇₋₉ spiro heterobicyclyl, wherein R¹ may be optionally independently substituted with one or more R^{a};
or, R¹ is selected from the group consisting of benzo 7- to 9-membered fused heterobicyclyl, benzo C₇₋₉ spiro bicycloalkyl, and benzo C₇₋₉ spiro heterobicyclyl, wherein in R¹, the ring connected to the structural unit 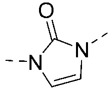 is a benzene ring, and R¹ may be optionally independently substituted with one or more R^{a};
or, R¹ is selected from the group consisting of benzo 5-membered heteroaryl fused 5-membered heterocyclyl, benzo 5-membered heteroaryl fused 6-membered heterocyclyl, benzo 5-membered heterocyclyl fused 5-membered heteroaryl, benzo 5-membered cycloalkyl spiro 3-membered cycloalkyl, benzo 5-membered heterocyclyl spiro 3-membered cycloalkyl, benzo 5-membered heterocyclyl spiro 4-membered cycloalkyl, benzo 5-membered cycloalkyl spiro 4-membered heterocyclyl, pyridino 5-membered heteroaryl fused 5-membered cycloalkyl, and pyridino 5-membered cycloalkyl spiro 3-membered cycloalkyl, wherein R¹ may be optionally independently substituted with one or more R^{a};
or, R¹ is selected from the group consisting of benzo 5-membered heteroaryl fused 5-membered heterocyclyl, benzo 5-membered heteroaryl fused 6-membered heterocyclyl, benzo 5-membered heterocyclyl fused 5-membered heteroaryl, benzo 5-membered cycloalkyl spiro 3-membered cycloalkyl, benzo 5-membered heterocyclyl spiro 3-membered cycloalkyl, benzo 5-membered heterocyclyl spiro 4-membered cycloalkyl, benzo 5-membered cycloalkyl spiro 4-membered heterocyclyl, pyridino 5-membered heteroaryl fused 5-membered cycloalkyl, and pyridino 5-membered cycloalkyl spiro 3-membered cycloalkyl, wherein in R¹, the ring connected to the structural unit 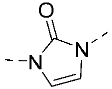 is a benzene ring or a pyridine ring, and R¹ may be optionally independently substituted with one or more R^{a};
or, R¹ is selected from the group consisting of benzo 5-membered heteroaryl fused 5-membered cycloalkyl, benzo 5-membered heteroaryl fused 5-membered heterocyclyl, benzo 5-membered heteroaryl fused 6-membered heterocyclyl, benzo 5-membered heterocyclyl fused 5-membered heteroaryl, benzo 5-membered cycloalkyl spiro 3-membered cycloalkyl, benzo 5-membered heterocyclyl spiro 3-membered cycloalkyl, benzo 5-membered heterocyclyl spiro 4-membered cycloalkyl, benzo 5-membered cycloalkyl spiro 4-membered heterocyclyl, and pyridino 5-membered cycloalkyl spiro 3-membered cycloalkyl, wherein R¹ may be optionally independently substituted with one or more R^{a};
or, R¹ is selected from the group consisting of benzo 5-membered heteroaryl fused 5-membered cycloalkyl, benzo 5-membered heteroaryl fused 5-membered heterocyclyl, benzo 5-membered heteroaryl fused 6-membered heterocyclyl, benzo 5-membered heterocyclyl fused 5-membered heteroaryl, benzo 5-membered cycloalkyl spiro 3-membered cycloalkyl, benzo 5-membered heterocyclyl spiro 3-membered cycloalkyl, benzo 5-membered heterocyclyl spiro 4-membered cycloalkyl, benzo 5-membered cycloalkyl spiro 4-membered heterocyclyl, and pyridino 5-membered cycloalkyl spiro 3-membered cycloalkyl, wherein in R¹, the ring connected to the structural unit 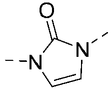 is a benzene ring or a pyridine ring, and R¹ may be optionally independently substituted with one or more R^{a};
or, R¹ is selected from the group consisting of benzo 5-membered cycloalkyl spiro 3-membered cycloalkyl and benzo 5-membered heterocyclyl spiro 3-membered cycloalkyl, wherein R¹ may be optionally independently substituted with one or more R^{a};
or, R¹ is selected from the group consisting of benzo 5-membered cycloalkyl spiro 3-membered cycloalkyl and benzo 5-membered heterocyclyl spiro 3-membered cycloalkyl, wherein in R¹, the ring connected to the structural unit 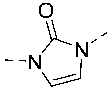 is a benzene ring, and R¹ may be optionally independently substituted with one or more R^{a};
or, R¹ is selected from the group consisting of 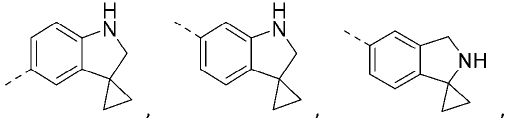 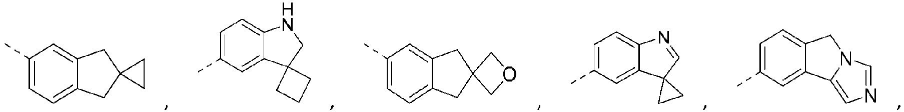 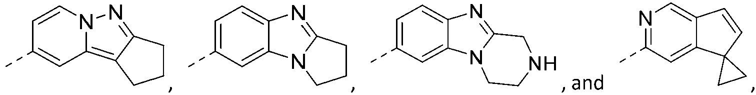 wherein R¹ may be optionally independently substituted with one or more R^{a};
or, R¹ is selected from the group consisting of 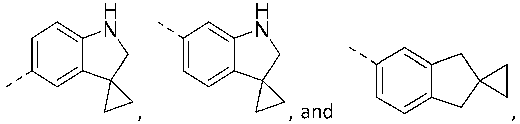 wherein
R¹ may be optionally independently substituted with one or more R^{a};
or, R¹ is selected from the group consisting of 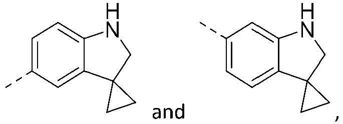 wherein R¹ may be optionally independently substituted with one or more R^{a};
or, R¹ is selected from 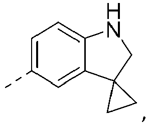 wherein R¹ may be optionally independently substituted with one or more R^{a};
or, R¹ is selected from the group consisting of phenyl and 5- to 6-membered heteroaryl, wherein R¹ is substituted with one C₂₋₄ alkynyl, R¹ may further be optionally independently substituted with one or more R^{a}, and the C₂₋₄ alkynyl may be optionally substituted with one or more R^{b};
or, R¹ is selected from the group consisting of phenyl, pyridinyl, pyrimidinyl, pyrazinyl, and pyridazinyl, wherein R¹ is substituted with one C₂₋₄ alkynyl, R¹ may further be optionally independently substituted with one or more R^{a}, and the C₂₋₄ alkynyl may be optionally substituted with one or more R^{b};
or, R¹ is selected from the group consisting of phenyl and pyridinyl, wherein R¹ is substituted with one C₂₋₄ alkynyl, R¹ may further be optionally independently substituted with one or more R^{a}, and the C₂₋₄ alkynyl may be optionally substituted with one or more R^{b};
or, R¹ is selected from the group consisting of phenyl and pyridinyl, wherein R¹ is substituted with one ethynyl or propynyl, R¹ may further be optionally independently substituted with one or more R^{a}, and the ethynyl or propynyl may be optionally substituted with one or more R^{b};
or, R¹ is selected from the group consisting of phenyl and pyridinyl, wherein R¹ is substituted with one ethynyl or 1-propynyl, R¹ may further be optionally independently substituted with one or more R^{a}, and the ethynyl or propynyl may be optionally substituted with one or more R^{b};
or, R¹ is selected from the group consisting of phenyl and pyridinyl, wherein R¹ is substituted with one ethynyl or propynyl, R¹ may further be optionally independently substituted with 1, 2, or 3 substituents selected from the group consisting of F and methyl, and the ethynyl or propynyl may be optionally substituted with 1, 2, or 3 methoxy;
or, R¹ is selected from the group consisting of phenyl and pyridinyl, wherein R¹ is substituted with one 1-propynyl, R¹ may further be optionally independently substituted with 1, 2, or 3 F, and the 1-propynyl may be optionally substituted with 1 methoxy.

3. The compound, the stereoisomer thereof, or the pharmaceutically acceptable salt thereof according to claim 1 or 2, wherein R^{a} is each independently selected from the group consisting of halogen, =O, deuterated C₁₋₅ alkyl, - OH, -CN, NH₂, C₁₋₆ alkyl, C₂₋₄ alkenyl, C₂₋₄ alkynyl, C₁₋₆ alkoxy, C₁₋₆ alkoxy C₁₋₃ alkylene, C₃₋₆ cycloalkyl, phenyl, 5- to 6-membered heteroaryl, and 3- to 6-membered heterocyclyl, wherein the deuterated C₁₋₅ alkyl, C₁₋₅ alkyl, C₂₋₄ alkenyl, C₂₋₄ alkynyl, C₁₋₅ alkoxy, or C₁₋₆ alkoxy C₁₋₃ alkylene is optionally independently substituted with one or more R^{c1}, and the C₃₋₆ cycloalkyl, phenyl, 5- to 6-membered heteroaryl, or 3- to 6-membered heterocyclyl is optionally independently substituted with one or more R^{d1}; R^{b} is each independently selected from the group consisting of halogen, -CN, -OH, -NH₂, methyl, ethyl, methoxy, and ethoxy, wherein the methyl, ethyl, methoxy, or ethoxy is optionally independently substituted with one or more substituents selected from the group consisting of deuterium, halogen, OH, CN, and NH₂;
or, R^{a} is each independently selected from the group consisting of halogen, =O, deuterated C₁₋₃ alkyl, -OH, -CN, NH₂, C₁₋₃ alkyl, C₂₋₄ alkenyl, C₂₋₄ alkynyl, C₁₋₃ alkoxy, C₁₋₃ alkoxymethylene, cyclopropyl, cyclobutyl, cyclopentyl, phenyl, 5-to 6-membered heteroaryl, and 3- to 5-membered heterocyclyl, wherein the deuterated C₁₋₃ alkyl, C₁₋₃ alkyl, C₂₋₄ alkenyl, C₂₋₄ alkynyl, C₁₋₃ alkoxy, or C₁₋₃ alkoxymethylene is optionally independently substituted with one or more R^{c1}, and the cyclopropyl, cyclobutyl, cyclopentyl, phenyl, 5- to 6-membered heteroaryl, or 3- to 5-membered heterocyclyl is optionally independently substituted with one or more R^{d1};
or, R^{a} is each independently selected from the group consisting of halogen, =O, deuterated C₁₋₃ alkyl, C₁₋₃ alkyl, and C₃₋₆ cycloalkyl, wherein the deuterated C₁₋₃ alkyl or C₁₋₃ alkyl is optionally independently substituted with one or more R^{c1}, and the C₃₋₆ cycloalkyl is optionally independently substituted with one or more R^{d1};
or, R^{a} is each independently selected from the group consisting of F, Cl, Br, I, =O, -CD₃, -C₂D₅, -OH, -CN, methyl, ethyl, propyl, ethenyl, propenyl, ethynyl, propynyl, methoxy, ethoxy, propoxy, methoxymethylene, ethoxymethylene, cyclopropyl, cyclobutyl, cyclopentyl, and phenyl, wherein the methyl, ethyl, propyl, ethenyl, propenyl, ethynyl, propynyl, methoxy, ethoxy, propoxy, methoxymethylene, or ethoxymethylene is optionally independently substituted with one or more R^{c1}, and the cyclopropyl, cyclobutyl, cyclopentyl, or phenyl is optionally independently substituted with one or more R^{d1};
or, R^{a} is each independently selected from the group consisting of F, Cl, Br, I, =O, -CD₃, -C₂D₅, -OH, -CN, methyl, ethyl, propyl, methoxymethylene, ethoxymethylene, cyclopropyl, cyclobutyl, and cyclopentyl, wherein the methyl, ethyl, propyl, methoxymethylene, or ethoxymethylene is optionally independently substituted with one or more R^{c1}, and the cyclopropyl, cyclobutyl, or cyclopentyl is optionally independently substituted with one or more R^{d1};
or, R^{a} is each independently selected from the group consisting of F, Cl, Br, =O, -CD₃, -C₂D₅, methyl, ethyl, propyl, cyclopropyl, cyclobutyl, and cyclopentyl, wherein the methyl, ethyl, or propyl is optionally independently substituted with one or more R^{c1}, and the cyclopropyl, cyclobutyl, or cyclopentyl is optionally independently substituted with one or more R^{d1};
or, R^{a} is each independently selected from the group consisting of F, methyl, =O, -CD₃, and cyclopropyl;
or, R^{a} is each independently selected from the group consisting of methyl and =O;
or, R^{a} is each independently selected from the group consisting of F and methyl;
or, R^{b} is each independently selected from C₁₋₃ alkoxy, wherein the C₁₋₃ alkoxy is optionally independently substituted with one or more substituents selected from the group consisting of deuterium, halogen, OH, CN, and NH₂;
or, R^{b} is each independently selected from the group consisting of F, Cl, Br, I, and methoxy;
or, R^{b} is each independently selected from methoxy.

4. The compound, the stereoisomer thereof, or the pharmaceutically acceptable salt thereof according to any one of claims 1-3, wherein R² is each independently selected from the group consisting of halogen, -OH, -CN, C₁₋₅ alkyl, C₂₋₆ alkenyl, C₂₋₆ alkynyl, C₁₋₅ alkoxy, C₃₋₆ cycloalkyl, phenyl, 5- to 6-membered heteroaryl, and 3- to 6-membered heterocyclyl, wherein the C₁₋₅ alkyl, C₂₋₆ alkenyl, C₂₋₆ alkynyl, or C₁₋₅ alkoxy is optionally independently substituted with one or more R^{c2}, and the C₃₋₆ cycloalkyl, phenyl, 5- to 6-membered heteroaryl, or 3- to 6-membered heterocyclyl is optionally independently substituted with one or more R^{d2};
or, R² is each independently selected from the group consisting of halogen, -OH, -CN, C₁₋₃ alkyl, C₂₋₄ alkenyl, C₂₋₄ alkynyl, C₁₋₃ alkoxy, C₃₋₅ cycloalkyl, phenyl, 5- to 6-membered heteroaryl, and 3- to 5-membered heterocyclyl, wherein the C₁₋₃ alkyl, C₂₋₄ alkenyl, C₂₋₄ alkynyl, or C₁₋₃ alkoxy is optionally independently substituted with one or more R^{c2}, and the C₃₋₆ cycloalkyl, phenyl, 5- to 6-membered heteroaryl, or 3- to 6-membered heterocyclyl is optionally independently substituted with one or more R^{d2};
or, R² is each independently selected from the group consisting of halogen, C₁₋₃ alkyl, and C₃₋₅ cycloalkyl, wherein the C₁₋₃ alkyl is optionally independently substituted with one or more R^{c2}, and the C₃₋₆ cycloalkyl is optionally independently substituted with one or more R^{d2};
or, R² is each independently selected from the group consisting of F, Cl, Br, I, methyl, ethyl, propyl, cyclopropyl, cyclobutyl, and cyclopentyl, wherein the methyl, ethyl, or propyl is optionally independently substituted with one or more R^{c2}, and the cyclopropyl, cyclobutyl, or cyclopentyl is optionally independently substituted with one or more R^{d2};
or, R² is each independently selected from the group consisting of F, methyl, and cyclopropyl, wherein the methyl is optionally independently substituted with one or more R^{c2}, and the cyclopropyl is optionally independently substituted with one or more R^{d2};
or, R² is each independently selected from the group consisting of F, methyl, and cyclopropyl;
or, R² on two adjacent carbon atoms, together with the carbon atoms connected thereto, form C₅₋₆ cycloalkyl, phenyl, 5- to 6-membered heteroaryl, or 5- to 6-membered heterocyclyl, wherein the C₅₋₆ cycloalkyl, phenyl, 5- to 6-membered heteroaryl, or 5- to 6-membered heterocyclyl is optionally independently substituted with one or more R^{d3};
or, R² on two adjacent carbon atoms, together with the carbon atoms connected thereto, form C₅₋₆ cycloalkyl or 5-to 6-membered heterocyclyl, wherein the C₅₋₆ cycloalkyl or 5- to 6-membered heterocyclyl is optionally independently substituted with one or more R^{d3};
or, R² on two adjacent carbon atoms, together with the carbon atoms connected thereto, form C₅₋₆ cycloalkyl, wherein the C₅₋₆ cycloalkyl is optionally independently substituted with one or more R^{d3};
or, R² on two adjacent carbon atoms, together with the carbon atoms connected thereto, form cyclopentyl, pyrrolyl, tetrahydrofuranyl, or tetrahydrothienyl, wherein the cyclopentyl, pyrrolyl, tetrahydrofuranyl, or tetrahydrothienyl is optionally independently substituted with one or more R^{d3}; or, R² on two adjacent carbon atoms, together with the carbon atoms connected thereto, form cyclopentyl, wherein the cyclopentyl is optionally independently substituted with one or more R^{d3};
or, R² on two adjacent carbon atoms, together with the carbon atoms connected thereto, form cyclopentyl.

5. The compound, the stereoisomer thereof, or the pharmaceutically acceptable salt thereof according to any one of claims 1-4, wherein one of X¹ and X² is selected from C, and the other is selected from N; Y¹, Y², and Y³ are each independently selected from the group consisting of C and N, and Y⁴ is selected from CH;
or, at least one of Y¹, Y², and Y³ is selected from N, and Y⁴ is selected from CH;
or, Y¹ is selected from C, Y² is selected from N, Y³ is selected from C, and Y⁴ is selected from CH;
or, Y¹ is selected from C, Y² is selected from C, Y³ is selected from N, and Y⁴ is selected from CH;
or, Y¹ is selected from N, Y² is selected from C, Y³ is selected from C, and Y⁴ is selected from CH.

6. The compound, the stereoisomer thereof, or the pharmaceutically acceptable salt thereof according to any one of claims 1-5, wherein R³ is each independently selected from the group consisting of deuterium, halogen, C₁₋₃ alkyl, C₂₋₄ alkenyl, C₂₋₄ alkynyl, C₃₋₆ cycloalkyl, phenyl, 5- to 6-membered heteroaryl, and 3- to 6-membered heterocyclyl, wherein the C₁₋₃ alkyl, C₂₋₄ alkenyl, or C₂₋₄ alkynyl is optionally independently substituted with one or more R^{c3}, and the C₃₋₆ cycloalkyl, phenyl, 5- to 6-membered heteroaryl, or 3- to 6-membered heterocyclyl is optionally independently substituted with one or more R^{d4}; R⁴ is selected from the group consisting of H, deuterium, halogen,
- CN, C₁₋₃ alkyl, deuterated C₁₋₃ alkyl, and C₁₋₃ alkoxy, wherein the C₁₋₃ alkyl, deuterated C₁₋₃ alkyl, or C₁₋₃ alkoxy is optionally independently substituted with one or more R^{c4}; R⁵ is selected from the group consisting of halogen, -CN, C₁₋₃ alkyl, deuterated C₁₋₃ alkyl, and C₁₋₃ alkoxy, wherein the C₁₋₃ alkyl, deuterated C₁₋₃ alkyl, or C₁₋₃ alkoxy is optionally independently substituted with one or more R^{c5};
or, R³ is each independently selected from the group consisting of deuterium, halogen, and C₁₋₃ alkyl, wherein the C₁₋₃ alkyl is optionally independently substituted with one or more R^{c3};
or, R³ is each independently selected from C₁₋₃ alkyl, wherein the C₁₋₃ alkyl is optionally independently substituted with one or more R^{c3};
or, R³ is each independently selected from the group consisting of deuterium, F, Cl, Br, I, methyl, ethyl, propyl, cyclopropyl, cyclobutyl, and cyclopentyl, wherein the methyl, ethyl, or propyl is optionally independently substituted with one or more R^{c3}, and the cyclopropyl, cyclobutyl, or cyclopentyl is optionally independently substituted with one or more R^{d4};
or, R³ is each independently selected from the group consisting of methyl, ethyl, and propyl, wherein the methyl, ethyl, or propyl is optionally substituted with one or more R^{c3};
or, R³ is selected from methyl;
or, R⁴ is selected from the group consisting of H and C₁₋₃ alkyl, wherein the C₁₋₃ alkyl is optionally independently substituted with one or more R^{c4};
or, R⁴ is selected from the group consisting of H and methyl, wherein the methyl is optionally substituted with one or more R^{c4};
or, R⁴ is selected from the group consisting of H and methyl;
or, R⁴ is selected from H;
or, R⁴ is selected from methyl;
or, R⁵ is selected from C₁₋₃ alkyl, wherein the C₁₋₃ alkyl is optionally independently substituted with one or more R^{c5};
or, R⁵ is selected from methyl.

7. The compound, the stereoisomer thereof, or the pharmaceutically acceptable salt thereof according to any one of claims 1-6, wherein R' and R" are each independently selected from the group consisting of H, deuterium, halogen, -CN, C₁₋₃ alkyl, deuterated C₁₋₃ alkyl, and C₁₋₃ alkoxy, wherein the C₁₋₃ alkyl, deuterated C₁₋₃ alkyl, or C₁₋₃ alkoxy is optionally independently substituted with one or more R^{c6};
or, R' and R" are each independently selected from the group consisting of H and C₁₋₃ alkyl, wherein the C₁₋₃ alkyl is optionally independently substituted with one or more R^{c6};
or, R' and R" are each independently selected from the group consisting of H and methyl, wherein the methyl is optionally substituted with one or more R^{c6};
or, R' and R" are selected from methyl;
or, R' and R", together with the carbon atom connected thereto, form C₃₋₄ cycloalkyl or 3- to 4-membered heterocycloalkyl, wherein the C₃₋₄ cycloalkyl or 3- to 4-membered heterocycloalkyl is optionally independently substituted with one or more R^{d5};
or, R' and R", together with the carbon atom connected thereto, form cyclopropyl, wherein the cyclopropyl is optionally independently substituted with one or more R^{d5};
or, R' and R", together with the carbon atom connected thereto, form cyclopropyl.

8. The compound, the stereoisomer thereof, or the pharmaceutically acceptable salt thereof according to any one of claims 1-7, wherein q is selected from the group consisting of 0, 1, 2, and 3; or, q is selected from the group consisting of 1, 2, and 3; or, q is selected from 2; or, q is selected from 3;
optionally, m is selected from the group consisting of 0, 1, 2, and 3; or, m is selected from the group consisting of 0, 1, and 2; or, m is selected from 0; or, m is selected from 1;
optionally, n is selected from the group consisting of 0, 1, 2, and 3; or, n is selected from the group consisting of 1, 2, and 3; or, n is selected from 1.

9. The compound, the stereoisomer thereof, or the pharmaceutically acceptable salt thereof according to any one of claims 1-8, wherein R^{c1}, R^{c2}, R^{c3}, R^{c4}, R^{c5}, and R^{c6} are each independently selected from the group consisting of halogen, -CN, -OH, and -NH₂;
or, R^{c1}, R^{c2}, R^{c3}, R^{c4}, R^{c5}, and R^{c6} are each independently selected from the group consisting of F, -CN, -OH, and -NH₂;
or, R^{c1}, R^{c2}, R^{c3}, R^{c4}, R^{c5}, and R^{c6} are each independently selected from the group consisting of F and OH;
or, R^{c1}, R^{c2}, R^{c3}, R^{c4}, R^{c5}, and R^{c6} are each independently selected from F;
optionally, R^{d1}, R^{d2}, R^{d3}, R^{d4}, and R^{d5} are each independently selected from the group consisting of halogen, -CN, - OH, -NH₂, C₁₋₃ alkyl, and C₁₋₃ alkoxy, wherein the C₁₋₃ alkyl or C₁₋₃ alkoxy is optionally independently substituted with one or more substituents selected from the group consisting of halogen, OH, CN, and NH₂;
or, R^{d1}, R^{d2}, R^{d3}, R^{d4}, and R^{d5} are each independently selected from the group consisting of F, -CN, -OH, -NH₂, methyl, and methoxy, wherein the methyl or methoxy is optionally independently substituted with one or more substituents selected from the group consisting of F, OH, CN, and NH₂;
or, R^{d1}, R^{d2}, R^{d3}, R^{d4}, and R^{d5} are each independently selected from the group consisting of F, -CN, -OH, -NH₂, methyl, and methoxy;
or, R^{d1}, R^{d2}, R^{d3}, R^{d4}, and R^{d5} are each independently selected from the group consisting of F and methyl;
or, R^{d1}, R^{d2}, R^{d3}, R^{d4}, and R^{d5} are each independently selected from F.

10. The compound, the stereoisomer thereof, or the pharmaceutically acceptable salt thereof according to any one of claims 1-9, being selected from the group consisting of compounds of formulas (I-A), (I-B), (I-C), and (I-D), stereoisomers thereof, or pharmaceutically acceptable salts thereof,

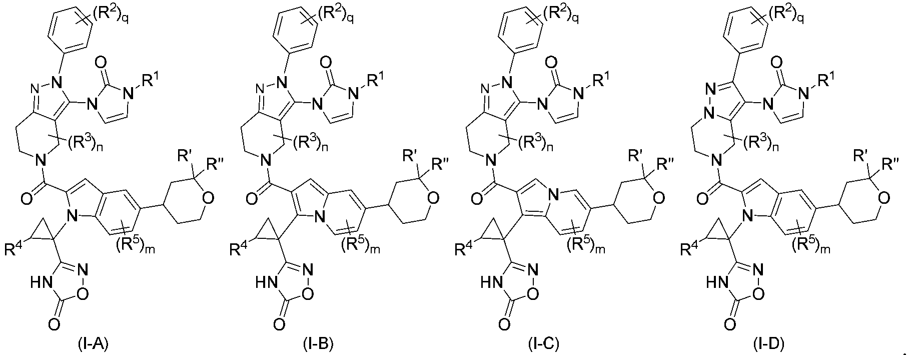

11. The compound, the stereoisomer thereof, or the pharmaceutically acceptable salt thereof according to any one of claims 1-10, being selected from the group consisting of compounds of formulas (II-A), (II-B), (II-C), (II-D), (II-E), (II-F), (II-G), (II-H), (II-I), (II-J), (II-K), and (II-L), stereoisomers thereof, or pharmaceutically acceptable salts thereof, 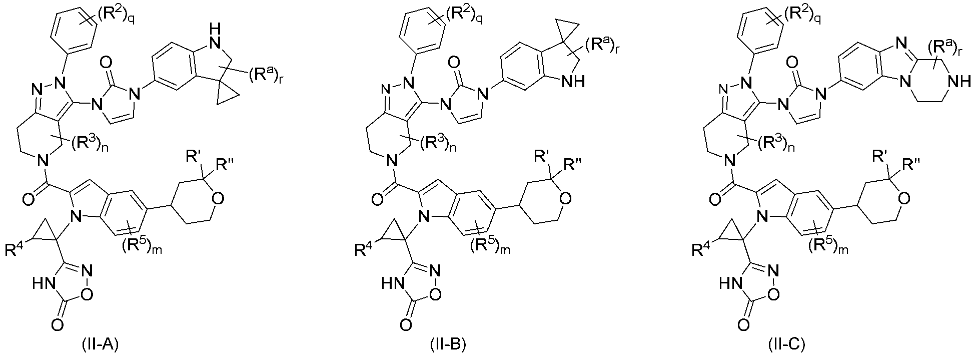 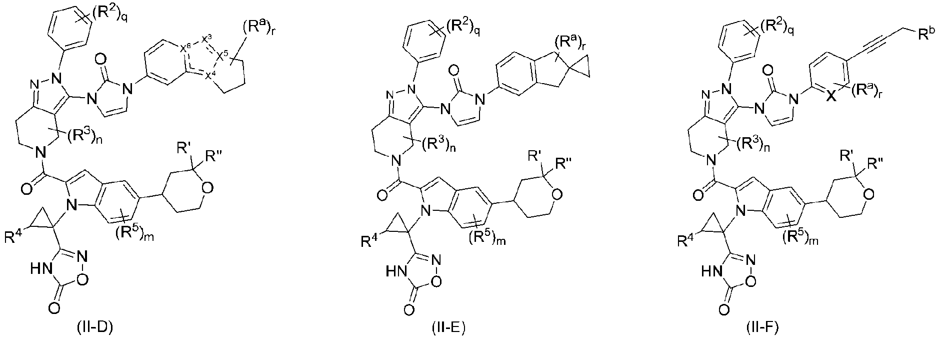 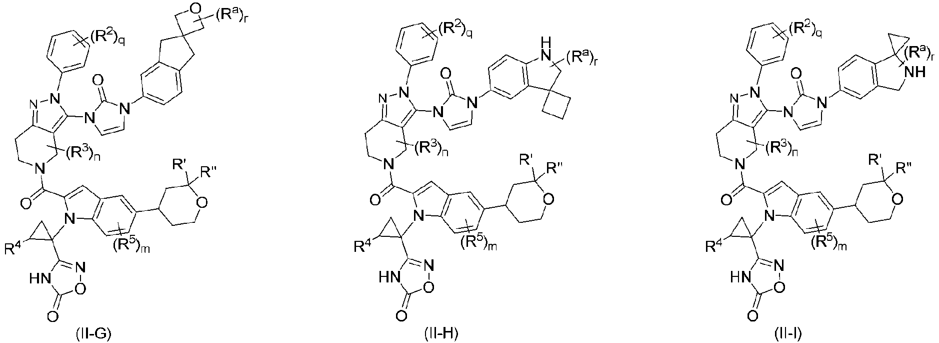 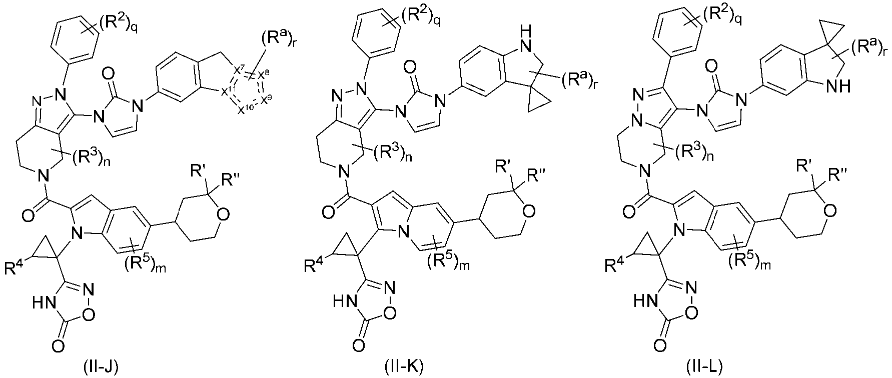
wherein, r is selected from the group consisting of 0, 1, 2, 3, and 4; X is selected from the group consisting of C and N; X³, X⁴, X⁵, and X⁶ are each independently selected from the group consisting of C and N; X⁷, X⁸, X⁹, X¹⁰, and X¹¹ are each independently selected from the group consisting of C, CH, and N;
or, X is selected from C; or, X is selected from N;
or, X³ is selected from N, and X⁴, X⁵, and X⁶ are each independently selected from the group consisting of C and N;
or, X³ and X⁶ are selected from N, and X⁴ and X⁵ are selected from C;
or, X³ and X⁴ are selected from N, and X⁵ and X⁶ are selected from C;
or, one of X⁷ and X¹¹ is selected from N, and the other is selected from C;
or, X⁷ and X⁹ are selected from N, and X¹¹ is selected from C, and X⁸ and X¹⁰ are selected from CH;
or, r is selected from the group consisting of 0, 1, and 2;
or, r is selected from the group consisting of 0 and 2.

12. The compound, the stereoisomer thereof, or the pharmaceutically acceptable salt thereof according to any one of claims 1-11, being selected from a compound of formula (I'), a stereoisomer thereof, or a pharmaceutically acceptable salt thereof,

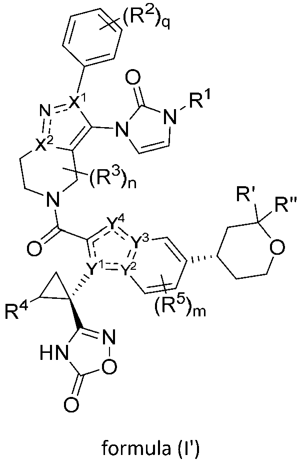

13. The compound, the stereoisomer thereof, or the pharmaceutically acceptable salt thereof according to any one of claims 1-12, being selected from the group consisting of compounds of formulas (I-A'), (I-B'), (I-C'), and (I-D'), stereoisomers thereof, or pharmaceutically acceptable salts thereof,

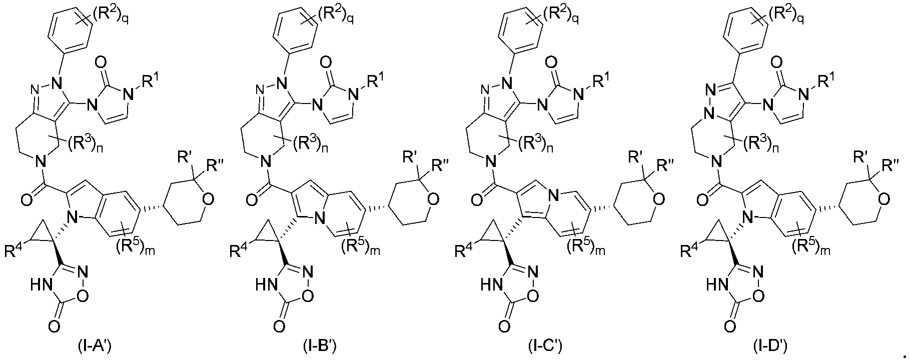

14. The compound, the stereoisomer thereof, or the pharmaceutically acceptable salt thereof according to any one of claims 1-13, being selected from the group consisting of compounds of formulas (II-A'), (II-B'), (II-C'), (II-D'), (II-E'), (II-F'), (II-G'), (II-H'), (II-I'), (II-J'), (II-K'), and (II-L'), stereoisomers thereof, or pharmaceutically acceptable salts thereof, 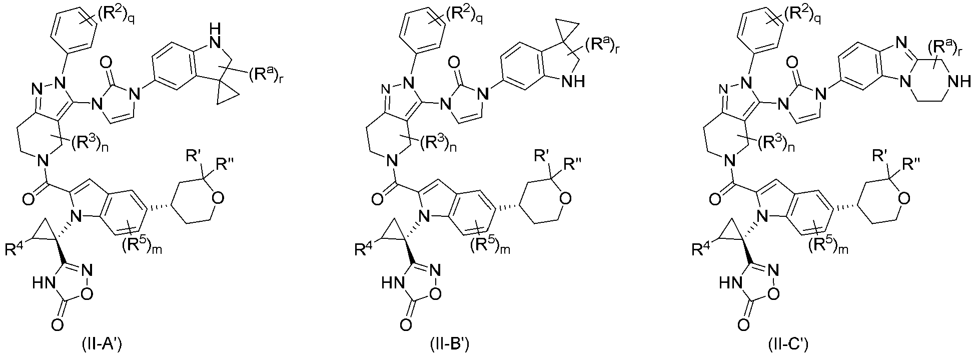 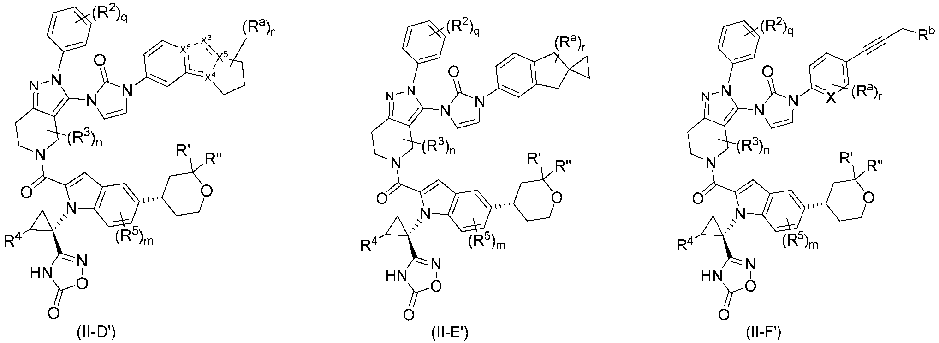 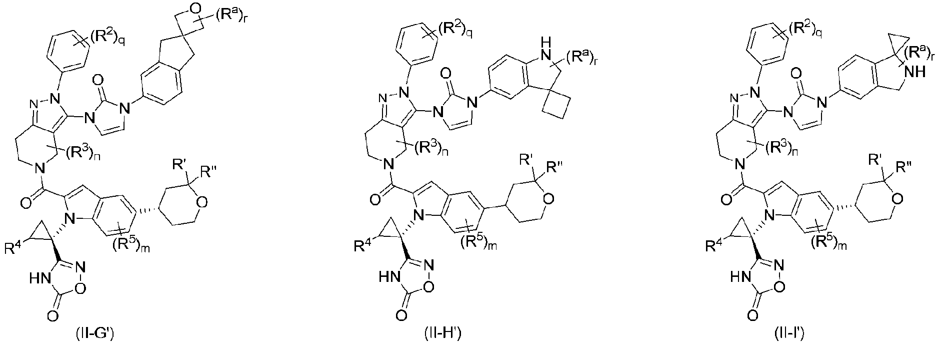 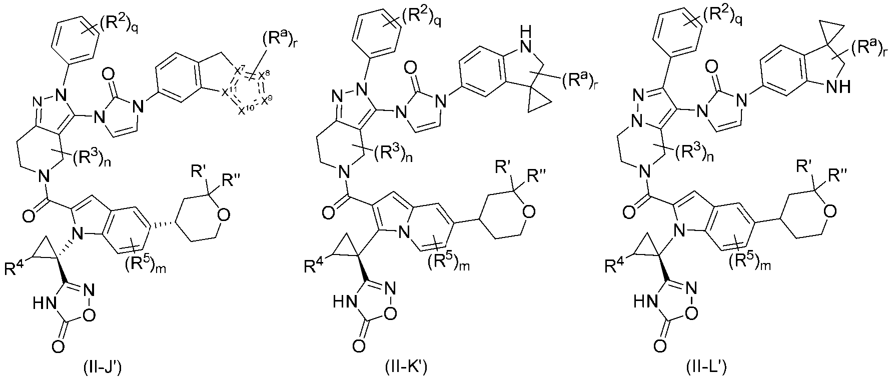
wherein, r is selected from the group consisting of 0, 1, 2, 3, and 4; X is selected from the group consisting of C and N; X³, X⁴, X⁵, and X⁶ are each independently selected from the group consisting of C and N; X⁷, X⁸, X⁹, X¹⁰, and X¹¹ are each independently selected from the group consisting of C, CH, and N;
or, X, X³, X⁴, X⁵, X⁸, X⁷, X⁸, X⁹, X¹⁰, or X¹¹ is as defined in the compound of formula (II-D), (II-F), or (II-J) in claim 11.

15. The compound, the stereoisomer thereof, or the pharmaceutically acceptable salt thereof according to any one of claims 1-14, being selected from the group consisting of compounds of formulas (IV-A), (IV-A'), (IV-B'), and (IV-C'), stereoisomers thereof, or pharmaceutically acceptable salts thereof,

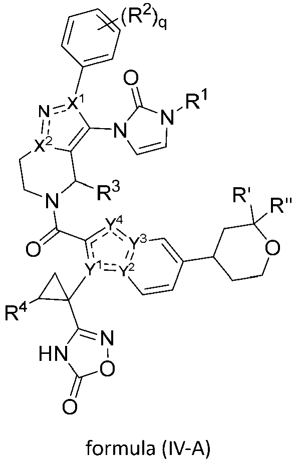

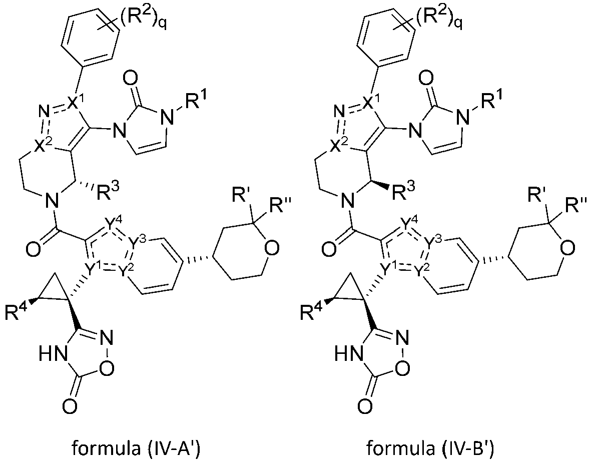

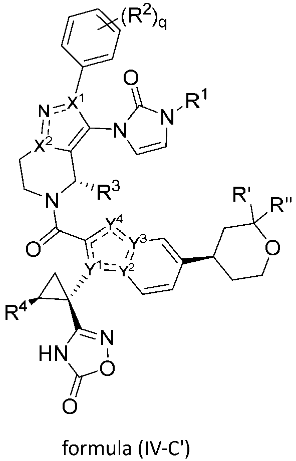

16. A compound of one of the following formulas, a stereoisomer thereof, or a pharmaceutically acceptable salt thereof:

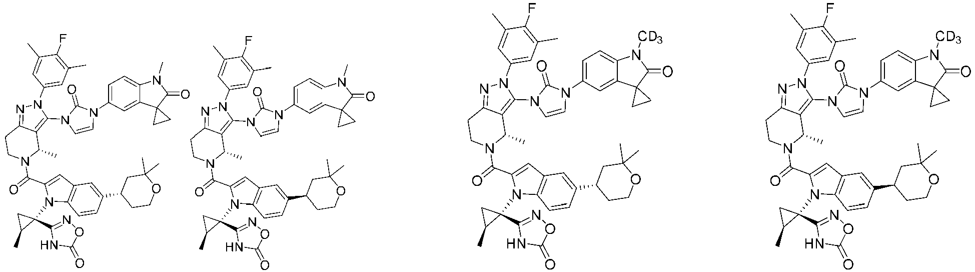

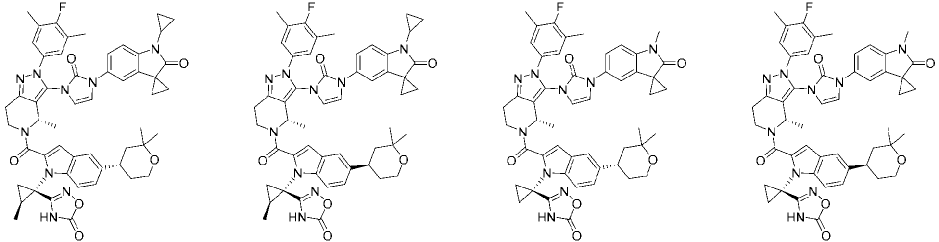

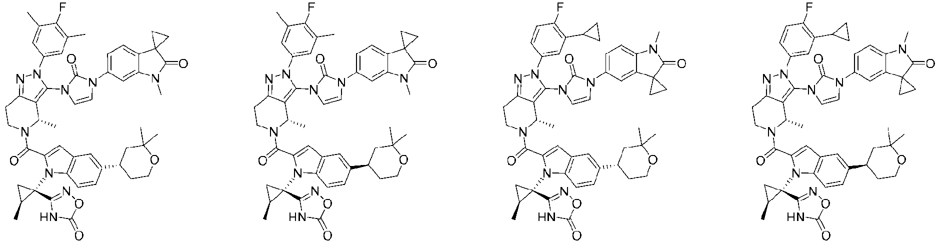

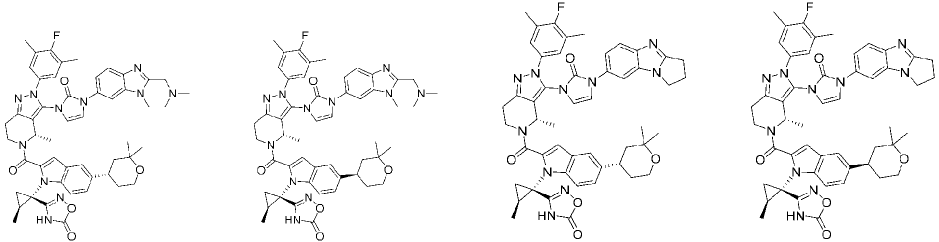

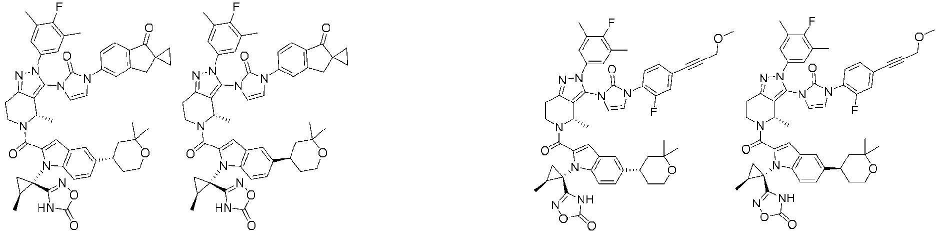

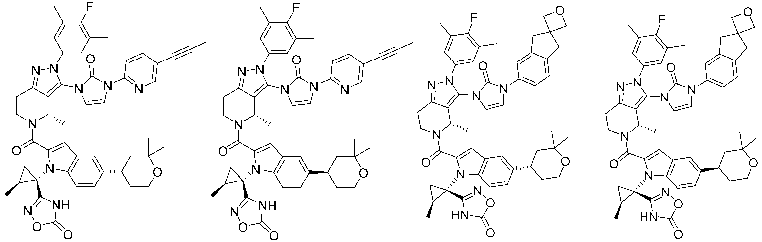

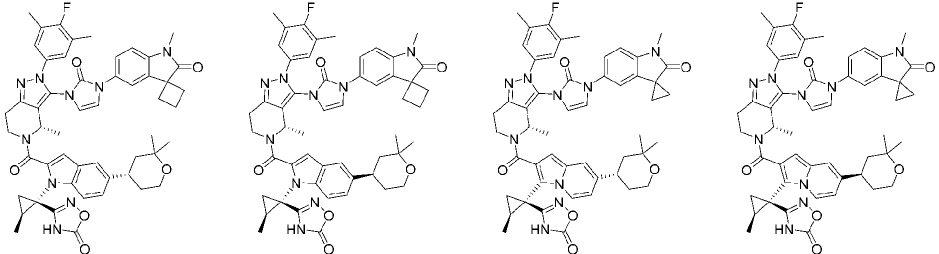

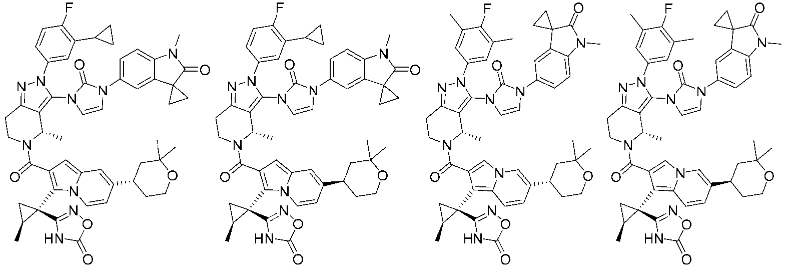

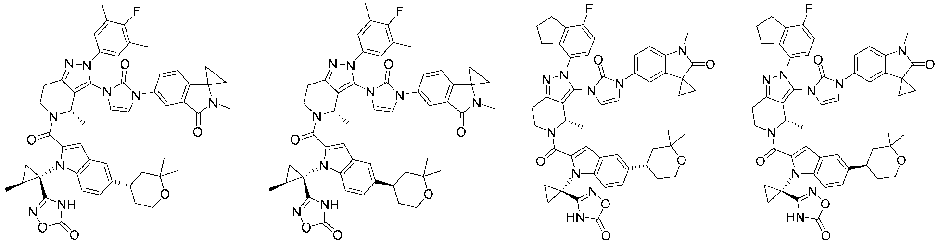

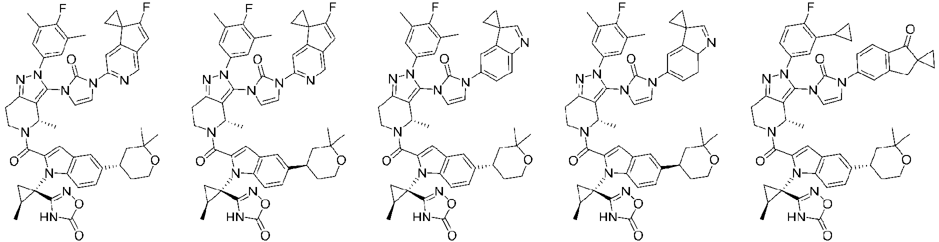

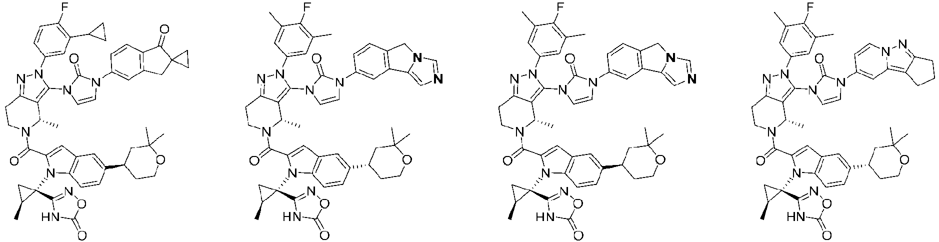

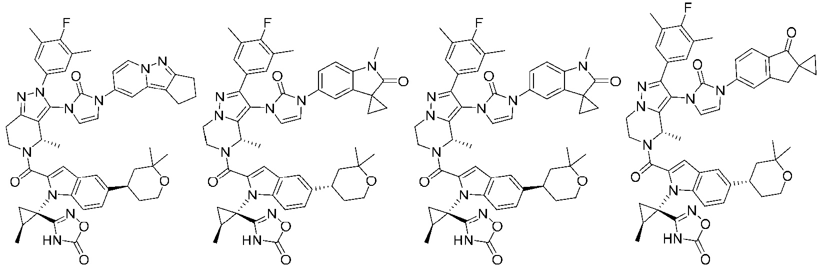

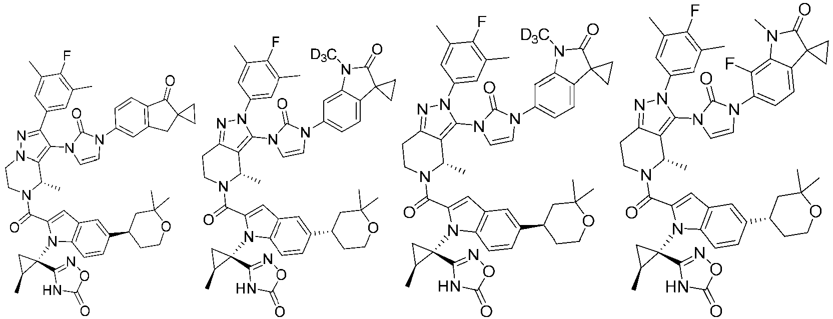

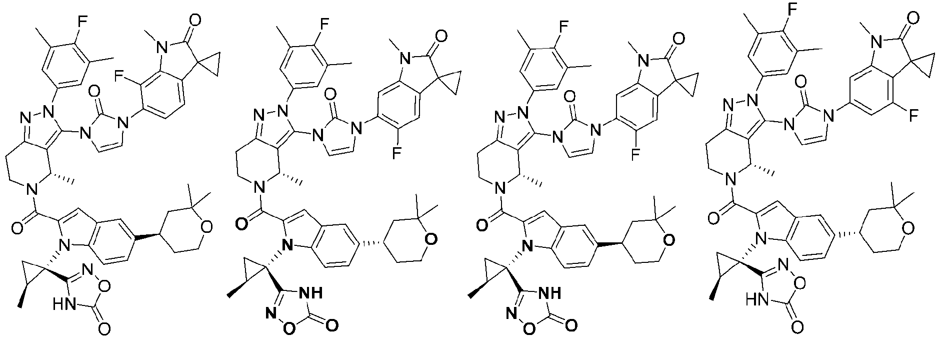

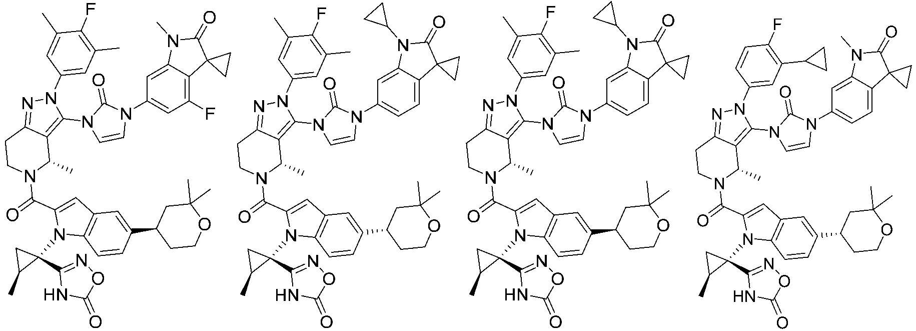

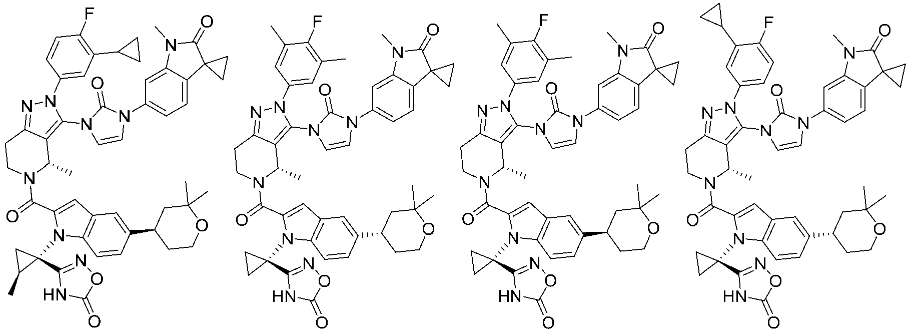

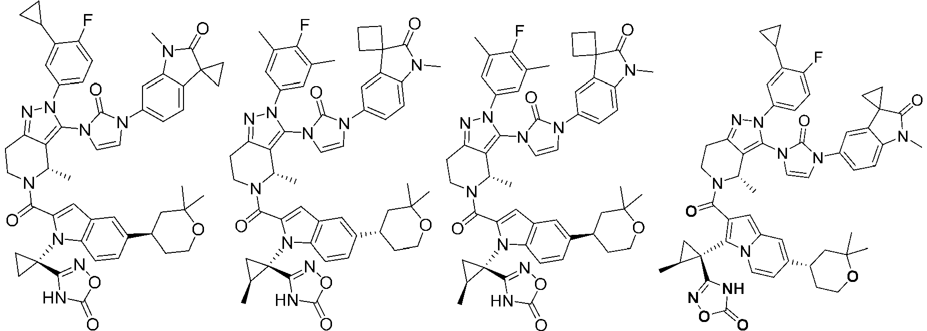

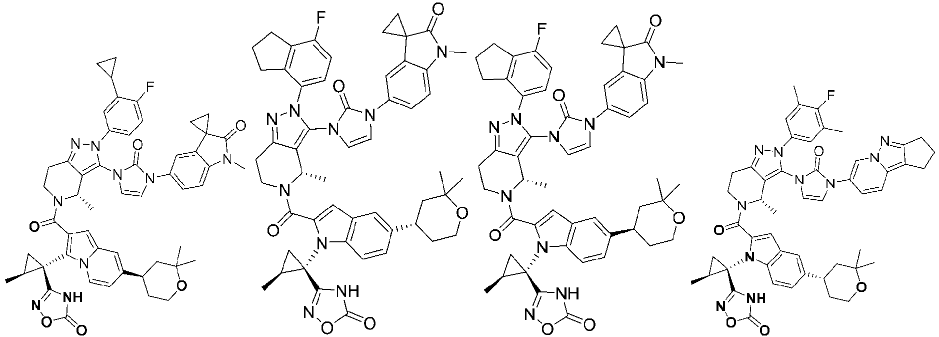

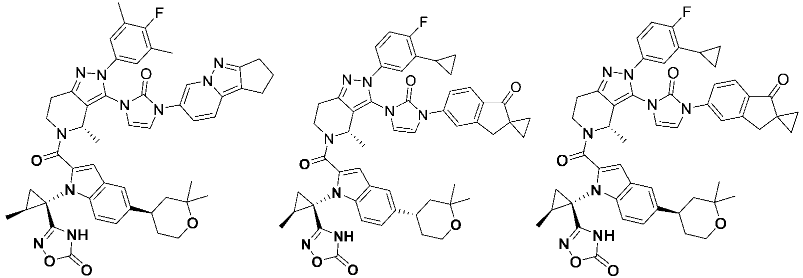

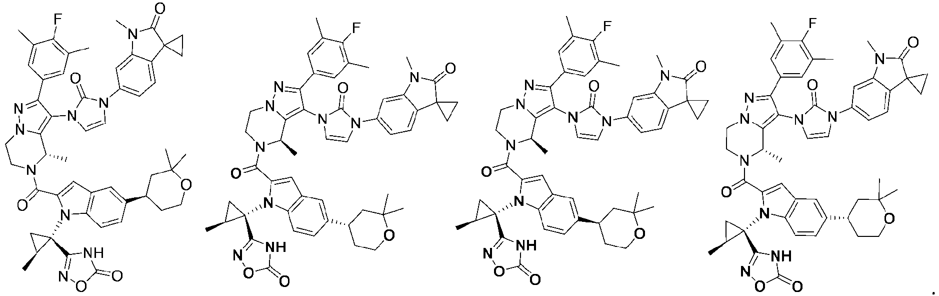

17. A pharmaceutical composition, comprising the compound, the stereoisomer thereof, or the pharmaceutically acceptable salt thereof according to any one of claims 1-16, and further comprising a pharmaceutically acceptable excipient.

18. Use of the compound, the stereoisomer thereof, or the pharmaceutically acceptable salt thereof according to any one of claims 1-16 or the pharmaceutical composition according to claim 17 for preparing a medicament for treating a GLP-1-associated disease.

19. A method for treating or preventing a GLP-1-associated disease, comprising administering to a mammal, preferably a human, in need of the treatment a therapeutically effective amount of the compound, the stereoisomer thereof, or the pharmaceutically acceptable salt thereof according to any one of claims 1-16, or the pharmaceutical composition according to claim 17.

20. Use of the compound, the stereoisomer thereof, or the pharmaceutically acceptable salt thereof according to any one of claims 1-16 or the pharmaceutical composition according to claim 17 for treating a GLP-1-associated disease.
